# EUROPEAN PATENT APPLICATION

(11) **EP 4 640 835 A1**
(43) Date of publication of application: **29.10.2025**
(21) Application number: 23906095.7
(22) Date of filing: 22.12.2023
(51) Int. Cl.: C12N 15/62, C12N 9/22, A61K 48/00

(54) **FUSION AND USE THEREOF**

(30) Priority: 23.12.2022 CN 202211663492; 27.09.2023 CN 202311263736
(71) Applicant: Epigenic Therapeutics Pte. Ltd., Singapore 068908 (SG)
(72) Inventor: MAO, Shaoshuai, Shanghai 200131 (CN); ZHAO, Junzheng, Shanghai 200131 (CN); ZANG, Ying, Shanghai 200131 (CN); LV, Ruimin, Shanghai 200131 (CN); LUO, Hao, Shanghai 200131 (CN)
(74) Representative: Murgitroyd & Company
(86) International application number: PCT/CN2023/141011
(87) International publication number: WO 2024/131940

(57) **Abstract**

Provided are a fusion and use thereof. The fusion comprises a nucleic acid-binding domain and one or more effector domains; the effector domain comprises one or more of an epigenetic modification domain and/or a transcriptional regulation domain, and the fusion comprises at least two types of epigenetic modification domains and/or transcriptional regulation domains. Also provided is use of the fusion for preparing a product for regulating the expression of a target gene and preparing a related drug.

## Description

### TECHNICAL FIELD

The present application relates to the field of biomedicine, and specifically to a fusion for gene editing and use thereof.

### BACKGROUND

Abnormalities in genomic epigenetic modifications are closely related to the occurrence and development of many diseases, such as common metabolic disorders, cardiovascular diseases, and cancers in life. Gene epigenetic editing tools can achieve the corresponding gene transcription regulation purpose without changing the gene sequence. This process will not cause permanent DNA damage, nor will it produce harmful mutations and off-target effects. In addition, epigenetic therapy can provide better therapeutic effects by simultaneously regulating the activity of multiple genes, making up for the shortcomings of gene therapy and providing new hope for the treatment of such diseases. In another aspect, the development of gene-directed modification technology, especially the development of CRISPR/Cas9 technology, makes it possible to perform targeted epigenome editing and transcriptional regulation in the natural chromatin environment.

At present, the design of epigenetic modification and transcriptional regulation is mainly based on the combined use of nucleases, among which the combined use of engineered defective nucleases (dCas9) is the most effective. The design principle is to fuse various epigenetic regulatory effectors to dCas9, and achieve epigenome editing at specific genomic sites through the characteristics of the targeting and binding of the nuclease to the target DNA. These trans-regulatory domains and proteins function by blocking the binding of RNA polymerase or recruiting endogenous transcriptional fusions to the dCas9 targeting site in the promoter region. Currently, there are only a few studies on the use of transcriptional regulation and epigenetic site-directed modification technology to treat diseases caused by epigenetic abnormalities in vivo, and existing editing tools have problems such as low transcriptional regulation efficiency and limited scope of modification of target genes (such as methylation modification).

### SUMMARY

The present application provides a fusion for gene editing and use thereof, the fusion comprises a nucleic acid-binding domain and an effector domain, wherein the effector domain comprises an epigenetic regulatory modifier and a transcriptional regulation factor, and the effector domain is linked to the N-terminus, C-terminus, or both the N-terminus and the C-terminus of the nucleic acid-binding domain. Compared with fusions composed of the same or similar domains and regulatory elements in the art, the fusion of the present invention has a more efficient target gene regulation efficiency, and the epigenetic modification range of the target gene can be enriched by fusing different epigenetic modifiers and transcriptional regulation factors into the effector domain. Futhermore, the fusion of the present application can be used to prepare a product that inhibits the expression of a target gene and to prepare a drug.

In one aspect, the present application provides a fusion comprising a nucleic acid-binding domain and one or more effector domains, wherein the effector domain comprises one or more of an epigenetic modification domain and/or a transcriptional regulation domain and the fusion comprises at least two types of epigenetic modification domains and/or transcriptional regulation domains.

In some embodiments, the nucleic acid-binding domain is a DNA-binding domain.

In some embodiments, the DNA-binding domain is selected from a TALE domain, a zinc finger domain, a tetR domain, a large-range nuclease, a Cas protein, an Argonaute (Ago) protein, and a homolog or modified form thereof.

In some embodiments, the DNA-binding domain is capable of binding to a target sequence of a target locus.

In some embodiments, the DNA-binding domain is capable of binding to a guide RNA.

In some embodiments, the guide RNA is capable of specifically recognizing and hybridizing to the target sequence of the target locus.

In some embodiments, the DNA-binding domain is a Cas protein, and the Cas protein is a class II Cas nuclease.

In some embodiments, the Cas protein is a class II type II Cas nuclease and a class II type V Cas nuclease.

In some embodiments, the DNA-binding domain is a Cas9 protein.

In some embodiments, the Cas9 protein is a dead Cas protein (dCas9).

In some embodiments, the nucleic acid-binding domain comprises the amino acid sequence as set forth in any one of SEQ ID NOs: 1-7, 353 and 354.

In some embodiments, the epigenetic modification domain is selected from DNA deamination activity, DNA methyltransferase activity, DNA demethylase activity, DNA amination activity, DNA oxidation activity, DNA helicase activity, histone acetyltransferase activity, histone deacetylase activity, histone methyltransferase activity, histone demethylase activity, histone kinase activity, histone phosphatase activity, histone ubiquitin ligase activity, and histone deubiquitination activity.

In some embodiments, the epigenetic modification domain comprises a DNA methyltransferase and/or a functionally active fragment thereof.

In some embodiments, the DNA methyltransferase is selected from DNMT3A, DNMT3B, Dnmt3c, DNMT1, DNMT2 and DNMT3L.

In some embodiments, the epigenetic modification domain comprises a plurality of DNA methyltransferases and/or functionally active fragments thereof, and the plurality of DNA methyltransferases and/or functionally active fragments thereof are linked via a linker sequence.

In some embodiments, the epigenetic modification domain comprises at least one DNMT3A and at least one DNMT3L.

In some embodiments, the DNA methyltransferase comprises an amino acid sequence as set forth in any one of SEQ ID NOs: 8-21.

In some embodiments, the transcriptional regulation domain is a transcriptional activation domain or a transcriptional repressor domain.

In some embodiments, the transcriptional repressor domain is selected from KRAB, ZIM3, ZNF680, ZNF554, ZNF264, ZNF582, ZNF324, ZNF669, ZNF354A, ZNF82, ZNF595, ZNF419, ZNF566, ZIM2, EHMT2, SUV39H1, ZFPM1, TRIM28, EZH2, MXD1, SID, LSD1, HP1a, HDAC3, ZNF436, ZNF257, ZNF675, ZNF490, ZNF320, ZNF331, ZNF816, ZNF41, ZNF189, ZNF528, ZNF543, ZNF140, ZNF610, ZNF350, ZNF8, ZNF30, ZNF98, ZNF677, ZNF596, ZNF214, ZNF37A, ZNF34, ZNF250, ZNF547, ZNF273, ZFP82, ZNF224, ZNF33A, ZNF45, ZNF175, ZNF184, ZFP28-1, ZFP28-2, ZNF18, ZNF213, ZNF394, ZFP1, ZFP14, ZNF416, ZNF557, ZNF729, ZNF254, ZNF764, ZNF785, ZNF10, CBX5, RYBP, YAF2, MGA, CBX1, SCMH1, MPP8, SUMO3, HERC2, BIN1, PCGF2, TOX, FOXA1, FOXA2, IRF2BP1, IRF2BP2, IRF2BPL IRF-2BP1_2 N-terminal domain, HOXA13, HOXB13, HOXC13, HOXA11, HOXC11, HOXC10, HOXA10, HOXB9, HOXA9, ZFP28, ZN334, ZN568, ZN37A, ZN181, ZN510, ZN862, ZN140, ZN208, ZN248, ZN571, ZN699, ZN726, ZIK1, ZNF2, Z705F, ZNF14, ZN471, ZN624, ZNF84, ZNF7, ZN891, ZN337, Z705G, ZN529, ZN729, ZN419, Z705A, ZN302, ZN486, ZN621, ZN688, ZN33A, ZN554, ZN878, ZN772, ZN224, ZN184, ZN544, ZNF57, ZN283, ZN549, ZN211, ZN615, ZN253, ZN226, ZN730, Z585A, ZN732, ZN681, ZN667, ZN649, ZN470, ZN484, ZN431, ZN382, ZN254, ZN124, ZN607, ZN317, ZN620, ZN141, ZN584, ZN540, ZN75D, ZN555, ZN658, ZN684, RBAK, ZN829, ZN582, ZN112, ZN716, HKR1, ZN350, ZN480, ZN416, ZNF92, ZN100, ZN736, ZNF74, ZN443, ZN195, ZN530, ZN782, ZN791, ZN331, Z354C, ZN157, ZN727, ZN550, ZN793, ZN235, ZN724, ZN573, ZN577, ZN789, ZN718, ZN300, ZN383, ZN429, ZN677, ZN850, ZN454, ZN257, ZN264, ZN485, ZN737, ZNF44, ZN596, ZN565, ZN543, ZFP69, SUMO1, ZNF12, ZN169, ZN433, ZN175, ZN347, ZNF25, ZN519, Z585B, ZN517, ZN846, ZN230, ZNF66, ZN713, ZN816, ZN426, ZN674, ZN627, ZNF20, Z587B, ZN316, ZN233, ZN611, ZN556, ZN234, ZN560, ZNF77, ZN682, ZN614, ZN785, ZN445, ZFP30, ZN225, ZN551, ZN610, ZN528, ZN284, ZN418, ZN490, ZN805, Z780B, ZN763, ZN285, ZNF85, ZN223, ZNF90, ZN557, ZN425, ZN229, ZN606, ZN155, ZN222, ZN442, ZNF91, ZN135, ZN778, ZN534, ZN586, ZN567, ZN440, ZN583, ZN441, ZNF43, ZN589, ZN563, ZN561, ZN136, ZN630, ZN527, ZN333, Z324B, ZN786, ZN709, ZN792, ZN599, ZN613, ZF69B, ZN799, ZN569, ZN564, ZN546, ZFP92, ZN723, ZN439, ZFP57, ZNF19, ZN404, ZN274, CBX3, ZN250, ZN570, ZN675, ZN695, ZN548, ZN132, ZN738, ZN420, ZN626, ZN559, ZN460, ZN268, ZN304, ZN605, ZN844, SUMO5, ZN101, ZN783, ZN417, ZN182, ZN823, ZN177, ZN197, ZN717, ZN669, ZN256, ZN251, CBX4, CDY2, CDYL2, ZN562, ZN461, Z324A, ZN766, ID2, ZN214, CBX7, ID1, CREM, SCX, ASCL1, ZN764, SCML2, TWST1, CREB1, TERF1, ID3, CBX8, GSX1, NKX22, ATF1, TWST2, ZNF17, TOX3, TOX4, ZMYM3, I2BP1, RHXF1, SSX2, I2BPL, ZN680, TRI68, HXA13, PHC3, TCF24, HXB13, HEY1, PHC2, ZNF81, FIGLA, SAM11, KMT2B, HEY2, JDP2, HXC13, ASCL4, HHEX, GSX2, ETV7, ASCL3, PHC1, OTP, I2BP2, VGLL2, HXA11, PDLI4, ASCL2, CDX4, ZN860, LMBL4, PDIP3, NKX25, CEBPB, ISL1, CDX2, PROP1, SIN3B, SMBT1, HXC11, HXC10, PRS6A, VSX1, NKX23, MTG16, HMX3, HMX1, KIF22, CSTF2, CEBPE, DLX2, PPARG, PRIC1, UNC4, BARX2, ALX3, TCF15, TERA, VSX2, HXD12, CDX1, TCF23, ALX1, HXA10, RX, CXXC5, SCML1, NFIL3, DLX6, MTG8, CEBPD, SEC13, FIP1, ALX4, LHX3, PRIC2, MAGI3, NELL1, PRRX1, MTG8R, RAX2, DLX3, DLX1, NKX26, NAB1, SAMD7, PITX3, WDR5, MEOX2, NAB2, DHX8, CBX6, EMX2, CPSF6, HXC12, KDM4B, LMBL3, PHX2A, EMX1, NC2B, DLX4, SRY, ZN777, ZN398, GATA3, BSH, SF3B4, TEAD1, TEAD3, RGAP1, PHF1, GATA2, FOXO3, ZN212, IRX4, ZBED6, LHX4, SIN3A, RBBP7, NKX61, R51A1, MB3L1, DLX5, NOTC1, TERF2, ZN282, RGS12, ZN840, SPI2B, PAX7, NKX62, ASXL2, FOXO1, GATA1, ZMYM5, LRP1, MIXL1, SGT1, LMCD1, CEBPA, SOX14, WTIP, PRP19, NKX11, RBBP4, DMRT2, SMCA2, and a fragment thereof.

In some embodiments, the transcriptional repressor domain comprises an amino acid sequence as set forth in any one of SEQ ID NOs: 22-46.

In some embodiments, one or more of the nucleic acid-binding domain, the epigenetic modification domain and the transcriptional regulation domain are linked via a linker sequence.

In some embodiments, the linker sequence comprises at least 16 amino acids.

In some embodiments, the linker sequence comprises an XTEN linker sequence.

In some embodiments, the linker sequence comprises one or more fragment sequences truncated from an amino acid sequence as set forth in SEQ ID NO: 59, and the one or more fragment sequences comprise no less than 16 consecutive amino acids.

In some embodiments, the linker sequence comprises a GS linking peptide, and the GS linking peptide comprises the sequence (GS)a(GGS)b(GGGS)c(GGGGS)d, in which G represents a glycine residue (Gly), S represents a serine residue (Ser), and a, b, c and d represent integers greater than or equal to 0.

In some embodiments, the linker sequence comprises an amino acid sequence selected from one or more of SEQ ID NOs: 47-58.

In some embodiments, the one or more effector domains are N-terminal or C-terminal to the nucleic acid-binding domain.

In some embodiments, the effector domain comprises at least one type of the epigenetic modification domain and at least one type of the transcriptional regulation domain.

In some embodiments, the epigenetic modification domain comprises DNMT3A and DNMT3L, and the C-terminus of the DNMT3A is linked to the N-terminus of the DNMT3L.

In some embodiments, the epigenetic modification domain comprises DNMT3A and DNMT3L, and the C-terminus of the DNMT3L is linked to the N-terminus of the DNMT3A.

In some embodiments, the transcriptional regulation domain comprises a transcriptional repressor domain.

In some embodiments, the transcriptional regulation domain comprises a zinc finger protein-based transcription factor or a functionally active fragment thereof.

In some embodiments, the zinc finger protein-based transcription factor comprises KRAB.

In some embodiments, the zinc finger protein-based transcription factor is selected from ZIM3 KRAB and KOX1 KRAB.

In some embodiments, the transcriptional regulation domain comprises two or more of the zinc finger protein-based transcription factors or functionally active fragments thereof described above.

In some embodiments, the transcriptional regulation domain comprises two or more of the KRAB domains described above, and the two or more of the KRAB domains described above are of the same type or of different types.

In some embodiments, the two or more of the KRAB domains described above are linked via an XTEN linker sequence.

In some embodiments, the one or more effector domains are N-terminal to the nucleic acid-binding domain, and the epigenetic editing domain in the effector domain is N-terminal or C-terminal to the transcriptional regulation domain.

In some embodiments, the fusion comprises the following substances linked in sequence from the N-terminus to the C-terminus: (1) at least one type of the epigenetic editing domain, at least one type of the transcriptional regulation domain and the nucleic acid-binding domain; (2) at least one type of the transcriptional regulation domain, at least one type of the epigenetic editing domain and the nucleic acid-binding domain; or (3) at least one type of the transcriptional regulation domain, at least one type of the epigenetic editing domain, at least one type of the transcriptional regulation domain and the nucleic acid-binding domain.

In some embodiments, the fusion comprises the following substances linked in sequence from the N-terminus to the C-terminus: (1) at least one DNA methyltransferase domain, at least one transcriptional repressor domain and the nucleic acid-binding domain; (2) at least one transcriptional repressor domain, at least one DNA methyltransferase domain and the nucleic acid-binding domain; or (3) at least one transcriptional repressor domain, at least one DNA methyltransferase domain, at least one transcriptional repressor domain and the nucleic acid-binding domain.

In some embodiments, the fusion comprises the following substances linked in sequence from the N-terminus to the C-terminus: (1) one or a combination of DNMT3A and DNMT3L, one or more of the zinc finger protein-based transcription factors described above, and dCas9; (2) one or more of the zinc finger protein-based transcription factors described above, one or a combination of DNMT3A and DNMT3L, and dCas9; or (3) one or more of the zinc finger protein-based transcription factors described above, one or a combination of DNMT3A and DNMT3L, one or more of the zinc finger protein-based transcription factors described above, and dCas9.

In some embodiments, the fusion comprises the following domains: DNMT3A-DNMT3L-KRAB-dCas9, KRAB-DNMT3A-DNMT3L-dCas9, DNMT3A-DNMT3L-ZNF582-dCas9, DNMT3A-DNMT3L-ZNF324-dCas9, DNMT3A-DNMT3L-ZNF680-dCas9, DNMT3A-DNMT3L-ZNF354a-dCas9, DNMT3A-DNMT3L-ZNF419-dCas9, KRAB-DNMT3A-DNMT3L-KRAB-dCas9, KRAB-KRAB-DNMT3A-DNMT3L-dCas9, or DNMT3A-DNMT3L-KRAB-KRAB-dCas9, wherein - indicates that the domains of the fusion are each directly and/or indirectly linked, and the domains are arranged in the order from N-terminus to C-terminus.

In some embodiments, the one or more effector domains are C-terminal to the nucleic acid-binding domain, and the epigenetic editing domain in the effector domain is N-terminal or C-terminal to the transcriptional regulation domain.

In some embodiments, the fusion comprises the following substances linked in sequence from the N-terminus to the C-terminus: (1) the nucleic acid-binding domain, at least one type of the epigenetic editing domain and at least one type of the transcriptional regulation domain; (2) the nucleic acid-binding domain, at least one type of the transcriptional regulation domain and at least one type of the epigenetic editing domain; or (3) the nucleic acid-binding domain, at least one type of the transcriptional regulation domain, at least one type of the epigenetic editing domain and at least one type of the transcriptional regulation domain.

In some embodiments, the fusion comprises the following substances linked in sequence from the N-terminus to the C-terminus: (1) the nucleic acid-binding domain, at least one DNA methyltransferase domain and at least one transcriptional repressor domain; (2) the nucleic acid-binding domain, at least one transcriptional repressor domain and at least one DNA methyltransferase domain; or (3) the nucleic acid-binding domain, at least one transcriptional repressor domain, at least one DNA methyltransferase domain and at least one transcriptional repressor domain.

In some embodiments, the fusion comprises the following substances linked in sequence from the N-terminus to the C-terminus: (1) dCas9, one or a combination of DNMT3A and DNMT3L, and one or more of the zinc finger protein-based transcription factors described above; (2) dCas9, one or more of the zinc finger protein-based transcription factors described above, and one or a combination of DNMT3A and DNMT3L; or (3) dCas9, one or more of the zinc finger protein-based transcription factors described above, one or a combination of DNMT3A and DNMT3L, and one or more of the zinc finger protein-based transcription factors described above.

In some embodiments, the fusion comprises the following domains: dCas9-DNMT3A-DNMT3L-KRAB, dCas9-DNMT3L-DNMT3A-KRAB, dCas9-KRAB-DNMT3A-DNMT3L, dCas9-KRAB-DNMT3L-DNMT3A, dCas9-ZNF582-DNMT3L-DNMT3A, dCas9-ZNF324-DNMT3L-DNMT3A, dCas9-DNMT3L-DNMT3A-KRAB-KRAB, dCas9-DNMT3L-DNMT3A-KRAB-KRAB-KRAB, or dCas9-KRAB-DNMT3L-DNMT3A-KRAB, wherein - indicates that the domains of the fusion are each directly and/or indirectly linked, and the domains are arranged in the order from N-terminus to C-terminus.

In some embodiments, the fusion comprises an amino acid sequence as set forth in any one of SEQ ID NOs: 60-67, 70-107 and 337-344.

In some embodiments, the fusion comprises one or more effector domains, and the effector domains are N-terminal and C-terminal to the nucleic acid-binding domain.

In some embodiments, the one or more effector domains comprise at least one epigenetic editing domain.

In some embodiments, the one or more effector domains comprise at least one epigenetic editing domain that provides histone modification.

In some embodiments, the effector domain that is C-terminal to the nucleic acid-binding domain comprises at least one type of epigenetic modification domain.

In some embodiments, the effector domain that is C-terminal to the nucleic acid-binding domain comprises at least one epigenetic modification domain that provides histone modification.

In some embodiments, the effector domain that is N-terminal to the nucleic acid-binding domain comprises one or more of the epigenetic modification domain and the transcriptional regulation domain.

In some embodiments, the effector domain that is N-terminal to the nucleic acid-binding domain comprises one or more of the epigenetic modification domain that provides histone modification, an epigenetic modification domain that provides DNA modification, and the transcriptional repressor domain.

In some embodiments, the effector domain that is N-terminal to the nucleic acid-binding domain comprises: (1) the epigenetic modification domain that provides histone modification; (2) the transcriptional repressor domain; (3) the epigenetic modification domain that provides DNA modification; or (4) the transcriptional repressor domain and the epigenetic modification domain that provides DNA modification.

In some embodiments, the effector domain that is N-terminal to the nucleic acid-binding domain comprises: (1) one or more epigenetic modification domains selected from EZH2, HDAC3, HDAC1, EHMT2, PRMT1, PRMT5, SETDB1, hSIRT1, HP1a, LSD1, and functionally active fragments thereof; (2) KRAB or a functionally active fragment thereof; (3) DNMT3A, DNMT3L, or a combination of DNMT3A and DNMT3L; or (4) a combination of KRAB and DNMT3A, a combination of KRAB and DNMT3L, or a combination of KRAB, DNMT3A, and DNMT3L.

In some embodiments, the fusion comprises the following substances linked in sequence from the N-terminus to the C-terminus: (1) the epigenetic modification domain that provides histone modification, the nucleic acid-binding domain and the epigenetic modification domain that provides histone modification; (2) the transcriptional repressor domain, the nucleic acid-binding domain and the epigenetic modification domain that provides histone modification; (3) the epigenetic modification domain that provides DNA modification, the nucleic acid-binding domain and the epigenetic modification domain that provides histone modification; (4) the transcriptional repressor domain, the epigenetic modification domain that provides DNA modification, the nucleic acid-binding domain, and the epigenetic modification domain that provides histone modification; or (5) the epigenetic modification domain that provides DNA modification, the transcriptional repressor domain, the nucleic acid-binding domain, and the epigenetic modification domain that provides histone modification.

In some embodiments, the epigenetic modification domain that provides histone modification is selected from EZH2, HDAC3, HDAC1, EHMT2, PRMT1, PRMT5, SETDB1, hSIRT1, HP1a, LSD1, and a functionally active fragment thereof.

In some embodiments, the epigenetic modification domain that provides DNA modification is selected from DNMT3A, DNMT3L, a combination of DNMT3A and DNMT3L, or a functionally active fragment thereof.

In some embodiments, the transcriptional repressor domain is KRAB and/or a functionally active fragment thereof.

In some embodiments, the fusion comprises the following domains: DNMT3A-DNMT3L-dCas9-KRAB, HDAC3-dCas9-EZH2, KRAB-dCas9-EZH2, KRAB-DNMT3A-dCas9-EZH2, KRAB-DNMT3A-dCas9-HDAC3, DNMT3A-DNMT3L-dCas9-(EZH2)ₙ₌₁₋₁₁, DNMT3A-DNMT3L-dCas9-HDAC3, DNMT3A-DNMT3L-dCas9-EHMT2, DNMT3A-DNMT3L-dCas9-HDAC1, DNTM3A-DNMT3L-dCas9-PRMT1, DNMT3A-DNMT3L-dCas9-SETDB1, DNMT3A-DNMT3L-dCas9-hSIRT1, DNMT3A-DNMT3L-dCas9-PRMT5, DNMT3A-DNMT3L-dCas9-HP1a, DNMT3A-DNMT3L-dCas9-LSD1, or DNMT3A-DNMT3L-TALE-KRAB, wherein - indicates that the domains of the fusion are each directly and/or indirectly linked, and the domains are arranged in the order from N-terminus to C-terminus.

In some embodiments, the fusion comprises an amino acid sequence as set forth in any one of SEQ ID NOs: 68, 69, 108-126, 355 and 356.

In some embodiments, the fusion further comprises a nuclear localization signal and/or a tag domain.

In another aspect, the present application provides a nucleic acid encoding the fusion described in the present application.

In another aspect, the present application provides a recombinant vector comprising the nucleic acid described in the present application.

**In** another aspect, the present application provides a delivery vector comprising the fusion described in the present application, the nucleic acid described in the present application, and/or the recombinant vector described in the present application, and optionally comprising a liposome and/or a lipid nanoparticle.

**In** another aspect, the present application provides a composition comprising the fusion described in the present application, the nucleic acid described in the present application, the recombinant vector described in the present application, and/or the delivery vector described in the present application.

**In** another aspect, the present application provides a cell comprising the fusion described in the present application, the nucleic acid described in the present application, the recombinant vector described in the present application, the delivery vector described in the present application, and/or the composition described in the present application.

In another aspect, the present application provides a kit comprising the fusion described in the present application, the nucleic acid described in the present application, the recombinant vector described in the present application, the delivery vector described in the present application, the composition described in the present application, and/or the cell described in the present application.

In some embodiments, the kit further comprises at least one container for placing the fusion, the nucleic acid, the recombinant vector, the delivery vector, the composition, and/or the cell.

In some embodiments, the kit further comprises instructions in physical form and/or in machine-readable electronic form.

In another aspect, the present application provides a method for regulating the expression of a target gene, wherein the method comprises administering the fusion described in the present application, the nucleic acid described in the present application, the recombinant vector described in the present application, the delivery vector described in the present application, the composition described in the present application, the cell described in the present application, and/or the kit described in the present application.

In some embodiments, the method comprises introducing the fusion, the nucleic acid, the recombinant vector, the delivery vector, the composition, the cell, and/or the kit into a cell containing the target gene.

In some embodiments, the method comprises contacting the fusion, the nucleic acid, the recombinant vector, the delivery vector, and/or the composition with a regulatory element near and/or of the target gene.

In some embodiments, the regulatory element comprises a core promoter, a proximal promoter, a distal enhancer, a silencer, an insulator element, a boundary element, and/or a locus control region.

In another aspect, the present application provides a method for treating or alleviating a disease or condition associated with abnormal expression and/or abnormal activity of a target gene, wherein the method comprises administering to a subject in need thereof an effective amount of the fusion described in the present application, the nucleic acid described in the present application, the recombinant vector described in the present application, the delivery vector described in the present application, the composition described in the present application, the cell described in the present application, and/or the kit described in the present application.

In another aspect, the present application provides use of the fusion described in the present application, the nucleic acid described in the present application, the recombinant vector described in the present application, the delivery vector described in the present application, the composition described in the present application, the cell described in the present application and/or the kit described in the present application for preparing a drug, wherein the drug is used to treat or alleviate a disease or condition thereof associated with abnormal expression and/or activity of a target gene.

In another aspect, the present application provides the fusion described in the present application, the nucleic acid described in the present application, the recombinant vector described in the present application, the delivery vector described in the present application, the composition described in the present application, the cell described in the present application, or the kit described in the present application, which is used to treat or alleviate a disease or condition thereof associated with abnormal expression and/or activity of a target gene.

Those skilled in the art can easily discern other aspects and advantages of the present application from the following detailed description. Only exemplary embodiments of the present application are shown and described in the following detailed description. As those skilled in the art will realize, the contents of the present application enable those skilled in the art to make changes to the specific embodiments disclosed without departing from the spirit and scope of the invention covered by the present application. Accordingly, the drawings and descriptions in the specification of the present application are illustrative only and not restrictive.

### BRIEF DESCRIPTION OF DRAWINGS

The specific features of the invention to which the present application relates are set forth in the appended claims. The features and advantages of the invention to which the present application relates can be better understood by reference to the exemplary embodiments described in detail below and the drawings. The accompanying drawings are briefly described as follows:
FIG. 1 shows a schematic diagram of the composition of an example fusion of the present application.
FIG. 2 shows a schematic diagram of the composition of an example fusion of the present application containing different transcriptional repressor domains.
FIG. 3 shows the inhibition effect of an example fusion of the present application on the VEGFA gene.
FIG. 4 shows the inhibition effect of an example fusion of the present application on the CLTA gene.
Figures 5A-5C show the inhibition effect of an example fusion of the present application on the CD151 gene.
Figures 6A-6F show the inhibition effect of an example fusion of the present application on the PCSK9 gene.
FIG. 7 shows the inhibition effect of an example fusion of the present application on the PCSK9 gene.

### DETAILED DESCRIPTION

The embodiments of the invention of the present application will be described below with specific examples. Those skilled in the art may easily understand other advantages and effects of the invention of the present application from the disclosure of the specification.

### DEFINITION OF TERMS

In the present application, the term "epigenetic modification domain" typically refers to a domain that is capable of changing the gene expression or cell phenotype of a cell population after administration. It is understood that such changes refer to one or more functionally relevant modifications to the genome without involving changes in the nucleic acid sequence. Examples of such modifications are DNA methylation and histone modification, both of which are important for the regulation of gene expression without changing the underlying DNA sequence.

In the present application, the term "DNA-binding domain" typically refers to an independently folded protein domain that contains at least one motif that recognizes double-stranded or single-stranded DNA. For example, the DNA-binding domain may recognize a specific DNA sequence (recognition or regulation sequence) or have a general affinity for DNA. In certain instances, other domains of the DNA-binding domain often regulate the activity of the DNA-binding domain; the DNA binding function may be structural regulation or include transcriptional regulation, and sometimes these two functions overlap. In certain embodiments of the method and gene expression regulatory molecules provided in the present application, the DNA-binding domain may comprise a (DNA) nuclease, such as a nuclease that can target DNA in a sequence-specific manner or can be guided or instructed to target DNA in a sequence-specific manner, such as a CRISPR-Cas system, a zinc finger nuclease (ZFN), a transcription activator-like effector nuclease (TALEN) or a large-range nuclease. In some embodiments, the DNA-binding domain is a DNA nuclease derived from a CRISPR-Cas system. For example, the DNA nuclease derived from a CRISPR-Cas system is a Cas protein.

In the present application, the term "TALE DNA-binding domain" or "TALE" is a polypeptide containing one or more TALE repeating domains/units. Naturally occurring TALEs or "wild-type TALEs" are nucleic acid-binding proteins secreted by numerous species of Proteobacteria. TALE polypeptides contain a nucleic acid-binding domain composed of tandem repeats of highly conserved monomeric polypeptides that are primarily 33, 34, or 35 amino acids in length and differ from each other primarily in amino acid positions 12 and 13. In a preferred embodiment, the nucleic acid is DNA. As used herein, the polypeptide monomer of TALE is used to refer to a repeating polypeptide sequence that is highly conserved within the nucleic acid binding domain of TALE, and the term "repeating variable di-residue" or "RVD" is used to refer to amino acids that are highly variable at positions 12 and 13 of the polypeptide monomer. A general representation of a TALE monomer contained within a DNA-binding domain is X₁₋₁₁-(X₁₂X₁₃)-X_{14-33 or 34 or 35}, wherein the subscripts indicate the amino acid position and X represents any amino acid. X₁₂X₁₃ indicates the RVD. In some TALE polypeptide monomers, the variable amino acid at position 13 is deleted or absent, and in such monomers, the RVD consists of a single amino acid. In such cases, the RVD may alternatively be represented as X*, wherein X represents X₁₂ and (*) indicates that X₁₃ is absent. The DNA-binding domain comprises several repeats of the TALE monomer and this can be represented as (X₁₋₁₁-(X₁₂X₁₃)-X_{14-33 or 34 or 35})_{z}, wherein in a preferred embodiment z is at least 5-40. In a further preferred embodiment, z is at least 10-26.

The TALE monomer has a nucleotide binding affinity determined by the type of amino acid within its RVD. For example, a polypeptide monomer having an RVD of NI preferentially binds to adenine (A), a polypeptide monomer having an RVD of NG preferentially binds to thymine (T), a polypeptide monomer having an RVD of HD preferentially binds to cytosine (C), and a monomer having an RVD of NN preferentially binds to adenine (A) and guanine (G). In other embodiments, a monomer having an RVD of IG preferentially binds to T. Thus, the number and order of polypeptide monomer repeats in the nucleic acid-binding domain of a TALE determines the nucleic acid target specificity of the TALE. In a further embodiment of the present application, a monomer having an RVD of NS recognizes all four base pairs and can bind to A, T, G or C. The structure and function of TALEs are further described, e.g., in Moscou et al., Science 326: 1501 (2009); Boch et al., Science 326: 1509-1512 (2009); and Zhang et al., Nature Biotechnology 29: 149-153 (2011), each of which is incorporated by reference in its entirety. The repeat domain of a TALE is involved in the binding of the TALE to its cognate target DNA sequence. These repeat units (or "repeat sequences") exhibit at least some sequence homology to other TALE repeat sequences within naturally occurring TALE proteins. See, e.g., U.S. Patent Publication No. 20110301073. The TALE-binding domains contemplated in the present application can be "engineered" to bind to a predetermined nucleotide sequence, for example, via engineering (changing one or more amino acids) of the recognition helix region of a naturally occurring TALE protein. Thus, engineered DNA-binding proteins (TALEs) are non-naturally occurring proteins. Non-limiting examples of methods for engineering DNA-binding proteins are design and selection. Designed DNA-binding proteins are non-naturally occurring proteins whose design and/or composition are primarily derived from rational criteria. Rational design criteria include the application of substitution rules and computational algorithms for processing information in information databases storing existing TALE design and binding data. See, e.g., U.S. Patents 6,140,081; 6,453,242; and 6,534,261; see also WO 98/53058; WO 98/53059; WO 98/53060; WO 02/016536 and WO 03/016496 and U.S. Publication No. 20110301073.

In the present application, "Cas enzyme" can be used interchangeably with "Cas protein", "CRISPR protein", "CRISPR enzyme", "CRISPR-Cas protein", "CRISPR-Cas enzyme", "Cas", "CRISPR effector" or "Cas effector protein", which typically refers to a class of enzymes that are complementary to the CRISPR sequence and can use the CRISPR sequence as a guide to recognize and cleave a specific DNA strand. Non-limiting examples of Cas proteins include: Cas1, CaslB, Cas2, Cas3, Cas4, Cas5, Cas6, Cas7, Cas8, Cas9 (also known as Csnl and Csxl2), CaslO, Csyl, Csy2, Csy3, Csel, Cse2, Cscl, Csc2, Csa5, Csn2, Csm2, Csm3, Csm4, Csm5, Csm6, Cmrl, Cmr3, Cmr4, Cmr5, Cmr6, Csbl, Csb2, Csb3, Csxl7, Csxl4, CsxlO, Csxl6, CsaX, Csx3, Csxl, Csxl5, Csf1, Csf2, Csf3, Csf4, and/or homologs thereof, or modified forms thereof. These proteins are known, for example, the amino acid sequence of the Streptococcus pyogenes Cas9 protein can be found in the SwissProt database under accession number Q99ZW2.

In the present application, the term "dCas9 enzyme" is also referred to as "dead Cas9 protein" or "dead Cas9 enzyme". Known methods for generating Cas9 proteins (or fragments thereof) with inactivated DNA cleavage domains are described in, for example, Jinek et al., Science. 337: 816-821 (2012); Qi et al., "Repurposing CRISPR as an RNA-Guided Platform for Sequence-Specific Control of Gene Expression", Cell. 28, 152(5): 1173-83 (2013), each of which is incorporated herein by reference in its entirety). For example, it is known that the DNA cleavage domain of Cas9 includes two subdomains, the HNH nuclease subdomain and the RuvC1 subdomain. The HNH subdomain cuts the strand complementary to the gRNA, while the RuvC1 subdomain cleaves the non-complementary strand. Mutations in these subdomains can silence the nuclease activity of Cas9. For example, mutations D10A and H840A completely inactivate the nuclease activity of Streptococcus pyogenes Cas9 (Jinek et al., Science. 337:816-821 (2012); Qi et al., Cell. 28;152(5):1173-83 (2013)). Suitable CRISPR inactivating or nicking DNA-binding domains include, but are not limited to, nuclease-inactive variant Cas9 domains including D10A, D10A/D839A/H840A and D10A/D839A/H840A/N863A mutant domains as described in WO2015089406A1, which is incorporated herein by reference. In some cases, the endonuclease-inactive dCas9 from Streptococcus pyogenes has been targeted to genes in bacteria, yeast, and human cells by gRNA to silence gene expression through steric hindrance. As used herein, "dCas" may refer to a dCas protein or a fragment thereof. As used herein, "dCas9" may refer to a dCas9 protein or a fragment thereof. As used herein, the terms "iCas" and "dCas" are used interchangeably to refer to a catalytically inactive CRISPR-associated protein. In one embodiment, the dCas protein comprises one or more mutations in the DNA cleavage domain. In one embodiment, the dCas protein comprises one or more mutations in the RuvC domain. In one embodiment, the dCas molecule comprises one or more mutations in both the RuvC and HNH domains. In one embodiment, the dCas protein is a fragment of a wild-type Cas protein. In one embodiment, the dCas protein comprises a functional domain from a wild-type Cas protein, wherein the functional domain is selected from a Reel domain, a bridge helix domain, or a PAM interaction domain. In one embodiment, the nuclease activity of dCas is reduced by at least 40%, at least 45%, at least 50%, at least 55%, at least 60%, at least 65%, at least 70%, at least 75%, at least 80%, at least 85%, at least 90%, at least 95%, or at least 99% compared with the nuclease activity of the corresponding wild-type Cas protein.

A suitable dCas can be derived from a wild-type Cas protein. The Cas protein can be from a type I, type II, or type III CRISPR-Cas system. In one embodiment, a suitable dCas may be derived from Cas1, Cas2, Cas3, Cas4, Cas5, Cas6, Cas7, Cas8, Cas9, or Cas10. In one embodiment, the dCas is derived from a Cas9 protein. For example, dCas9 can be obtained by introducing point mutations (e.g., substitutions, deletions or additions) in the DNA cleavage domain (e.g., the nuclease domain, such as RuvC and/or HNH domains) of the Cas9 protein. See, e.g., Jinek et al., Science (2012) 337:816-21, incorporated herein by reference in its entirety. For example, the introduction of two point mutations in the RuvC and HNH domains reduced Cas9 nuclease activity while retaining Cas9 sgRNA- and DNA-binding activities. In one embodiment, the two point mutations within the RuvC and HNH active sites are the D10A and H840A mutations of Streptococcus pyogenes Cas9. Alternatively, D10 and H840 of Streptococcus pyogenes Cas9 can be deleted to eliminate Cas9 nuclease activity while retaining its sgRNA-and DNA-binding activities. In one embodiment, the two point mutations within the RuvC and HNH active sites are the D10A and N580A mutations of Streptococcus pyogenes Cas9.

In various embodiments, the present application relates to a dCas protein or any variant or mutant thereof. All variants and mutants of dCas9 can be used in the methods, compositions, fusion molecules or kits disclosed herein, and include but not limited to those variants and mutants derived from SpCas9 (Cas9 isolated from Streptococcus pyogenes), SaCas9 (Cas9 isolated from Staphylococcus aureus), StCas9 (Cas9 isolated from Streptococcus thermophilus), NmCas9 (Cas9 isolated from Neisseria meningitidis), FnCas9 (Cas9 isolated from Francisella novicida), CjCas9 (Cas9 isolated from Campylobacter jejuni), ScCas9 (Cas9 isolated from Streptococcus canis), and any variants and mutant forms of Cas9 listed above, such as high-fidelity Cas9 (Kleinstiver et al., Nature. January 28, 2016) and enhanced SpCas9 (Slaymaker et al., Sciences. January 1, 2016). For example, the dCas9 sequences as set forth in SEQ ID NOs: 1162-1179 of the present application only provide a few exemplary options and are not exclusive. In one embodiment, the dCas protein is a Streptococcus pyogenes dCas9 protein comprising a mutation at D10 and/or H840 (as set forth in SEQ ID NO: 1162). In one embodiment, the dCas protein is a Streptococcus pyogenes dCas9 protein comprising the D10A and/or H840A mutations (as set forth in SEQ ID NO: 1162). In one embodiment, the dCas9 protein is a Staphylococcus aureus dCas9 protein, which comprises an amino acid sequence as set forth in SEQ ID NO: 1163 or 1164, a sequence substantially identical to SEQ ID NO: 1163 or 1164 (e.g., at least 80%, at least 85%, at least 90%, at least 91%, at least 92%, at least 93%, at least 94%, at least 95%, at least 96%, at least 97%, at least 98%, at least 99% or more sequence identity), or a sequence having 1, 2, 3, 4, 5 or more changes (e.g., amino acid substitutions, insertions or deletions) relative to SEQ ID NO: 1163 or 1164, or any fragment thereof.

Similar mutations can also be applied to any other naturally occurring Cas9 (e.g., Cas9 from other species) or engineered Cas9. In certain embodiments, the dCas9 comprises Streptococcus pyogenes dCas9, Staphylococcus aureus dCas9, Campylobacter jejuni dCas9, Corynebacterium diphtheria dCas9, Eubacterium ventriosum dCas9, Streptococcus pasteurianus dCas9, Lactobacillus farciminis dCas9, Sphaerochaeta globus dCas9, Azospirillum (e.g., strain B510) dCas9, Gluconacetobacter diazotrophicus dCas9, Neisseria cinerea dCas9, Roseburia intestinalis dCas9, Parvibaculum lavamentivorans dCas9, Nitratifractor salsuginis (e.g., strain DSM 16511) dCas9, Campylobacter lari (e.g., strain CF89-12) dCas9, Streptococcus thermophilus (e.g., strain LMD-9) dCas9, or fragments thereof. In certain embodiments, the present application further provides a vector comprising a nucleotide encoding the following protein molecules: Streptococcus pyogenes dCas9, Staphylococcus aureus dCas9, Campylobacter jejuni dCas9, Corynebacterium diphtheria dCas9, Eubacterium ventriosum dCas9, Streptococcus pasteurianus dCas9, Lactobacillus farciminis dCas9, Sphaerochaeta globus dCas9, Azospirillum (strain B510) dCas9, Gluconacetobacter diazotrophicus dCas9, Neisseria cinerea dCas9, Roseburia intestinalis dCas9, Parvibaculum lavamentivorans dCas9, Nitratifractor salsuginis (strain DSM 16511) dCas9, Campylobacter lari (strain CF89-12) dCas9, Streptococcus thermophilus (strain LMD-9) dCas9, or fragments thereof.

In the present application, the term "capable of binding" can be used interchangeably with "binding to", "specifically recognizing", "targeting", etc., and typically means that the binding molecule (for example, the gene expression regulatory molecule of the present application) is able to interact with the nucleotides on the target gene or target site, or the binding molecule (for example, the gene expression regulatory molecule of the present application) has sufficient affinity for the target gene or target site, and such interaction may be by means of conjugation, coupling, attachment, providing complementarity, providing covalent force or providing non-covalent force, improving binding stability, etc.

In the present application, the terms "guide RNA", "guidance DNA" and "gRNA" are used interchangeably and typically refer to a DNA molecule capable of directing a nuclease (e.g., Argonaute, or Ago) to bind and/or cleave a target gene. In some preferred embodiments, the guide DNA may include a single-stranded DNA molecule (ssDNA), a single-stranded DNA molecule that is phosphorylated at the 5' end, a single-stranded DNA molecule that is hydroxylated at the 5' end, or a base fragment that is complementary to the target gene, and/or has a length of 8-35 nt. In some embodiments of the present application, the term "guide RNA" refers to an RNA comprising: (1) an "activating" nucleotide sequence that binds to a guide RNA-directed endonuclease (e.g., a class II Cas nuclease, such as a type II, type V, or type VI Cas endonuclease) and activates the RNA-directed endonuclease; and (2) a "target" nucleotide sequence comprising a nucleotide sequence that hybridizes to a target nucleic acid. The "activating" nucleotide sequence and the "target" nucleotide sequence can be on separate RNA molecules (e.g., "dual-guide RNAs"); or can be on the same RNA molecule ("single-guide RNA", also called sgRNA).

In the present application, the term "class II Cas nuclease" typically refers to a class of Cas proteins that perform recognition and/or cleavage functions in the form of a single protein, as defined by the updated classification scheme for CRISPR/Cas loci (Makarova et al., (2015) Nat Rev Microbiol, 13(11):722-36; Shmakov et al., (2015) Mol Cell, 60:385-397).

In the present application, the term "class II type II Cas nuclease and class II type V Cas nuclease" typically refers to RNA-directed endonucleases in the form of a single protein among class II Cas nucleases. Among them, type II and type V-B Cas nucleases in type V require the joint action of tracrRNA (trans-activating CRISPR RNA) and crRNA (CRISPR RNA) to function normally, and crRNA and tracrRNA can be artificially combined into a single guide RNA (sgRNA); type V-A Cas nucleases in type V require use of crRNA alone to perform their guide function. Non-limiting examples of class II type II Cas nucleases include Cas9 and family-related nucleases thereof, and non-limiting examples of class II type V Cas nucleases include Cas12a (also known as Cpf1), Cas12b (also known as C2c1), Cas12c (also known as C2c3), Cas12d (CasY), Cas12e (CasX), Cas12g, Cas12h, Cas12i, C2c1, C2c4, C2c5, C2c8, C2c9, C2c10, Cas14a, Cas14b, Cas14c nuclease and/or TnpB.

In the present application, the term "DNA methyltransferase" typically refers to an enzyme that catalyzes the transfer of a methyl group to DNA. Non-limiting examples of DNA methyltransferases include DNMT1, DNMT 3A, DNMT 3B, and DNMT 3L. For example, through DNA methylation, DNA methyltransferases can modify the activity of DNA fragments (such as regulating gene expression) without changing the DNA sequence. As described herein, gene expression regulatory molecules may include one or more (e.g., two) DNA methyltransferases. When a DNA methyltransferase is included as part of a gene expression regulatory molecule, the DNA methyltransferase may be referred to as a "DNA methyltransferase domain." In various aspects, the DNA methyltransferase domain comprises a variant or homolog of an amino acid sequence having at least 50%, at least 55%, at least 60%, at least 65%, at least 70%, at least 75%, at least 80%, at least 85%, 90%, at least 91%, at least 92%, at least 93%, at least 94%, at least 95%, at least 96%, at least 97%, at least 98%, at least 99%, or 100% sequence identity to DNMT 3A. In various aspects, the DNA methyltransferase domain comprises a variant or homolog of an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to DNMT 3L.

In the present application, the term "functionally active fragment" typically refers to a fragment that has a partial region of a full-length protein or nucleic acid but retains or partially retains the biological activity or function of the full-length protein or nucleic acid. For example, the functionally active fragment can retain or partially retain the ability of the full-length protein to bind to another molecule. For example, the functionally active fragment of the DNA methyltransferase can retain or partially retain the biologically active function of the full-length DNA methyltransferase to catalyze the transfer of a methyl group to DNA.

In the present application, the term "linker" typically refers to a linker that links 2 or more moieties. In various embodiments, the linker is linked to the amino acid sequence of the remainder of the compound (e.g., a fusion protein provided herein) at the N-terminus and the C-terminus. For example, the terms "XTEN," "XTEN linker," or "XTEN polypeptide," as used herein, refer to a recombinant polypeptide lacking hydrophobic amino acid residues (e.g., an unstructured recombinant peptide); the development and use of XTEN can be found, for example, in Schellenberger et al., Nature Biotechnology 27, 1186-1190 (2009), which is incorporated herein by reference in its entirety.

In the present application, the terms "inhibit", "repress", "silencing" and the like typically refer to a reduction in gene expression and/or activity. For example, administration of a substance of the present application (e.g., fusion protein, complex, nucleic acid, and vector) may negatively affect (e.g., reduce) the activity of a nucleic acid sequence relative to the activity of the nucleic acid sequence in the absence of the substance (control). For example, inhibition may refer to a decrease in a disease or a symptom of a disease. For example, inhibition includes at least partially, partially or completely blocking activation (e.g., transcription) of a nucleic acid sequence, or reducing, preventing or delaying activation of a nucleic acid sequence. For example, the inhibitory activity may be 90%, 80%, 70%, 60%, 50%, 40%, 30%, 20%, 10% or less of that of the control.

In the present application, the term "transcriptional repressor" typically refers to a substance and/or agent, such as a protein (e.g., a transcription factor or a fragment thereof), that binds to a target nucleic acid sequence and causes a reduction in the expression level of a gene product associated with the target nucleic acid sequence. For example, the gene product can be an RNA (e.g., mRNA) transcribed from a gene or a polypeptide translated from an mRNA transcribed from a gene. Typically, an increase or decrease in the level of an mRNA results in an increase or decrease in the level of a polypeptide translated therefrom. Expression levels can be determined using standard techniques for measuring mRNA or protein. Non-limiting examples of transcriptional repressors include: mSin3 interacting domain (SID) protein, methyl-CpG-binding domain 2 (MBD2), MBD3, DNAmethyltransferase (DNMT) 1 (DNMT1), DNMT2A, DNMT3A, DNMT3B, DNMT3L, retinoblastoma protein (Rb), methyl CpG binding protein 2 (Mecp2), GATA-1 and cofactor Fog1 thereof, MAT2 regulator (ROM2), Arabidopsis HD2A protein (AtHD2A), lysine-specific demethylase 1 (LSD1) and/or Krüppel-related box (KRAB).

In the present application, the term "KRAB" is also referred to as "Krüppel-related box domain" or "Krüppel-associated box domain", which typically refers to about 45 to about 75 amino acid residues of the transcriptional repression domain present in the transcription factors of human zinc finger proteins. In various aspects, the KRAB domain can include a variant or homolog of an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to a ZIM3 KRAB domain or a KOX1 KRAB domain.

In the present application, the term "nuclear localization sequence" or "nuclear localization signal" or "NLS" typically refers to a peptide that directs a protein to the nucleus of a cell. For example, the NLS includes five basic positively charged amino acids. For example, the NLS can be located at any position on the peptide chain.

In the present application, the term "tag" refers to a peptide, which can be introduced into an expression vector and can be used to allow the deletion and/or purification of the expression product of one or more insert fragments in the vector. Such tags are well known in the art and include radiolabeled amino acids or polypeptides linked to a biotin moiety detectable by labeled avidin (e.g., streptavidin containing a fluorescent label or enzymatic activity detectable by optical or colorimetric methods). Affinity tags are such as FLAG, glutathione-S-transferase, maltose-binding protein, cellulose-binding domain, thioredoxin, NusA, mistin, chitin-binding domain, cutinase, AGT, GFP and other widely used tags, such as those used in protein expression and purification systems. Further non-limiting examples for use in polypeptides include, but are not limited to, the following: histidine tags, radioisotopes or radionuclides (e.g., 3H, 14C, 35S, 90Y, 99Tc, 1111In, 125I, 177Lu, 166Ho or 153Sm); fluorescent tags (e.g., FITC, rhodamine, lanthanide phosphides), enzyme tags (e.g., horseradish peroxidase, luciferase, alkaline phosphatase); chemiluminescent tags; biotin groups; pendant polypeptide antigenic determinants recognized by secondary reporters (e.g., leucine zipper pair sequences, binding sites for secondary antibodies, metal-binding domains, antigenic determinant tags); and magnetic agents, such as gadolinium chelates.

In the present application, the specific protein (e.g., KRAB, dCas9, Dnmt3A, or Dnmt3L) may include any native form of the protein or a variant or homolog that maintains the activity of the protein (e.g., at least 50%, at least 60%, at least 70%, at least 80%, at least 90%, at least 95%, at least 96%, at least 97%, at least 98%, at least 99% or 100% of the activity compared with the native protein). In various aspects, a variant or homolog has at least 50%, at least 55%, at least 60%, at least 65%, at least 70%, at least 75%, at least 80%, at least 85%, at least 90%, at least 95%, at least 96%, at least 97%, at least 98%, at least 99% or 100% amino acid sequence identity over the entire sequence or a portion of the sequence (e.g., a portion with 50, 100, 150 or 200 contiguous amino acids) compared with the naturally occurring form.

In the present application, the term "nucleic acid" is used interchangeably with "polynucleotide", "nucleotides", "nucleotide sequence" and "oligonucleotide" and typically refers to nucleotides (e.g., deoxyribonucleotides or ribonucleotides) and polymers thereof in single-stranded, double-stranded or multi-stranded form, or complements thereof. For example, the nucleotides can be ribonucleotides, deoxyribonucleotides, or a modified version thereof. For example, the nucleotides can be single-stranded and double-stranded DNA, single-stranded and double-stranded RNA, and a hybrid molecule having a mixture of single-stranded and double-stranded DNA and RNA. For example, the nucleotides may include, but are not limited to, any type of RNA, such as mRNA, siRNA, miRNA, sgRNA, and guide RNA, and any type of DNA, genomic DNA, plasmid DNA, and minicircle DNA, and any fragments thereof. The terms also encompass nucleic acids containing known nucleotide analogs or modified backbone residues or linkages, and the nucleic acids are synthetic, naturally occurring, and non-naturally occurring.

In the present application, the term "sequence encoding" or "nucleic acid encoding" typically refers to a nucleic acid (RNA or DNA molecule) comprising a nucleotide sequence encoding a protein. The coding sequence may also include start and stop signals operably linked to regulatory elements comprising a promoter capable of directing expression in the cells of an individual or mammal to which the nucleic acid is administered and a polyadenylation signal. The coding sequence may be codon optimized.

As used herein, the term "delivery vector" typically refers to a transfer vehicle capable of delivering an agent (e.g., a nucleic acid molecule) to a target cell. The delivery vector can deliver the agent to a specific subtype of cells. For example, the delivery vector can be targeted to certain cell types by virtue of an inherent feature of the delivery vector or by a moiety coupled to the vector, a moiety contained within the vector (or a moiety associated with the vector such that the moiety and the delivery vector are maintained together, thereby making the moiety sufficient to target the delivery vector to certain cell types). The delivery vector may also increase the in vivo half-life of the agent to be delivered and/or the bioavailability of the agent to be delivered. The delivery vector may include a viral vector, a virus-like particle, a polycationic vector, a peptide vector, a liposome, and/or a hybrid vector. For example, if the target cell is a hepatocyte, the properties of the delivery vector (e.g., size, charge, and/or pH) can effectively deliver the delivery vector and/or the molecule enclosed therein to the target cell, reduce immune clearance, and/or promote retention in the target cell.

In the present application, the term "liposome" typically refers to a vesicle having an internal space separated from the external medium by one or more bilayer membranes. In some embodiments, the bilayer membrane can be formed by amphiphilic molecules, such as synthetic or naturally derived lipids containing spatially separated hydrophilic and hydrophobic domains; in other embodiments, the bilayer membrane can be formed by amphiphilic polymers and surfactants. In some embodiments, the liposome is a spherical vesicle structure composed of a unilamellar or multilamellar lipid bilayer surrounding an inner aqueous compartment, and a relatively impermeable outer lipophilic phospholipid bilayer. In some embodiments, the liposome is biocompatible, nontoxic, can deliver both hydrophilic and lipophilic drug molecules, protect their cargo from degradation by plasma enzymes, and transport their loads across biological membranes and the blood-brain barrier (BBB). The liposome can be made from several different types of lipids, such as phospholipids. The liposome may comprise natural phospholipids and lipids such as 1,2-distearoyl-sn-glycero-3-phosphatidylcholine (DSPC), sphingomyelin, egg phosphatidylcholine, monosialoganglioside, or any combination thereof. Several other additives can be added to the liposome in order to modify the structure and properties of the liposome. For example, the liposome may further comprise cholesterol, sphingomyelin and/or 1,2-dioleoyl-sn-glycero-3-phosphoethanolamine (DOPE), for example, to increase stability and/or prevent leakage of cargo inside the liposome.

The term "lipid nanoparticle (LNP)" typically refers to a particle comprising a plurality (i.e., more than one) lipid molecules physically bound to each other (e.g., covalently or non-covalently) by intermolecular forces. LNPs can be, for example, microspheres (including unilamellar and multilamellar vesicles such as liposomes), dispersed phases in emulsions, micelles, or internal phases in suspensions. LNPs can encapsulate nucleic acids within cationic lipid particles (e.g., liposomes) and can be delivered to cells relatively easily. In some examples, the lipid nanoparticles do not contain any viral components, which helps minimize safety and immunogenicity issues. The lipid particles can be used for in vitro, ex vivo, and in vivo delivery. The lipid particles can also be used for cell populations of various sizes. The LNPs of the present application can be easily prepared by various methods known in the art, such as by mixing an organic phase with an aqueous phase. The mixing of the two phases can be achieved by a microfluidic device and an impinging stream reactor. The more thoroughly the organic phase and the aqueous phase are mixed, the better the encapsulation efficiency and particle size distribution of the obtained LNPs will be. Preferably, the particle size of the LNPs can be adjusted by changing the mixing speed of the organic phase and the aqueous phase. The faster the mixing speed, the smaller the particle size of the prepared LNP will be. The encapsulation efficiency can be optimized by adjusting the N/P (ionizable lipid/nucleic acid) ratio of the LNP system. In some examples, LNPs can be used to deliver DNA molecules (e.g., molecules comprising coding sequences for DNA-binding proteins and/or sgRNAs) and/or RNA molecules (e.g., mRNAs for Cas or sgRNAs). In some cases, LNPs can be used to deliver Cas/gRNA RNP complexes. In some embodiments, LNPs are used to deliver mRNAs and gRNAs (e.g., mRNA fusion molecules comprising DNMT3A-DNMT3L(3A-3L)-dCas9-KRAB and at least one sgRNA targeting a target gene).

In the present application, the term "recombinant vector" typically refers to a nucleic acid molecule capable of transporting itself as well as another nucleic acid to which it is linked. One type of vector is a "plasmid", which refers to a circular double-stranded DNA loop into which additional DNA segments can be ligated. Alternatively, the vector can be linear. Another type of vector is a viral vector, in which additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non- attached mammalian vectors) can be integrated into the genome of the host cell upon introduction into the host cell and thereby replicate along with the host genome.

In the present application, the term "regulatory element" refers to a genetic element capable of controlling the expression of a nucleic acid sequence. For example, splicing signals, promoter sequences, polyadenylation signals, transcription termination sequences, upstream regulatory domains, replication origins, internal ribosome entry sites ("IRES"), enhancers, etc., which together provide for the replication, transcription and translation of the coding sequence in the recipient cell. Not all of these control sequences need to be present. Transcriptional control signals in eukaryotes typically contain "promoter" and "enhancer" elements. Promoters and enhancers consist of short arrays of DNA sequences, with promoters being regulatory elements that promote the initiation of transcription of an operably linked coding region and enhancers being regulatory elements that increase the rate of genetic transcription by increasing the activity of the closest promoter located on the same DNA molecule, all these sequences specifically interact with cellular proteins involved in transcription (Maniatis et al., Science 236:1237 (1987), incorporated herein by reference in its entirety). Promoter and enhancer elements have been isolated from a variety of eukaryotic sources, including genes in yeast cells, insect cells, mammalian cells, and viruses (similar control sequences, known as promoters, are also found in prokaryotes). The choice of specific promoters and enhancers depends on the recipient cell type. Some eukaryotic promoters and enhancers have a broad host range, while others are functional in a limited subset of cell types (for reviews, see, e.g., Voss et al., Trends Biochem. Sci., 11:287 (1986); and Maniatis et al., supra, incorporated herein by reference in their entireties). For example, the SV40 early gene enhancer is very active in a variety of cell types from many mammalian species and has been used to express proteins in a variety of mammalian cells (Dijkema et al., EMBO J. 4:761 (1985), incorporated herein by reference in its entirety). Promoter and enhancer elements derived from the human elongation factor 1-alpha gene (Uetsuki et al., J. Biol. Chem., 264:5791 (1989); Kim et al., Gene 91:217 (1990); and Mizushima and Nagata, Nucl. Acids. Res., 18:5322 (1990)), the long terminal repeat of Rous sarcoma virus (Gorman et al., Proc. Natl. Acad. Sci. U.S.A. 79:6777 (1982)), and human cytomegalovirus (Boshart et al., Cell 41:521 (1985)), which references are incorporated herein by reference in their entireties, can also be used to express proteins in various mammalian cell types. Promoters and enhancers can occur naturally separately or together. For example, the retroviral long terminal repeat comprises both promoter and enhancer elements. In general, promoters and enhancers function independently of the gene being transcribed or translated. Thus, the enhancers and promoters used may be "endogenous," "exogenous," or "heterologous" relative to the gene to which they are operably linked. An "endogenous" enhancer/promoter is one that is naturally linked to a given gene in the genome. An "exogenous" or "heterologous" enhancer or promoter is one that is placed in juxtaposition with a gene via genetic manipulation (i.e., molecular biology techniques), with this juxtaposition enabling the transcription of the gene to be directed by the linked enhancer/promoter. The presence of a "splicing signal" on an expression vector typically results in high levels of expression of the recombinant transcript. In certain embodiments, a "splicing signal" mediates the removal of introns from a primary RNA transcript and consists of a splice donor and acceptor site (Sambrook et al., Molecular Cloning: A Laboratory Manual, 2nd ed., Cold Spring Harbor Laboratory Press, New York (1989), pp. 16.7-16.8, incorporated herein by reference in its entirety). Commonly used splice donor and acceptor sites are the splice sites of the 16S RNA from SV40. In certain embodiments, a "transcription termination signal" is typically present downstream of the polyadenylation signal and is several hundred nucleotides in length. For example, the term "poly A signal" or "poly A sequence" refers to a DNA sequence that directs the termination and polyadenylation of a nascent RNA transcript. Efficient polyadenylation of recombinant transcripts is often necessary because transcripts lacking a poly A signal are unstable and rapidly degraded. The poly A signal used in an expression vector can be either "heterologous" or "endogenous." An endogenous poly A signal is a signal that is naturally present at the 3' end of the coding region of a given gene in the genome. A heterologous poly A signal is a signal that is isolated from one gene and operably linked to the 3' end of another gene. A commonly used heterologous poly A signal is the SV40 poly A signal. The SV40 poly A signal is contained on the 237 bp BamHI/BclI restriction fragment and directs termination and polyadenylation (Sambrook et al., supra, 16.6-16.7, incorporated by reference in its entirety).

In the present application, the term "subject" typically refers to animals, usually mammals, such as humans, non-human primates (apes, gibbons, gorillas, chimpanzees, orangutans, and macaques), livestock (dogs and cats), farm animals (poultry such as chickens and ducks, horses, cows, goats, sheep, and pigs), and laboratory animals (mice, rats, rabbits, and guinea pigs). Human subjects include fetal, neonatal, infant, adolescent, and adult subjects. Subjects include animal disease models, such as mice and other animal models of blood coagulation diseases (such as HemA), and other animal models known to those skilled in the art.

In this application, the term "comprise" typically refers to the inclusion of explicitly specified features, but not the exclusion of other elements.

In the present application, the term "selected from" typically refers to the inclusion of selected objects and all combinations thereof. For example, "selected from (:) A, B, and C" means including all combinations of A, B, and C, for example, A, B, C, A + B, A + C, B + C, or A + B + C.

In the present application, the term "about" typically refers to a variation within a range of 0.5%-10% above or below a specified value, for example, 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below a specified value.

### DETAILED DESCRIPTION

In one aspect, the present application provides a fusion comprising a nucleic acid-binding domain and one or more effector domains, wherein the effector domain comprises one or more of an epigenetic modification domain and/or a transcriptional regulation domain and the fusion comprises at least two types of epigenetic modification domains and/or transcriptional regulation domains.

In another aspect, the present application provides a nucleic acid encoding the fusion described in the present application. For example, the nucleic acid comprises DNA and/or mRNA. For example, the nucleic acid can be used to treat or alleviate a disease or condition thereof associated with abnormal expression and/or activity of a target gene. In some embodiments, the nucleic acid is mRNA; one or more modification techniques can be used to generate a more stable mRNA. Known mRNA modification technologies can be roughly divided into three categories: using artificially synthesized non-natural RNA instead of natural RNA to synthesize mRNA; adding 5' caps, 3'poly (A) "tail" and UTR (untranslated region) sequences; using special new formulation technology to effectively protect mRNA. Among them, the preferred mRNA modification technology can synthesize mRNA by artificially synthesizing non-natural ribonucleic acid instead of natural ribonucleic acid. Chemical modifications on eukaryotic mRNA can be roughly divided into three categories: methylation, pseudouridine (Ψ) and hypoxanthine. For example, the chemical modifications can be selected from pseudouridine, N1-methylpseudouridine, N1-ethylpseudouridine, 2-thiouridine, 4'-thiouridine, 5-methylcytosine, 2-thio-1-methyl-1-deaza-pseudouridine, 2-thio-1-methyl-pseudouridine, 2-thio-5-aza-uridine, 2-thio-dihydropseudouridine, 2-thio-dihydrouridine, 2-thio-pseudouridine, 4-methoxy-2-thio-pseudouridine, 4-methoxy-pseudouridine, 4-thio-1-methyl-pseudouridine, 4-thio-pseudouridine, 5-aza-uridine, dihydropseudouridine, 5-methyluridine, 5-methoxyuridine and 2'-O-methyluridine.

In another aspect, the present application provides a recombinant vector comprising the nucleic acid described in the present application. For example, a recombinant vector may refer to a nucleic acid molecule capable of transporting another nucleic acid linked thereto. The recombinant vector can include single-stranded, double-stranded, or partially double-stranded nucleic acid molecules; nucleic acid molecules with one or more free ends, or without free ends (e.g., circular); nucleic acid molecules containing DNA, RNA, or both; and other types of polynucleotides known in the art. For example, viral vectors can be used. Viral vectors may comprise virally derived DNA or RNA sequences for packaging into viruses (e.g., retroviruses, replication-defective retroviruses, adenoviruses, replication-defective adenoviruses, and adeno-associated viruses AAV). Viruses and viral vectors may be used for in vitro, ex vivo, and/or in vivo delivery.

In another aspect, the present application provides a delivery vector comprising the fusion described in the present application, the nucleic acid described in the present application, and/or the recombinant vector described in the present application, and optionally comprising a liposome and/or a lipid nanoparticle. For example, the delivery vector can comprise one or more Cas proteins and one or more guide RNAs, e.g., in the form of a ribonucleoprotein complex (RNP). For example, the ribonucleoprotein can be delivered via a polypeptide-based shuttle agent. For example, the ribonucleoprotein can be delivered using a synthetic peptide. For example, the delivery vector can be introduced into cells by physical delivery methods. Examples of physical methods include microinjection, electroporation, and hydrodynamic delivery. For example, LNPs can encapsulate nucleic acids in cationic lipid particles (e.g., liposomes) and can be delivered to cells relatively easily. In some examples, lipid nanoparticles do not contain any viral components, which helps minimize safety and immunogenicity issues. Lipid particles can be used for in vitro, ex vivo, and in vivo delivery. The components of LNPs may include cationic lipids, ionizable lipids, PEGylated lipids and/or supporting lipids, and optionally cholesterol components. In some embodiments, the LNPs may comprise ionizable lipids (20%-70%, molar ratio), PEGylated lipids (0%-30%, molar ratio), supporting lipids (30%-50%, molar ratio), and cholesterol (10%-50%, molar ratio).

In another aspect, the present application provides a composition comprising the fusion described in the present application, the nucleic acid described in the present application, the recombinant vector described in the present application, and/or the delivery vector described in the present application. For example, the fusion, the nucleic acid encoding the fusion, the recombinant vector, and the delivery vector in the composition can be contained in one composition simultaneously, or separately contained in different compositions. For example, when the fusion in the composition, the nucleic acid encoding the fusion, the recombinant vector and/or the delivery vector are used, they can be used simultaneously or separately.

In another aspect, the present application provides a cell comprising the fusion described in the present application, the nucleic acid described in the present application, the recombinant vector described in the present application, the delivery vector described in the present application, and/or the composition described in the present application.

In another aspect, the present application provides a kit comprising the fusion described in the present application, the nucleic acid described in the present application, the recombinant vector described in the present application, the delivery vector described in the present application, the composition described in the present application, and/or the cell described in the present application.

In another aspect, the present application provides a method for regulating the expression of a target gene, wherein the method comprises administering the fusion described in the present application, the nucleic acid described in the present application, the recombinant vector described in the present application, the delivery vector described in the present application, the composition described in the present application, the cell described in the present application, and/or the kit described in the present application. For example, the method of the present application may be a method for non-therapeutic purposes. For example, the method of the present application may be a method that is not directly used on the human body. For example, the method of the present application may be an in vitro or ex vivo method. For example, the method of the present application may be a method for therapeutic purposes. For example, the method of the present application may be an in vivo method. For example, the method can reduce the expression and/or activity of the target gene. For example, compared with the expression and/or activity of the target gene in the absence of the substance of the present application, the administration of the substance of the present application may have a negative impact (e.g., reduction) on the activity of a nucleic acid sequence, for example, may include at least partially, partially or completely blocking activation (e.g., transcription) of a nucleic acid sequence, or reducing, preventing or delaying activation of a nucleic acid sequence. For example, the inhibitory activity may be about 90%, about 80%, about 70%, about 60%, about 50%, about 40%, about 30%, about 20%, about 10% or less of that of the control.

**In** another aspect, the present application provides a method for treating or alleviating a disease or condition associated with abnormal expression and/or abnormal activity of a target gene, wherein the method comprises administering to a subject in need thereof an effective amount of the fusion described in the present application, the nucleic acid described in the present application, the recombinant vector described in the present application, the delivery vector described in the present application, the composition described in the present application, the cell described in the present application, and/or the kit described in the present application. **In** some embodiments, the treatment is directed to a disease/condition of an organ, illustratively including liver diseases, eye diseases, muscle diseases, heart diseases, blood diseases, brain diseases, kidney diseases, or may include treatment of autoimmune diseases, central nervous system diseases, cancers and other proliferative diseases, neurodegenerative diseases, inflammatory diseases, metabolic diseases, musculoskeletal diseases, etc.

### Fusion

In some embodiments, the one or more effector domains are N-terminal or C-terminal to the nucleic acid-binding domain.

For example, the one or more effector domains may be N-terminal to the nucleic acid-binding domain. In some embodiments, the effector domain may comprise at least one type of the epigenetic modification domain and at least one type of the transcriptional regulation domain; and, the epigenetic editing domain in the effector domain may be N-terminal to the transcriptional regulation domain, or may be C-terminal to the transcriptional regulation domain. In these cases, the epigenetic modification domain may comprise DNMT3A and DNMT3L, the C-terminus of the DNMT3Ais linked to the N-terminus of the DNMT3L, or the C-terminus of the DNMT3L is linked to the N-terminus of the DNMT3A. Also in these cases, the transcriptional regulation domain may comprise a transcriptional repressor domain. For example, the transcriptional regulation domain comprises a zinc finger protein-based transcription factor or a functionally active fragment thereof. Specifically, the zinc finger protein-based transcription factor may include KRAB. For example, in some embodiments of the present application, the zinc finger protein-based transcription factor may be selected from ZIM3 KRAB and KOX1 KRAB.

In some embodiments of the above cases, the transcriptional regulation domain may comprise two or more of the KRAB domains described above, and the two or more of the KRAB domains described above are of the same type or of different types. For example, the transcriptional regulation domain comprises two or more ZIM3 KRABs, two or more KOX1 KRABs, or both ZIM3 KRAB and KOX1 KRAB, and the total number thereof is two or more. In these cases, the two or more KRAB domains (of the same type or different types) can be linked via an XTEN linker sequence.

The following are exemplary fusions of the present application, and the components in the amino acid sequence of the fusion are as follows: bold-DNMT3A, underlined bold-DNMT3L, italic-linker sequence, bold italic-transcriptional regulation domain, underlined bold italic-dCas9, and * is any amino acid.

The amino acid sequence of DNMT3A-DNMT3L-XTEN16-ZIM3-XTEN80-dCas9 (V7 or V7' scheme, SEQ ID NO: 60 or 337) is as follows: 
MNHDQEFDPPKVYPPVPAEKRKPIRVLSLFDGIATGLLVLKDLGIQVDRYIASEV
CEDSITVGMVRHQGKIMYVGDVRSVTQKHIQEWGPFDLVIGGSPCNDLSIVNPARK
GLYEGTGRLFFEFYRLLHDARPKEGDDRPFFWLFENVVAMGVSDKRDISRFLESNP
VMIDAKEVSAAHRARYFWGNLPGMNRPLASTVNDKLELQECLEHGRIAKFSKVRT
ITTRSNSIKQGKDQHFPVFMNEKEDILWCTEMERVFGFPVHYTDVSNMSRLARQRL
LGRSWSVPVIRHLFAPLKEYFACVSSGNSNANSRGPSFSSGLVPLSLRGSHMGPMEIYKT
VSAWKRQPVRVLSLFRNIDKVLKSLGFLESGSGSGGGTLKYVEDVTNVVRRDVEK
WGPFDLVYGSTQPLGSSCDRCPGWYMFQFHRILQYALPRQESQRPFFWIFMDNLL
LTEDDQETTTRFLQTEAVTLQDVRGRDYQNAMRVWSNIPGLKSKHAPLTPKEEEY
LQAQVRSRSKLDAPKVDLLVKNCLLPLREYFKYFSQNSLPLDGSGSETPGTSESATPES
MNNSQGRVTFEDVTVNFTQGEWQRLNPEQRNLYRDVMLENYSNLVSVGQGETTKPDVI
LRLEQGKEPWLEEEEVLGSGRAEKNGDIGGQIWKPKDVKESLGGPSSGAPPPSGGSPAGS
PTSTEEGTSESATPESGPGTSTEPSEGSAPGSPAGSPTSTEEGTSTEPSEGSAPGTSTEPSEPKK
KRKVMDKKYSIGLAIGTNSVGWAVITDEYKVPSKKFKVLGNTDRHSIKKNLIGALLFDSG
ETAEATRLKRTARRRYTRRKNRICYLQEIFSNEMAKVDDSFFHRLEESFLVEEDKKHER
HPIFGNIVDEVAYHEKYPTIYHLRKKLVDSTDKADLRLIYLALAHMIKFRGHFLIEGDLN
PDNSDVDKLFIQLVQTYNQLFEENPINASGVDAKAILSARLSKSRRLENLIAQLPGEKKN
GLFGNLIALSLGLTPNFKSNFDLAEDAKLQLSKDTYDDDLDNLLAQIGDQYADLFLAAK
NLSDAILLSDILRVNTEITKAPLSASMIKRYDEHHQDLTLLKALVRQQLPEKYKEIFFDQS
KNGYAGYIDGGASQEEFYKFIKPILEKMDGTEELLVKLNREDLLRKQRTFDNGSIPHQIH
LGELHAILRRQEDFYPFLKDNREKIEKILTFRIPYYVGPLARGNSRFAWMTRKSEETITP
WNFEEVVDKGASAQSFIERMTNFDKNLPNEKVLPKHSLLYEYFTVYNELTKVKYVTEG
MRKPAFLSGEQKKAIVDLLFKTNRKVTVKQLKEDYFKKIECFDSVEISGVEDRFNASLGT
 
YHDLLKIIKDKDFLDNEENEDILEDIVLTLTLFEDREMIEERLKTYAHLFDDKVMKQLK
RRRYTGWGRLSRKLINGIRDKQSGKTILDFLKSDGFANRNFMQLIHDDSLTFKEDIQKA
QVSGQGDSLHEHIANLAGSPAIKKGILQTVKVVDELVKVMGRHKPENIVIEMARENQTT
QKGQKNSRERMKRIEEGIKELGSQILKEHPVENTQLQNEKLYLYYLQNGRDMYVDQEL
DINRLSDYDVDAIVPQSFLKDDSIDNKVLTRSDKNRGKSDNVPSEEVVKKMKNYWRQLL
NAKLITQRKFDNLTKAERGGLSELDKAGFIKRQLVETRQITKHVAQILDSRMNTKYDEN
DKLIREVKVITLKSKLVSDFRKDFQFYKVREINNYHHAHDAYLNAVVGTALIKKYPKLES
EFVYGDYKVYDVRKMIAKSEQEIGKATAKYFFYSNIMNFFKTEITLANGEIRKRPLIETN
GETGEIVWDKGRDFATVRKVLSMPQVNIVKKTEVQTGGFSKESILPKRNSDKLIARKKD
WDPKKYGGFDSPTVAYSVLVVAKVEKGKSKKLKSVKELLGITIMERSSFEKNPIDFLEAK
GYKEVKKDLIIKLPKYSLFELENGRKRMLASAGELQKGNELALPSKYVNFLYLASHYEKL
KGSPEDNEQKQLFVEQHKHYLDEIIEQISEFSKRVILADANLDKVLSAYNKHRDKPIREQ
AENIIHLFTLTNLGAPAAFKYFDTTIDRKRYTSTKEVLDATLIHQSITGLYETRIDLSQLGG
DAYPYDVPDYASLGSGSPKKKRKVEDPKKKRKVDGLE* (V7 scheme).
 or V7' scheme:

MPAAKRVKLDGSMNHDQEFDPPKVYPPVPAEKRKPIRVLSLFDGIATGLLVLKDLGI
QVDRYIASEVCEDSITVGMVRHQGKIMYVGDVRSVTQKHIQEWGPFDLVIGGSPCNDLS
IVNPARKGLYEGTGRLFFEFYRLLHDARPKEGDDRPFFWLFENVVAMGVSDKRDISRFL
ESNPVMIDAKEVSAAHRARYFWGNLPGMNRPLASTVNDKLELQECLEHGRIAKFSKVR
TITTRSNSIKQGKDQHFPVFMNEKEDILWCTEMERVFGFPVHYTDVSNMSRLARQRLLG
RSWSVPVIRHLFAPLKEYFACVSSGNSNANSRGPSFSSGLVPLSLRGSHMGPMEIYKTVS
AWKRQPVRVLSLFRNIDKVLKSLGFLESGSGSGGGTLKYVEDVTNVVRRDVEKWGPFD
LVYGSTQPLGSSCDRCPGWYMFQFHRILQYALPRQESQRPFFWIFMDNLLLTEDDQETT
TRFLQTEAVTLQDVRGRDYQNAMRVWSNIPGLKSKHAPLTPKEEEYLQAQVRSRSKLD
APKVDLLVKNCLLPLREYFKYFSQNSLPLDGSGSETPGTSESATPESMNNSQGRVTFEDV
TVNFTQGEWQRLNPEQRNLYRDVMLENYSNLVSVGQGETTKPDVILRLEQGKEPWLEE
EEVLGSGRAEKNGDIGGQIWKPKDVKESLGGPSSGAPPPSGGSPAGSPTSTEEGTSESATP

ESGPGTSTEPSEGSAPGSPAGSPTSTEEGTSTEPSEGSAPGTSTEPSEPKKKRKVMDKKYSI
GLAIGTNSVGWAVITDEYKVPSKKFKVLGNTDRHSIKKNLIGALLFDSGETAEATRLKRT
ARRRYTRRKNRICYLQEIFSNEMAKVDDSFFHRLEESFLVEEDKKHERHPIFGNIVDEVA
YHEKYPTIYHLRKKLVDSTDKADLRLIYLALAHMIKFRGHFLIEGDLNPDNSDVDKLFIQ
LVQTYNQLFEENPINASGVDAKAILSARLSKSRRLENLIAQLPGEKKNGLFGNLIALSLGL
TPNFKSNFDLAEDAKLQLSKDTYDDDLDNLLAQIGDQYADLFLAAKNLSDAILLSDILRV
NTEITKAPLSASMIKRYDEHHQDLTLLKALVRQQLPEKYKEIFFDQSKNGYAGYIDGGAS
QEEFYKFIKPILEKMDGTEELLVKLNREDLLRKQRTFDNGSIPHQIHLGELHAILRRQEDF
YPFLKDNREKIEKILTFRIPYYVGPLARGNSRFAWMTRKSEETITPWNFEEVVDKGASAQ
SFIERMTNFDKNLPNEKVLPKHSLLYEYFTVYNELTKVKYVTEGMRKPAFLSGEQKKAI
VDLLFKTNRKVTVKQLKEDYFKKIECFDSVEISGVEDRFNASLGTYHDLLKIIKDKDFLD
NEENEDILEDIVLTLTLFEDREMIEERLKTYAHLFDDKVMKQLKRRRYTGWGRLSRKLIN
GIRDKQSGKTILDFLKSDGFANRNFMQLIHDDSLTFKEDIQKAQVSGQGDSLHEHIANLA
GSPAIKKGILQTVKVVDELVKVMGRHKPENIVIEMARENQTTQKGQKNSRERMKRIEEG
IKELGSQILKEHPVENTQLQNEKLYLYYLQNGRDMYVDQELDINRLSDYDVDAIVPQSF
LKDDSIDNKVLTRSDKNRGKSDNVPSEEVVKKMKNYWRQLLNAKLITQRKFDNLTKAE
RGGLSELDKAGFIKRQLVETRQITKHVAQILDSRMNTKYDENDKLIREVKVITLKSKLVS
DFRKDFQFYKVREINNYHHAHDAYLNAVVGTALIKKYPKLESEFVYGDYKVYDVRKMI
AKSEQEIGKATAKYFFYSNIMNFFKTEITLANGEIRKRPLIETNGETGEIVWDKGRDFATV
RKVLSMPQVNIVKKTEVQTGGFSKESILPKRNSDKLIARKKDWDPKKYGGFDSPTVAYS
VLVVAKVEKGKSKKLKSVKELLGITIMERSSFEKNPIDFLEAKGYKEVKKDLIIKLPKYSL
FELENGRKRMLASAGELQKGNELALPSKYVNFLYLASHYEKLKGSPEDNEQKQLFVEQ
HKHYLDEIIEQISEFSKRVILADANLDKVLSAYNKHRDKPIREQAENIIHLFTLTNLGAPA
AFKYFDTTIDRKRYTSTKEVLDATLIHQSITGLYETRIDLSQLGGDAYPYDVPDYASLGS
GSPKKKRKVEDPKKKRKV

The amino acid sequence of ZIM3-XTEN16-DNMT3A-DNMT3L-XTEN80-dCas9 (V8 or V8' scheme, SEQ ID NO: 61 or 338) is as follows: 
MNNSQGRVTFEDVTVNFTQGEWQRLNPEQRNLYRDVMLENYSNLVSVGQGETTKP
DVILRLEQGKEPWLEEEEVLGSGRAEKNGDIGGQIWKPKDVKESLSGSETPGTSESATPES
MNHDQEFDPPKVYPPVPAEKRKPIRVLSLFDGIATGLLVLKDLGIQVDRYIASEVCE
DSITVGMVRHQGKIMYVGDVRSVTQKHIQEWGPFDLVIGGSPCNDLSIVNPARKGL
YEGTGRLFFEFYRLLHDARPKEGDDRPFFWLFENVVAMGVSDKRDISRFLESNPVM
IDAKEVSAAHRARYFWGNLPGMNRPLASTVNDKLELQECLEHGRIAKFSKVRTITT
RSNSIKQGKDQHFPVFMNEKEDILWCTEMERVFGFPVHYTDVSNMSRLARQRLLG
RSWSVPVIRHLFAPLKEYFACVSSGNSNANSRGPSFSSGLVPLSLRGSHMGPMEIYKTVS
AWKRQPVRVLSLFRNIDKVLKSLGFLESGSGSGGGTLKYVEDVTNVVRRDVEKWG
PFDLVYGSTQPLGSSCDRCPGWYMFQFHRILQYALPRQESQRPFFWIFMDNLLLTE
DDQETTTRFLQTEAVTLQDVRGRDYQNAMRVWSNIPGLKSKHAPLTPKEEEYLQA
QVRSRSKLDAPKVDLLVKNCLLPLREYFKYFSQNSLPLGGPSSGAPPPSGGSPAGSPTS
TEEGTSESATPESGPGTSTEPSEGSAPGSPAGSPTSTEEGTSTEPSEGSAPGTSTEPSEPKKKRK
VMDKKYSIGLAIGTNSVGWAVITDEYKVPSKKFKVLGNTDRHSIKKNLIGALLFDSGETA
EATRLKRTARRRYTRRKNRICYLQEIFSNEMAKVDDSFFHRLEESFLVEEDKKHERHPI
FGNIVDEVAYHEKYPTIYHLRKKLVDSTDKADLRLIYLALAHMIKFRGHFLIEGDLNPDN
SDVDKLFIQLVQTYNQLFEENPINASGVDAKAILSARLSKSRRLENLIAQLPGEKKNGLF
GNLIALSLGLTPNFKSNFDLAEDAKLQLSKDTYDDDLDNLLAQIGDQYADLFLAAKNLS
DAILLSDILRVNTEITKAPLSASMIKRYDEHHQDLTLLKALVRQQLPEKYKEIFFDQSKNG
YAGYIDGGASQEEFYKFIKPILEKMDGTEELLVKLNREDLLRKQRTFDNGSIPHQIHLGE
LHAILRRQEDFYPFLKDNREKIEKILTFRIPYYVGPLARGNSRFAWMTRKSEETITPWNF
EEVVDKGASAQSFIERMTNFDKNLPNEKVLPKHSLLYEYFTVYNELTKVKYVTEGMRKP
AFLSGEQKKAIVDLLFKTNRKVTVKQLKEDYFKKIECFDSVEISGVEDRFNASLGTYHDL
LKIIKDKDFLDNEENEDILEDIVLTLTLFEDREMIEERLKTYAHLFDDKVMKQLKRRRYT
GWGRLSRKLINGIRDKQSGKTILDFLKSDGFANRNFMQLIHDDSLTFKEDIQKAQVSGQ
GDSLHEHIANLAGSPAIKKGILQTVKVVDELVKVMGRHKPENIVIEMARENQTTQKGQK
NSRERMKRIEEGIKELGSQILKEHPVENTQLQNEKLYLYYLQNGRDMYVDQELDINRLS
 
DYDVDAIVPQSFLKDDSIDNKVLTRSDKNRGKSDNVPSEEVVKKMKNYWRQLLNAKLIT
QRKFDNLTKAERGGLSELDKAGFIKRQLVETRQITKHVAQILDSRMNTKYDENDKLIRE
VKVITLKSKLVSDFRKDFQFYKVREINNYHHAHDAYLNAVVGTALIKKYPKLESEFVYGD
YKVYDVRKMIAKSEQEIGKATAKYFFYSNIMNFFKTEITLANGEIRKRPLIETNGETGEIV
WDKGRDFATVRKVLSMPQVNIVKKTEVQTGGFSKESILPKRNSDKLIARKKDWDPKKYG
GFDSPTVAYSVLVVAKVEKGKSKKLKSVKELLGITIMERSSFEKNPIDFLEAKGYKEVKK
DLIIKLPKYSLFELENGRKRMLASAGELQKGNELALPSKYVNFLYLASHYEKLKGSPEDN
EQKQLFVEQHKHYLDEIIEQISEFSKRVILADANLDKVLSAYNKHRDKPIREQAENIIHLF
TLTNLGAPAAFKYFDTTIDRKRYTSTKEVLDATLIHQSITGLYETRIDLSQLGGDAYPYDV
PDYASLGSGSPKKKRKVEDPKKKRKVDGLE* (V8 scheme).
 or V8' scheme:

MPAAKRVKLDGSNNSQGRVTFEDVTVNFTQGEWQRLNPEQRNLYRDVMLENYSNL
VSVGQGETTKPDVILRLEQGKEPWLEEEEVLGSGRAEKNGDIGGQIWKPKDVKESLSGS
ETPGTSESATPESMNHDQEFDPPKVYPPVPAEKRKPIRVLSLFDGIATGLLVLKDLGIQVD
RYIASEVCEDSITVGMVRHQGKIMYVGDVRSVTQKHIQEWGPFDLVIGGSPCNDLSIVNP
ARKGLYEGTGRLFFEFYRLLHDARPKEGDDRPFFWLFENVVAMGVSDKRDISRFLESNP
VMIDAKEVSAAHRARYFWGNLPGMNRPLASTVNDKLELQECLEHGRIAKFSKVRTITTR
SNSIKQGKDQHFPVFMNEKEDILWCTEMERVFGFPVHYTDVSNMSRLARQRLLGRSWS
VPVIRHLFAPLKEYFACVSSGNSNANSRGPSFSSGLVPLSLRGSHMGPMEIYKTVSAWKR
QPVRVLSLFRNIDKVLKSLGFLESGSGSGGGTLKYVEDVTNVVRRDVEKWGPFDLVYGS
TQPLGSSCDRCPGWYMFQFHRILQYALPRQESQRPFFWIFMDNLLLTEDDQETTTRFLQT
EAVTLQDVRGRDYQNAMRVWSNIPGLKSKHAPLTPKEEEYLQAQVRSRSKLDAPKVDL
LVKNCLLPLREYFKYFSQNSLPLGGPSSGAPPPSGGSPAGSPTSTEEGTSESATPESGPGTS
TEPSEGSAPGSPAGSPTSTEEGTSTEPSEGSAPGTSTEPSEPKKKRKVMDKKYSIGLAIGTN
SVGWAVITDEYKVPSKKFKVLGNTDRHSIKKNLIGALLFDSGETAEATRLKRTARRRYT
RRKNRICYLQEIFSNEMAKVDDSFFHRLEESFLVEEDKKHERHPIFGNIVDEVAYHEKYP
TIYHLRKKLVDSTDKADLRLIYLALAHMIKFRGHFLIEGDLNPDNSDVDKLFIQLVQTYN

QLFEENPINASGVDAKAILSARLSKSRRLENLIAQLPGEKKNGLFGNLIALSLGLTPNFKS
NFDLAEDAKLQLSKDTYDDDLDNLLAQIGDQYADLFLAAKNLSDAILLSDILRVNTEITK
APLSASMIKRYDEHHQDLTLLKALVRQQLPEKYKEIFFDQSKNGYAGYIDGGASQEEFY
KFIKPILEKMDGTEELLVKLNREDLLRKQRTFDNGSIPHQIHLGELHAILRRQEDFYPFLK
DNREKIEKILTFRIPYYVGPLARGNSRFAWMTRKSEETITPWNFEEVVDKGASAQSFIER
MTNFDKNLPNEKVLPKHSLLYEYFTVYNELTKVKYVTEGMRKPAFLSGEQKKAIVDLLF
KTNRKVTVKQLKEDYFKKIECFDSVEISGVEDRFNASLGTYHDLLKIIKDKDFLDNEENE
DILEDIVLTLTLFEDREMIEERLKTYAHLFDDKVMKQLKRRRYTGWGRLSRKLINGIRDK
QSGKTILDFLKSDGFANRNFMQLIHDDSLTFKEDIQKAQVSGQGDSLHEHIANLAGSPAI
KKGILQTVKVVDELVKVMGRHKPENIVIEMARENQTTQKGQKNSRERMKRIEEGIKELG
SQILKEHPVENTQLQNEKLYLYYLQNGRDMYVDQELDINRLSDYDVDAIVPQSFLKDDS
IDNKVLTRSDKNRGKSDNVPSEEVVKKMKNYWRQLLNAKLITQRKFDNLTKAERGGLS
ELDKAGFIKRQLVETRQITKHVAQILDSRMNTKYDENDKLIREVKVITLKSKLVSDFRKD
FQFYKVREINNYHHAHDAYLNAVVGTALIKKYPKLESEFVYGDYKVYDVRKMIAKSEQ
EIGKATAKYFFYSNIMNFFKTEITLANGEIRKRPLIETNGETGEIVWDKGRDFATVRKVLS
MPQVNIVKKTEVQTGGFSKESILPKRNSDKLIARKKDWDPKKYGGFDSPTVAYSVLVVA
KVEKGKSKKLKSVKELLGITIMERSSFEKNPIDFLEAKGYKEVKKDLIIKLPKYSLFELEN
GRKRMLASAGELQKGNELALPSKYVNFLYLASHYEKLKGSPEDNEQKQLFVEQHKHYL
DEIIEQISEFSKRVILADANLDKVLSAYNKHRDKPIREQAENIIHLFTLTNLGAPAAFKYFD
TTIDRKRYTSTKEVLDATLIHQSITGLYETRIDLSQLGGDAYPYDVPDYASLGSGSPKKKR
KVEDPKKKRKV

The amino acid sequence of DNMT3A-DNMT3L-XTEN16-KRAB-XTEN80-dCas9 (V19 scheme, SEQ ID NO: 70) is as follows:

MPAAKRVKLDGSNHDQEFDPPKVYPPVPAEKRKPIRVLSLFDGIATGLLVLKDL
GIQVDRYIASEVCEDSITVGMVRHQGKIMYVGDVRSVTQKHIQEWGPFDLVIGGSP
CNDLSIVNPARKGLYEGTGRLFFEFYRLLHDARPKEGDDRPFFWLFENVVAMGVSD
KRDISRFLESNPVMIDAKEVSAAHRARYFWGNLPGMNRPLASTVNDKLELQECLEH

GRIAKFSKVRTITTRSNSIKQGKDQHFPVFMNEKEDILWCTEMERVFGFPVHYTDV
SNMSRLARQRLLGRSWSVPVIRHLFAPLKEYFACVSSGNSNANSRGPSFSSGLVPLSLRG
SHMGPMEIYKTVSAWKRQPVRVLSLFRNIDKVLKSLGFLESGSGSGGGTLKYVEDV
TNVVRRDVEKWGPFDLVYGSTQPLGSSCDRCPGWYMFQFHRILQYALPRQESQRP
FFWIFMDNLLLTEDDQETTTRFLQTEAVTLQDVRGRDYQNAMRVWSNIPGLKSKH
APLTPKEEEYLQAQVRSRSKLDAPKVDLLVKNCLLPLREYFKYFSQNSLPLDGSGSE
TPGTSESATPESRTLVTFKDVFVDFTREEWKLLDTAQQIVYRNVMLENYKNLVSLGYQLT
KPDVILRLEKGEEPGGPSSGAPPPSGGSPAGSPTSTEEGTSESATPESGPGTSTEPSEGSAPGS
PAGSPTSTEEGTSTEPSEGSAPGTSTEPSEPKKKRKVMDKKYSIGLAIGTNSVGWAVITDEY
KVPSKKFKVLGNTDRHSIKKNLIGALLFDSGETAEATRLKRTARRRYTRRKNRICYLQEIF
SNEMAKVDDSFFHRLEESFLVEEDKKHERHPIFGNIVDEVAYHEKYPTIYHLRKKLVDS
TDKADLRLIYLALAHMIKFRGHFLIEGDLNPDNSDVDKLFIQLVQTYNQLFEENPINASG
VDAKAILSARLSKSRRLENLIAQLPGEKKNGLFGNLIALSLGLTPNFKSNFDLAEDAKLQ
LSKDTYDDDLDNLLAQIGDQYADLFLAAKNLSDAILLSDILRVNTEITKAPLSASMIKRYD
EHHQDLTLLKALVRQQLPEKYKEIFFDQSKNGYAGYIDGGASQEEFYKFIKPILEKMDG
TEELLVKLNREDLLRKQRTFDNGSIPHQIHLGELHAILRRQEDFYPFLKDNREKIEKILT
FRIPYYVGPLARGNSRFAWMTRKSEETITPWNFEEVVDKGASAQSFIERMTNFDKNLPN
EKVLPKHSLLYEYFTVYNELTKVKYVTEGMRKPAFLSGEQKKAIVDLLFKTNRKVTVKQ
LKEDYFKKIECFDSVEISGVEDRFNASLGTYHDLLKIIKDKDFLDNEENEDILEDIVLTLT
LFEDREMIEERLKTYAHLFDDKVMKQLKRRRYTGWGRLSRKLINGIRDKQSGKTILDFL
KSDGFANRNFMQLIHDDSLTFKEDIQKAQVSGQGDSLHEHIANLAGSPAIKKGILQTVK
VVDELVKVMGRHKPENIVIEMARENQTTQKGQKNSRERMKRIEEGIKELGSQILKEHPV
ENTQLQNEKLYLYYLQNGRDMYVDQELDINRLSDYDVDAIVPQSFLKDDSIDNKVLTRS
DKNRGKSDNVPSEEVVKKMKNYWRQLLNAKLITQRKFDNLTKAERGGLSELDKAGFIK
RQLVETRQITKHVAQILDSRMNTKYDENDKLIREVKVITLKSKLVSDFRKDFQFYKVREI
NNYHHAHDAYLNAVVGTALIKKYPKLESEFVYGDYKVYDVRKMIAKSEQEIGKATAKYF
FYSNIMNFFKTEITLANGEIRKRPLIETNGETGEIVWDKGRDFATVRKVLSMPQVNIVKK

TEVQTGGFSKESILPKRNSDKLIARKKDWDPKKYGGFDSPTVAYSVLVVAKVEKGKSKKL

KSVKELLGITIMERSSFEKNPIDFLEAKGYKEVKKDLIIKLPKYSLFELENGRKRMLASAG

ELQKGNELALPSKYVNFLYLASHYEKLKGSPEDNEQKQLFVEQHKHYLDEIIEQISEFSK

RVILADANLDKVLSAYNKHRDKPIREQAENIIHLFTLTNLGAPAAFKYFDTTIDRKRYTST

KEVLDATLIHQSITGLYETRIDLSQLGGDAYPYDVPDYASLGSGSPKKKRKVEDPKKKR

KVDGLE*

The amino acid sequence of DNMT3A-(human)DNMT3L-XTEN16-ZIM3-XTEN80-dCas9 (V20 scheme, SEQ ID NO: 71) is as follows:

MNHDQEFDPPKVYPPVPAEKRKPIRVLSLFDGIATGLLVLKDLGIQVDRYIASEV
CEDSITVGMVRHQGKIMYVGDVRSVTQKHIQEWGPFDLVIGGSPCNDLSIVNPARK
GLYEGTGRLFFEFYRLLHDARPKEGDDRPFFWLFENVVAMGVSDKRDISRFLESNP
VMIDAKEVSAAHRARYFWGNLPGMNRPLASTVNDKLELQECLEHGRIAKFSKVRT
ITTRSNSIKQGKDQHFPVFMNEKEDILWCTEMERVFGFPVHYTDVSNMSRLARQRL
LGRSWSVPVIRHLFAPLKEYFACVSSGNSNANSRGPSFSSGLVPLSLRGSHMFETVPVW
RRQPVRVLSLFEDIKKELTSLGFLESGSDPGQLKHVVDVTDTVRKDVEEWGPFDLV
YGATPPLGHTCDRPPSWYLFQFHRLLQYARPKPGSPRPFFWMFVDNLVLNKEDLD
VASRFLEMEPVTIPDVHGGSLQNAVRVWSNIPAIRSSRHWALVSEEELSLLAQNKQS
SKLAAKWPTKLVKNCFLPLREYFKYFSDGSGSETPGTSESATPESMNNSQGRVTFEDV
TVNFTQGEWQRLNPEQRNLYRDVMLENYSNLVSVGQGETTKPDVILRLEQGKEPWLEE
EEVLGSGRAEKNGDIGGQIWKPKDVKESLGGPSSGAPPPSGGSPAGSPTSTEEGTSESATPE
SGPGTSTEPSEGSAPGSPAGSPTSTEEGTSTEPSEGSAPGTSTEPSEPKKKRKVMDKKYSIGL
AIGTNSVGWAVITDEYKVPSKKFKVLGNTDRHSIKKNLIGALLFDSGETAEATRLKRTAR
RRYTRRKNRICYLQEIFSNEMAKVDDSFFHRLEESFLVEEDKKHERHPIFGNIVDEVAY
HEKYPTIYHLRKKLVDSTDKADLRLIYLALAHMIKFRGHFLIEGDLNPDNSDVDKLFIQL
VQTYNQLFEENPINASGVDAKAILSARLSKSRRLENLIAQLPGEKKNGLFGNLIALSLGLT
PNFKSNFDLAEDAKLQLSKDTYDDDLDNLLAQIGDQYADLFLAAKNLSDAILLSDILRVN
TEITKAPLSASMIKRYDEHHQDLTLLKALVRQQLPEKYKEIFFDQSKNGYAGYIDGGASQ

EEFYKFIKPILEKMDGTEELLVKLNREDLLRKQRTFDNGSIPHQIHLGELHAILRRQEDF
YPFLKDNREKIEKILTFRIPYYVGPLARGNSRFAWMTRKSEETITPWNFEEVVDKGASAQ
SFIERMTNFDKNLPNEKVLPKHSLLYEYFTVYNELTKVKYVTEGMRKPAFLSGEQKKAI
VDLLFKTNRKVTVKQLKEDYFKKIECFDSVEISGVEDRFNASLGTYHDLLKIIKDKDFLD
NEENEDILEDIVLTLTLFEDREMIEERLKTYAHLFDDKVMKQLKRRRYTGWGRLSRKLI
NGIRDKQSGKTILDFLKSDGFANRNFMQLIHDDSLTFKEDIQKAQVSGQGDSLHEHIAN
LAGSPAIKKGILQTVKVVDELVKVMGRHKPENIVIEMARENQTTQKGQKNSRERMKRIE
EGIKELGSQILKEHPVENTQLQNEKLYLYYLQNGRDMYVDQELDINRLSDYDVDAIVPQS
FLKDDSIDNKVLTRSDKNRGKSDNVPSEEVVKKMKNYWRQLLNAKLITQRKFDNLTKAE
RGGLSELDKAGFIKRQLVETRQITKHVAQILDSRMNTKYDENDKLIREVKVITLKSKLVS
DFRKDFQFYKVREINNYHHAHDAYLNAVVGTALIKKYPKLESEFVYGDYKVYDVRKMIA
KSEQEIGKATAKYFFYSNIMNFFKTEITLANGEIRKRPLIETNGETGEIVWDKGRDFATV
RKVLSMPQVNIVKKTEVQTGGFSKESILPKRNSDKLIARKKDWDPKKYGGFDSPTVAYSV
LVVAKVEKGKSKKLKSVKELLGITIMERSSFEKNPIDFLEAKGYKEVKKDLIIKLPKYSLF
ELENGRKRMLASAGELQKGNELALPSKYVNFLYLASHYEKLKGSPEDNEQKQLFVEQH
KHYLDEIIEQISEFSKRVILADANLDKVLSAYNKHRDKPIREQAENIIHLFTLTNLGAPAA
FKYFDTTIDRKRYTSTKEVLDATLIHQSITGLYETRIDLSQLGGDAYPYDVPDYASLGSGS
PKKKRKVEDPKKKRKVDGLE*

The amino acid sequence of ZIM3-XTEN16-DNMT3A-(human)DNMT3L-XTEN80-dCas9 (V21 scheme, SEQ ID NO: 72) is as follows:

MNNSQGRVTFEDVTVNFTQGEWQRLNPEQRNLYRDVMLENYSNLVSVGQGETTKP
DVILRLEQGKEPWLEEEEVLGSGRAEKNGDIGGQIWKPKDVKESLSGSETPGTSESATPES
MNHDQEFDPPKVYPPVPAEKRKPIRVLSLFDGIATGLLVLKDLGIQVDRYIASEVCE
DSITVGMVRHQGKIMYVGDVRSVTQKHIQEWGPFDLVIGGSPCNDLSIVNPARKGL
YEGTGRLFFEFYRLLHDARPKEGDDRPFFWLFENVVAMGVSDKRDISRFLESNPVM
IDAKEVSAAHRARYFWGNLPGMNRPLASTVNDKLELQECLEHGRIAKFSKVRTITT
RSNSIKQGKDQHFPVFMNEKEDILWCTEMERVFGFPVHYTDVSNMSRLARQRLLG

RSWSVPVIRHLFAPLKEYFACVSSGNSNANSRGPSFSSGLVPLSLRGSHMFETVPVWRR
QPVRVLSLFEDIKKELTSLGFLESGSDPGQLKHVVDVTDTVRKDVEEWGPFDLVYG
ATPPLGHTCDRPPSWYLFQFHRLLQYARPKPGSPRPFFWMFVDNLVLNKEDLDVA
SRFLEMEPVTIPDVHGGSLQNAVRVWSNIPAIRSSRHWALVSEEELSLLAQNKQSSK
LAAKWPTKLVKNCFLPLREYFKYFSGGPSSGAPPPSGGSPAGSPTSTEEGTSESATPESGP
GTSTEPSEGSAPGSPAGSPTSTEEGTSTEPSEGSAPGTSTEPSEPKKKRKVMDKKYSIGLAIGT
NSVGWAVITDEYKVPSKKFKVLGNTDRHSIKKNLIGALLFDSGETAEATRLKRTARRRYT
RRKNRICYLQEIFSNEMAKVDDSFFHRLEESFLVEEDKKHERHPIFGNIVDEVAYHEKY
PTIYHLRKKLVDSTDKADLRLIYLALAHMIKFRGHFLIEGDLNPDNSDVDKLFIQLVQTY
NQLFEENPINASGVDAKAILSARLSKSRRLENLIAQLPGEKKNGLFGNLIALSLGLTPNFK
SNFDLAEDAKLQLSKDTYDDDLDNLLAQIGDQYADLFLAAKNLSDAILLSDILRVNTEIT
KAPLSASMIKRYDEHHQDLTLLKALVRQQLPEKYKEIFFDQSKNGYAGYIDGGASQEEF
YKFIKPILEKMDGTEELLVKLNREDLLRKQRTFDNGSIPHQIHLGELHAILRRQEDFYPF
LKDNREKIEKILTFRIPYYVGPLARGNSRFAWMTRKSEETITPWNFEEVVDKGASAQSFI
ERMTNFDKNLPNEKVLPKHSLLYEYFTVYNELTKVKYVTEGMRKPAFLSGEQKKAIVDL
LFKTNRKVTVKQLKEDYFKKIECFDSVEISGVEDRFNASLGTYHDLLKIIKDKDFLDNEE
NEDILEDIVLTLTLFEDREMIEERLKTYAHLFDDKVMKQLKRRRYTGWGRLSRKLINGIR
DKQSGKTILDFLKSDGFANRNFMQLIHDDSLTFKEDIQKAQVSGQGDSLHEHIANLAGS
PAIKKGILQTVKVVDELVKVMGRHKPENIVIEMARENQTTQKGQKNSRERMKRIEEGIK
ELGSQILKEHPVENTQLQNEKLYLYYLQNGRDMYVDQELDINRLSDYDVDAIVPQSFLK
DDSIDNKVLTRSDKNRGKSDNVPSEEVVKKMKNYWRQLLNAKLITQRKFDNLTKAERG
GLSELDKAGFIKRQLVETRQITKHVAQILDSRMNTKYDENDKLIREVKVITLKSKLVSDF
RKDFQFYKVREINNYHHAHDAYLNAVVGTALIKKYPKLESEFVYGDYKVYDVRKMIAKS
EQEIGKATAKYFFYSNIMNFFKTEITLANGEIRKRPLIETNGETGEIVWDKGRDFATVRK
VLSMPQVNIVKKTEVQTGGFSKESILPKRNSDKLIARKKDWDPKKYGGFDSPTVAYSVLV
VAKVEKGKSKKLKSVKELLGITIMERSSFEKNPIDFLEAKGYKEVKKDLIIKLPKYSLFEL
ENGRKRMLASAGELQKGNELALPSKYVNFLYLASHYEKLKGSPEDNEQKQLFVEQHKH

YLDEIIEQISEFSKRVILADANLDKVLSAYNKHRDKPIREQAENIIHLFTLTNLGAPAAFK

YFDTTIDRKRYTSTKEVLDATLIHQSITGLYETRIDLSQLGGDAYPYDVPDYASLGSGSPK

KKRKVEDPKKKRKVDGLE*

The amino acid sequence of KRAB-XTEN16-DNMT3A-DNMT3L-XTEN80-dCas9 (V22 scheme, SEQ ID NO: 73) is as follows:

MRTLVTFKDVFVDFTREEWKLLDTAQQIVYRNVMLENYKNLVSLGYQLTKPDVILRL
EKGEEPSGSETPGTSESATPESMNHDQEFDPPKVYPPVPAEKRKPIRVLSLFDGIATGL
LVLKDLGIQVDRYIASEVCEDSITVGMVRHQGKIMYVGDVRSVTQKHIQEWGPFDL
VIGGSPCNDLSIVNPARKGLYEGTGRLFFEFYRLLHDARPKEGDDRPFFWLFENVV
AMGVSDKRDISRFLESNPVMIDAKEVSAAHRARYFWGNLPGMNRPLASTVNDKLE
LQECLEHGRIAKFSKVRTITTRSNSIKQGKDQHFPVFMNEKEDILWCTEMERVFGF
PVHYTDVSNMSRLARQRLLGRSWSVPVIRHLFAPLKEYFACVSSGNSNANSRGPSFSS
GLVPLSLRGSHMGPMEIYKTVSAWKRQPVRVLSLFRNIDKVLKSLGFLESGSGSGGG
TLKYVEDVTNVVRRDVEKWGPFDLVYGSTQPLGSSCDRCPGWYMFQFHRILQYAL
PRQESQRPFFWIFMDNLLLTEDDQETTTRFLQTEAVTLQDVRGRDYQNAMRVWSN
IPGLKSKHAPLTPKEEEYLQAQVRSRSKLDAPKVDLLVKNCLLPLREYFKYFSQNSL
PLGGPSSGAPPPSGGSPAGSPTSTEEGTSESATPESGPGTSTEPSEGSAPGSPAGSPTSTEEGTS
TEPSEGSAPGTSTEPSEPKKKRKVMDKKYSIGLAIGTNSVGWAVITDEYKVPSKKFKVLGN
TDRHSIKKNLIGALLFDSGETAEATRLKRTARRRYTRRKNRICYLQEIFSNEMAKVDDSF
FHRLEESFLVEEDKKHERHPIFGNIVDEVAYHEKYPTIYHLRKKLVDSTDKADLRLIYLA
LAHMIKFRGHFLIEGDLNPDNSDVDKLFIQLVQTYNQLFEENPINASGVDAKAILSARLS
KSRRLENLIAQLPGEKKNGLFGNLIALSLGLTPNFKSNFDLAEDAKLQLSKDTYDDDLD
NLLAQIGDQYADLFLAAKNLSDAILLSDILRVNTEITKAPLSASMIKRYDEHHQDLTLLKA
LVRQQLPEKYKEIFFDQSKNGYAGYIDGGASQEEFYKFIKPILEKMDGTEELLVKLNRED
LLRKQRTFDNGSIPHQIHLGELHAILRRQEDFYPFLKDNREKIEKILTFRIPYYVGPLARG
NSRFAWMTRKSEETITPWNFEEVVDKGASAQSFIERMTNFDKNLPNEKVLPKHSLLYEY
FTVYNELTKVKYVTEGMRKPAFLSGEQKKAIVDLLFKTNRKVTVKQLKEDYFKKIECFD

SVEISGVEDRFNASLGTYHDLLKIIKDKDFLDNEENEDILEDIVLTLTLFEDREMIEERLK
TYAHLFDDKVMKQLKRRRYTGWGRLSRKLINGIRDKQSGKTILDFLKSDGFANRNFMQ
LIHDDSLTFKEDIQKAQVSGQGDSLHEHIANLAGSPAIKKGILQTVKVVDELVKVMGRH
KPENIVIEMARENQTTQKGQKNSRERMKRIEEGIKELGSQILKEHPVENTQLQNEKLYLY
YLQNGRDMYVDQELDINRLSDYDVDAIVPQSFLKDDSIDNKVLTRSDKNRGKSDNVPSE
EVVKKMKNYWRQLLNAKLITQRKFDNLTKAERGGLSELDKAGFIKRQLVETRQITKHVA
QILDSRMNTKYDENDKLIREVKVITLKSKLVSDFRKDFQFYKVREINNYHHAHDAYLNA
VVGTALIKKYPKLESEFVYGDYKVYDVRKMIAKSEQEIGKATAKYFFYSNIMNFFKTEITL
ANGEIRKRPLIETNGETGEIVWDKGRDFATVRKVLSMPQVNIVKKTEVQTGGFSKESILP
KRNSDKLIARKKDWDPKKYGGFDSPTVAYSVLVVAKVEKGKSKKLKSVKELLGITIMERS
SFEKNPIDFLEAKGYKEVKKDLIIKLPKYSLFELENGRKRMLASAGELQKGNELALPSKY
VNFLYLASHYEKLKGSPEDNEQKQLFVEQHKHYLDEIIEQISEFSKRVILADANLDKVLS
AYNKHRDKPIREQAENIIHLFTLTNLGAPAAFKYFDTTIDRKRYTSTKEVLDATLIHQSIT
GLYETRIDLSQLGGDAYPYDVPDYASLGSGSPKKKRKVEDPKKKRKVDGLE*

The amino acid sequence of ZIM3-BFP-DNMT3A-(human)DNMT3L-XTEN80-dCas9 (V38 scheme, SEQ ID NO: 89) is as follows (underlined dashed bold italic-BFP, SEQ ID NO: 55):

MNNSQGRVTFEDVTVNFTQGEWQRLNPEQRNLYRDVMLENYSNLVSVGQGETTKP
DVILRLEQGKEPWLEEEEVLGSGRAEKNGDIGGQIWKPKDVKESLSGSETPGTSESATPES
HVSELIKENMHMKLYMEGTVDNHHFKCTSEGEGKPYEGTQTMRIKVVEGGPLPFAFDI
LATSFLYGSKTFINHTQGIPDFFKQSFPEGFTWERVTTYEDGGVLTATQDTSLQDGCLIY
NVKIRGVNFTSNGPVMQKKTLGWEAFTETLYPADGGLEGRNDMALKLVGGSHLIANIK
TTYRSKKPAKNLKMPGVYYVDYRLERIKEANNETYVEQHEVAVARYCDLPSKLGHKLNS
GSETPGTSESATPESMNHDQEFDPPKVYPPVPAEKRKPIRVLSLFDGIATGLLVLKDLG
IQVDRYIASEVCEDSITVGMVRHQGKIMYVGDVRSVTQKHIQEWGPFDLVIGGSPC
NDLSIVNPARKGLYEGTGRLFFEFYRLLHDARPKEGDDRPFFWLFENVVAMGVSD
KRDISRFLESNPVMIDAKEVSAAHRARYFWGNLPGMNRPLASTVNDKLELQECLEH
GRIAKFSKVRTITTRSNSIKQGKDQHFPVFMNEKEDILWCTEMERVFGFPVHYTDV

SNMSRLARQRLLGRSWSVPVIRHLFAPLKEYFACVSSGNSNANSRGPSFSSGLVPLSLRG
SHMFETVPVWRRQPVRVLSLFEDIKKELTSLGFLESGSDPGQLKHVVDVTDTVRKD
VEEWGPFDLVYGATPPLGHTCDRPPSWYLFQFHRLLQYARPKPGSPRPFFWMFVD
NLVLNKEDLDVASRFLEMEPVTIPDVHGGSLQNAVRVWSNIPAIRSSRHWALVSEE
ELSLLAQNKQSSKLAAKWPTKLVKNCFLPLREYFKYFSGGPSSGAPPPSGGSPAGSPT
STEEGTSESATPESGPGTSTEPSEGSAPGSPAGSPTSTEEGTSTEPSEGSAPGTSTEPSEPKKKR
KVMDKKYSIGLAIGTNSVGWAVITDEYKVPSKKFKVLGNTDRHSIKKNLIGALLFDSGET
AEATRLKRTARRRYTRRKNRICYLQEIFSNEMAKVDDSFFHRLEESFLVEEDKKHERHP
IFGNIVDEVAYHEKYPTIYHLRKKLVDSTDKADLRLIYLALAHMIKFRGHFLIEGDLNPD
NSDVDKLFIQLVQTYNQLFEENPINASGVDAKAILSARLSKSRRLENLIAQLPGEKKNGL
FGNLIALSLGLTPNFKSNFDLAEDAKLQLSKDTYDDDLDNLLAQIGDQYADLFLAAKNL
SDAILLSDILRVNTEITKAPLSASMIKRYDEHHQDLTLLKALVRQQLPEKYKEIFFDQSKN
GYAGYIDGGASQEEFYKFIKPILEKMDGTEELLVKLNREDLLRKQRTFDNGSIPHQIHLG
ELHAILRRQEDFYPFLKDNREKIEKILTFRIPYYVGPLARGNSRFAWMTRKSEETITPWN
FEEVVDKGASAQSFIERMTNFDKNLPNEKVLPKHSLLYEYFTVYNELTKVKYVTEGMRK
PAFLSGEQKKAIVDLLFKTNRKVTVKQLKEDYFKKIECFDSVEISGVEDRFNASLGTYHD
LLKIIKDKDFLDNEENEDILEDIVLTLTLFEDREMIEERLKTYAHLFDDKVMKQLKRRRY
TGWGRLSRKLINGIRDKQSGKTILDFLKSDGFANRNFMQLIHDDSLTFKEDIQKAQVSG
QGDSLHEHIANLAGSPAIKKGILQTVKVVDELVKVMGRHKPENIVIEMARENQTTQKGQ
KNSRERMKRIEEGIKELGSQILKEHPVENTQLQNEKLYLYYLQNGRDMYVDQELDINRL
SDYDVDAIVPQSFLKDDSIDNKVLTRSDKNRGKSDNVPSEEVVKKMKNYWRQLLNAKLI
TQRKFDNLTKAERGGLSELDKAGFIKRQLVETRQITKHVAQILDSRMNTKYDENDKLIR
EVKVITLKSKLVSDFRKDFQFYKVREINNYHHAHDAYLNAVVGTALIKKYPKLESEFVYG
DYKVYDVRKMIAKSEQEIGKATAKYFFYSNIMNFFKTEITLANGEIRKRPLIETNGETGEI
VWDKGRDFATVRKVLSMPQVNIVKKTEVQTGGFSKESILPKRNSDKLIARKKDWDPKKY
GGFDSPTVAYSVLVVAKVEKGKSKKLKSVKELLGITIMERSSFEKNPIDFLEAKGYKEVK
KDLIIKLPKYSLFELENGRKRMLASAGELQKGNELALPSKYVNFLYLASHYEKLKGSPED

NEQKQLFVEQHKHYLDEIIEQISEFSKRVILADANLDKVLSAYNKHRDKPIREQAENIIHL

FTLTNLGAPAAFKYFDTTIDRKRYTSTKEVLDATLIHQSITGLYETRIDLSQLGGDAYPYD

VPDYASLGSGSPKKKRKVEDPKKKRKVDGLE*

The amino acid sequence of DNMT3A-(human)DNMT3L-XTEN80-ZIM3-XTEN80-dCas9 (V39 scheme, SEQ ID NO: 90) is as follows:

MVPAAKRVKLDGSNHDQEFDPPKVYPPVPAEKRKPIRVLSLFDGIATGLLVLKDL
GIQVDRYIASEVCEDSITVGMVRHQGKIMYVGDVRSVTQKHIQEWGPFDLVIGGSP
CNDLSIVNPARKGLYEGTGRLFFEFYRLLHDARPKEGDDRPFFWLFENVVAMGVSD
KRDISRFLESNPVMIDAKEVSAAHRARYFWGNLPGMNRPLASTVNDKLELQECLEH
GRIAKFSKVRTITTRSNSIKQGKDQHFPVFMNEKEDILWCTEMERVFGFPVHYTDV
SNMSRLARQRLLGRSWSVPVIRHLFAPLKEYFACVSSGNSNANSRGPSFSSGLVPLSLRG
SHMFETVPVWRRQPVRVLSLFEDIKKELTSLGFLESGSDPGQLKHVVDVTDTVRKD
VEEWGPFDLVYGATPPLGHTCDRPPSWYLFQFHRLLQYARPKPGSPRPFFWMFVD
NLVLNKEDLDVASRFLEMEPVTIPDVHGGSLQNAVRVWSNIPAIRSSRHWALVSEE
ELSLLAQNKQSSKLAAKWPTKLVKNCFLPLREYFKYFSGGPSSGAPPPSGGSPAGSPT
STEEGTSESATPESGPGTSTEPSEGSAPGSPAGSPTSTEEGTSTEPSEGSAPGTSTEPSEMNNSQ
GRVTFEDVTVNFTQGEWQRLNPEQRNLYRDVMLENYSNLVSVGQGETTKPDVILRLEQ
GKEPWLEEEEVLGSGRAEKNGDIGGQIWKPKDVKESLSGSETPGTSEGGPGTSTEPSEGSA
PGSPAGSPTSTEEGTSTEPSEGSAPGTSTEPSEPKKKRKVGSDKKYSIGLAIGTNSVGWAVIT
DEYKVPSKKFKVLGNTDRHSIKKNLIGALLFDSGETAEATRLKRTARRRYTRRKNRICYL
QEIFSNEMAKVDDSFFHRLEESFLVEEDKKHERHPIFGNIVDEVAYHEKYPTIYHLRKK
LVDSTDKADLRLIYLALAHMIKFRGHFLIEGDLNPDNSDVDKLFIQLVQTYNQLFEENPI
NASGVDAKAILSARLSKSRRLENLIAQLPGEKKNGLFGNLIALSLGLTPNFKSNFDLAED
AKLQLSKDTYDDDLDNLLAQIGDQYADLFLAAKNLSDAILLSDILRVNTEITKAPLSASMI
KRYDEHHQDLTLLKALVRQQLPEKYKEIFFDQSKNGYAGYIDGGASQEEFYKFIKPILEK
MDGTEELLVKLNREDLLRKQRTFDNGSIPHQIHLGELHAILRRQEDFYPFLKDNREKIE
KILTFRIPYYVGPLARGNSRFAWMTRKSEETITPWNFEEVVDKGASAQSFIERMTNFDK

NLPNEKVLPKHSLLYEYFTVYNELTKVKYVTEGMRKPAFLSGEQKKAIVDLLFKTNRKV
TVKQLKEDYFKKIECFDSVEISGVEDRFNASLGTYHDLLKIIKDKDFLDNEENEDILEDIV
LTLTLFEDREMIEERLKTYAHLFDDKVMKQLKRRRYTGWGRLSRKLINGIRDKQSGKTI
LDFLKSDGFANRNFMQLIHDDSLTFKEDIQKAQVSGQGDSLHEHIANLAGSPAIKKGIL
QTVKVVDELVKVMGRHKPENIVIEMARENQTTQKGQKNSRERMKRIEEGIKELGSQILK
EHPVENTQLQNEKLYLYYLQNGRDMYVDQELDINRLSDYDVDAIVPQSFLKDDSIDNKV
LTRSDKNRGKSDNVPSEEVVKKMKNYWRQLLNAKLITQRKFDNLTKAERGGLSELDKA
GFIKRQLVETRQITKHVAQILDSRMNTKYDENDKLIREVKVITLKSKLVSDFRKDFQFYK
VREINNYHHAHDAYLNAVVGTALIKKYPKLESEFVYGDYKVYDVRKMIAKSEQEIGKAT
AKYFFYSNIMNFFKTEITLANGEIRKRPLIETNGETGEIVWDKGRDFATVRKVLSMPQVN
IVKKTEVQTGGFSKESILPKRNSDKLIARKKDWDPKKYGGFDSPTVAYSVLVVAKVEKGK
SKKLKSVKELLGITIMERSSFEKNPIDFLEAKGYKEVKKDLIIKLPKYSLFELENGRKRML
ASAGELQKGNELALPSKYVNFLYLASHYEKLKGSPEDNEQKQLFVEQHKHYLDEIIEQIS
EFSKRVILADANLDKVLSAYNKHRDKPIREQAENIIHLFTLTNLGAPAAFKYFDTTIDRK
RYTSTKEVLDATLIHQSITGLYETRIDLSQLGGDAYPYDVPDYASLGSGSPKKKRKVEDP
KKKRKV*

The amino acid sequence of DNMT3A-(human)DNMT3L-XTEN80-ZNF582-XTEN80-dCas9 (V40 scheme, SEQ ID NO: 91) is as follows:

MPAAKRVKLDGSNHDQEFDPPKVYPPVPAEKRKPIRVLSLFDGIATGLLVLKDL
GIQVDRYIASEVCEDSITVGMVRHQGKIMYVGDVRSVTQKHIQEWGPFDLVIGGSP
CNDLSIVNPARKGLYEGTGRLFFEFYRLLHDARPKEGDDRPFFWLFENVVAMGVSD
KRDISRFLESNPVMIDAKEVSAAHRARYFWGNLPGMNRPLASTVNDKLELQECLEH
GRIAKFSKVRTITTRSNSIKQGKDQHFPVFMNEKEDILWCTEMERVFGFPVHYTDV
SNMSRLARQRLLGRSWSVPVIRHLFAPLKEYFACVSSGNSNANSRGPSFSSGLVPLSLRG
SHMFETVPVWRRQPVRVLSLFEDIKKELTSLGFLESGSDPGQLKHVVDVTDTVRKD
VEEWGPFDLVYGATPPLGHTCDRPPSWYLFQFHRLLQYARPKPGSPRPFFWMFVD
NLVLNKEDLDVASRFLEMEPVTIPDVHGGSLQNAVRVWSNIPAIRSSRHWALVSEE

ELSLLAQNKQSSKLAAKWPTKLVKNCFLPLREYFKYFSGGPSSGAPPPSGGSPAGSPT
STEEGTSESATPESGPGTSTEPSEGSAPGSPAGSPTSTEEGTSTEPSEGSAPGTSTEPSERTLEL
FRDVAIVFSQEEWQWLAPAQRDLYRDVMLETYSNLVSLGLAVSKPDVISFLEQGKEPW
MSGSETPGTSESGPGTSTEPSEGSAPGSPAGSPTSTEEGTSTEPSEGSAPGTSTEPSEPKKKRK
VMDKKYSIGLAIGTNSVGWAVITDEYKVPSKKFKVLGNTDRHSIKKNLIGALLFDSGETA
EATRLKRTARRRYTRRKNRICYLQEIFSNEMAKVDDSFFHRLEESFLVEEDKKHERHPI
FGNIVDEVAYHEKYPTIYHLRKKLVDSTDKADLRLIYLALAHMIKFRGHFLIEGDLNPDN
SDVDKLFIQLVQTYNQLFEENPINASGVDAKAILSARLSKSRRLENLIAQLPGEKKNGLF
GNLIALSLGLTPNFKSNFDLAEDAKLQLSKDTYDDDLDNLLAQIGDQYADLFLAAKNLS
DAILLSDILRVNTEITKAPLSASMIKRYDEHHQDLTLLKALVRQQLPEKYKEIFFDQSKNG
YAGYIDGGASQEEFYKFIKPILEKMDGTEELLVKLNREDLLRKQRTFDNGSIPHQIHLGE
LHAILRRQEDFYPFLKDNREKIEKILTFRIPYYVGPLARGNSRFAWMTRKSEETITPWNF
EEVVDKGASAQSFIERMTNFDKNLPNEKVLPKHSLLYEYFTVYNELTKVKYVTEGMRKP
AFLSGEQKKAIVDLLFKTNRKVTVKQLKEDYFKKIECFDSVEISGVEDRFNASLGTYHDL
LKIIKDKDFLDNEENEDILEDIVLTLTLFEDREMIEERLKTYAHLFDDKVMKQLKRRRYT
GWGRLSRKLINGIRDKQSGKTILDFLKSDGFANRNFMQLIHDDSLTFKEDIQKAQVSGQ
GDSLHEHIANLAGSPAIKKGILQTVKVVDELVKVMGRHKPENIVIEMARENQTTQKGQK
NSRERMKRIEEGIKELGSQILKEHPVENTQLQNEKLYLYYLQNGRDMYVDQELDINRLS
DYDVDAIVPQSFLKDDSIDNKVLTRSDKNRGKSDNVPSEEVVKKMKNYWRQLLNAKLIT
QRKFDNLTKAERGGLSELDKAGFIKRQLVETRQITKHVAQILDSRMNTKYDENDKLIRE
VKVITLKSKLVSDFRKDFQFYKVREINNYHHAHDAYLNAVVGTALIKKYPKLESEFVYGD
YKVYDVRKMIAKSEQEIGKATAKYFFYSNIMNFFKTEITLANGEIRKRPLIETNGETGEIV
WDKGRDFATVRKVLSMPQVNIVKKTEVQTGGFSKESILPKRNSDKLIARKKDWDPKKYG
GFDSPTVAYSVLVVAKVEKGKSKKLKSVKELLGITIMERSSFEKNPIDFLEAKGYKEVKK
DLIIKLPKYSLFELENGRKRMLASAGELQKGNELALPSKYVNFLYLASHYEKLKGSPEDN
EQKQLFVEQHKHYLDEIIEQISEFSKRVILADANLDKVLSAYNKHRDKPIREQAENIIHLF

TLTNLGAPAAFKYFDTTIDRKRYTSTKEVLDATLIHQSITGLYETRIDLSQLGGDAYPYDV

PDYASLGSGSPKKKRKVEDPKKKRKVDGLE*

The amino acid sequence of DNMT3A-(human)DNMT3L-XTEN80-ZNF324-XTEN80-dCas9 (V41 scheme, SEQ ID NO: 92) is as follows:

MPAAKRVKLDGSNHDQEFDPPKVYPPVPAEKRKPIRVLSLFDGIATGLLVLKDL
GIQVDRYIASEVCEDSITVGMVRHQGKIMYVGDVRSVTQKHIQEWGPFDLVIGGSP
CNDLSIVNPARKGLYEGTGRLFFEFYRLLHDARPKEGDDRPFFWLFENVVAMGVSD
KRDISRFLESNPVMIDAKEVSAAHRARYFWGNLPGMNRPLASTVNDKLELQECLEH
GRIAKFSKVRTITTRSNSIKQGKDQHFPVFMNEKEDILWCTEMERVFGFPVHYTDV
SNMSRLARQRLLGRSWSVPVIRHLFAPLKEYFACVSSGNSNANSRGPSFSSGLVPLSLRG
SHMFETVPVWRRQPVRVLSLFEDIKKELTSLGFLESGSDPGQLKHVVDVTDTVRKD
VEEWGPFDLVYGATPPLGHTCDRPPSWYLFQFHRLLQYARPKPGSPRPFFWMFVD
NLVLNKEDLDVASRFLEMEPVTIPDVHGGSLQNAVRVWSNIPAIRSSRHWALVSEE
ELSLLAQNKQSSKLAAKWPTKLVKNCFLPLREYFKYFSGGPSSGAPPPSGGSPAGSPT
STEEGTSESATPESGPGTSTEPSEGSAPGSPAGSPTSTEEGTSTEPSEGSAPGTSTEPSERTLMA
FEDVAVYFSQEEWGLLDTAQRALYRRVMLDNFALVASLGLSTSRPRVVIQLERGEEPWV
SGSETPGTSEGGPGTSTEPSEGSAPGSPAGSPTSTEEGTSTEPSEGSAPGTSTEPSEPKKKRKV
MDKKYSIGLAIGTNSVGWAVITDEYKVPSKKFKVLGNTDRHSIKKNLIGALLFDSGETAE
ATRLKRTARRRYTRRKNRICYLQEIFSNEMAKVDDSFFHRLEESFLVEEDKKHERHPIF
GNIVDEVAYHEKYPTIYHLRKKLVDSTDKADLRLIYLALAHMIKFRGHFLIEGDLNPDNS
DVDKLFIQLVQTYNQLFEENPINASGVDAKAILSARLSKSRRLENLIAQLPGEKKNGLFG
NLIALSLGLTPNFKSNFDLAEDAKLQLSKDTYDDDLDNLLAQIGDQYADLFLAAKNLSD
AILLSDILRVNTEITKAPLSASMIKRYDEHHQDLTLLKALVRQQLPEKYKEIFFDQSKNGY
AGYIDGGASQEEFYKFIKPILEKMDGTEELLVKLNREDLLRKQRTFDNGSIPHQIHLGEL
HAILRRQEDFYPFLKDNREKIEKILTFRIPYYVGPLARGNSRFAWMTRKSEETITPWNFE
EVVDKGASAQSFIERMTNFDKNLPNEKVLPKHSLLYEYFTVYNELTKVKYVTEGMRKPA
FLSGEQKKAIVDLLFKTNRKVTVKQLKEDYFKKIECFDSVEISGVEDRFNASLGTYHDLL

KIIKDKDFLDNEENEDILEDIVLTLTLFEDREMIEERLKTYAHLFDDKVMKQLKRRRYTG
WGRLSRKLINGIRDKQSGKTILDFLKSDGFANRNFMQLIHDDSLTFKEDIQKAQVSGQG
DSLHEHIANLAGSPAIKKGILQTVKVVDELVKVMGRHKPENIVIEMARENQTTQKGQKN
SRERMKRIEEGIKELGSQILKEHPVENTQLQNEKLYLYYLQNGRDMYVDQELDINRLSD
YDVDAIVPQSFLKDDSIDNKVLTRSDKNRGKSDNVPSEEVVKKMKNYWRQLLNAKLITQ
RKFDNLTKAERGGLSELDKAGFIKRQLVETRQITKHVAQILDSRMNTKYDENDKLIREV
KVITLKSKLVSDFRKDFQFYKVREINNYHHAHDAYLNAVVGTALIKKYPKLESEFVYGDY
KVYDVRKMIAKSEQEIGKATAKYFFYSNIMNFFKTEITLANGEIRKRPLIETNGETGEIV
WDKGRDFATVRKVLSMPQVNIVKKTEVQTGGFSKESILPKRNSDKLIARKKDWDPKKYG
GFDSPTVAYSVLVVAKVEKGKSKKLKSVKELLGITIMERSSFEKNPIDFLEAKGYKEVKK
DLIIKLPKYSLFELENGRKRMLASAGELQKGNELALPSKYVNFLYLASHYEKLKGSPEDN
EQKQLFVEQHKHYLDEIIEQISEFSKRVILADANLDKVLSAYNKHRDKPIREQAENIIHLF
TLTNLGAPAAFKYFDTTIDRKRYTSTKEVLDATLIHQSITGLYETRIDLSQLGGDAYPYDV
PDYASLGSGSPKKKRKVEDPKKKRKVDGLE*

The amino acid sequence of DNMT3A-(human)DNMT3L-BFP-XTEN80-ZIM3-XTEN80-dCas9 (V42 scheme, SEQ ID NO: 93) is as follows (underlined dashed bold italic-BFP, SEQ ID NO: 55):

MPAAKRVKLDGSNHDQEFDPPKVYPPVPAEKRKPIRVLSLFDGIATGLLVLKDL
GIQVDRYIASEVCEDSITVGMVRHQGKIMYVGDVRSVTQKHIQEWGPFDLVIGGSP
CNDLSIVNPARKGLYEGTGRLFFEFYRLLHDARPKEGDDRPFFWLFENVVAMGVSD
KRDISRFLESNPVMIDAKEVSAAHRARYFWGNLPGMNRPLASTVNDKLELQECLEH
GRIAKFSKVRTITTRSNSIKQGKDQHFPVFMNEKEDILWCTEMERVFGFPVHYTDV
SNMSRLARQRLLGRSWSVPVIRHLFAPLKEYFACVSSGNSNANSRGPSFSSGLVPLSLRG
SHMFETVPVWRRQPVRVLSLFEDIKKELTSLGFLESGSDPGQLKHVVDVTDTVRKD
VEEWGPFDLVYGATPPLGHTCDRPPSWYLFQFHRLLQYARPKPGSPRPFFWMFVD
NLVLNKEDLDVASRFLEMEPVTIPDVHGGSLQNAVRVWSNIPAIRSSRHWALVSEE
ELSLLAQNKQSSKLAAKWPTKLVKNCFLPLREYFKYFSGGPSSGSELIKENMHMKLY

MEGTVDNHHFKCTSEGEGKPYEGTQTMRIKVVEGGPLPFAFDILATSFLYGSKTFINHT
QGIPDFFKQSFPEGFTWERVTTYEDGGVLTATQDTSLQDGCLIYNVKIRGVNFTSNGPV
MQKKTLGWEAFTETLYPADGGLEGRNDMALKLVGGSHLIANIKTTYRSKKPAKNLKMP
GVYYVDYRLERIKEANNETYVEQHEVAVARYCDLPSKLGHKLNAPPPSGGSPAGSPTSTE
EGTSESATPESGPGTSTEPSEGSAPGSPAGSPTSTEEGTSTEPSEGSAPGTSTEPSEMNNSQGR
VTFEDVTVNFTQGEWQRLNPEQRNLYRDVMLENYSNLVSVGQGETTKPDVILRLEQGK
EPWLEEEEVLGSGRAEKNGDIGGQIWKPKDVKESLSGSETPGTSEGGPGTSTEPSEGSAPG
SPAGSPTSTEEGTSTEPSEGSAPGTSTEPSEPKKKRKVMDKKYSIGLAIGTNSVGWAVITDE
YKVPSKKFKVLGNTDRHSIKKNLIGALLFDSGETAEATRLKRTARRRYTRRKNRICYLQEI
FSNEMAKVDDSFFHRLEESFLVEEDKKHERHPIFGNIVDEVAYHEKYPTIYHLRKKLVD
STDKADLRLIYLALAHMIKFRGHFLIEGDLNPDNSDVDKLFIQLVQTYNQLFEENPINAS
GVDAKAILSARLSKSRRLENLIAQLPGEKKNGLFGNLIALSLGLTPNFKSNFDLAEDAKL
QLSKDTYDDDLDNLLAQIGDQYADLFLAAKNLSDAILLSDILRVNTEITKAPLSASMIKRY
DEHHQDLTLLKALVRQQLPEKYKEIFFDQSKNGYAGYIDGGASQEEFYKFIKPILEKMD
GTEELLVKLNREDLLRKQRTFDNGSIPHQIHLGELHAILRRQEDFYPFLKDNREKIEKIL
TFRIPYYVGPLARGNSRFAWMTRKSEETITPWNFEEVVDKGASAQSFIERMTNFDKNLP
NEKVLPKHSLLYEYFTVYNELTKVKYVTEGMRKPAFLSGEQKKAIVDLLFKTNRKVTVK
QLKEDYFKKIECFDSVEISGVEDRFNASLGTYHDLLKIIKDKDFLDNEENEDILEDIVLTL
TLFEDREMIEERLKTYAHLFDDKVMKQLKRRRYTGWGRLSRKLINGIRDKQSGKTILDF
LKSDGFANRNFMQLIHDDSLTFKEDIQKAQVSGQGDSLHEHIANLAGSPAIKKGILQTV
KVVDELVKVMGRHKPENIVIEMARENQTTQKGQKNSRERMKRIEEGIKELGSQILKEHP
VENTQLQNEKLYLYYLQNGRDMYVDQELDINRLSDYDVDAIVPQSFLKDDSIDNKVLTR
SDKNRGKSDNVPSEEVVKKMKNYWRQLLNAKLITQRKFDNLTKAERGGLSELDKAGFIK
RQLVETRQITKHVAQILDSRMNTKYDENDKLIREVKVITLKSKLVSDFRKDFQFYKVREI
NNYHHAHDAYLNAVVGTALIKKYPKLESEFVYGDYKVYDVRKMIAKSEQEIGKATAKYF
FYSNIMNFFKTEITLANGEIRKRPLIETNGETGEIVWDKGRDFATVRKVLSMPQVNIVKK
TEVQTGGFSKESILPKRNSDKLIARKKDWDPKKYGGFDSPTVAYSVLVVAKVEKGKSKKL

KSVKELLGITIMERSSFEKNPIDFLEAKGYKEVKKDLIIKLPKYSLFELENGRKRMLASAG
ELQKGNELALPSKYVNFLYLASHYEKLKGSPEDNEQKQLFVEQHKHYLDEIIEQISEFSK
RVILADANLDKVLSAYNKHRDKPIREQAENIIHLFTLTNLGAPAAFKYFDTTIDRKRYTST
KEVLDATLIHQSITGLYETRIDLSQLGGDAYPYDVPDYASLGSGSPKKKRKVEDPKKKR
KVDGLE*

The amino acid sequence of DNMT3A-(human)DNMT3L-Beta2-XTEN80-ZIM3-XTEN80-dCas9 (V43 scheme, SEQ ID NO: 94) is as follows:

MPAAKRVKLDGSNHDQEFDPPKVYPPVPAEKRKPIRVLSLFDGIATGLLVLKDL
GIQVDRYIASEVCEDSITVGMVRHQGKIMYVGDVRSVTQKHIQEWGPFDLVIGGSP
CNDLSIVNPARKGLYEGTGRLFFEFYRLLHDARPKEGDDRPFFWLFENVVAMGVSD
KRDISRFLESNPVMIDAKEVSAAHRARYFWGNLPGMNRPLASTVNDKLELQECLEH
GRIAKFSKVRTITTRSNSIKQGKDQHFPVFMNEKEDILWCTEMERVFGFPVHYTDV
SNMSRLARQRLLGRSWSVPVIRHLFAPLKEYFACVSSGNSNANSRGPSFSSGLVPLSLRG
SHMFETVPVWRRQPVRVLSLFEDIKKELTSLGFLESGSDPGQLKHVVDVTDTVRKD
VEEWGPFDLVYGATPPLGHTCDRPPSWYLFQFHRLLQYARPKPGSPRPFFWMFVD
NLVLNKEDLDVASRFLEMEPVTIPDVHGGSLQNAVRVWSNIPAIRSSRHWALVSEE
ELSLLAQNKQSSKLAAKWPTKLVKNCFLPLREYFKYFSGGPSSGSELIKENGLEGRND
MALKLVGGSHLIANIKTTYRSKKPAKNLKMPGVYYVDYRLERIKEANNETYVEQHEVAVARYC
DLPSKLGHKLNAPPPSGGSPAGSPTSTEEGTSESATPESGPGTSTEPSEGSAPGSPAGSPTSTEE
GTSTEPSEGSAPGTSTEPSEMNNSQGRVTFEDVTVNFTQGEWQRLNPEQRNLYRDVMLE
NYSNLVSVGQGETTKPDVILRLEQGKEPWLEEEEVLGSGRAEKNGDIGGQIWKPKDVKE
SLSGSETPGTSEGGPGTSTEPSEGSAPGSPAGSPTSTEEGTSTEPSEGSAPGTSTEPSEPKKKR
KVMDKKYSIGLAIGTNSVGWAVITDEYKVPSKKFKVLGNTDRHSIKKNLIGALLFDSGET
AEATRLKRTARRRYTRRKNRICYLQEIFSNEMAKVDDSFFHRLEESFLVEEDKKHERHP
IFGNIVDEVAYHEKYPTIYHLRKKLVDSTDKADLRLIYLALAHMIKFRGHFLIEGDLNPD
NSDVDKLFIQLVQTYNQLFEENPINASGVDAKAILSARLSKSRRLENLIAQLPGEKKNGL
FGNLIALSLGLTPNFKSNFDLAEDAKLQLSKDTYDDDLDNLLAQIGDQYADLFLAAKNL

SDAILLSDILRVNTEITKAPLSASMIKRYDEHHQDLTLLKALVRQQLPEKYKEIFFDQSKN
GYAGYIDGGASQEEFYKFIKPILEKMDGTEELLVKLNREDLLRKQRTFDNGSIPHQIHLG
ELHAILRRQEDFYPFLKDNREKIEKILTFRIPYYVGPLARGNSRFAWMTRKSEETITPWN
FEEVVDKGASAQSFIERMTNFDKNLPNEKVLPKHSLLYEYFTVYNELTKVKYVTEGMRK
PAFLSGEQKKAIVDLLFKTNRKVTVKQLKEDYFKKIECFDSVEISGVEDRFNASLGTYHD
LLKIIKDKDFLDNEENEDILEDIVLTLTLFEDREMIEERLKTYAHLFDDKVMKQLKRRRY
TGWGRLSRKLINGIRDKQSGKTILDFLKSDGFANRNFMQLIHDDSLTFKEDIQKAQVSG
QGDSLHEHIANLAGSPAIKKGILQTVKVVDELVKVMGRHKPENIVIEMARENQTTQKGQ
KNSRERMKRIEEGIKELGSQILKEHPVENTQLQNEKLYLYYLQNGRDMYVDQELDINRL
SDYDVDAIVPQSFLKDDSIDNKVLTRSDKNRGKSDNVPSEEVVKKMKNYWRQLLNAKLI
TQRKFDNLTKAERGGLSELDKAGFIKRQLVETRQITKHVAQILDSRMNTKYDENDKLIR
EVKVITLKSKLVSDFRKDFQFYKVREINNYHHAHDAYLNAVVGTALIKKYPKLESEFVYG
DYKVYDVRKMIAKSEQEIGKATAKYFFYSNIMNFFKTEITLANGEIRKRPLIETNGETGEI
VWDKGRDFATVRKVLSMPQVNIVKKTEVQTGGFSKESILPKRNSDKLIARKKDWDPKKY
GGFDSPTVAYSVLVVAKVEKGKSKKLKSVKELLGITIMERSSFEKNPIDFLEAKGYKEVK
KDLIIKLPKYSLFELENGRKRMLASAGELQKGNELALPSKYVNFLYLASHYEKLKGSPED
NEQKQLFVEQHKHYLDEIIEQISEFSKRVILADANLDKVLSAYNKHRDKPIREQAENIIHL
FTLTNLGAPAAFKYFDTTIDRKRYTSTKEVLDATLIHQSITGLYETRIDLSQLGGDAYPYD
VPDYASLGSGSPKKKRKVEDPKKKRKVDGLE*

The amino acid sequence of DNMT3A-(human)DNMT3L-beta1-XTEN80-ZIM3-XTEN80-dCas9 (V44 scheme, SEQ ID NO: 95) is as follows:

MPAAKRVKLDGSNHDQEFDPPKVYPPVPAEKRKPIRVLSLFDGIATGLLVLKDL
GIQVDRYIASEVCEDSITVGMVRHQGKIMYVGDVRSVTQKHIQEWGPFDLVIGGSP
CNDLSIVNPARKGLYEGTGRLFFEFYRLLHDARPKEGDDRPFFWLFENVVAMGVSD
KRDISRFLESNPVMIDAKEVSAAHRARYFWGNLPGMNRPLASTVNDKLELQECLEH
GRIAKFSKVRTITTRSNSIKQGKDQHFPVFMNEKEDILWCTEMERVFGFPVHYTDV
SNMSRLARQRLLGRSWSVPVIRHLFAPLKEYFACVSSGNSNANSRGPSFSSGLVPLSLRG

SHMFETVPVWRRQPVRVLSLFEDIKKELTSLGFLESGSDPGQLKHVVDVTDTVRKD
VEEWGPFDLVYGATPPLGHTCDRPPSWYLFQFHRLLQYARPKPGSPRPFFWMFVD
NLVLNKEDLDVASRFLEMEPVTIPDVHGGSLQNAVRVWSNIPAIRSSRHWALVSEE
ELSLLAQNKQSSKLAAKWPTKLVKNCFLPLREYFKYFSGGPSSGSELIKENYYVDYRLE
RIKEANNETYVEQHEVAVARYCDLPSKLGHKLNAPPPSGGSPAGSPTSTEEGTSESATPESGPG
TSTEPSEGSAPGSPAGSPTSTEEGTSTEPSEGSAPGTSTEPSEMNNSQGRVTFEDVTVNFTQG
EWQRLNPEQRNLYRDVMLENYSNLVSVGQGETTKPDVILRLEQGKEPWLEEEEVLGSG
RAEKNGDIGGQIWKPKDVKESLSGSETPGTSEGGPGTSTEPSEGSAPGSPAGSPTSTEEGTST
EPSEGSAPGTSTEPSEPKKKRKVMDKKYSIGLAIGTNSVGWAVITDEYKVPSKKFKVLGNT
DRHSIKKNLIGALLFDSGETAEATRLKRTARRRYTRRKNRICYLQEIFSNEMAKVDDSFF
HRLEESFLVEEDKKHERHPIFGNIVDEVAYHEKYPTIYHLRKKLVDSTDKADLRLIYLAL
AHMIKFRGHFLIEGDLNPDNSDVDKLFIQLVQTYNQLFEENPINASGVDAKAILSARLSK
SRRLENLIAQLPGEKKNGLFGNLIALSLGLTPNFKSNFDLAEDAKLQLSKDTYDDDLDNL
LAQIGDQYADLFLAAKNLSDAILLSDILRVNTEITKAPLSASMIKRYDEHHQDLTLLKALV
RQQLPEKYKEIFFDQSKNGYAGYIDGGASQEEFYKFIKPILEKMDGTEELLVKLNREDLL
RKQRTFDNGSIPHQIHLGELHAILRRQEDFYPFLKDNREKIEKILTFRIPYYVGPLARGNS
RFAWMTRKSEETITPWNFEEVVDKGASAQSFIERMTNFDKNLPNEKVLPKHSLLYEYFT
VYNELTKVKYVTEGMRKPAFLSGEQKKAIVDLLFKTNRKVTVKQLKEDYFKKIECFDSV
EISGVEDRFNASLGTYHDLLKIIKDKDFLDNEENEDILEDIVLTLTLFEDREMIEERLKTY
AHLFDDKVMKQLKRRRYTGWGRLSRKLINGIRDKQSGKTILDFLKSDGFANRNFMQLI
HDDSLTFKEDIQKAQVSGQGDSLHEHIANLAGSPAIKKGILQTVKVVDELVKVMGRHKP
ENIVIEMARENQTTQKGQKNSRERMKRIEEGIKELGSQILKEHPVENTQLQNEKLYLYYL
QNGRDMYVDQELDINRLSDYDVDAIVPQSFLKDDSIDNKVLTRSDKNRGKSDNVPSEEV
VKKMKNYWRQLLNAKLITQRKFDNLTKAERGGLSELDKAGFIKRQLVETRQITKHVAQI
LDSRMNTKYDENDKLIREVKVITLKSKLVSDFRKDFQFYKVREINNYHHAHDAYLNAVV
GTALIKKYPKLESEFVYGDYKVYDVRKMIAKSEQEIGKATAKYFFYSNIMNFFKTEITLA
NGEIRKRPLIETNGETGEIVWDKGRDFATVRKVLSMPQVNIVKKTEVQTGGFSKESILPK

RNSDKLIARKKDWDPKKYGGFDSPTVAYSVLVVAKVEKGKSKKLKSVKELLGITIMERSS
FEKNPIDFLEAKGYKEVKKDLIIKLPKYSLFELENGRKRMLASAGELQKGNELALPSKYV
NFLYLASHYEKLKGSPEDNEQKQLFVEQHKHYLDEIIEQISEFSKRVILADANLDKVLSA
YNKHRDKPIREQAENIIHLFTLTNLGAPAAFKYFDTTIDRKRYTSTKEVLDATLIHQSITG
LYETRIDLSQLGGDAYPYDVPDYASLGSGSPKKKRKVEDPKKKRKVDGLE*

The amino acid sequence of DNMT3A-(human)DNMT3L-XTEN80-ZIM3-GS-XTEN80-dCas9 (V45 scheme, SEQ ID NO: 96) is as follows:

MPAAKRVKLDGSNHDQEFDPPKVYPPVPAEKRKPIRVLSLFDGIATGLLVLKDL
GIQVDRYIASEVCEDSITVGMVRHQGKIMYVGDVRSVTQKHIQEWGPFDLVIGGSP
CNDLSIVNPARKGLYEGTGRLFFEFYRLLHDARPKEGDDRPFFWLFENVVAMGVSD
KRDISRFLESNPVMIDAKEVSAAHRARYFWGNLPGMNRPLASTVNDKLELQECLEH
GRIAKFSKVRTITTRSNSIKQGKDQHFPVFMNEKEDILWCTEMERVFGFPVHYTDV
SNMSRLARQRLLGRSWSVPVIRHLFAPLKEYFACVSSGNSNANSRGPSFSSGLVPLSLRG
SHMFETVPVWRRQPVRVLSLFEDIKKELTSLGFLESGSDPGQLKHVVDVTDTVRKD
VEEWGPFDLVYGATPPLGHTCDRPPSWYLFQFHRLLQYARPKPGSPRPFFWMFVD
NLVLNKEDLDVASRFLEMEPVTIPDVHGGSLQNAVRVWSNIPAIRSSRHWALVSEE
ELSLLAQNKQSSKLAAKWPTKLVKNCFLPLREYFKYFSGGPSSGAPPPSGGSPAGSPT
STEEGTSESATPESGPGTSTEPSEGSAPGSPAGSPTSTEEGTSTEPSEGSAPGTSTEPSEMNNSQ
GRVTFEDVTVNFTQGEWQRLNPEQRNLYRDVMLENYSNLVSVGQGETTKPDVILRLEQ
GKEPWLEEEEVLGSGRAEKNGDIGGQIWKPKDVKESLSGSGSGSGGSGSGGSGSGSGGSG
SGGSGSGETPGTSEGGPGTSTEPSEGSAPGSPAGSPTSTEEGTSTEPSEGSAPGTSTEPSEPKK
KRKVMDKKYSIGLAIGTNSVGWAVITDEYKVPSKKFKVLGNTDRHSIKKNLIGALLFDSG
ETAEATRLKRTARRRYTRRKNRICYLQEIFSNEMAKVDDSFFHRLEESFLVEEDKKHER
HPIFGNIVDEVAYHEKYPTIYHLRKKLVDSTDKADLRLIYLALAHMIKFRGHFLIEGDLN
PDNSDVDKLFIQLVQTYNQLFEENPINASGVDAKAILSARLSKSRRLENLIAQLPGEKKN
GLFGNLIALSLGLTPNFKSNFDLAEDAKLQLSKDTYDDDLDNLLAQIGDQYADLFLAAK
NLSDAILLSDILRVNTEITKAPLSASMIKRYDEHHQDLTLLKALVRQQLPEKYKEIFFDQS

KNGYAGYIDGGASQEEFYKFIKPILEKMDGTEELLVKLNREDLLRKQRTFDNGSIPHQIH
LGELHAILRRQEDFYPFLKDNREKIEKILTFRIPYYVGPLARGNSRFAWMTRKSEETITP
WNFEEVVDKGASAQSFIERMTNFDKNLPNEKVLPKHSLLYEYFTVYNELTKVKYVTEG
MRKPAFLSGEQKKAIVDLLFKTNRKVTVKQLKEDYFKKIECFDSVEISGVEDRFNASLGT
YHDLLKIIKDKDFLDNEENEDILEDIVLTLTLFEDREMIEERLKTYAHLFDDKVMKQLK
RRRYTGWGRLSRKLINGIRDKQSGKTILDFLKSDGFANRNFMQLIHDDSLTFKEDIQKA
QVSGQGDSLHEHIANLAGSPAIKKGILQTVKVVDELVKVMGRHKPENIVIEMARENQTT
QKGQKNSRERMKRIEEGIKELGSQILKEHPVENTQLQNEKLYLYYLQNGRDMYVDQEL
DINRLSDYDVDAIVPQSFLKDDSIDNKVLTRSDKNRGKSDNVPSEEVVKKMKNYWRQLL
NAKLITQRKFDNLTKAERGGLSELDKAGFIKRQLVETRQITKHVAQILDSRMNTKYDEN
DKLIREVKVITLKSKLVSDFRKDFQFYKVREINNYHHAHDAYLNAVVGTALIKKYPKLES
EFVYGDYKVYDVRKMIAKSEQEIGKATAKYFFYSNIMNFFKTEITLANGEIRKRPLIETN
GETGEIVWDKGRDFATVRKVLSMPQVNIVKKTEVQTGGFSKESILPKRNSDKLIARKKD
WDPKKYGGFDSPTVAYSVLVVAKVEKGKSKKLKSVKELLGITIMERSSFEKNPIDFLEAK
GYKEVKKDLIIKLPKYSLFELENGRKRMLASAGELQKGNELALPSKYVNFLYLASHYEKL
KGSPEDNEQKQLFVEQHKHYLDEIIEQISEFSKRVILADANLDKVLSAYNKHRDKPIREQ
AENIIHLFTLTNLGAPAAFKYFDTTIDRKRYTSTKEVLDATLIHQSITGLYETRIDLSQLGG
DAYPYDVPDYASLGSGSPKKKRKVEDPKKKRKVDGLE*

The amino acid sequence of DNMT3A-(human)DNMT3L-XTEN80-xten-ZIM3-GS-xten80-dCas9 (V46 scheme, SEQ ID NO: 97) is as follows:

MVPAAKRVKLDGSNHDQEFDPPKVYPPVPAEKRKPIRVLSLFDGIATGLLVLKDL
GIQVDRYIASEVCEDSITVGMVRHQGKIMYVGDVRSVTQKHIQEWGPFDLVIGGSP
CNDLSIVNPARKGLYEGTGRLFFEFYRLLHDARPKEGDDRPFFWLFENVVAMGVSD
KRDISRFLESNPVMIDAKEVSAAHRARYFWGNLPGMNRPLASTVNDKLELQECLEH
GRIAKFSKVRTITTRSNSIKQGKDQHFPVFMNEKEDILWCTEMERVFGFPVHYTDV
SNMSRLARQRLLGRSWSVPVIRHLFAPLKEYFACVSSGNSNANSRGPSFSSGLVPLSLRG
SHMFETVPVWRRQPVRVLSLFEDIKKELTSLGFLESGSDPGQLKHVVDVTDTVRKD

VEEWGPFDLVYGATPPLGHTCDRPPSWYLFQFHRLLQYARPKPGSPRPFFWMFVD
NLVLNKEDLDVASRFLEMEPVTIPDVHGGSLQNAVRVWSNIPAIRSSRHWALVSEE
ELSLLAQNKQSSKLAAKWPTKLVKNCFLPLREYFKYFSGGPSSGAPPPSGGSPAGSPT
STEEGTSESATPESGPGTSTEPSEGSAPGSPAGSPTSTEEGTSTEPSEGSAPGTSTEPSEPKKKR
KVGSGSETPGTSESATPESMNNSQGRVTFEDVTVNFTQGEWQRLNPEQRNLYRDVMLEN
YSNLVSVGQGETTKPDVILRLEQGKEPWLEEEEVLGSGRAEKNGDIGGQIWKPKDVKES
LSGSGSGSGGSGSGGSGSGSGGSGSGGSGSGETPGTSEGGPGTSTEPSEGSAPGSPAGSPTST
EEGTSTEPSEGSAPGTSTEPSEPKKKRKVGSDKKYSIGLAIGTNSVGWAVITDEYKVPSKKF
KVLGNTDRHSIKKNLIGALLFDSGETAEATRLKRTARRRYTRRKNRICYLQEIFSNEMAK
VDDSFFHRLEESFLVEEDKKHERHPIFGNIVDEVAYHEKYPTIYHLRKKLVDSTDKADL
RLIYLALAHMIKFRGHFLIEGDLNPDNSDVDKLFIQLVQTYNQLFEENPINASGVDAKAI
LSARLSKSRRLENLIAQLPGEKKNGLFGNLIALSLGLTPNFKSNFDLAEDAKLQLSKDTY
DDDLDNLLAQIGDQYADLFLAAKNLSDAILLSDILRVNTEITKAPLSASMIKRYDEHHQD
LTLLKALVRQQLPEKYKEIFFDQSKNGYAGYIDGGASQEEFYKFIKPILEKMDGTEELLV
KLNREDLLRKQRTFDNGSIPHQIHLGELHAILRRQEDFYPFLKDNREKIEKILTFRIPYYV
GPLARGNSRFAWMTRKSEETITPWNFEEVVDKGASAQSFIERMTNFDKNLPNEKVLPK
HSLLYEYFTVYNELTKVKYVTEGMRKPAFLSGEQKKAIVDLLFKTNRKVTVKQLKEDYF
KKIECFDSVEISGVEDRFNASLGTYHDLLKIIKDKDFLDNEENEDILEDIVLTLTLFEDRE
MIEERLKTYAHLFDDKVMKQLKRRRYTGWGRLSRKLINGIRDKQSGKTILDFLKSDGFA
NRNFMQLIHDDSLTFKEDIQKAQVSGQGDSLHEHIANLAGSPAIKKGILQTVKVVDELV
KVMGRHKPENIVIEMARENQTTQKGQKNSRERMKRIEEGIKELGSQILKEHPVENTQLQ
NEKLYLYYLQNGRDMYVDQELDINRLSDYDVDAIVPQSFLKDDSIDNKVLTRSDKNRGK
SDNVPSEEVVKKMKNYWRQLLNAKLITQRKFDNLTKAERGGLSELDKAGFIKRQLVETR
QITKHVAQILDSRMNTKYDENDKLIREVKVITLKSKLVSDFRKDFQFYKVREINNYHHAH
DAYLNAVVGTALIKKYPKLESEFVYGDYKVYDVRKMIAKSEQEIGKATAKYFFYSNIMNF
FKTEITLANGEIRKRPLIETNGETGEIVWDKGRDFATVRKVLSMPQVNIVKKTEVQTGGF
SKESILPKRNSDKLIARKKDWDPKKYGGFDSPTVAYSVLVVAKVEKGKSKKLKSVKELLG

ITITMERSSFEKNPIDFLEAKGYKEVKKDLIIKLPKYSLFELENGRKRMLASAGELQKGNE
LALPSKYVNFLYLASHYEKLKGSPEDNEQKQLFVEQHKHYLDEIIEQISEFSKRVILADA
NLDKVLSAYNKHRDKPIREQAENIIHLFTLTNLGAPAAFKYFDTTIDRKRYTSTKEVLDA
TLIHQSITGLYETRIDLSQLGGDAYPYDVPDYASLGSGSPKKKRKVEDPKKKRKV*

The amino acid sequence of DNMT3A-(human)DNMT3L-XTEN80-ZNF680-GS-XTEN80-dCas9 (V47 scheme, SEQ ID NO: 98) is as follows:

MPAAKRVKLDGSNHDQEFDPPKVYPPVPAEKRKPIRVLSLFDGIATGLLVLKDL
GIQVDRYIASEVCEDSITVGMVRHQGKIMYVGDVRSVTQKHIQEWGPFDLVIGGSP
CNDLSIVNPARKGLYEGTGRLFFEFYRLLHDARPKEGDDRPFFWLFENVVAMGVSD
KRDISRFLESNPVMIDAKEVSAAHRARYFWGNLPGMNRPLASTVNDKLELQECLEH
GRIAKFSKVRTITTRSNSIKQGKDQHFPVFMNEKEDILWCTEMERVFGFPVHYTDV
SNMSRLARQRLLGRSWSVPVIRHLFAPLKEYFACVSSGNSNANSRGPSFSSGLVPLSLRG
SHMFETVPVWRRQPVRVLSLFEDIKKELTSLGFLESGSDPGQLKHVVDVTDTVRKD
VEEWGPFDLVYGATPPLGHTCDRPPSWYLFQFHRLLQYARPKPGSPRPFFWMFVD
NLVLNKEDLDVASRFLEMEPVTIPDVHGGSLQNAVRVWSNIPAIRSSRHWALVSEE
ELSLLAQNKQSSKLAAKWPTKLVKNCFLPLREYFKYFSGGPSSGAPPPSGGSPAGSPT
STEEGTSESATPESGPGTSTEPSEGSAPGSPAGSPTSTEEGTSTEPSEGSAPGTSTEPSERTLLT
FRDVAIEFSLEEWQCLDTAQRNLYRKVMFENYRNLVFLGIAVSKPHLITCLEQGKEPWN
SGSGSGSGGSGSGGSGSGSGGSGSGGSGSGETPGTSEGGPGTSTEPSEGSAPGSPAGSPTSTE
EGTSTEPSEGSAPGTSTEPSEPKKKRKVMDKKYSIGLAIGTNSVGWAVITDEYKVPSKKFK
VLGNTDRHSIKKNLIGALLFDSGETAEATRLKRTARRRYTRRKNRICYLQEIFSNEMAKV
DDSFFHRLEESFLVEEDKKHERHPIFGNIVDEVAYHEKYPTIYHLRKKLVDSTDKADLR
LIYLALAHMIKFRGHFLIEGDLNPDNSDVDKLFIQLVQTYNQLFEENPINASGVDAKAIL
SARLSKSRRLENLIAQLPGEKKNGLFGNLIALSLGLTPNFKSNFDLAEDAKLQLSKDTYD
DDLDNLLAQIGDQYADLFLAAKNLSDAILLSDILRVNTEITKAPLSASMIKRYDEHHQDL
TLLKALVRQQLPEKYKEIFFDQSKNGYAGYIDGGASQEEFYKFIKPILEKMDGTEELLVK
LNREDLLRKQRTFDNGSIPHQIHLGELHAILRRQEDFYPFLKDNREKIEKILTFRIPYYVG

PLARGNSRFAWMTRKSEETITPWNFEEVVDKGASAQSFIERMTNFDKNLPNEKVLPKH
SLLYEYFTVYNELTKVKYVTEGMRKPAFLSGEQKKAIVDLLFKTNRKVTVKQLKEDYFK
KIECFDSVEISGVEDRFNASLGTYHDLLKIIKDKDFLDNEENEDILEDIVLTLTLFEDRE
MIEERLKTYAHLFDDKVMKQLKRRRYTGWGRLSRKLINGIRDKQSGKTILDFLKSDGFA
NRNFMQLIHDDSLTFKEDIQKAQVSGQGDSLHEHIANLAGSPAIKKGILQTVKVVDELV
KVMGRHKPENIVIEMARENQTTQKGQKNSRERMKRIEEGIKELGSQILKEHPVENTQLQ
NEKLYLYYLQNGRDMYVDQELDINRLSDYDVDAIVPQSFLKDDSIDNKVLTRSDKNRGK
SDNVPSEEVVKKMKNYWRQLLNAKLITQRKFDNLTKAERGGLSELDKAGFIKRQLVETR
QITKHVAQILDSRMNTKYDENDKLIREVKVITLKSKLVSDFRKDFQFYKVREINNYHHAH
DAYLNAVVGTALIKKYPKLESEFVYGDYKVYDVRKMIAKSEQEIGKATAKYFFYSNIMNF
FKTEITLANGEIRKRPLIETNGETGEIVWDKGRDFATVRKVLSMPQVNIVKKTEVQTGGF
SKESILPKRNSDKLIARKKDWDPKKYGGFDSPTVAYSVLVVAKVEKGKSKKLKSVKELLG
ITIMERSSFEKNPIDFLEAKGYKEVKKDLIIKLPKYSLFELENGRKRMLASAGELQKGNE
LALPSKYVNFLYLASHYEKLKGSPEDNEQKQLFVEQHKHYLDEIIEQISEFSKRVILADA
NLDKVLSAYNKHRDKPIREQAENIIHLFTLTNLGAPAAFKYFDTTIDRKRYTSTKEVLDA
TLIHQSITGLYETRIDLSQLGGDAYPYDVPDYASLGSGSPKKKRKVEDPKKKRKVDGLE
*

The amino acid sequence of DNMT3A-(human)DNMT3L-XTEN80-ZNF354a-GS-XTEN80-dCas9 (V48 scheme, SEQ ID NO: 99) is as follows:

MPAAKRVKLDGSNHDQEFDPPKVYPPVPAEKRKPIRVLSLFDGIATGLLVLKDL
GIQVDRYIASEVCEDSITVGMVRHQGKIMYVGDVRSVTQKHIQEWGPFDLVIGGSP
CNDLSIVNPARKGLYEGTGRLFFEFYRLLHDARPKEGDDRPFFWLFENVVAMGVSD
KRDISRFLESNPVMIDAKEVSAAHRARYFWGNLPGMNRPLASTVNDKLELQECLEH
GRIAKFSKVRTITTRSNSIKQGKDQHFPVFMNEKEDILWCTEMERVFGFPVHYTDV
SNMSRLARQRLLGRSWSVPVIRHLFAPLKEYFACVSSGNSNANSRGPSFSSGLVPLSLRG
SHMFETVPVWRRQPVRVLSLFEDIKKELTSLGFLESGSDPGQLKHVVDVTDTVRKD
VEEWGPFDLVYGATPPLGHTCDRPPSWYLFQFHRLLQYARPKPGSPRPFFWMFVD

NLVLNKEDLDVASRFLEMEPVTIPDVHGGSLQNAVRVWSNIPAIRSSRHWALVSEE
ELSLLAQNKQSSKLAAKWPTKLVKNCFLPLREYFKYFSGGPSSGAPPPSGGSPAGSPT
STEEGTSESATPESGPGTSTEPSEGSAPGSPAGSPTSTEEGTSTEPSEGSAPGTSTEPSERTLLT
FEDVAVLFTRDEWRKLAPSQRNLYRDVMLENYRNLVSLGLPFTKPKVISLLQQGEDPW
ESGSGSGSGGSGSGGSGSGSGGSGSGGSGSGETPGTSEGGPGTSTEPSEGSAPGSPAGSPTST
EEGTSTEPSEGSAPGTSTEPSEPKKKRKVMDKKYSIGLAIGTNSVGWAVITDEYKVPSKKF
KVLGNTDRHSIKKNLIGALLFDSGETAEATRLKRTARRRYTRRKNRICYLQEIFSNEMAK
VDDSFFHRLEESFLVEEDKKHERHPIFGNIVDEVAYHEKYPTIYHLRKKLVDSTDKADL
RLIYLALAHMIKFRGHFLIEGDLNPDNSDVDKLFIQLVQTYNQLFEENPINASGVDAKAI
LSARLSKSRRLENLIAQLPGEKKNGLFGNLIALSLGLTPNFKSNFDLAEDAKLQLSKDTY
DDDLDNLLAQIGDQYADLFLAAKNLSDAILLSDILRVNTEITKAPLSASMIKRYDEHHQD
LTLLKALVRQQLPEKYKEIFFDQSKNGYAGYIDGGASQEEFYKFIKPILEKMDGTEELLV
KLNREDLLRKQRTFDNGSIPHQIHLGELHAILRRQEDFYPFLKDNREKIEKILTFRIPYYV
GPLARGNSRFAWMTRKSEETITPWNFEEVVDKGASAQSFIERMTNFDKNLPNEKVLPK
HSLLYEYFTVYNELTKVKYVTEGMRKPAFLSGEQKKAIVDLLFKTNRKVTVKQLKEDYF
KKIECFDSVEISGVEDRFNASLGTYHDLLKIIKDKDFLDNEENEDILEDIVLTLTLFEDRE
MIEERLKTYAHLFDDKVMKQLKRRRYTGWGRLSRKLINGIRDKQSGKTILDFLKSDGFA
NRNFMQLIHDDSLTFKEDIQKAQVSGQGDSLHEHIANLAGSPAIKKGILQTVKVVDELV
KVMGRHKPENIVIEMARENQTTQKGQKNSRERMKRIEEGIKELGSQILKEHPVENTQLQ
NEKLYLYYLQNGRDMYVDQELDINRLSDYDVDAIVPQSFLKDDSIDNKVLTRSDKNRGK
SDNVPSEEVVKKMKNYWRQLLNAKLITQRKFDNLTKAERGGLSELDKAGFIKRQLVETR
QITKHVAQILDSRMNTKYDENDKLIREVKVITLKSKLVSDFRKDFQFYKVREINNYHHAH
DAYLNAVVGTALIKKYPKLESEFVYGDYKVYDVRKMIAKSEQEIGKATAKYFFYSNIMNF
FKTEITLANGEIRKRPLIETNGETGEIVWDKGRDFATVRKVLSMPQVNIVKKTEVQTGGF
SKESILPKRNSDKLIARKKDWDPKKYGGFDSPTVAYSVLVVAKVEKGKSKKLKSVKELLG
ITIMERSSFEKNPIDFLEAKGYKEVKKDLIIKLPKYSLFELENGRKRMLASAGELQKGNE
LALPSKYVNFLYLASHYEKLKGSPEDNEQKQLFVEQHKHYLDEIIEQISEFSKRVILADA

NLDKVLSAYNKHRDKPIREQAENIIHLFTLTNLGAPAAFKYFDTTIDRKRYTSTKEVLDA
TLIHQSITGLYETRIDLSQLGGDAYPYDVPDYASLGSGSPKKKRKVEDPKKKRKVDGLE
*

The amino acid sequence of DNMT3A-(human)DNMT3L-XTEN80-ZNF419-GS-XTEN80-dCas9 (V49 scheme, SEQ ID NO: 100) is as follows:

MPAAKRVKLDGSNHDQEFDPPKVYPPVPAEKRKPIRVLSLFDGIATGLLVLKDL
GIQVDRYIASEVCEDSITVGMVRHQGKIMYVGDVRSVTQKHIQEWGPFDLVIGGSP
CNDLSIVNPARKGLYEGTGRLFFEFYRLLHDARPKEGDDRPFFWLFENVVAMGVSD
KRDISRFLESNPVMIDAKEVSAAHRARYFWGNLPGMNRPLASTVNDKLELQECLEH
GRIAKFSKVRTITTRSNSIKQGKDQHFPVFMNEKEDILWCTEMERVFGFPVHYTDV
SNMSRLARQRLLGRSWSVPVIRHLFAPLKEYFACVSSGNSNANSRGPSFSSGLVPLSLRG
SHMFETVPVWRRQPVRVLSLFEDIKKELTSLGFLESGSDPGQLKHVVDVTDTVRKD
VEEWGPFDLVYGATPPLGHTCDRPPSWYLFQFHRLLQYARPKPGSPRPFFWMFVD
NLVLNKEDLDVASRFLEMEPVTIPDVHGGSLQNAVRVWSNIPAIRSSRHWALVSEE
ELSLLAQNKQSSKLAAKWPTKLVKNCFLPLREYFKYFSGGPSSGAPPPSGGSPAGSPT
STEEGTSESATPESGPGTSTEPSEGSAPGSPAGSPTSTEEGTSTEPSEGSAPGTSTEPSERTLVT
FEDVAVYFSQEEWRLLDDAQRLLYRNVMLENFTLLASLGLASSKTHEITQLESWEEPF
MSGSGSGSGGSGSGGSGSGSGGSGSGGSGSGETPGTSEGGPGTSTEPSEGSAPGSPAGSPTST
EEGTSTEPSEGSAPGTSTEPSEPKKKRKVMDKKYSIGLAIGTNSVGWAVITDEYKVPSKKF
KVLGNTDRHSIKKNLIGALLFDSGETAEATRLKRTARRRYTRRKNRICYLQEIFSNEMAK
VDDSFFHRLEESFLVEEDKKHERHPIFGNIVDEVAYHEKYPTIYHLRKKLVDSTDKADL
RLIYLALAHMIKFRGHFLIEGDLNPDNSDVDKLFIQLVQTYNQLFEENPINASGVDAKAI
LSARLSKSRRLENLIAQLPGEKKNGLFGNLIALSLGLTPNFKSNFDLAEDAKLQLSKDTY
DDDLDNLLAQIGDQYADLFLAAKNLSDAILLSDILRVNTEITKAPLSASMIKRYDEHHQD
LTLLKALVRQQLPEKYKEIFFDQSKNGYAGYIDGGASQEEFYKFIKPILEKMDGTEELLV
KLNREDLLRKQRTFDNGSIPHQIHLGELHAILRRQEDFYPFLKDNREKIEKILTFRIPYYV
GPLARGNSRFAWMTRKSEETITPWNFEEVVDKGASAQSFIERMTNFDKNLPNEKVLPK

HSLLYEYFTVYNELTKVKYVTEGMRKPAFLSGEQKKAIVDLLFKTNRKVTVKQLKEDYF
KKIECFDSVEISGVEDRFNASLGTYHDLLKIIKDKDFLDNEENEDILEDIVLTLTLFEDRE
MIEERLKTYAHLFDDKVMKQLKRRRYTGWGRLSRKLINGIRDKQSGKTILDFLKSDGFA
NRNFMQLIHDDSLTFKEDIQKAQVSGQGDSLHEHIANLAGSPAIKKGILQTVKVVDELV
KVMGRHKPENIVIEMARENQTTQKGQKNSRERMKRIEEGIKELGSQILKEHPVENTQLQ
NEKLYLYYLQNGRDMYVDQELDINRLSDYDVDAIVPQSFLKDDSIDNKVLTRSDKNRGK
SDNVPSEEVVKKMKNYWRQLLNAKLITQRKFDNLTKAERGGLSELDKAGFIKRQLVETR
QITKHVAQILDSRMNTKYDENDKLIREVKVITLKSKLVSDFRKDFQFYKVREINNYHHAH
DAYLNAVVGTALIKKYPKLESEFVYGDYKVYDVRKMIAKSEQEIGKATAKYFFYSNIMNF
FKTEITLANGEIRKRPLIETNGETGEIVWDKGRDFATVRKVLSMPQVNIVKKTEVQTGGF
SKESILPKRNSDKLIARKKDWDPKKYGGFDSPTVAYSVLVVAKVEKGKSKKLKSVKELLG
ITIMERSSFEKNPIDFLEAKGYKEVKKDLIIKLPKYSLFELENGRKRMLASAGELQKGNE
LALPSKYVNFLYLASHYEKLKGSPEDNEQKQLFVEQHKHYLDEIIEQISEFSKRVILADA
NLDKVLSAYNKHRDKPIREQAENIIHLFTLTNLGAPAAFKYFDTTIDRKRYTSTKEVLDA
TLIHQSITGLYETRIDLSQLGGDAYPYDVPDYASLGSGSPKKKRKVEDPKKKRKVDGLE
*

The amino acid sequence of KRAB-XTEN80-DNMT3A-(human)DNMT3L-XTEN16-ZIM3-dCas9 (V54 scheme, SEQ ID NO: 105) is as follows:

MVRTLVTFKDVFVDFTREEWKLLDTAQQIVYRNVMLENYKNLVSLGYQLTKPDVIL
RLEKGEEPGGPSSGAPPPSGGSPAGSPTSTEEGTSESATPESGPGTSTEPSEGSAPGSPAGSPT
STEEGTSTEPSEGSAPGTSTEPSEPKKKRKVMNHDQEFDPPKVYPPVPAEKRKPIRVLSL
FDGIATGLLVLKDLGIQVDRYIASEVCEDSITVGMVRHQGKIMYVGDVRSVTQKHI
QEWGPFDLVIGGSPCNDLSIVNPARKGLYEGTGRLFFEFYRLLHDARPKEGDDRPF
FWLFENVVAMGVSDKRDISRFLESNPVMIDAKEVSAAHRARYFWGNLPGMNRPLA
STVNDKLELQECLEHGRIAKFSKVRTITTRSNSIKQGKDQHFPVFMNEKEDILWCT
EMERVFGFPVHYTDVSNMSRLARQRLLGRSWSVPVIRHLFAPLKEYFACVSSGNSNA
NSRGPSFSSGLVPLSLRGSHMFETVPVWRRQPVRVLSLFEDIKKELTSLGFLESGSDPG

QLKHVVDVTDTVRKDVEEWGPFDLVYGATPPLGHTCDRPPSWYLFQFHRLLQYA
RPKPGSPRPFFWMFVDNLVLNKEDLDVASRFLEMEPVTIPDVHGGSLQNAVRVWS
NIPAIRSSRHWALVSEEELSLLAQNKQSSKLAAKWPTKLVKNCFLPLREYFKYFSDG
SGSETPGTSESATPESMNNSQGRVTFEDVTVNFTQGEWQRLNPEQRNLYRDVMLENYSN
LVSVGQGETTKPDVILRLEQGKEPWLEEEEVLGSGRAEKNGDIGGQIWKPKDVKESLGG
PSSGAPPPSGGSPAGSPTSTEEGTSESATPESGPGTSTEPSEGSAPGSPAGSPTSTEEGTSTEPSE
GSAPGTSTEPSEPKKKRKVMDKKYSIGLAIGTNSVGWAVITDEYKVPSKKFKVLGNTDRH
SIKKNLIGALLFDSGETAEATRLKRTARRRYTRRKNRICYLQEIFSNEMAKVDDSFFHRL
EESFLVEEDKKHERHPIFGNIVDEVAYHEKYPTIYHLRKKLVDSTDKADLRLIYLALAHM
IKFRGHFLIEGDLNPDNSDVDKLFIQLVQTYNQLFEENPINASGVDAKAILSARLSKSRRL
ENLIAQLPGEKKNGLFGNLIALSLGLTPNFKSNFDLAEDAKLQLSKDTYDDDLDNLLAQI
GDQYADLFLAAKNLSDAILLSDILRVNTEITKAPLSASMIKRYDEHHQDLTLLKALVRQQ
LPEKYKEIFFDQSKNGYAGYIDGGASQEEFYKFIKPILEKMDGTEELLVKLNREDLLRKQ
RTFDNGSIPHQIHLGELHAILRRQEDFYPFLKDNREKIEKILTFRIPYYVGPLARGNSRFA
WMTRKSEETITPWNFEEVVDKGASAQSFIERMTNFDKNLPNEKVLPKHSLLYEYFTVYN
ELTKVKYVTEGMRKPAFLSGEQKKAIVDLLFKTNRKVTVKQLKEDYFKKIECFDSVEISG
VEDRFNASLGTYHDLLKIIKDKDFLDNEENEDILEDIVLTLTLFEDREMIEERLKTYAHL
FDDKVMKQLKRRRYTGWGRLSRKLINGIRDKQSGKTILDFLKSDGFANRNFMQLIHDDS
LTFKEDIQKAQVSGQGDSLHEHIANLAGSPAIKKGILQTVKVVDELVKVMGRHKPENIVI
EMARENQTTQKGQKNSRERMKRIEEGIKELGSQILKEHPVENTQLQNEKLYLYYLQNGR
DMYVDQELDINRLSDYDVDAIVPQSFLKDDSIDNKVLTRSDKNRGKSDNVPSEEVVKKM
KNYWRQLLNAKLITQRKFDNLTKAERGGLSELDKAGFIKRQLVETRQITKHVAQILDSR
MNTKYDENDKLIREVKVITLKSKLVSDFRKDFQFYKVREINNYHHAHDAYLNAVVGTAL
IKKYPKLESEFVYGDYKVYDVRKMIAKSEQEIGKATAKYFFYSNIMNFFKTEITLANGEIR
KRPLIETNGETGEIVWDKGRDFATVRKVLSMPQVNIVKKTEVQTGGFSKESILPKRNSDK
LIARKKDWDPKKYGGFDSPTVAYSVLVVAKVEKGKSKKLKSVKELLGITIMERSSFEKNP
IDFLEAKGYKEVKKDLIIKLPKYSLFELENGRKRMLASAGELQKGNELALPSKYVNFLYL

ASHYEKLKGSPEDNEQKQLFVEQHKHYLDEIIEQISEFSKRVILADANLDKVLSAYNKHR
DKPIREQAENIIHLFTLTNLGAPAAFKYFDTTIDRKRYTSTKEVLDATLIHQSITGLYETRI
DLSQLGGDAYPYDVPDYASLGSGSPKKKRKVEDPKKKRKVDGLE*

The amino acid sequence of KRAB-XTEN80-ZIM3-XTEN16-DNMT3A-(human)DNMT3L-dCas9 (V55 scheme, SEQ ID NO: 106) is as follows:

MVRTLVTFKDVFVDFTREEWKLLDTAQQIVYRNVMLENYKNLVSLGYQLTKPDVIL
RLEKGEEPGGPSSGAPPPSGGSPAGSPTSTEEGTSESATPESGPGTSTEPSEGSAPGSPAGSPT
STEEGTSTEPSEGSAPGTSTEPSEPKKKRKVNNSQGRVTFEDVTVNFTQGEWQRLNPEQR
NLYRDVMLENYSNLVSVGQGETTKPDVILRLEQGKEPWLEEEEVLGSGRAEKNGDIGG
QIWKPKDVKESLSGSETPGTSESATPESMNHDQEFDPPKVYPPVPAEKRKPIRVLSLFD
GIATGLLVLKDLGIQVDRYIASEVCEDSITVGMVRHQGKIMYVGDVRSVTQKHIQE
WGPFDLVIGGSPCNDLSIVNPARKGLYEGTGRLFFEFYRLLHDARPKEGDDRPFFW
LFENVVAMGVSDKRDISRFLESNPVMIDAKEVSAAHRARYFWGNLPGMNRPLAST
VNDKLELQECLEHGRIAKFSKVRTITTRSNSIKQGKDQHFPVFMNEKEDILWCTEM
ERVFGFPVHYTDVSNMSRLARQRLLGRSWSVPVIRHLFAPLKEYFACVSSGNSNANS
RGPSFSSGLVPLSLRGSHMFETVPVWRRQPVRVLSLFEDIKKELTSLGFLESGSDPGQL
KHVVDVTDTVRKDVEEWGPFDLVYGATPPLGHTCDRPPSWYLFQFHRLLQYARPK
PGSPRPFFWMFVDNLVLNKEDLDVASRFLEMEPVTIPDVHGGSLQNAVRVWSNIPA
IRSSRHWALVSEEELSLLAQNKQSSKLAAKWPTKLVKNCFLPLREYFKYFSGGPSSG
APPPSGGSPAGSPTSTEEGTSESATPESGPGTSTEPSEGSAPGSPAGSPTSTEEGTSTEPSEGSA
PGTSTEPSEPKKKRKVMDKKYSIGLAIGTNSVGWAVITDEYKVPSKKFKVLGNTDRHSIK
KNLIGALLFDSGETAEATRLKRTARRRYTRRKNRICYLQEIFSNEMAKVDDSFFHRLEES
FLVEEDKKHERHPIFGNIVDEVAYHEKYPTIYHLRKKLVDSTDKADLRLIYLALAHMIKF
RGHFLIEGDLNPDNSDVDKLFIQLVQTYNQLFEENPINASGVDAKAILSARLSKSRRLEN
LIAQLPGEKKNGLFGNLIALSLGLTPNFKSNFDLAEDAKLQLSKDTYDDDLDNLLAQIGD
QYADLFLAAKNLSDAILLSDILRVNTEITKAPLSASMIKRYDEHHQDLTLLKALVRQQLP
EKYKEIFFDQSKNGYAGYIDGGASQEEFYKFIKPILEKMDGTEELLVKLNREDLLRKQRT

FDNGSIPHQIHLGELHAILRRQEDFYPFLKDNREKIEKILTFRIPYYVGPLARGNSRFAW
MTRKSEETITPWNFEEVVDKGASAQSFIERMTNFDKNLPNEKVLPKHSLLYEYFTVYNE
LTKVKYVTEGMRKPAFLSGEQKKAIVDLLFKTNRKVTVKQLKEDYFKKIECFDSVEISGV
EDRFNASLGTYHDLLKIIKDKDFLDNEENEDILEDIVLTLTLFEDREMIEERLKTYAHLF
DDKVMKQLKRRRYTGWGRLSRKLINGIRDKQSGKTILDFLKSDGFANRNFMQLIHDDSL
TFKEDIQKAQVSGQGDSLHEHIANLAGSPAIKKGILQTVKVVDELVKVMGRHKPENIVIE
MARENQTTQKGQKNSRERMKRIEEGIKELGSQILKEHPVENTQLQNEKLYLYYLQNGRD
MYVDQELDINRLSDYDVDAIVPQSFLKDDSIDNKVLTRSDKNRGKSDNVPSEEVVKKMK
NYWRQLLNAKLITQRKFDNLTKAERGGLSELDKAGFIKRQLVETRQITKHVAQILDSRM
NTKYDENDKLIREVKVITLKSKLVSDFRKDFQFYKVREINNYHHAHDAYLNAVVGTALIK
KYPKLESEFVYGDYKVYDVRKMIAKSEQEIGKATAKYFFYSNIMNFFKTEITLANGEIRK
RPLIETNGETGEIVWDKGRDFATVRKVLSMPQVNIVKKTEVQTGGFSKESILPKRNSDKL
IARKKDWDPKKYGGFDSPTVAYSVLVVAKVEKGKSKKLKSVKELLGITIMERSSFEKNPI
DFLEAKGYKEVKKDLIIKLPKYSLFELENGRKRMLASAGELQKGNELALPSKYVNFLYLA
SHYEKLKGSPEDNEQKQLFVEQHKHYLDEIIEQISEFSKRVILADANLDKVLSAYNKHRD
KPIREQAENIIHLFTLTNLGAPAAFKYFDTTIDRKRYTSTKEVLDATLIHQSITGLYETRID
LSQLGGDAYPYDVPDYASLGSGSPKKKRKVEDPKKKRKVDGLE*

The amino acid sequence of DNMT3A-(human)DNMT3L-XTEN80-KRAB-XTEN80-ZIM3-XTEN80-dCas9 (V56 scheme, SEQ ID NO: 107) is as follows:

MNHDQEFDPPKVYPPVPAEKRKPIRVLSLFDGIATGLLVLKDLGIQVDRYIASEV
CEDSITVGMVRHQGKIMYVGDVRSVTQKHIQEWGPFDLVIGGSPCNDLSIVNPARK
GLYEGTGRLFFEFYRLLHDARPKEGDDRPFFWLFENVVAMGVSDKRDISRFLESNP
VMIDAKEVSAAHRARYFWGNLPGMNRPLASTVNDKLELQECLEHGRIAKFSKVRT
ITTRSNSIKQGKDQHFPVFMNEKEDILWCTEMERVFGFPVHYTDVSNMSRLARQRL
LGRSWSVPVIRHLFAPLKEYFACVSSGNSNANSRGPSFSSGLVPLSLRGSHMFETVPVW
RRQPVRVLSLFEDIKKELTSLGFLESGSDPGQLKHVVDVTDTVRKDVEEWGPFDLV
YGATPPLGHTCDRPPSWYLFQFHRLLQYARPKPGSPRPFFWMFVDNLVLNKEDLD

VASRFLEMEPVTIPDVHGGSLQNAVRVWSNIPAIRSSRHWALVSEEELSLLAQNKQS
SKLAAKWPTKLVKNCFLPLREYFKYFSGGPSSGAPPPSGGSPAGSPTSTEEGTSESATPE
SGPGTSTEPSEGSAPGSPAGSPTSTEEGTSTEPSEGSAPGTSTEPSERTLVTFKDVFVDFTREE
WKLLDTAQQIVYRNVMLENYKNLVSLGYQLTKPDVILRLEKGEEPGGPSSGAPPPSGGSP
AGSPTSTEEGTSESATPESGPGTSTEPSEGSAPGSPAGSPTSTEEGTSTEPSEGSAPGTSTEPSEP
KKKRKVNNSQGRVTFEDVTVNFTQGEWQRLNPEQRNLYRDVMLENYSNLVSVGQGET
TKPDVILRLEQGKEPWLEEEEVLGSGRAEKNGDIGGQIWKPKDVKESLGGPSSGAPPPSG
GSPAGSPTSTEEGTSESATPESGPGTSTEPSEGSAPGSPAGSPTSTEEGTSTEPSEGSAPGTSTEP
SEPKKKRKVMDKKYSIGLAIGTNSVGWAVITDEYKVPSKKFKVLGNTDRHSIKKNLIGAL
LFDSGETAEATRLKRTARRRYTRRKNRICYLQEIFSNEMAKVDDSFFHRLEESFLVEED
KKHERHPIFGNIVDEVAYHEKYPTIYHLRKKLVDSTDKADLRLIYLALAHMIKFRGHFLI
EGDLNPDNSDVDKLFIQLVQTYNQLFEENPINASGVDAKAILSARLSKSRRLENLIAQLP
GEKKNGLFGNLIALSLGLTPNFKSNFDLAEDAKLQLSKDTYDDDLDNLLAQIGDQYADL
FLAAKNLSDAILLSDILRVNTEITKAPLSASMIKRYDEHHQDLTLLKALVRQQLPEKYKEI
FFDQSKNGYAGYIDGGASQEEFYKFIKPILEKMDGTEELLVKLNREDLLRKQRTFDNGSI
PHQIHLGELHAILRRQEDFYPFLKDNREKIEKILTFRIPYYVGPLARGNSRFAWMTRKSE
ETITPWNFEEVVDKGASAQSFIERMTNFDKNLPNEKVLPKHSLLYEYFTVYNELTKVKY
VTEGMRKPAFLSGEQKKAIVDLLFKTNRKVTVKQLKEDYFKKIECFDSVEISGVEDRFN
ASLGTYHDLLKIIKDKDFLDNEENEDILEDIVLTLTLFEDREMIEERLKTYAHLFDDKVM
KQLKRRRYTGWGRLSRKLINGIRDKQSGKTILDFLKSDGFANRNFMQLIHDDSLTFKEDI
QKAQVSGQGDSLHEHIANLAGSPAIKKGILQTVKVVDELVKVMGRHKPENIVIEMAREN
QTTQKGQKNSRERMKRIEEGIKELGSQILKEHPVENTQLQNEKLYLYYLQNGRDMYVDQ
ELDINRLSDYDVDAIVPQSFLKDDSIDNKVLTRSDKNRGKSDNVPSEEVVKKMKNYWRQ
LLNAKLITQRKFDNLTKAERGGLSELDKAGFIKRQLVETRQITKHVAQILDSRMNTKYDE
NDKLIREVKVITLKSKLVSDFRKDFQFYKVREINNYHHAHDAYLNAVVGTALIKKYPKLE
SEFVYGDYKVYDVRKMIAKSEQEIGKATAKYFFYSNIMNFFKTEITLANGEIRKRPLIETN
GETGEIVWDKGRDFATVRKVLSMPQVNIVKKTEVQTGGFSKESILPKRNSDKLIARKKD

WDPKKYGGFDSPTVAYSVLVVAKVEKGKSKKLKSVKELLGITIMERSSFEKNPIDFLEAK
GYKEVKKDLIIKLPKYSLFELENGRKRMLASAGELQKGNELALPSKYVNFLYLASHYEKL
KGSPEDNEQKQLFVEQHKHYLDEIIEQISEFSKRVILADANLDKVLSAYNKHRDKPIREQ
AENIIHLFTLTNLGAPAAFKYFDTTIDRKRYTSTKEVLDATLIHQSITGLYETRIDLSQLGG
DAYPYDVPDYASLGSGSPKKKRKVEDPKKKRKVDGLE*

For another example, the one or more effector domains may be C-terminal to the nucleic acid-binding domain. In some embodiments, the effector domain may comprise at least one type of the epigenetic modification domain and at least one type of the transcriptional regulation domain; and, the epigenetic editing domain in the effector domain may be N-terminal to the transcriptional regulation domain, or may be C-terminal to the transcriptional regulation domain. In these cases, the epigenetic modification domain may comprise DNMT3A and DNMT3L, the C-terminus of the DNMT3Ais linked to the N-terminus of the DNMT3L, or the C-terminus of the DNMT3L is linked to the N-terminus of the DNMT3A. Also in these cases, the transcriptional regulation domain may comprise a transcriptional repressor domain. For example, the transcriptional regulation domain comprises a zinc finger protein-based transcription factor or a functionally active fragment thereof. Specifically, the zinc finger protein-based transcription factor may include KRAB. For example, in some embodiments of the present application, the zinc finger protein-based transcription factor may be selected from ZIM3 KRAB and KOX1 KRAB.

In some embodiments of the above cases, the transcriptional regulation domain may comprise two or more of the KRAB domains described above, and the two or more of the KRAB domains described above are of the same type or of different types. For example, the transcriptional regulation domain comprises two or more ZIM3 KRABs, two or more KOX1 KRABs, or both ZIM3 KRAB and KOX1 KRAB, and the total number thereof is two or more. In these cases, the two or more KRAB domains (of the same type or different types) can be linked via an XTEN linker sequence.

The following are some other exemplary fusions of the present application, and the components in the amino acid sequence of the fusion are as follows: bold-DNMT3A, underlined bold-DNMT3L, italic-linker sequence, bold italic-transcriptional regulation domain, underlined bold italic-dCas9, and * is any amino acid.

The amino acid sequence of dCas9-XTEN80-DNMT3A-DNMT3L-XTEN16-ZIM3 (V9 or V9' scheme, SEQ ID NO: 62 or 339) is as follows: 
MVPAAKRVKLDGSDKKYSIGLAIGTNSVGWAVITDEYKVPSKKFKVLGNTDRHSIKK
NLIGALLFDSGETAEATRLKRTARRRYTRRKNRICYLQEIFSNEMAKVDDSFFHRLEESF
LVEEDKKHERHPIFGNIVDEVAYHEKYPTIYHLRKKLVDSTDKADLRLIYLALAHMIKFR
GHFLIEGDLNPDNSDVDKLFIQLVQTYNQLFEENPINASGVDAKAILSARLSKSRRLENLI
AQLPGEKKNGLFGNLIALSLGLTPNFKSNFDLAEDAKLQLSKDTYDDDLDNLLAQIGDQ
YADLFLAAKNLSDAILLSDILRVNTEITKAPLSASMIKRYDEHHQDLTLLKALVRQQLPEK
YKEIFFDQSKNGYAGYIDGGASQEEFYKFIKPILEKMDGTEELLVKLNREDLLRKQRTFD
NGSIPHQIHLGELHAILRRQEDFYPFLKDNREKIEKILTFRIPYYVGPLARGNSRFAWMT
RKSEETITPWNFEEVVDKGASAQSFIERMTNFDKNLPNEKVLPKHSLLYEYFTVYNELT
KVKYVTEGMRKPAFLSGEQKKAIVDLLFKTNRKVTVKQLKEDYFKKIECFDSVEISGVED
RFNASLGTYHDLLKIIKDKDFLDNEENEDILEDIVLTLTLFEDREMIEERLKTYAHLFDD
KVMKQLKRRRYTGWGRLSRKLINGIRDKQSGKTILDFLKSDGFANRNFMQLIHDDSLTF
KEDIQKAQVSGQGDSLHEHIANLAGSPAIKKGILQTVKVVDELVKVMGRHKPENIVIEM
ARENQTTQKGQKNSRERMKRIEEGIKELGSQILKEHPVENTQLQNEKLYLYYLQNGRDM
YVDQELDINRLSDYDVDAIVPQSFLKDDSIDNKVLTRSDKNRGKSDNVPSEEVVKKMKN
YWRQLLNAKLITQRKFDNLTKAERGGLSELDKAGFIKRQLVETRQITKHVAQILDSRMN
TKYDENDKLIREVKVITLKSKLVSDFRKDFQFYKVREINNYHHAHDAYLNAVVGTALIKK
YPKLESEFVYGDYKVYDVRKMIAKSEQEIGKATAKYFFYSNIMNFFKTEITLANGEIRKR
PLIETNGETGEIVWDKGRDFATVRKVLSMPQVNIVKKTEVQTGGFSKESILPKRNSDKLI
ARKKDWDPKKYGGFDSPTVAYSVLVVAKVEKGKSKKLKSVKELLGITIMERSSFEKNPID
FLEAKGYKEVKKDLIIKLPKYSLFELENGRKRMLASAGELQKGNELALPSKYVNFLYLAS
HYEKLKGSPEDNEQKQLFVEQHKHYLDEIIEQISEFSKRVILADANLDKVLSAYNKHRDK
PIREQAENIIHLFTLTNLGAPAAFKYFDTTIDRKRYTSTKEVLDATLIHQSITGLYETRIDL
 
SQLGGDAYPYDVPDYASLGSGSPKKKRKVEDPKKKRKVDGGGPSSGAPPPSGGSPAGSP
TSTEEGTSESATPESGPGTSTEPSEGSAPGSPAGSPTSTEEGTSTEPSEGSAPGTSTEPSEMNHD
QEFDPPKVYPPVPAEKRKPIRVLSLFDGIATGLLVLKDLGIQVDRYIASEVCEDSITV
GMVRHQGKIMYVGDVRSVTQKHIQEWGPFDLVIGGSPCNDLSIVNPARKGLYEGT
GRLFFEFYRLLHDARPKEGDDRPFFWLFENVVAMGVSDKRDISRFLESNPVMIDAK
EVSAAHRARYFWGNLPGMNRPLASTVNDKLELQECLEHGRIAKFSKVRTITTRSNS
IKQGKDQHFPVFMNEKEDILWCTEMERVFGFPVHYTDVSNMSRLARQRLLGRSWS
VPVIRHLFAPLKEYFACVSSGNSNANSRGPSFSSGLVPLSLRGSHMGPMEIYKTVSAWK
RQPVRVLSLFRNIDKVLKSLGFLESGSGSGGTLKYVEDVTNVVRRDVEKWGPFDL
VYGSTQPLGSSCDRCPGWYMFQFHRILQYALPRQESQRPFFWIFMDNLLLTEDDQE
TTTRFLQTEAVTLQDVRGRDYQNAMRVWSNIPGLKSKHAPLTPKEEEYLQAQVRS
RSKLDAPKVDLLVKNCLLPLREYFKYFSQNSLPLDGSGSETPGTSESATPESMNNSQGR
VTFEDVTVNFTQGEWQRLNPEQRNLYRDVMLENYSNLVSVGQGETTKPDVILRLEQGK
EPWLEEEEVLGSGRAEKNGDIGGQIWKPKDVKESLLE* (V9 scheme).
 or V9' scheme:

MPAAKRVKLDGSMDKKYSIGLAIGTNSVGWAVITDEYKVPSKKFKVLGNTDRHSIKK
NLIGALLFDSGETAEATRLKRTARRRYTRRKNRICYLQEIFSNEMAKVDDSFFHRLEESFL
VEEDKKHERHPIFGNIVDEVAYHEKYPTIYHLRKKLVDSTDKADLRLIYLALAHMIKFRG
HFLIEGDLNPDNSDVDKLFIQLVQTYNQLFEENPINASGVDAKAILSARLSKSRRLENLIA
QLPGEKKNGLFGNLIALSLGLTPNFKSNFDLAEDAKLQLSKDTYDDDLDNLLAQIGDQY
ADLFLAAKNLSDAILLSDILRVNTEITKAPLSASMIKRYDEHHQDLTLLKALVRQQLPEKY
KEIFFDQSKNGYAGYIDGGASQEEFYKFIKPILEKMDGTEELLVKLNREDLLRKQRTFDN
GSIPHQIHLGELHAILRRQEDFYPFLKDNREKIEKILTFRIPYYVGPLARGNSRFAWMTRKS
EETITPWNFEEVVDKGASAQSFIERMTNFDKNLPNEKVLPKHSLLYEYFTVYNELTKVKY
VTEGMRKPAFLSGEQKKAIVDLLFKTNRKVTVKQLKEDYFKKIECFDSVEISGVEDRFNA
SLGTYHDLLKIIKDKDFLDNEENEDILEDIVLTLTLFEDREMIEERLKTYAHLFDDKVMKQ
LKRRRYTGWGRLSRKLINGIRDKQSGKTILDFLKSDGFANRNFMQLIHDDSLTFKEDIQK

AQVSGQGDSLHEHIANLAGSPAIKKGILQTVKVVDELVKVMGRHKPENIVIEMARENQT
TQKGQKNSRERMKRIEEGIKELGSQILKEHPVENTQLQNEKLYLYYLQNGRDMYVDQEL
DINRLSDYDVDAIVPQSFLKDDSIDNKVLTRSDKNRGKSDNVPSEEVVKKMKNYWRQL
LNAKLITQRKFDNLTKAERGGLSELDKAGFIKRQLVETRQITKHVAQILDSRMNTKYDEN
DKLIREVKVITLKSKLVSDFRKDFQFYKVREINNYHHAHDAYLNAVVGTALIKKYPKLES
EFVYGDYKVYDVRKMIAKSEQEIGKATAKYFFYSNIMNFFKTEITLANGEIRKRPLIETNG
ETGEIVWDKGRDFATVRKVLSMPQVNIVKKTEVQTGGFSKESILPKRNSDKLIARKKDW
DPKKYGGFDSPTVAYSVLVVAKVEKGKSKKLKSVKELLGITIMERSSFEKNPIDFLEAKG
YKEVKKDLIIKLPKYSLFELENGRKRMLASAGELQKGNELALPSKYVNFLYLASHYEKL
KGSPEDNEQKQLFVEQHKHYLDEIIEQISEFSKRVILADANLDKVLSAYNKHRDKPIREQA
ENIIHLFTLTNLGAPAAFKYFDTTIDRKRYTSTKEVLDATLIHQSITGLYETRIDLSQLGGD
AYPYDVPDYASLGSGSPKKKRKVEDPKKKRKVDGGGPSSGAPPPSGGSPAGSPTSTEEGT
SESATPESGPGTSTEPSEGSAPGSPAGSPTSTEEGTSTEPSEGSAPGTSTEPSEMNHDQEFDP
PKVYPPVPAEKRKPIRVLSLFDGIATGLLVLKDLGIQVDRYIASEVCEDSITVGMVRHQGK
IMYVGDVRSVTQKHIQEWGPFDLVIGGSPCNDLSIVNPARKGLYEGTGRLFFEFYRLLHD
ARPKEGDDRPFFWLFENVVAMGVSDKRDISRFLESNPVMIDAKEVSAAHRARYFWGNL
PGMNRPLASTVNDKLELQECLEHGRIAKFSKVRTITTRSNSIKQGKDQHFPVFMNEKEDI
LWCTEMERVFGFPVHYTDVSNMSRLARQRLLGRSWSVPVIRHLFAPLKEYFACVSSGNS
NANSRGPSFSSGLVPLSLRGSHMGPMEIYKTVSAWKRQPVRVLSLFRNIDKVLKSLGFLE
SGSGSGGGTLKYVEDVTNVVRRDVEKWGPFDLVYGSTQPLGSSCDRCPGWYMFQFHRI
LQYALPRQESQRPFFWIFMDNLLLTEDDQETTTRFLQTEAVTLQDVRGRDYQNAMRVWS
NIPGLKSKHAPLTPKEEEYLQAQVRSRSKLDAPKVDLLVKNCLLPLREYFKYFSQNSLPL
DGSGSETPGTSESATPESMNNSQGRVTFEDVTVNFTQGEWQRLNPEQRNLYRDVMLENY
SNLVSVGQGETTKPDVILRLEQGKEPWLEEEEVLGSGRAEKNGDIGGQIWKPKDVKESL

The amino acid sequence of dCas9-XTEN80-ZIM3-XTEN16-DNMT3A-DNMT3L (V10 or V10' scheme, SEQ ID NO: 63 or 340) is as follows: 
MPKKKRKVMDKKYSIGLAIGTNSVGWAVITDEYKVPSKKFKVLGNTDRHSIKKNLIG
ALLFDSGETAEATRLKRTARRRYTRRKNRICYLQEIFSNEMAKVDDSFFHRLEESFLVEE
DKKHERHPIFGNIVDEVAYHEKYPTIYHLRKKLVDSTDKADLRLIYLALAHMIKFRGHFL
IEGDLNPDNSDVDKLFIQLVQTYNQLFEENPINASGVDAKAILSARLSKSRRLENLIAQLP
GEKKNGLFGNLIALSLGLTPNFKSNFDLAEDAKLQLSKDTYDDDLDNLLAQIGDQYADL
FLAAKNLSDAILLSDILRVNTEITKAPLSASMIKRYDEHHQDLTLLKALVRQQLPEKYKEI
FFDQSKNGYAGYIDGGASQEEFYKFIKPILEKMDGTEELLVKLNREDLLRKQRTFDNGSI
PHQIHLGELHAILRRQEDFYPFLKDNREKIEKILTFRIPYYVGPLARGNSRFAWMTRKSE
ETITPWNFEEVVDKGASAQSFIERMTNFDKNLPNEKVLPKHSLLYEYFTVYNELTKVKY
VTEGMRKPAFLSGEQKKAIVDLLFKTNRKVTVKQLKEDYFKKIECFDSVEISGVEDRFN
ASLGTYHDLLKIIKDKDFLDNEENEDILEDIVLTLTLFEDREMIEERLKTYAHLFDDKVM
KQLKRRRYTGWGRLSRKLINGIRDKQSGKTILDFLKSDGFANRNFMQLIHDDSLTFKEDI
QKAQVSGQGDSLHEHIANLAGSPAIKKGILQTVKVVDELVKVMGRHKPENIVIEMAREN
QTTQKGQKNSRERMKRIEEGIKELGSQILKEHPVENTQLQNEKLYLYYLQNGRDMYVDQ
ELDINRLSDYDVDAIVPQSFLKDDSIDNKVLTRSDKNRGKSDNVPSEEVVKKMKNYWRQ
LLNAKLITQRKFDNLTKAERGGLSELDKAGFIKRQLVETRQITKHVAQILDSRMNTKYDE
NDKLIREVKVITLKSKLVSDFRKDFQFYKVREINNYHHAHDAYLNAVVGTALIKKYPKLE
SEFVYGDYKVYDVRKMIAKSEQEIGKATAKYFFYSNIMNFFKTEITLANGEIRKRPLIETN
GETGEIVWDKGRDFATVRKVLSMPQVNIVKKTEVQTGGFSKESILPKRNSDKLIARKKD
WDPKKYGGFDSPTVAYSVLVVAKVEKGKSKKLKSVKELLGITIMERSSFEKNPIDFLEAK
GYKEVKKDLIIKLPKYSLFELENGRKRMLASAGELQKGNELALPSKYVNFLYLASHYEKL
KGSPEDNEQKQLFVEQHKHYLDEIIEQISEFSKRVILADANLDKVLSAYNKHRDKPIREQ
AENIIHLFTLTNLGAPAAFKYFDTTIDRKRYTSTKEVLDATLIHQSITGLYETRIDLSQLGG
DAYPYDVPDYASLGSGSPKKKRKVEDPKKKRKVDGGGPSSGAPPPSGGSPAGSPTSTEEG
TSESATPESGPGTSTEPSEGSAPGSPAGSPTSTEEGTSTEPSEGSAPGTSTEPSEMNNSQGRVT
FEDVTVNFTQGEWQRLNPEQRNLYRDVMLENYSNLVSVGQGETTKPDVILRLEQGKEP
WLEEEEVLGSGRAEKNGDIGGQIWKPKDVKESLSGSETPGTSESATPESMNHDQEFDPP
 
KVYPPVPAEKRKPIRVLSLFDGIATGLLVLKDLGIQVDRYIASEVCEDSITVGMVRH
QGKIMYVGDVRSVTQKHIQEWGPFDLVIGGSPCNDLSIVNPARKGLYEGTGRLFFE
FYRLLHDARPKEGDDRPFFWLFENVVAMGVSDKRDISRFLESNPVMIDAKEVSAAH
RARYFWGNLPGMNRPLASTVNDKLELQECLEHGRIAKFSKVRTITTRSNSIKQGKD
QHFPVFMNEKEDILWCTEMERVFGFPVHYTDVSNMSRLARQRLLGRSWSVPVIRH
LFAPLKEYFACVSSGNSNANSRGPSFSSGLVPLSLRGSHMGPMEIYKTVSAWKRQPVRV
LSLFRNIDKVLKSLGFLESGSGSGGGTLKYVEDVTNVVRRDVEKWGPFDLVYGSTQ
PLGSSCDRCPGWYMFQFHRILQYALPRQESQRPFFWIFMDNLLLTEDDQETTTRFL
QTEAVTLQDVRGRDYQNAMRVWSNIPGLKSKHAPLTPKEEEYLQAQVRSRSKLDA
PKVDLLVKNCLLPLREYFKYFSQNSLPLLE* (V10 scheme).
 or V10' scheme:

MPAAKRVKLDGSMDKKYSIGLAIGTNSVGWAVITDEYKVPSKKFKVLGNTDRHSIKK
NLIGALLFDSGETAEATRLKRTARRRYTRRKNRICYLQEIFSNEMAKVDDSFFHRLEESFL
VEEDKKHERHPIFGNIVDEVAYHEKYPTIYHLRKKLVDSTDKADLRLIYLALAHMIKFRG
HFLIEGDLNPDNSDVDKLFIQLVQTYNQLFEENPINASGVDAKAILSARLSKSRRLENLIA
QLPGEKKNGLFGNLIALSLGLTPNFKSNFDLAEDAKLQLSKDTYDDDLDNLLAQIGDQY
ADLFLAAKNLSDAILLSDILRVNTEITKAPLSASMIKRYDEHHQDLTLLKALVRQQLPEKY
KEIFFDQSKNGYAGYIDGGASQEEFYKFIKPILEKMDGTEELLVKLNREDLLRKQRTFDN
GSIPHQIHLGELHAILRRQEDFYPFLKDNREKIEKILTFRIPYYVGPLARGNSRFAWMTRKS
EETITPWNFEEVVDKGASAQSFIERMTNFDKNLPNEKVLPKHSLLYEYFTVYNELTKVKY
VTEGMRKPAFLSGEQKKAIVDLLFKTNRKVTVKQLKEDYFKKIECFDSVEISGVEDRFNA
SLGTYHDLLKIIKDKDFLDNEENEDILEDIVLTLTLFEDREMIEERLKTYAHLFDDKVMKQ
LKRRRYTGWGRLSRKLINGIRDKQSGKTILDFLKSDGFANRNFMQLIHDDSLTFKEDIQK
AQVSGQGDSLHEHIANLAGSPAIKKGILQTVKVVDELVKVMGRHKPENIVIEMARENQT
TQKGQKNSRERMKRIEEGIKELGSQILKEHPVENTQLQNEKLYLYYLQNGRDMYVDQEL
DINRLSDYDVDAIVPQSFLKDDSIDNKVLTRSDKNRGKSDNVPSEEVVKKMKNYWRQL
LNAKLITQRKFDNLTKAERGGLSELDKAGFIKRQLVETRQITKHVAQILDSRMNTKYDEN

DKLIREVKVITLKSKLVSDFRKDFQFYKVREINNYHHAHDAYLNAVVGTALIKKYPKLES
EFVYGDYKVYDVRKMIAKSEQEIGKATAKYFFYSNIMNFFKTEITLANGEIRKRPLIETNG
ETGEIVWDKGRDFATVRKVLSMPQVNIVKKTEVQTGGFSKESILPKRNSDKLIARKKDW
DPKKYGGFDSPTVAYSVLVVAKVEKGKSKKLKSVKELLGITIMERSSFEKNPIDFLEAKG
YKEVKKDLIIKLPKYSLFELENGRKRMLASAGELQKGNELALPSKYVNFLYLASHYEKL
KGSPEDNEQKQLFVEQHKHYLDEIIEQISEFSKRVILADANLDKVLSAYNKHRDKPIREQA
ENIIHLFTLTNLGAPAAFKYFDTTIDRKRYTSTKEVLDATLIHQSITGLYETRIDLSQLGGD
AYPYDVPDYASLGSGSPKKKRKVEDPKKKRKVDGGGPSSGAPPPSGGSPAGSPTSTEEGT
SESATPESGPGTSTEPSEGSAPGSPAGSPTSTEEGTSTEPSEGSAPGTSTEPSEMNNSQGRV
TFEDVTVNFTQGEWQRLNPEQRNLYRDVMLENYSNLVSVGQGETTKPDVILRLEQGKEP
WLEEEEVLGSGRAEKNGDIGGQIWKPKDVKESLSGSETPGTSESATPESMNHDQEFDPPK
VYPPVPAEKRKPIRVLSLFDGIATGLLVLKDLGIQVDRYIASEVCEDSITVGMVRHQGKIM
YVGDVRSVTQKHIQEWGPFDLVIGGSPCNDLSIVNPARKGLYEGTGRLFFEFYRLLHDAR
PKEGDDRPFFWLFENVVAMGVSDKRDISRFLESNPVMIDAKEVSAAHRARYFWGNLPG
MNRPLASTVNDKLELQECLEHGRIAKFSKVRTITTRSNSIKQGKDQHFPVFMNEKEDILW
CTEMERVFGFPVHYTDVSNMSRLARQRLLGRSWSVPVIRHLFAPLKEYFACVSSGNSNA
NSRGPSFSSGLVPLSLRGSHMGPMEIYKTVSAWKRQPVRVLSLFRNIDKVLKSLGFLESGS
GSGGGTLKYVEDVTNVVRRDVEKWGPFDLVYGSTQPLGSSCDRCPGWYMFQFHRILQY
ALPRQESQRPFFWIFMDNLLLTEDDQETTTRFLQTEAVTLQDVRGRDYQNAMRVWSNIP
GLKSKHAPLTPKEEEYLQAQVRSRSKLDAPKVDLLVKNCLLPLREYFKYFSQNSLPL

The amino acid sequence of dCas9-XTEN80-ZIM3-XTEN16-DNMT3L-DNMT3A (V11 or V11' scheme, SEQ ID NO: 64 or 341) is as follows: 
MPKKKRKVMDKKYSIGLAIGTNSVGWAVITDEYKVPSKKFKVLGNTDRHSIKKNLIG
ALLFDSGETAEATRLKRTARRRYTRRKNRICYLQEIFSNEMAKVDDSFFHRLEESFLVEE
DKKHERHPIFGNIVDEVAYHEKYPTIYHLRKKLVDSTDKADLRLIYLALAHMIKFRGHFL
IEGDLNPDNSDVDKLFIQLVQTYNQLFEENPINASGVDAKAILSARLSKSRRLENLIAQLP
GEKKNGLFGNLIALSLGLTPNFKSNFDLAEDAKLQLSKDTYDDDLDNLLAQIGDQYADL
 
FLAAKNLSDAILLSDILRVNTEITKAPLSASMIKRYDEHHQDLTLLKALVRQQLPEKYKEI
FFDQSKNGYAGYIDGGASQEEFYKFIKPILEKMDGTEELLVKLNREDLLRKQRTFDNGSI
PHQIHLGELHAILRRQEDFYPFLKDNREKIEKILTFRIPYYVGPLARGNSRFAWMTRKSE
ETITPWNFEEVVDKGASAQSFIERMTNFDKNLPNEKVLPKHSLLYEYFTVYNELTKVKY
VTEGMRKPAFLSGEQKKAIVDLLFKTNRKVTVKQLKEDYFKKIECFDSVEISGVEDRFN
ASLGTYHDLLKIIKDKDFLDNEENEDILEDIVLTLTLFEDREMIEERLKTYAHLFDDKVM
KQLKRRRYTGWGRLSRKLINGIRDKQSGKTILDFLKSDGFANRNFMQLIHDDSLTFKEDI
QKAQVSGQGDSLHEHIANLAGSPAIKKGILQTVKVVDELVKVMGRHKPENIVIEMAREN
QTTQKGQKNSRERMKRIEEGIKELGSQILKEHPVENTQLQNEKLYLYYLQNGRDMYVDQ
ELDINRLSDYDVDAIVPQSFLKDDSIDNKVLTRSDKNRGKSDNVPSEEVVKKMKNYWRQ
LLNAKLITQRKFDNLTKAERGGLSELDKAGFIKRQLVETRQITKHVAQILDSRMNTKYDE
NDKLIREVKVITLKSKLVSDFRKDFQFYKVREINNYHHAHDAYLNAVVGTALIKKYPKLE
SEFVYGDYKVYDVRKMIAKSEQEIGKATAKYFFYSNIMNFFKTEITLANGEIRKRPLIETN
GETGEIVWDKGRDFATVRKVLSMPQVNIVKKTEVQTGGFSKESILPKRNSDKLIARKKD
WDPKKYGGFDSPTVAYSVLVVAKVEKGKSKKLKSVKELLGITIMERSSFEKNPIDFLEAK
GYKEVKKDLIIKLPKYSLFELENGRKRMLASAGELQKGNELALPSKYVNFLYLASHYEKL
KGSPEDNEQKQLFVEQHKHYLDEIIEQISEFSKRVILADANLDKVLSAYNKHRDKPIREQ
AENIIHLFTLTNLGAPAAFKYFDTTIDRKRYTSTKEVLDATLIHQSITGLYETRIDLSQLGG
DAYPYDVPDYASLGSGSPKKKRKVEDPKKKRKVDGGGPSSGAPPPSGGSPAGSPTSTEEG
TSESATPESGPGTSTEPSEGSAPGSPAGSPTSTEEGTSTEPSEGSAPGTSTEPSEMNNSQGRVT
FEDVTVNFTQGEWQRLNPEQRNLYRDVMLENYSNLVSVGQGETTKPDVILRLEQGKEP
WLEEEEVLGSGRAEKNGDIGGQIWKPKDVKESLSGSETPGTSESATPESMGPMEIYKTVS
AWKRQPVRVLSLFRNIDKVLKSLGFLESGSGSGGGTLKYVEDVTNVVRRDVEKWG
PFDLVYGSTQPLGSSCDRCPGWYMFQFHRILQYALPRQESQRPFFWIFMDNLLLTE
DDQETTTRFLQTEAVTLQDVRGRDYQNAMRVWSNIPGLKSKHAPLTPKEEEYLQA
QVRSRSKLDAPKVDLLVKNCLLPLREYFKYFSQNSLPLSSGNSNANSRGPSFSSGLVPLS
LRGSHMNHDQEFDPPKVYPPVPAEKRKPIRVLSLFDGIATGLLVLKDLGIQVDRYIAS
 
EVCEDSITVGMVRHQGKIMYVGDVRSVTQKHIQEWGPFDLVIGGSPCNDLSIVNPA
RKGLYEGTGRLFFEFYRLLHDARPKEGDDRPFFWLFENVVAMGVSDKRDISRFLES
NPVMIDAKEVSAAHRARYFWGNLPGMNRPLASTVNDKLELQECLEHGRIAKFSKV
RTITTRSNSIKQGKDQHFPVFMNEKEDILWCTEMERVFGFPVHYTDVSNMSRLARQ
RLLGRSWSVPVIRHLFAPLKEYFACVLE* (V11 scheme).
 or V11' scheme:

MPAAKRVKLDGSMDKKYSIGLAIGTNSVGWAVITDEYKVPSKKFKVLGNTDRHSIK
KNLIGALLFDSGETAEATRLKRTARRRYTRRKNRICYLQEIFSNEMAKVDDSFFHRLEES
FLVEEDKKHERHPIFGNIVDEVAYHEKYPTIYHLRKKLVDSTDKADLRLIYLALAHMIKF
RGHFLIEGDLNPDNSDVDKLFIQLVQTYNQLFEENPINASGVDAKAILSARLSKSRRLENL
IAQLPGEKKNGLFGNLIALSLGLTPNFKSNFDLAEDAKLQLSKDTYDDDLDNLLAQIGDQ
YADLFLAAKNLSDAILLSDILRVNTEITKAPLSASMIKRYDEHHQDLTLLKALVRQQLPE
KYKEIFFDQSKNGYAGYIDGGASQEEFYKFIKPILEKMDGTEELLVKLNREDLLRKQRTF
DNGSIPHQIHLGELHAILRRQEDFYPFLKDNREKIEKILTFRIPYYVGPLARGNSRFAWMT
RKSEETITPWNFEEVVDKGASAQSFIERMTNFDKNLPNEKVLPKHSLLYEYFTVYNELTK
VKYVTEGMRKPAFLSGEQKKAIVDLLFKTNRKVTVKQLKEDYFKKIECFDSVEISGVED
RFNASLGTYHDLLKIIKDKDFLDNEENEDILEDIVLTLTLFEDREMIEERLKTYAHLFDDK
VMKQLKRRRYTGWGRLSRKLINGIRDKQSGKTILDFLKSDGFANRNFMQLIHDDSLTFK
EDIQKAQVSGQGDSLHEHIANLAGSPAIKKGILQTVKVVDELVKVMGRHKPENIVIEMA
RENQTTQKGQKNSRERMKRIEEGIKELGSQILKEHPVENTQLQNEKLYLYYLQNGRDMY
VDQELDINRLSDYDVDAIVPQSFLKDDSIDNKVLTRSDKNRGKSDNVPSEEVVKKMKNY
WRQLLNAKLITQRKFDNLTKAERGGLSELDKAGFIKRQLVETRQITKHVAQILDSRMNT
KYDENDKLIREVKVITLKSKLVSDFRKDFQFYKVREINNYHHAHDAYLNAVVGTALIKK
YPKLESEFVYGDYKVYDVRKMIAKSEQEIGKATAKYFFYSNIMNFFKTEITLANGEIRKR
PLIETNGETGEIVWDKGRDFATVRKVLSMPQVNIVKKTEVQTGGFSKESILPKRNSDKLI
ARKKDWDPKKYGGFDSPTVAYSVLVVAKVEKGKSKKLKSVKELLGITIMERSSFEKNPI
DFLEAKGYKEVKKDLIIKLPKYSLFELENGRKRMLASAGELQKGNELALPSKYVNFLYL

ASHYEKLKGSPEDNEQKQLFVEQHKHYLDEIIEQISEFSKRVILADANLDKVLSAYNKHR
DKPIREQAENIIHLFTLTNLGAPAAFKYFDTTIDRKRYTSTKEVLDATLIHQSITGLYETRI
DLSQLGGDAYPYDVPDYASLGSGSPKKKRKVEDPKKKRKVDGGGPSSGAPPPSGGSPA
GSPTSTEEGTSESATPESGPGTSTEPSEGSAPGSPAGSPTSTEEGTSTEPSEGSAPGTSTEPS
EMNNSQGRVTFEDVTVNFTQGEWQRLNPEQRNLYRDVMLENYSNLVSVGQGETTKPD
VILRLEQGKEPWLEEEEVLGSGRAEKNGDIGGQIWKPKDVKESLSGSETPGTSESATPES
MGPMEIYKTVSAWKRQPVRVLSLFRNIDKVLKSLGFLESGSGSGGGTLKYVEDVTNVV
RRDVEKWGPFDLVYGSTQPLGSSCDRCPGWYMFQFHRILQYALPRQESQRPFFWIFMD
NLLLTEDDQETTTRFLQTEAVTLQDVRGRDYQNAMRVWSNIPGLKSKHAPLTPKEEEYL
QAQVRSRSKLDAPKVDLLVKNCLLPLREYFKYFSQNSLPLSSGNSNANSRGPSFSSGLVP
LSLRGSHMNHDQEFDPPKVYPPVPAEKRKPIRVLSLFDGIATGLLVLKDLGIQVDRYIAS
EVCEDSITVGMVRHQGKIMYVGDVRSVTQKHIQEWGPFDLVIGGSPCNDLSIVNPARKG
LYEGTGRLFFEFYRLLHDARPKEGDDRPFFWLFENVVAMGVSDKRDISRFLESNPVMID
AKEVSAAHRARYFWGNLPGMNRPLASTVNDKLELQECLEHGRIAKFSKVRTITTRSNSI
KQGKDQHFPVFMNEKEDILWCTEMERVFGFPVHYTDVSNMSRLARQRLLGRSWSVPVI
RHLFAPLKEYFACV

The amino acid sequence of dCas9-XTEN80-ZIM3-XTEN80-DNMT3L-DNMT3A (V14 or V14' scheme, SEQ ID NO: 65 or 342) is as follows: 
MPKKKRKVMDKKYSIGLAIGTNSVGWAVITDEYKVPSKKFKVLGNTDRHSIKKNLIG
ALLFDSGETAEATRLKRTARRRYTRRKNRICYLQEIFSNEMAKVDDSFFHRLEESFLVEE
DKKHERHPIFGNIVDEVAYHEKYPTIYHLRKKLVDSTDKADLRLIYLALAHMIKFRGHFL
IEGDLNPDNSDVDKLFIQLVQTYNQLFEENPINASGVDAKAILSARLSKSRRLENLIAQLP
GEKKNGLFGNLIALSLGLTPNFKSNFDLAEDAKLQLSKDTYDDDLDNLLAQIGDQYADL
FLAAKNLSDAILLSDILRVNTEITKAPLSASMIKRYDEHHQDLTLLKALVRQQLPEKYKEI
FFDQSKNGYAGYIDGGASQEEFYKFIKPILEKMDGTEELLVKLNREDLLRKQRTFDNGSI
PHQIHLGELHAILRRQEDFYPFLKDNREKIEKILTFRIPYYVGPLARGNSRFAWMTRKSE
ETITPWNFEEVVDKGASAQSFIERMTNFDKNLPNEKVLPKHSLLYEYFTVYNELTKVKY
 
VTEGMRKPAFLSGEQKKAIVDLLFKTNRKVTVKQLKEDYFKKIECFDSVEISGVEDRFN
ASLGTYHDLLKIIKDKDFLDNEENEDILEDIVLTLTLFEDREMIEERLKTYAHLFDDKVM
KQLKRRRYTGWGRLSRKLINGIRDKQSGKTILDFLKSDGFANRNFMQLIHDDSLTFKEDI
QKAQVSGQGDSLHEHIANLAGSPAIKKGILQTVKVVDELVKVMGRHKPENIVIEMAREN
QTTQKGQKNSRERMKRIEEGIKELGSQILKEHPVENTQLQNEKLYLYYLQNGRDMYVDQ
ELDINRLSDYDVDAIVPQSFLKDDSIDNKVLTRSDKNRGKSDNVPSEEVVKKMKNYWRQ
LLNAKLITQRKFDNLTKAERGGLSELDKAGFIKRQLVETRQITKHVAQILDSRMNTKYDE
NDKLIREVKVITLKSKLVSDFRKDFQFYKVREINNYHHAHDAYLNAVVGTALIKKYPKLE
SEFVYGDYKVYDVRKMIAKSEQEIGKATAKYFFYSNIMNFFKTEITLANGEIRKRPLIETN
GETGEIVWDKGRDFATVRKVLSMPQVNIVKKTEVQTGGFSKESILPKRNSDKLIARKKD
WDPKKYGGFDSPTVAYSVLVVAKVEKGKSKKLKSVKELLGITIMERSSFEKNPIDFLEAK
GYKEVKKDLIIKLPKYSLFELENGRKRMLASAGELQKGNELALPSKYVNFLYLASHYEKL
KGSPEDNEQKQLFVEQHKHYLDEIIEQISEFSKRVILADANLDKVLSAYNKHRDKPIREQ
AENIIHLFTLTNLGAPAAFKYFDTTIDRKRYTSTKEVLDATLIHQSITGLYETRIDLSQLGG
DAYPYDVPDYASLGSGSPKKKRKVEDPKKKRKVDGGGPSSGAPPPSGGSPAGSPTSTEEG
TSESATPESGPGTSTEPSEGSAPGSPAGSPTSTEEGTSTEPSEGSAPGTSTEPSEMNNSQGRVT
FEDVTVNFTQGEWQRLNPEQRNLYRDVMLENYSNLVSVGQGETTKPDVILRLEQGKEP
WLEEEEVLGSGRAEKNGDIGGQIWKPKDVKESLSGSETPGTSEGGPSSGAPPPSGGSPAGS
PTSTEEGTSTEPSEGSAPGTSTEPSESATPESMGPMEIYKTVSAWKRQPVRVLSLFRNIDK
VLKSLGFLESGSGSGGGTLKYVEDVTNVVRRDVEKWGPFDLVYGSTQPLGSSCDR
CPGWYMFQFHRILQYALPRQESQRPFFWIFMDNLLLTEDDQETTTRFLQTEAVTL
QDVRGRDYQNAMRVWSNIPGLKSKHAPLTPKEEEYLQAQVRSRSKLDAPKVDLLV
KNCLLPLREYFKYFSQNSLPLSSGNSNANSRGPSFSSGLVPLSLRGSHMNHDQEFDPPKV
YPPVPAEKRKPIRVLSLFDGIATGLLVLKDLGIQVDRYIASEVCEDSITVGMVRHQG
KIMYVGDVRSVTQKHIQEWGPFDLVIGGSPCNDLSIVNPARKGLYEGTGRLFFEFY
RLLHDARPKEGDDRPFFWLFENVVAMGVSDKRDISRFLESNPVMIDAKEVSAAHRA
RYFWGNLPGMNRPLASTVNDKLELQECLEHGRIAKFSKVRTITTRSNSIKQGKDQH
 
FPVFMNEKEDILWCTEMERVFGFPVHYTDVSNMSRLARQRLLGRSWSVPVIRHLF

APLKEYFACVLE* (V14 scheme).
 or V14' scheme:

MPAAKRVKLDGSMDKKYSIGLAIGTNSVGWAVITDEYKVPSKKFKVLGNTDRHSIK
KNLIGALLFDSGETAEATRLKRTARRRYTRRKNRICYLQEIFSNEMAKVDDSFFHRLEES
FLVEEDKKHERHPIFGNIVDEVAYHEKYPTIYHLRKKLVDSTDKADLRLIYLALAHMIKF
RGHFLIEGDLNPDNSDVDKLFIQLVQTYNQLFEENPINASGVDAKAILSARLSKSRRLENL
IAQLPGEKKNGLFGNLIALSLGLTPNFKSNFDLAEDAKLQLSKDTYDDDLDNLLAQIGDQ
YADLFLAAKNLSDAILLSDILRVNTEITKAPLSASMIKRYDEHHQDLTLLKALVRQQLPE
KYKEIFFDQSKNGYAGYIDGGASQEEFYKFIKPILEKMDGTEELLVKLNREDLLRKQRTF
DNGSIPHQIHLGELHAILRRQEDFYPFLKDNREKIEKILTFRIPYYVGPLARGNSRFAWMT
RKSEETITPWNFEEVVDKGASAQSFIERMTNFDKNLPNEKVLPKHSLLYEYFTVYNELTK
VKYVTEGMRKPAFLSGEQKKAIVDLLFKTNRKVTVKQLKEDYFKKIECFDSVEISGVED
RFNASLGTYHDLLKIIKDKDFLDNEENEDILEDIVLTLTLFEDREMIEERLKTYAHLFDDK
VMKQLKRRRYTGWGRLSRKLINGIRDKQSGKTILDFLKSDGFANRNFMQLIHDDSLTFK
EDIQKAQVSGQGDSLHEHIANLAGSPAIKKGILQTVKVVDELVKVMGRHKPENIVIEMA
RENQTTQKGQKNSRERMKRIEEGIKELGSQILKEHPVENTQLQNEKLYLYYLQNGRDMY
VDQELDINRLSDYDVDAIVPQSFLKDDSIDNKVLTRSDKNRGKSDNVPSEEVVKKMKNY
WRQLLNAKLITQRKFDNLTKAERGGLSELDKAGFIKRQLVETRQITKHVAQILDSRMNT
KYDENDKLIREVKVITLKSKLVSDFRKDFQFYKVREINNYHHAHDAYLNAVVGTALIKK
YPKLESEFVYGDYKVYDVRKMIAKSEQEIGKATAKYFFYSNIMNFFKTEITLANGEIRKR
PLIETNGETGEIVWDKGRDFATVRKVLSMPQVNIVKKTEVQTGGFSKESILPKRNSDKLI
ARKKDWDPKKYGGFDSPTVAYSVLVVAKVEKGKSKKLKSVKELLGITIMERSSFEKNPI
DFLEAKGYKEVKKDLIIKLPKYSLFELENGRKRMLASAGELQKGNELALPSKYVNFLYL
ASHYEKLKGSPEDNEQKQLFVEQHKHYLDEIIEQISEFSKRVILADANLDKVLSAYNKHR
DKPIREQAENIIHLFTLTNLGAPAAFKYFDTTIDRKRYTSTKEVLDATLIHQSITGLYETRI
DLSQLGGDAYPYDVPDYASLGSGSPKKKRKVEDPKKKRKVDGGGPSSGAPPPSGGSPA

GSPTSTEEGTSESATPESGPGTSTEPSEGSAPGSPAGSPTSTEEGTSTEPSEGSAPGTSTEPS
EMNNSQGRVTFEDVTVNFTQGEWQRLNPEQRNLYRDVMLENYSNLVSVGQGETTKPD
VILRLEQGKEPWLEEEEVLGSGRAEKNGDIGGQIWKPKDVKESLSGSETPGTSEGGPSSG
APPPSGGSPAGSPTSTEEGTSESATPESGPGTSTEPSEGSAPGSPAGSPTSTEEGTSTEPSEG
SAPGTSTEPSESATPESMGPMEIYKTVSAWKRQPVRVLSLFRNIDKVLKSLGFLESGSGS
GGGTLKYVEDVTNVVRRDVEKWGPFDLVYGSTQPLGSSCDRCPGWYMFQFHRILQYA
LPRQESQRPFFWIFMDNLLLTEDDQETTTRFLQTEAVTLQDVRGRDYQNAMRVWSNIPG
LKSKHAPLTPKEEEYLQAQVRSRSKLDAPKVDLLVKNCLLPLREYFKYFSQNSLPLSSGN
SNANSRGPSFSSGLVPLSLRGSHMNHDQEFDPPKVYPPVPAEKRKPIRVLSLFDGIATGLL
VLKDLGIQVDRYIASEVCEDSITVGMVRHQGKIMYVGDVRSVTQKHIQEWGPFDLVIGG
SPCNDLSIVNPARKGLYEGTGRLFFEFYRLLHDARPKEGDDRPFFWLFENVVAMGVSDK
RDISRFLESNPVMIDAKEVSAAHRARYFWGNLPGMNRPLASTVNDKLELQECLEHGRIA
KFSKVRTITTRSNSIKQGKDQHFPVFMNEKEDILWCTEMERVFGFPVHYTDVSNMSRLA
RQRLLGRSWSVPVIRHLFAPLKEYFACV

The amino acid sequence of dCas9-XTEN80-ZIM3-NEW XTEN-DNMT3L-DNMT3A (V15 or V15' scheme, SEQ ID NO: 66 or 343) is as follows: 
MVPAAKRVKLDGSDKKYSIGLAIGTNSVGWAVITDEYKVPSKKFKVLGNTDRHSIKK
NLIGALLFDSGETAEATRLKRTARRRYTRRKNRICYLQEIFSNEMAKVDDSFFHRLEESF
LVEEDKKHERHPIFGNIVDEVAYHEKYPTIYHLRKKLVDSTDKADLRLIYLALAHMIKFR
GHFLIEGDLNPDNSDVDKLFIQLVQTYNQLFEENPINASGVDAKAILSARLSKSRRLENLI
AQLPGEKKNGLFGNLIALSLGLTPNFKSNFDLAEDAKLQLSKDTYDDDLDNLLAQIGDQ
YADLFLAAKNLSDAILLSDILRVNTEITKAPLSASMIKRYDEHHQDLTLLKALVRQQLPEK
YKEIFFDQSKNGYAGYIDGGASQEEFYKFIKPILEKMDGTEELLVKLNREDLLRKQRTFD
NGSIPHQIHLGELHAILRRQEDFYPFLKDNREKIEKILTFRIPYYVGPLARGNSRFAWMT
RKSEETITPWNFEEVVDKGASAQSFIERMTNFDKNLPNEKVLPKHSLLYEYFTVYNELT
KVKYVTEGMRKPAFLSGEQKKAIVDLLFKTNRKVTVKQLKEDYFKKIECFDSVEISGVED
RFNASLGTYHDLLKIIKDKDFLDNEENEDILEDIVLTLTLFEDREMIEERLKTYAHLFDD
 
KVMKQLKRRRYTGWGRLSRKLINGIRDKQSGKTILDFLKSDGFANRNFMQLIHDDSLTF
KEDIQKAQVSGQGDSLHEHIANLAGSPAIKKGILQTVKVVDELVKVMGRHKPENIVIEM
ARENQTTQKGQKNSRERMKRIEEGIKELGSQILKEHPVENTQLQNEKLYLYYLQNGRDM
YVDQELDINRLSDYDVDAIVPQSFLKDDSIDNKVLTRSDKNRGKSDNVPSEEVVKKMKN
YWRQLLNAKLITQRKFDNLTKAERGGLSELDKAGFIKRQLVETRQITKHVAQILDSRMN
TKYDENDKLIREVKVITLKSKLVSDFRKDFQFYKVREINNYHHAHDAYLNAVVGTALIKK
YPKLESEFVYGDYKVYDVRKMIAKSEQEIGKATAKYFFYSNIMNFFKTEITLANGEIRKR
PLIETNGETGEIVWDKGRDFATVRKVLSMPQVNIVKKTEVQTGGFSKESILPKRNSDKLI
ARKKDWDPKKYGGFDSPTVAYSVLVVAKVEKGKSKKLKSVKELLGITIMERSSFEKNPID
FLEAKGYKEVKKDLIIKLPKYSLFELENGRKRMLASAGELQKGNELALPSKYVNFLYLAS
HYEKLKGSPEDNEQKQLFVEQHKHYLDEIIEQISEFSKRVILADANLDKVLSAYNKHRDK
PIREQAENIIHLFTLTNLGAPAAFKYFDTTIDRKRYTSTKEVLDATLIHQSITGLYETRIDL
SQLGGDAYPYDVPDYASLGSGSPKKKRKVEDPKKKRKVDGGGPSSGAPPPSGGSPAGSP
TSTEEGTSESATPESGPGTSTEPSEGSAPGSPAGSPTSTEEGTSTEPSEGSAPGTSTEPSEMNNS
QGRVTFEDVTVNFTQGEWQRLNPEQRNLYRDVMLENYSNLVSVGQGETTKPDVILRLE
QGKEPWLEEEEVLGSGRAEKNGDIGGQIWKPKDVKESLSGSETPGTSESATPESGPGSPA
GSPTSTEEGTSESATPESGPGSEPATSGSETPGTSESATPESGPGTSTEPSEGSAPGTSTEPSESA
TPESMGPMEIYKTVSAWKRQPVRVLSLFRNIDKVLKSLGFLESGSGSGGGTLKYVE
DVTNVVRRDVEKWGPFDLVYGSTQPLGSSCDRCPGWYMFQFHRILQYALPRQESQ
RPFFWIFMDNLLLTEDDQETTTRFLQTEAVTLQDVRGRDYQNAMRVWSNIPGLKS
KHAPLTPKEEEYLQAQVRSRSKLDAPKVDLLVKNCLLPLREYFKYFSQNSLPLSSGN
SNANSRGPSFSSGLVPLSLRGSHMNHDQEFDPPKVYPPVPAEKRKPIRVLSLFDGIATGL
LVLKDLGIQVDRYIASEVCEDSITVGMVRHQGKIMYVGDVRSVTQKHIQEWGPFDL
VIGGSPCNDLSIVNPARKGLYEGTGRLFFEFYRLLHDARPKEGDDRPFFWLFENVV
AMGVSDKRDISRFLESNPVMIDAKEVSAAHRARYFWGNLPGMNRPLASTVNDKLE
LQECLEHGRIAKFSKVRTITTRSNSIKQGKDQHFPVFMNEKEDILWCTEMERVFGF
PVHYTDVSNMSRLARQRLLGRSWSVPVIRHLFAPLKEYFACV* (V15 scheme).
 or V15' scheme:

MPAAKRVKLDGSMDKKYSIGLAIGTNSVGWAVITDEYKVPSKKFKVLGNTDRHSIKK
NLIGALLFDSGETAEATRLKRTARRRYTRRKNRICYLQEIFSNEMAKVDDSFFHRLEESFL
VEEDKKHERHPIFGNIVDEVAYHEKYPTIYHLRKKLVDSTDKADLRLIYLALAHMIKFRG
HFLIEGDLNPDNSDVDKLFIQLVQTYNQLFEENPINASGVDAKAILSARLSKSRRLENLIA
QLPGEKKNGLFGNLIALSLGLTPNFKSNFDLAEDAKLQLSKDTYDDDLDNLLAQIGDQY
ADLFLAAKNLSDAILLSDILRVNTEITKAPLSASMIKRYDEHHQDLTLLKALVRQQLPEKY
KEIFFDQSKNGYAGYIDGGASQEEFYKFIKPILEKMDGTEELLVKLNREDLLRKQRTFDN
GSIPHQIHLGELHAILRRQEDFYPFLKDNREKIEKILTFRIPYYVGPLARGNSRFAWMTRKS
EETITPWNFEEVVDKGASAQSFIERMTNFDKNLPNEKVLPKHSLLYEYFTVYNELTKVKY
VTEGMRKPAFLSGEQKKAIVDLLFKTNRKVTVKQLKEDYFKKIECFDSVEISGVEDRFNA
SLGTYHDLLKIIKDKDFLDNEENEDILEDIVLTLTLFEDREMIEERLKTYAHLFDDKVMKQ
LKRRRYTGWGRLSRKLINGIRDKQSGKTILDFLKSDGFANRNFMQLIHDDSLTFKEDIQK
AQVSGQGDSLHEHIANLAGSPAIKKGILQTVKVVDELVKVMGRHKPENIVIEMARENQT
TQKGQKNSRERMKRIEEGIKELGSQILKEHPVENTQLQNEKLYLYYLQNGRDMYVDQEL
DINRLSDYDVDAIVPQSFLKDDSIDNKVLTRSDKNRGKSDNVPSEEVVKKMKNYWRQL
LNAKLITQRKFDNLTKAERGGLSELDKAGFIKRQLVETRQITKHVAQILDSRMNTKYDEN
DKLIREVKVITLKSKLVSDFRKDFQFYKVREINNYHHAHDAYLNAVVGTALIKKYPKLES
EFVYGDYKVYDVRKMIAKSEQEIGKATAKYFFYSNIMNFFKTEITLANGEIRKRPLIETNG
ETGEIVWDKGRDFATVRKVLSMPQVNIVKKTEVQTGGFSKESILPKRNSDKLIARKKDW
DPKKYGGFDSPTVAYSVLVVAKVEKGKSKKLKSVKELLGITIMERSSFEKNPIDFLEAKG
YKEVKKDLIIKLPKYSLFELENGRKRMLASAGELQKGNELALPSKYVNFLYLASHYEKL
KGSPEDNEQKQLFVEQHKHYLDEIIEQISEFSKRVILADANLDKVLSAYNKHRDKPIREQA
ENIIHLFTLTNLGAPAAFKYFDTTIDRKRYTSTKEVLDATLIHQSITGLYETRIDLSQLGGD
AYPYDVPDYASLGSGSPKKKRKVEDPKKKRKVDGGGPSSGAPPPSGGSPAGSPTSTEEGT
SESATPESGPGTSTEPSEGSAPGSPAGSPTSTEEGTSTEPSEGSAPGTSTEPSEMNNSQGRV
TFEDVTVNFTQGEWQRLNPEQRNLYRDVMLENYSNLVSVGQGETTKPDVILRLEQGKEP

WLEEEEVLGSGRAEKNGDIGGQIWKPKDVKESLSGSETPGTSESATPESGPGSPAGSPTST
EEGTSESATPESGPGSEPATSGSETPGTSESATPESGPGTSTEPSEGSAPGTSTEPSESATPES
MGPMEIYKTVSAWKRQPVRVLSLFRNIDKVLKSLGFLESGSGSGGGTLKYVEDVTNVVR
RDVEKWGPFDLVYGSTQPLGSSCDRCPGWYMFQFHRILQYALPRQESQRPFFWIFMDNL
LLTEDDQETTTRFLQTEAVTLQDVRGRDYQNAMRVWSNIPGLKSKHAPLTPKEEEYLQA
QVRSRSKLDAPKVDLLVKNCLLPLREYFKYFSQNSLPLSSGNSNANSRGPSFSSGLVPLSL
RGSHMNHDQEFDPPKVYPPVPAEKRKPIRVLSLFDGIATGLLVLKDLGIQVDRYIASEVCE
DSITVGMVRHQGKIMYVGDVRSVTQKHIQEWGPFDLVIGGSPCNDLSIVNPARKGLYEG
TGRLFFEFYRLLHDARPKEGDDRPFFWLFENVVAMGVSDKRDISRFLESNPVMIDAKEVS
AAHRARYFWGNLPGMNRPLASTVNDKLELQECLEHGRIAKFSKVRTITTRSNSIKQGKD
QHFPVFMNEKEDILWCTEMERVFGFPVHYTDVSNMSRLARQRLLGRSWSVPVIRHLFAP
LKEYFACV

The amino acid sequence of dCas9-XTEN80-ZIM3-XTEN80-(human)DNMT3L-DNMT3A (V16 or V16' scheme, SEQ ID NO: 67 or 344) is as follows: 
MVPAAKRVKLDGSDKKYSIGLAIGTNSVGWAVITDEYKVPSKKFKVLGNTDRHSIKK
NLIGALLFDSGETAEATRLKRTARRRYTRRKNRICYLQEIFSNEMAKVDDSFFHRLEESF
LVEEDKKHERHPIFGNIVDEVAYHEKYPTIYHLRKKLVDSTDKADLRLIYLALAHMIKFR
GHFLIEGDLNPDNSDVDKLFIQLVQTYNQLFEENPINASGVDAKAILSARLSKSRRLENLI
AQLPGEKKNGLFGNLIALSLGLTPNFKSNFDLAEDAKLQLSKDTYDDDLDNLLAQIGDQ
YADLFLAAKNLSDAILLSDILRVNTEITKAPLSASMIKRYDEHHQDLTLLKALVRQQLPEK
YKEIFFDQSKNGYAGYIDGGASQEEFYKFIKPILEKMDGTEELLVKLNREDLLRKQRTFD
NGSIPHQIHLGELHAILRRQEDFYPFLKDNREKIEKILTFRIPYYVGPLARGNSRFAWMT
RKSEETITPWNFEEVVDKGASAQSFIERMTNFDKNLPNEKVLPKHSLLYEYFTVYNELT
KVKYVTEGMRKPAFLSGEQKKAIVDLLFKTNRKVTVKQLKEDYFKKIECFDSVEISGVED
RFNASLGTYHDLLKIIKDKDFLDNEENEDILEDIVLTLTLFEDREMIEERLKTYAHLFDD
KVMKQLKRRRYTGWGRLSRKLINGIRDKQSGKTILDFLKSDGFANRNFMQLIHDDSLTF
KEDIQKAQVSGQGDSLHEHIANLAGSPAIKKGILQTVKVVDELVKVMGRHKPENIVIEM
 
ARENQTTQKGQKNSRERMKRIEEGIKELGSQILKEHPVENTQLQNEKLYLYYLQNGRDM
YVDQELDINRLSDYDVDAIVPQSFLKDDSIDNKVLTRSDKNRGKSDNVPSEEVVKKMKN
YWRQLLNAKLITQRKFDNLTKAERGGLSELDKAGFIKRQLVETRQITKHVAQILDSRMN
TKYDENDKLIREVKVITLKSKLVSDFRKDFQFYKVREINNYHHAHDAYLNAVVGTALIKK
YPKLESEFVYGDYKVYDVRKMIAKSEQEIGKATAKYFFYSNIMNFFKTEITLANGEIRKR
PLIETNGETGEIVWDKGRDFATVRKVLSMPQVNIVKKTEVQTGGFSKESILPKRNSDKLI
ARKKDWDPKKYGGFDSPTVAYSVLVVAKVEKGKSKKLKSVKELLGITIMERSSFEKNPID
FLEAKGYKEVKKDLIIKLPKYSLFELENGRKRMLASAGELQKGNELALPSKYVNFLYLAS
HYEKLKGSPEDNEQKQLFVEQHKHYLDEIIEQISEFSKRVILADANLDKVLSAYNKHRDK
PIREQAENIIHLFTLTNLGAPAAFKYFDTTIDRKRYTSTKEVLDATLIHQSITGLYETRIDL
SQLGGDAYPYDVPDYASLGSGSPKKKRKVEDPKKKRKVDGGGPSSGAPPPSGGSPAGSP
TSTEEGTSESATPESGPGTSTEPSEGSAPGSPAGSPTSTEEGTSTEPSEGSAPGTSTEPSEMNNS
QGRVTFEDVTVNFTQGEWQRLNPEQRNLYRDVMLENYSNLVSVGQGETTKPDVILRLE
QGKEPWLEEEEVLGSGRAEKNGDIGGQIWKPKDVKESLSGSETPGTSEGGPSSGAPPPSG
GSPAGSPTSTEEGTSESATPESGPGTSTEPSEGSAPGSPAGSPTSTEEGTSTEPSEGSAPGTSTEP
SESATPESMFETVPVWRRQPVRVLSLFEDIKKELTSLGFLESGSDPGQLKHVVDVTD
TVRKDVEEWGPFDLVYGATPPLGHTCDRPPSWYLFQFHRLLQYARPKPGSPRPFF
WMFVDNLVLNKEDLDVASRFLEMEPVTIPDVHGGSLQNAVRVWSNIPAIRSSRHW
ALVSEEELSLLAQNKQSSKLAAKWPTKLVKNCFLPLREYFKYFSTELTSSLSSGNSNA
NSRGPSFSSGLVPLSLRGSHMNHDQEFDPPKVYPPVPAEKRKPIRVLSLFDGIATGLLV
LKDLGIQVDRYIASEVCEDSITVGMVRHQGKIMYVGDVRSVTQKHIQEWGPFDLVI
GGSPCNDLSIVNPARKGLYEGTGRLFFEFYRLLHDARPKEGDDRPFFWLFENVVAM
GVSDKRDISRFLESNPVMIDAKEVSAAHRARYFWGNLPGMNRPLASTVNDKLELQE
CLEHGRIAKFSKVRTITTRSNSIKQGKDQHFPVFMNEKEDILWCTEMERVFGFPVH
YTDVSNMSRLARQRLLGRSWSVPVIRHLFAPLKEYFACV* (V16 scheme).
 or V16' scheme:

MPAAKRVKLDGSMDKKYSIGLAIGTNSVGWAVITDEYKVPSKKFKVLGNTDRHSIKK
NLIGALLFDSGETAEATRLKRTARRRYTRRKNRICYLQEIFSNEMAKVDDSFFHRLEESFL
VEEDKKHERHPIFGNIVDEVAYHEKYPTIYHLRKKLVDSTDKADLRLIYLALAHMIKFRG
HFLIEGDLNPDNSDVDKLFIQLVQTYNQLFEENPINASGVDAKAILSARLSKSRRLENLIA
QLPGEKKNGLFGNLIALSLGLTPNFKSNFDLAEDAKLQLSKDTYDDDLDNLLAQIGDQY
ADLFLAAKNLSDAILLSDILRVNTEITKAPLSASMIKRYDEHHQDLTLLKALVRQQLPEKY
KEIFFDQSKNGYAGYIDGGASQEEFYKFIKPILEKMDGTEELLVKLNREDLLRKQRTFDN
GSIPHQIHLGELHAILRRQEDFYPFLKDNREKIEKILTFRIPYYVGPLARGNSRFAWMTRKS
EETITPWNFEEVVDKGASAQSFIERMTNFDKNLPNEKVLPKHSLLYEYFTVYNELTKVKY
VTEGMRKPAFLSGEQKKAIVDLLFKTNRKVTVKQLKEDYFKKIECFDSVEISGVEDRFNA
SLGTYHDLLKIIKDKDFLDNEENEDILEDIVLTLTLFEDREMIEERLKTYAHLFDDKVMKQ
LKRRRYTGWGRLSRKLINGIRDKQSGKTILDFLKSDGFANRNFMQLIHDDSLTFKEDIQK
AQVSGQGDSLHEHIANLAGSPAIKKGILQTVKVVDELVKVMGRHKPENIVIEMARENQT
TQKGQKNSRERMKRIEEGIKELGSQILKEHPVENTQLQNEKLYLYYLQNGRDMYVDQEL
DINRLSDYDVDAIVPQSFLKDDSIDNKVLTRSDKNRGKSDNVPSEEVVKKMKNYWRQL
LNAKLITQRKFDNLTKAERGGLSELDKAGFIKRQLVETRQITKHVAQILDSRMNTKYDEN
DKLIREVKVITLKSKLVSDFRKDFQFYKVREINNYHHAHDAYLNAVVGTALIKKYPKLES
EFVYGDYKVYDVRKMIAKSEQEIGKATAKYFFYSNIMNFFKTEITLANGEIRKRPLIETNG
ETGEIVWDKGRDFATVRKVLSMPQVNIVKKTEVQTGGFSKESILPKRNSDKLIARKKDW
DPKKYGGFDSPTVAYSVLVVAKVEKGKSKKLKSVKELLGITIMERSSFEKNPIDFLEAKG
YKEVKKDLIIKLPKYSLFELENGRKRMLASAGELQKGNELALPSKYVNFLYLASHYEKL
KGSPEDNEQKQLFVEQHKHYLDEIIEQISEFSKRVILADANLDKVLSAYNKHRDKPIREQA
ENIIHLFTLTNLGAPAAFKYFDTTIDRKRYTSTKEVLDATLIHQSITGLYETRIDLSQLGGD
AYPYDVPDYASLGSGSPKKKRKVEDPKKKRKVDGGGPSSGAPPPSGGSPAGSPTSTEEGT
SESATPESGPGTSTEPSEGSAPGSPAGSPTSTEEGTSTEPSEGSAPGTSTEPSEMNNSQGRV
TFEDVTVNFTQGEWQRLNPEQRNLYRDVMLENYSNLVSVGQGETTKPDVILRLEQGKEP
WLEEEEVLGSGRAEKNGDIGGQIWKPKDVKESLSGSETPGTSESATPESGPGSPAGSPTST

EEGTSESATPESGPGSEPATSGSETPGTSESATPESGPGTSTEPSEGSAPGTSTEPSESATPES
MFETVPVWRRQPVRVLSLFEDIKKELTSLGFLESGSDPGQLKHVVDVTDTVRKDVEEWG
PFDLVYGATPPLGHTCDRPPSWYLFQFHRLLQYARPKPGSPRPFFWMFVDNLVLNKEDL
DVASRFLEMEPVTIPDVHGGSLQNAVRVWSNIPAIRSSRHWALVSEEELSLLAQNKQSSKL
AAKWPTKLVKNCFLPLREYFKYFSSSGNSNANSRGPSFSSGLVPLSLRGSHMNHDQEFDP
PKVYPPVPAEKRKPIRVLSLFDGIATGLLVLKDLGIQVDRYIASEVCEDSITVGMVRHQGK
IMYVGDVRSVTQKHIQEWGPFDLVIGGSPCNDLSIVNPARKGLYEGTGRLFFEFYRLLHD
ARPKEGDDRPFFWLFENVVAMGVSDKRDISRFLESNPVMIDAKEVSAAHRARYFWGNL
PGMNRPLASTVNDKLELQECLEHGRIAKFSKVRTITTRSNSIKQGKDQHFPVFMNEKEDI
LWCTEMERVFGFPVHYTDVSNMSRLARQRLLGRSWSVPVIRHLFAPLKEYFACV

The amino acid sequence of dCas9-XTEN80-DNMT3L-DNMT3A-XTEN16-ZIM3 (V23 scheme, SEQ ID NO: 74) is as follows:

MVPAAKRVKLDGSDKKYSIGLAIGTNSVGWAVITDEYKVPSKKFKVLGNTDRHSIKK
NLIGALLFDSGETAEATRLKRTARRRYTRRKNRICYLQEIFSNEMAKVDDSFFHRLEESF
LVEEDKKHERHPIFGNIVDEVAYHEKYPTIYHLRKKLVDSTDKADLRLIYLALAHMIKFR
GHFLIEGDLNPDNSDVDKLFIQLVQTYNQLFEENPINASGVDAKAILSARLSKSRRLENLI
AQLPGEKKNGLFGNLIALSLGLTPNFKSNFDLAEDAKLQLSKDTYDDDLDNLLAQIGDQ
YADLFLAAKNLSDAILLSDILRVNTEITKAPLSASMIKRYDEHHQDLTLLKALVRQQLPEK
YKEIFFDQSKNGYAGYIDGGASQEEFYKFIKPILEKMDGTEELLVKLNREDLLRKQRTFD
NGSIPHQIHLGELHAILRRQEDFYPFLKDNREKIEKILTFRIPYYVGPLARGNSRFAWMT
RKSEETITPWNFEEVVDKGASAQSFIERMTNFDKNLPNEKVLPKHSLLYEYFTVYNELT
KVKYVTEGMRKPAFLSGEQKKAIVDLLFKTNRKVTVKQLKEDYFKKIECFDSVEISGVED
RFNASLGTYHDLLKIIKDKDFLDNEENEDILEDIVLTLTLFEDREMIEERLKTYAHLFDD
KVMKQLKRRRYTGWGRLSRKLINGIRDKQSGKTILDFLKSDGFANRNFMQLIHDDSLTF
KEDIQKAQVSGQGDSLHEHIANLAGSPAIKKGILQTVKVVDELVKVMGRHKPENIVIEM
ARENQTTQKGQKNSRERMKRIEEGIKELGSQILKEHPVENTQLQNEKLYLYYLQNGRDM
YVDQELDINRLSDYDVDAIVPQSFLKDDSIDNKVLTRSDKNRGKSDNVPSEEVVKKMKN

YWRQLLNAKLITQRKFDNLTKAERGGLSELDKAGFIKRQLVETRQITKHVAQILDSRMN
TKYDENDKLIREVKVITLKSKLVSDFRKDFQFYKVREINNYHHAHDAYLNAVVGTALIKK
YPKLESEFVYGDYKVYDVRKMIAKSEQEIGKATAKYFFYSNIMNFFKTEITLANGEIRKR
PLIETNGETGEIVWDKGRDFATVRKVLSMPQVNIVKKTEVQTGGFSKESILPKRNSDKLI
ARKKDWDPKKYGGFDSPTVAYSVLVVAKVEKGKSKKLKSVKELLGITIMERSSFEKNPID
FLEAKGYKEVKKDLIIKLPKYSLFELENGRKRMLASAGELQKGNELALPSKYVNFLYLAS
HYEKLKGSPEDNEQKQLFVEQHKHYLDEIIEQISEFSKRVILADANLDKVLSAYNKHRDK
PIREQAENIIHLFTLTNLGAPAAFKYFDTTIDRKRYTSTKEVLDATLIHQSITGLYETRIDL
SQLGGDAYPYDVPDYASLGSGSPKKKRKVEDPKKKRKVDGGGPSSGAPPPSGGSPAGSP
TSTEEGTSESATPESGPGTSTEPSEGSAPGSPAGSPTSTEEGTSTEPSEGSAPGTSTEPSEMGP
MEIYKTVSAWKRQPVRVLSLFRNIDKVLKSLGFLESGSGSGGGTLKYVEDVTNVVR
RDVEKWGPFDLVYGSTQPLGSSCDRCPGWYMFQFHRILQYALPRQESQRPFFWIF
MDNLLLTEDDQETTTRFLQTEAVTLQDVRGRDYQNAMRVWSNIPGLKSKHAPLTP
KEEEYLQAQVRSRSKLDAPKVDLLVKNCLLPLREYFKYFSQNSLPLSSGNSNANSRGP
SFSSGLVPLSLRGSHMNHDQEFDPPKVYPPVPAEKRKPIRVLSLFDGIATGLLVLKDLG
IQVDRYIASEVCEDSITVGMVRHQGKIMYVGDVRSVTQKHIQEWGPFDLVIGGSPC
NDLSIVNPARKGLYEGTGRLFFEFYRLLHDARPKEGDDRPFFWLFENVVAMGVSD
KRDISRFLESNPVMIDAKEVSAAHRARYFWGNLPGMNRPLASTVNDKLELQECLEH
GRIAKFSKVRTITTRSNSIKQGKDQHFPVFMNEKEDILWCTEMERVFGFPVHYTDV
SNMSRLARQRLLGRSWSVPVIRHLFAPLKEYFACVDGSGSETPGTSESATPESMNNSQ
GRVTFEDVTVNFTQGEWQRLNPEQRNLYRDVMLENYSNLVSVGQGETTKPDVILRLEQ
GKEPWLEEEEVLGSGRAEKNGDIGGQIWKPKDVKESLLE*

The amino acid sequence of dCas9-XTEN80-DNMT3A-DNMT3L-XTEN16-KRAB (V24 scheme, SEQ ID NO: 75) is as follows:

MVPAAKRVKLDGSDKKYSIGLAIGTNSVGWAVITDEYKVPSKKFKVLGNTDRHSIKK
NLIGALLFDSGETAEATRLKRTARRRYTRRKNRICYLQEIFSNEMAKVDDSFFHRLEESF
LVEEDKKHERHPIFGNIVDEVAYHEKYPTIYHLRKKLVDSTDKADLRLIYLALAHMIKFR

GHFLIEGDLNPDNSDVDKLFIQLVQTYNQLFEENPINASGVDAKAILSARLSKSRRLENLI
AQLPGEKKNGLFGNLIALSLGLTPNFKSNFDLAEDAKLQLSKDTYDDDLDNLLAQIGDQ
YADLFLAAKNLSDAILLSDILRVNTEITKAPLSASMIKRYDEHHQDLTLLKALVRQQLPEK
YKEIFFDQSKNGYAGYIDGGASQEEFYKFIKPILEKMDGTEELLVKLNREDLLRKQRTFD
NGSIPHQIHLGELHAILRRQEDFYPFLKDNREKIEKILTFRIPYYVGPLARGNSRFAWMT
RKSEETITPWNFEEVVDKGASAQSFIERMTNFDKNLPNEKVLPKHSLLYEYFTVYNELT
KVKYVTEGMRKPAFLSGEQKKAIVDLLFKTNRKVTVKQLKEDYFKKIECFDSVEISGVED
RFNASLGTYHDLLKIIKDKDFLDNEENEDILEDIVLTLTLFEDREMIEERLKTYAHLFDD
KVMKQLKRRRYTGWGRLSRKLINGIRDKQSGKTILDFLKSDGFANRNFMQLIHDDSLTF
KEDIQKAQVSGQGDSLHEHIANLAGSPAIKKGILQTVKVVDELVKVMGRHKPENIVIEM
ARENQTTQKGQKNSRERMKRIEEGIKELGSQILKEHPVENTQLQNEKLYLYYLQNGRDM
YVDQELDINRLSDYDVDAIVPQSFLKDDSIDNKVLTRSDKNRGKSDNVPSEEVVKKMKN
YWRQLLNAKLITQRKFDNLTKAERGGLSELDKAGFIKRQLVETRQITKHVAQILDSRMN
TKYDENDKLIREVKVITLKSKLVSDFRKDFQFYKVREINNYHHAHDAYLNAVVGTALIKK
YPKLESEFVYGDYKVYDVRKMIAKSEQEIGKATAKYFFYSNIMNFFKTEITLANGEIRKR
PLIETNGETGEIVWDKGRDFATVRKVLSMPQVNIVKKTEVQTGGFSKESILPKRNSDKLI
ARKKDWDPKKYGGFDSPTVAYSVLVVAKVEKGKSKKLKSVKELLGITIMERSSFEKNPID
FLEAKGYKEVKKDLIIKLPKYSLFELENGRKRMLASAGELQKGNELALPSKYVNFLYLAS
HYEKLKGSPEDNEQKQLFVEQHKHYLDEIIEQISEFSKRVILADANLDKVLSAYNKHRDK
PIREQAENIIHLFTLTNLGAPAAFKYFDTTIDRKRYTSTKEVLDATLIHQSITGLYETRIDL
SQLGGDAYPYDVPDYASLGSGSPKKKRKVEDPKKKRKVDGGGPSSGAPPPSGGSPAGSP
TSTEEGTSESATPESGPGTSTEPSEGSAPGSPAGSPTSTEEGTSTEPSEGSAPGTSTEPSEMNHD
QEFDPPKVYPPVPAEKRKPIRVLSLFDGIATGLLVLKDLGIQVDRYIASEVCEDSITV
GMVRHQGKIMYVGDVRSVTQKHIQEWGPFDLVIGGSPCNDLSIVNPARKGLYEGT
GRLFFEFYRLLHDARPKEGDDRPFFWLFENVVAMGVSDKRDISRFLESNPVMIDAK
EVSAAHRARYFWGNLPGMNRPLASTVNDKLELQECLEHGRIAKFSKVRTITTRSNS
IKQGKDQHFPVFMNEKEDILWCTEMERVFGFPVHYTDVSNMSRLARQRLLGRSWS

VPVIRHLFAPLKEYFACVSSGNSNANSRGPSFSSGLVPLSLRGSHMGPMEIYKTVSAWK
RQPVRVLSLFRNIDKVLKSLGFLESGSGSGGGTLKYVEDVTNVVRRDVEKWGPFDL
VYGSTQPLGSSCDRCPGWYMFQFHRILQYALPRQESQRPFFWIFMDNLLLTEDDQE
TTTRFLQTEAVTLQDVRGRDYQNAMRVWSNIPGLKSKHAPLTPKEEEYLQAQVRS
RSKLDAPKVDLLVKNCLLPLREYFKYFSQNSLPLDGSGSETPGTSESATPESRTLVTFK
DVFVDFTREEWKLLDTAQQIVYRNVMLENYKNLVSLGYQLTKPDVILRLEKGEEP*

The amino acid sequence of dCas9-XTEN80-DNMT3L-DNMT3A-XTEN16-KRAB (V25 scheme, SEQ ID NO: 76) is as follows:

MVPAAKRVKLDGSDKKYSIGLAIGTNSVGWAVITDEYKVPSKKFKVLGNTDRHSIKK
NLIGALLFDSGETAEATRLKRTARRRYTRRKNRICYLQEIFSNEMAKVDDSFFHRLEESF
LVEEDKKHERHPIFGNIVDEVAYHEKYPTIYHLRKKLVDSTDKADLRLIYLALAHMIKFR
GHFLIEGDLNPDNSDVDKLFIQLVQTYNQLFEENPINASGVDAKAILSARLSKSRRLENLI
AQLPGEKKNGLFGNLIALSLGLTPNFKSNFDLAEDAKLQLSKDTYDDDLDNLLAQIGDQ
YADLFLAAKNLSDAILLSDILRVNTEITKAPLSASMIKRYDEHHQDLTLLKALVRQQLPEK
YKEIFFDQSKNGYAGYIDGGASQEEFYKFIKPILEKMDGTEELLVKLNREDLLRKQRTFD
NGSIPHQIHLGELHAILRRQEDFYPFLKDNREKIEKILTFRIPYYVGPLARGNSRFAWMT
RKSEETITPWNFEEVVDKGASAQSFIERMTNFDKNLPNEKVLPKHSLLYEYFTVYNELT
KVKYVTEGMRKPAFLSGEQKKAIVDLLFKTNRKVTVKQLKEDYFKKIECFDSVEISGVED
RFNASLGTYHDLLKIIKDKDFLDNEENEDILEDIVLTLTLFEDREMIEERLKTYAHLFDD
KVMKQLKRRRYTGWGRLSRKLINGIRDKQSGKTILDFLKSDGFANRNFMQLIHDDSLTF
KEDIQKAQVSGQGDSLHEHIANLAGSPAIKKGILQTVKVVDELVKVMGRHKPENIVIEM
ARENQTTQKGQKNSRERMKRIEEGIKELGSQILKEHPVENTQLQNEKLYLYYLQNGRDM
YVDQELDINRLSDYDVDAIVPQSFLKDDSIDNKVLTRSDKNRGKSDNVPSEEVVKKMKN
YWRQLLNAKLITQRKFDNLTKAERGGLSELDKAGFIKRQLVETRQITKHVAQILDSRMN
TKYDENDKLIREVKVITLKSKLVSDFRKDFQFYKVREINNYHHAHDAYLNAVVGTALIKK
YPKLESEFVYGDYKVYDVRKMIAKSEQEIGKATAKYFFYSNIMNFFKTEITLANGEIRKR
PLIETNGETGEIVWDKGRDFATVRKVLSMPQVNIVKKTEVQTGGFSKESILPKRNSDKLI

ARKKDWDPKKYGGFDSPTVAYSVLVVAKVEKGKSKKLKSVKELLGITIMERSSFEKNPID
FLEAKGYKEVKKDLIIKLPKYSLFELENGRKRMLASAGELQKGNELALPSKYVNFLYLAS
HYEKLKGSPEDNEQKQLFVEQHKHYLDEIIEQISEFSKRVILADANLDKVLSAYNKHRDK
PIREQAENIIHLFTLTNLGAPAAFKYFDTTIDRKRYTSTKEVLDATLIHQSITGLYETRIDL
SQLGGDAYPYDVPDYASLGSGSPKKKRKVEDPKKKRKVDGGGPSSGAPPPSGGSPAGSP
TSTEEGTSESATPESGPGTSTEPSEGSAPGSPAGSPTSTEEGTSTEPSEGSAPGTSTEPSEMGP
MEIYKTVSAWKRQPVRVLSLFRNIDKVLKSLGFLESGSGSGGGTLKYVEDVTNVVR
RDVEKWGPFDLVYGSTQPLGSSCDRCPGWYMFQFHRILQYALPRQESQRPFFWIF
MDNLLLTEDDQETTTRFLQTEAVTLQDVRGRDYQNAMRVWSNIPGLKSKHAPLTP
KEEEYLQAQVRSRSKLDAPKVDLLVKNCLLPLREYFKYFSQNSLPLSSGNSNANSRGP
SFSSGLVPLSLRGSHMNHDQEFDPPKVYPPVPAEKRKPIRVLSLFDGIATGLLVLKDLG
IQVDRYIASEVCEDSITVGMVRHQGKIMYVGDVRSVTQKHIQEWGPFDLVIGGSPC
NDLSIVNPARKGLYEGTGRLFFEFYRLLHDARPKEGDDRPFFWLFENVVAMGVSD
KRDISRFLESNPVMIDAKEVSAAHRARYFWGNLPGMNRPLASTVNDKLELQECLEH
GRIAKFSKVRTITTRSNSIKQGKDQHFPVFMNEKEDILWCTEMERVFGFPVHYTDV
SNMSRLARQRLLGRSWSVPVIRHLFAPLKEYFACVDGSGSETPGTSESATPESRTLVTF
KDVFVDFTREEWKLLDTAQQIVYRNVMLENYKNLVSLGYQLTKPDVILRLEKGEEP*

The amino acid sequence of dCas9-XTEN80-KRAB-NEW XTEN-DNMT3L-DNMT3A (V26 scheme, SEQ ID NO: 77) is as follows:

MVPAAKRVKLDGSDKKYSIGLAIGTNSVGWAVITDEYKVPSKKFKVLGNTDRHSIKK
NLIGALLFDSGETAEATRLKRTARRRYTRRKNRICYLQEIFSNEMAKVDDSFFHRLEESF
LVEEDKKHERHPIFGNIVDEVAYHEKYPTIYHLRKKLVDSTDKADLRLIYLALAHMIKFR
GHFLIEGDLNPDNSDVDKLFIQLVQTYNQLFEENPINASGVDAKAILSARLSKSRRLENLI
AQLPGEKKNGLFGNLIALSLGLTPNFKSNFDLAEDAKLQLSKDTYDDDLDNLLAQIGDQ
YADLFLAAKNLSDAILLSDILRVNTEITKAPLSASMIKRYDEHHQDLTLLKALVRQQLPEK
YKEIFFDQSKNGYAGYIDGGASQEEFYKFIKPILEKMDGTEELLVKLNREDLLRKQRTFD
NGSIPHQIHLGELHAILRRQEDFYPFLKDNREKIEKILTFRIPYYVGPLARGNSRFAWMT

RKSEETITPWNFEEVVDKGASAQSFIERMTNFDKNLPNEKVLPKHSLLYEYFTVYNELT
KVKYVTEGMRKPAFLSGEQKKAIVDLLFKTNRKVTVKQLKEDYFKKIECFDSVEISGVED
RFNASLGTYHDLLKIIKDKDFLDNEENEDILEDIVLTLTLFEDREMIEERLKTYAHLFDD
KVMKQLKRRRYTGWGRLSRKLINGIRDKQSGKTILDFLKSDGFANRNFMQLIHDDSLTF
KEDIQKAQVSGQGDSLHEHIANLAGSPAIKKGILQTVKVVDELVKVMGRHKPENIVIEM
ARENQTTQKGQKNSRERMKRIEEGIKELGSQILKEHPVENTQLQNEKLYLYYLQNGRDM
YVDQELDINRLSDYDVDAIVPQSFLKDDSIDNKVLTRSDKNRGKSDNVPSEEVVKKMKN
YWRQLLNAKLITQRKFDNLTKAERGGLSELDKAGFIKRQLVETRQITKHVAQILDSRMN
TKYDENDKLIREVKVITLKSKLVSDFRKDFQFYKVREINNYHHAHDAYLNAVVGTALIKK
YPKLESEFVYGDYKVYDVRKMIAKSEQEIGKATAKYFFYSNIMNFFKTEITLANGEIRKR
PLIETNGETGEIVWDKGRDFATVRKVLSMPQVNIVKKTEVQTGGFSKESILPKRNSDKLI
ARKKDWDPKKYGGFDSPTVAYSVLVVAKVEKGKSKKLKSVKELLGITIMERSSFEKNPID
FLEAKGYKEVKKDLIIKLPKYSLFELENGRKRMLASAGELQKGNELALPSKYVNFLYLAS
HYEKLKGSPEDNEQKQLFVEQHKHYLDEIIEQISEFSKRVILADANLDKVLSAYNKHRDK
PIREQAENIIHLFTLTNLGAPAAFKYFDTTIDRKRYTSTKEVLDATLIHQSITGLYETRIDL
SQLGGDAYPYDVPDYASLGSGSPKKKRKVEDPKKKRKVDGGGPSSGAPPPSGGSPAGSP
TSTEEGTSESATPESGPGTSTEPSEGSAPGSPAGSPTSTEEGTSTEPSEGSAPGTSTEPSERTLVT
FKDVFVDFTREEWKLLDTAQQIVYRNVMLENYKNLVSLGYQLTKPDVILRLEKGEEPSG
SETPGTSESATPESGPGSPAGSPTSTEEGTSESATPESGPGSEPATSGSETPGTSESATPESGPGT
STEPSEGSAPGTSTEPSESATPESMGPMEIYKTVSAWKRQPVRVLSLFRNIDKVLKSLGF
LESGSGSGGGTLKYVEDVTNVVRRDVEKWGPFDLVYGSTQPLGSSCDRCPGWYMF
QFHRILQYALPRQESQRPFFWIFMDNLLLTEDDQETTTRFLQTEAVTLQDVRGRDY
QNAMRVWSNIPGLKSKHAPLTPKEEEYLQAQVRSRSKLDAPKVDLLVKNCLLPLR
EYFKYFSQNSLPLSSGNSNANSRGPSFSSGLVPLSLRGSHMNHDQEFDPPKVYPPVPAEK
RKPIRVLSLFDGIATGLLVLKDLGIQVDRYIASEVCEDSITVGMVRHQGKIMYVGDV
RSVTQKHIQEWGPFDLVIGGSPCNDLSIVNPARKGLYEGTGRLFFEFYRLLHDARP
KEGDDRPFFWLFENVVAMGVSDKRDISRFLESNPVMIDAKEVSAAHRARYFWGNL

PGMNRPLASTVNDKLELQECLEHGRIAKFSKVRTITTRSNSIKQGKDQHFPVFMNE
KEDILWCTEMERVFGFPVHYTDVSNMSRLARQRLLGRSWSVPVIRHLFAPLKEYFA
CV*

The amino acid sequence of dCas9-XTEN80-KRAB-NEW XTEN-DNMT3A-DNMT3L (V27 scheme, SEQ ID NO: 78) is as follows:

MVPAAKRVKLDGSDKKYSIGLAIGTNSVGWAVITDEYKVPSKKFKVLGNTDRHSIKK
NLIGALLFDSGETAEATRLKRTARRRYTRRKNRICYLQEIFSNEMAKVDDSFFHRLEESF
LVEEDKKHERHPIFGNIVDEVAYHEKYPTIYHLRKKLVDSTDKADLRLIYLALAHMIKFR
GHFLIEGDLNPDNSDVDKLFIQLVQTYNQLFEENPINASGVDAKAILSARLSKSRRLENLI
AQLPGEKKNGLFGNLIALSLGLTPNFKSNFDLAEDAKLQLSKDTYDDDLDNLLAQIGDQ
YADLFLAAKNLSDAILLSDILRVNTEITKAPLSASMIKRYDEHHQDLTLLKALVRQQLPEK
YKEIFFDQSKNGYAGYIDGGASQEEFYKFIKPILEKMDGTEELLVKLNREDLLRKQRTFD
NGSIPHQIHLGELHAILRRQEDFYPFLKDNREKIEKILTFRIPYYVGPLARGNSRFAWMT
RKSEETITPWNFEEVVDKGASAQSFIERMTNFDKNLPNEKVLPKHSLLYEYFTVYNELT
KVKYVTEGMRKPAFLSGEQKKAIVDLLFKTNRKVTVKQLKEDYFKKIECFDSVEISGVED
RFNASLGTYHDLLKIIKDKDFLDNEENEDILEDIVLTLTLFEDREMIEERLKTYAHLFDD
KVMKQLKRRRYTGWGRLSRKLINGIRDKQSGKTILDFLKSDGFANRNFMQLIHDDSLTF
KEDIQKAQVSGQGDSLHEHIANLAGSPAIKKGILQTVKVVDELVKVMGRHKPENIVIEM
ARENQTTQKGQKNSRERMKRIEEGIKELGSQILKEHPVENTQLQNEKLYLYYLQNGRDM
YVDQELDINRLSDYDVDAIVPQSFLKDDSIDNKVLTRSDKNRGKSDNVPSEEVVKKMKN
YWRQLLNAKLITQRKFDNLTKAERGGLSELDKAGFIKRQLVETRQITKHVAQILDSRMN
TKYDENDKLIREVKVITLKSKLVSDFRKDFQFYKVREINNYHHAHDAYLNAVVGTALIKK
YPKLESEFVYGDYKVYDVRKMIAKSEQEIGKATAKYFFYSNIMNFFKTEITLANGEIRKR
PLIETNGETGEIVWDKGRDFATVRKVLSMPQVNIVKKTEVQTGGFSKESILPKRNSDKLI
ARKKDWDPKKYGGFDSPTVAYSVLVVAKVEKGKSKKLKSVKELLGITIMERSSFEKNPID
FLEAKGYKEVKKDLIIKLPKYSLFELENGRKRMLASAGELQKGNELALPSKYVNFLYLAS
HYEKLKGSPEDNEQKQLFVEQHKHYLDEIIEQISEFSKRVILADANLDKVLSAYNKHRDK

PIREQAENIIHLFTLTNLGAPAAFKYFDTTIDRKRYTSTKEVLDATLIHQSITGLYETRIDL
SQLGGDAYPYDVPDYASLGSGSPKKKRKVEDPKKKRKVDGGGPSSGAPPPSGGSPAGSP
TSTEEGTSESATPESGPGTSTEPSEGSAPGSPAGSPTSTEEGTSTEPSEGSAPGTSTEPSERTLVT
FKDVFVDFTREEWKLLDTAQQIVYRNVMLENYKNLVSLGYQLTKPDVILRLEKGEEPSG
SETPGTSESATPESGPGSPAGSPTSTEEGTSESATPESGPGSEPATSGSETPGTSESATPESGPGT
STEPSEGSAPGTSTEPSESATPESMNHDQEFDPPKVYPPVPAEKRKPIRVLSLFDGIATGL
LVLKDLGIQVDRYIASEVCEDSITVGMVRHQGKIMYVGDVRSVTQKHIQEWGPFDL
VIGGSPCNDLSIVNPARKGLYEGTGRLFFEFYRLLHDARPKEGDDRPFFWLFENVV
AMGVSDKRDISRFLESNPVMIDAKEVSAAHRARYFWGNLPGMNRPLASTVNDKLE
LQECLEHGRIAKFSKVRTITTRSNSIKQGKDQHFPVFMNEKEDILWCTEMERVFGF
PVHYTDVSNMSRLARQRLLGRSWSVPVIRHLFAPLKEYFACVSSGNSNANSRGPSFSS
GLVPLSLRGSHMGPMEIYKTVSAWKRQPVRVLSLFRNIDKVLKSLGFLESGSGSGGG
TLKYVEDVTNVVRRDVEKWGPFDLVYGSTQPLGSSCDRCPGWYMFQFHRILQYAL
PRQESQRPFFWIFMDNLLLTEDDQETTTRFLQTEAVTLQDVRGRDYQNAMRVWSN
IPGLKSKHAPLTPKEEEYLQAQVRSRSKLDAPKVDLLVKNCLLPLREYFKYFSQNSL
PL*

The amino acid sequence of dCas9-XTEN80-KRAB-XTEN80-(human)DNMT3L-DNMT3A (V28 scheme, SEQ ID NO: 79) is as follows:

MPKKKRKVMDKKYSIGLAIGTNSVGWAVITDEYKVPSKKFKVLGNTDRHSIKKNLIG
ALLFDSGETAEATRLKRTARRRYTRRKNRICYLQEIFSNEMAKVDDSFFHRLEESFLVEE
DKKHERHPIFGNIVDEVAYHEKYPTIYHLRKKLVDSTDKADLRLIYLALAHMIKFRGHFL
IEGDLNPDNSDVDKLFIQLVQTYNQLFEENPINASGVDAKAILSARLSKSRRLENLIAQLP
GEKKNGLFGNLIALSLGLTPNFKSNFDLAEDAKLQLSKDTYDDDLDNLLAQIGDQYADL
FLAAKNLSDAILLSDILRVNTEITKAPLSASMIKRYDEHHQDLTLLKALVRQQLPEKYKEI
FFDQSKNGYAGYIDGGASQEEFYKFIKPILEKMDGTEELLVKLNREDLLRKQRTFDNGSI
PHQIHLGELHAILRRQEDFYPFLKDNREKIEKILTFRIPYYVGPLARGNSRFAWMTRKSE
ETITPWNFEEVVDKGASAQSFIERMTNFDKNLPNEKVLPKHSLLYEYFTVYNELTKVKY

VTEGMRKPAFLSGEQKKAIVDLLFKTNRKVTVKQLKEDYFKKIECFDSVEISGVEDRFN
ASLGTYHDLLKIIKDKDFLDNEENEDILEDIVLTLTLFEDREMIEERLKTYAHLFDDKVM
KQLKRRRYTGWGRLSRKLINGIRDKQSGKTILDFLKSDGFANRNFMQLIHDDSLTFKEDI
QKAQVSGQGDSLHEHIANLAGSPAIKKGILQTVKVVDELVKVMGRHKPENIVIEMAREN
QTTQKGQKNSRERMKRIEEGIKELGSQILKEHPVENTQLQNEKLYLYYLQNGRDMYVDQ
ELDINRLSDYDVDAIVPQSFLKDDSIDNKVLTRSDKNRGKSDNVPSEEVVKKMKNYWRQ
LLNAKLITQRKFDNLTKAERGGLSELDKAGFIKRQLVETRQITKHVAQILDSRMNTKYDE
NDKLIREVKVITLKSKLVSDFRKDFQFYKVREINNYHHAHDAYLNAVVGTALIKKYPKLE
SEFVYGDYKVYDVRKMIAKSEQEIGKATAKYFFYSNIMNFFKTEITLANGEIRKRPLIETN
GETGEIVWDKGRDFATVRKVLSMPQVNIVKKTEVQTGGFSKESILPKRNSDKLIARKKD
WDPKKYGGFDSPTVAYSVLVVAKVEKGKSKKLKSVKELLGITIMERSSFEKNPIDFLEAK
GYKEVKKDLIIKLPKYSLFELENGRKRMLASAGELQKGNELALPSKYVNFLYLASHYEKL
KGSPEDNEQKQLFVEQHKHYLDEIIEQISEFSKRVILADANLDKVLSAYNKHRDKPIREQ
AENIIHLFTLTNLGAPAAFKYFDTTIDRKRYTSTKEVLDATLIHQSITGLYETRIDLSQLGG
DAYPYDVPDYASLGSGSPKKKRKVEDPKKKRKVDGGGPSSGAPPPSGGSPAGSPTSTEEG
TSESATPESGPGTSTEPSEGSAPGSPAGSPTSTEEGTSTEPSEGSAPGTSTEPSERTLVTFKDVF
VDFTREEWKLLDTAQQIVYRNVMLENYKNLVSLGYQLTKPDVILRLEKGEEPSGSETPGT
SEGGPSSGAPPPSGGSPAGSPTSTEEGTSESATPESGPGTSTEPSEGSAPGSPAGSPTSTEEGTS
TEPSEGSAPGTSTEPSESATPESMFETVPVWRRQPVRVLSLFEDIKKELTSLGFLESGSD
PGQLKHVVDVTDTVRKDVEEWGPFDLVYGATPPLGHTCDRPPSWYLFQFHRLLQ
YARPKPGSPRPFFWMFVDNLVLNKEDLDVASRFLEMEPVTIPDVHGGSLQNAVRV
WSNIPAIRSSRHWALVSEEELSLLAQNKQSSKLAAKWPTKLVKNCFLPLREYFKYF
STELTSSLSSGNSNANSRGPSFSSGLVPLSLRGSHMNHDQEFDPPKVYPPVPAEKRKPIRV
LSLFDGIATGLLVLKDLGIQVDRYIASEVCEDSITVGMVRHQGKIMYVGDVRSVTQ
KHIQEWGPFDLVIGGSPCNDLSIVNPARKGLYEGTGRLFFEFYRLLHDARPKEGDD
RPFFWLFENVVAMGVSDKRDISRFLESNPVMIDAKEVSAAHRARYFWGNLPGMNR

PLASTVNDKLELQECLEHGRIAKFSKVRTITTRSNSIKQGKDQHFPVFMNEKEDILW
CTEMERVFGFPVHYTDVSNMSRLARQRLLGRSWSVPVIRHLFAPLKEYFACVLE*

The amino acid sequence of dCas9-XTEN80-ZNF582-XTEN80-(human)DNMT3L-DNMT3A (V29 scheme, SEQ ID NO: 80) is as follows:

MPKKKRKVMDKKYSIGLAIGTNSVGWAVITDEYKVPSKKFKVLGNTDRHSIKKNLIG
ALLFDSGETAEATRLKRTARRRYTRRKNRICYLQEIFSNEMAKVDDSFFHRLEESFLVEE
DKKHERHPIFGNIVDEVAYHEKYPTIYHLRKKLVDSTDKADLRLIYLALAHMIKFRGHFL
IEGDLNPDNSDVDKLFIQLVQTYNQLFEENPINASGVDAKAILSARLSKSRRLENLIAQLP
GEKKNGLFGNLIALSLGLTPNFKSNFDLAEDAKLQLSKDTYDDDLDNLLAQIGDQYADL
FLAAKNLSDAILLSDILRVNTEITKAPLSASMIKRYDEHHQDLTLLKALVRQQLPEKYKEI
FFDQSKNGYAGYIDGGASQEEFYKFIKPILEKMDGTEELLVKLNREDLLRKQRTFDNGSI
PHQIHLGELHAILRRQEDFYPFLKDNREKIEKILTFRIPYYVGPLARGNSRFAWMTRKSE
ETITPWNFEEVVDKGASAQSFIERMTNFDKNLPNEKVLPKHSLLYEYFTVYNELTKVKY
VTEGMRKPAFLSGEQKKAIVDLLFKTNRKVTVKQLKEDYFKKIECFDSVEISGVEDRFN
ASLGTYHDLLKIIKDKDFLDNEENEDILEDIVLTLTLFEDREMIEERLKTYAHLFDDKVM
KQLKRRRYTGWGRLSRKLINGIRDKQSGKTILDFLKSDGFANRNFMQLIHDDSLTFKEDI
QKAQVSGQGDSLHEHIANLAGSPAIKKGILQTVKVVDELVKVMGRHKPENIVIEMAREN
QTTQKGQKNSRERMKRIEEGIKELGSQILKEHPVENTQLQNEKLYLYYLQNGRDMYVDQ
ELDINRLSDYDVDAIVPQSFLKDDSIDNKVLTRSDKNRGKSDNVPSEEVVKKMKNYWRQ
LLNAKLITQRKFDNLTKAERGGLSELDKAGFIKRQLVETRQITKHVAQILDSRMNTKYDE
NDKLIREVKVITLKSKLVSDFRKDFQFYKVREINNYHHAHDAYLNAVVGTALIKKYPKLE
SEFVYGDYKVYDVRKMIAKSEQEIGKATAKYFFYSNIMNFFKTEITLANGEIRKRPLIETN
GETGEIVWDKGRDFATVRKVLSMPQVNIVKKTEVQTGGFSKESILPKRNSDKLIARKKD
WDPKKYGGFDSPTVAYSVLVVAKVEKGKSKKLKSVKELLGITIMERSSFEKNPIDFLEAK
GYKEVKKDLIIKLPKYSLFELENGRKRMLASAGELQKGNELALPSKYVNFLYLASHYEKL
KGSPEDNEQKQLFVEQHKHYLDEIIEQISEFSKRVILADANLDKVLSAYNKHRDKPIREQ
AENIIHLFTLTNLGAPAAFKYFDTTIDRKRYTSTKEVLDATLIHQSITGLYETRIDLSQLGG

DAYPYDVPDYASLGSGSPKKKRKVEDPKKKRKVDGGGPSSGAPPPSGGSPAGSPTSTEEG
TSESATPESGPGTSTEPSEGSAPGSPAGSPTSTEEGTSTEPSEGSAPGTSTEPSERTLELFRDVA
IVFSQEEWQWLAPAQRDLYRDVMLETYSNLVSLGLAVSKPDVISFLEQGKEPWMSGSET
PGTSEGGPSSGAPPPSGGSPAGSPTSTEEGTSESATPESGPGTSTEPSEGSAPGSPAGSPTSTEE
GTSTEPSEGSAPGTSTEPSESATPESMFETVPVWRRQPVRVLSLFEDIKKELTSLGFLES
GSDPGQLKHVVDVTDTVRKDVEEWGPFDLVYGATPPLGHTCDRPPSWYLFQFHRL
LQYARPKPGSPRPFFWMFVDNLVLNKEDLDVASRFLEMEPVTIPDVHGGSLQNAVR
VWSNIPAIRSSRHWALVSEEELSLLAQNKQSSKLAAKWPTKLVKNCFLPLREYFKY
FSTELTSSLSSGNSNANSRGPSFSSGLVPLSLRGSHMNHDQEFDPPKVYPPVPAEKRKPIR
VLSLFDGIATGLLVLKDLGIQVDRYIASEVCEDSITVGMVRHQGKIMYVGDVRSVT
QKHIQEWGPFDLVIGGSPCNDLSIVNPARKGLYEGTGRLFFEFYRLLHDARPKEGD
DRPFFWLFENVVAMGVSDKRDISRFLESNPVMIDAKEVSAAHRARYFWGNLPGMN
RPLASTVNDKLELQECLEHGRIAKFSKVRTITTRSNSIKQGKDQHFPVFMNEKEDIL
WCTEMERVFGFPVHYTDVSNMSRLARQRLLGRSWSVPVIRHLFAPLKEYFACVLE*

The amino acid sequence of dCas9-XTEN80-ZNF324-XTEN80-(human)DNMT3L-DNMT3A (V30 scheme, SEQ ID NO: 81) is as follows:

MPKKKRKVMDKKYSIGLAIGTNSVGWAVITDEYKVPSKKFKVLGNTDRHSIKKNLIG
ALLFDSGETAEATRLKRTARRRYTRRKNRICYLQEIFSNEMAKVDDSFFHRLEESFLVEE
DKKHERHPIFGNIVDEVAYHEKYPTIYHLRKKLVDSTDKADLRLIYLALAHMIKFRGHFL
IEGDLNPDNSDVDKLFIQLVQTYNQLFEENPINASGVDAKAILSARLSKSRRLENLIAQLP
GEKKNGLFGNLIALSLGLTPNFKSNFDLAEDAKLQLSKDTYDDDLDNLLAQIGDQYADL
FLAAKNLSDAILLSDILRVNTEITKAPLSASMIKRYDEHHQDLTLLKALVRQQLPEKYKEI
FFDQSKNGYAGYIDGGASQEEFYKFIKPILEKMDGTEELLVKLNREDLLRKQRTFDNGSI
PHQIHLGELHAILRRQEDFYPFLKDNREKIEKILTFRIPYYVGPLARGNSRFAWMTRKSE
ETITPWNFEEVVDKGASAQSFIERMTNFDKNLPNEKVLPKHSLLYEYFTVYNELTKVKY
VTEGMRKPAFLSGEQKKAIVDLLFKTNRKVTVKQLKEDYFKKIECFDSVEISGVEDRFN
ASLGTYHDLLKIIKDKDFLDNEENEDILEDIVLTLTLFEDREMIEERLKTYAHLFDDKVM

KQLKRRRYTGWGRLSRKLINGIRDKQSGKTILDFLKSDGFANRNFMQLIHDDSLTFKEDI
QKAQVSGQGDSLHEHIANLAGSPAIKKGILQTVKVVDELVKVMGRHKPENIVIEMAREN
QTTQKGQKNSRERMKRIEEGIKELGSQILKEHPVENTQLQNEKLYLYYLQNGRDMYVDQ
ELDINRLSDYDVDAIVPQSFLKDDSIDNKVLTRSDKNRGKSDNVPSEEVVKKMKNYWRQ
LLNAKLITQRKFDNLTKAERGGLSELDKAGFIKRQLVETRQITKHVAQILDSRMNTKYDE
NDKLIREVKVITLKSKLVSDFRKDFQFYKVREINNYHHAHDAYLNAVVGTALIKKYPKLE
SEFVYGDYKVYDVRKMIAKSEQEIGKATAKYFFYSNIMNFFKTEITLANGEIRKRPLIETN
GETGEIVWDKGRDFATVRKVLSMPQVNIVKKTEVQTGGFSKESILPKRNSDKLIARKKD
WDPKKYGGFDSPTVAYSVLVVAKVEKGKSKKLKSVKELLGITIMERSSFEKNPIDFLEAK
GYKEVKKDLIIKLPKYSLFELENGRKRMLASAGELQKGNELALPSKYVNFLYLASHYEKL
KGSPEDNEQKQLFVEQHKHYLDEIIEQISEFSKRVILADANLDKVLSAYNKHRDKPIREQ
AENIIHLFTLTNLGAPAAFKYFDTTIDRKRYTSTKEVLDATLIHQSITGLYETRIDLSQLGG
DAYPYDVPDYASLGSGSPKKKRKVEDPKKKRKVDGGGPSSGAPPPSGGSPAGSPTSTEEG
TSESATPESGPGTSTEPSEGSAPGSPAGSPTSTEEGTSTEPSEGSAPGTSTEPSERTLMAFEDV
AVYFSQEEWGLLDTAQRALYRRVMLDNFALVASLGLSTSRPRVVIQLERGEEPWVSGSE
TPGTSEGGPSSGAPPPSGGSPAGSPTSTEEGTSESATPESGPGTSTEPSEGSAPGSPAGSPTSTE
EGTSTEPSEGSAPGTSTEPSESATPESMFETVPVWRRQPVRVLSLFEDIKKELTSLGFLES
GSDPGQLKHVVDVTDTVRKDVEEWGPFDLVYGATPPLGHTCDRPPSWYLFQFHRL
LQYARPKPGSPRPFFWMFVDNLVLNKEDLDVASRFLEMEPVTIPDVHGGSLQNAVR
VWSNIPAIRSSRHWALVSEEELSLLAQNKQSSKLAAKWPTKLVKNCFLPLREYFKY
FSTELTSSLSSGNSNANSRGPSFSSGLVPLSLRGSHMNHDQEFDPPKVYPPVPAEKRKPIR
VLSLFDGIATGLLVLKDLGIQVDRYIASEVCEDSITVGMVRHQGKIMYVGDVRSVT
QKHIQEWGPFDLVIGGSPCNDLSIVNPARKGLYEGTGRLFFEFYRLLHDARPKEGD
DRPFFWLFENVVAMGVSDKRDISRFLESNPVMIDAKEVSAAHRARYFWGNLPGMN
RPLASTVNDKLELQECLEHGRIAKFSKVRTITTRSNSIKQGKDQHFPVFMNEKEDIL
WCTEMERVFGFPVHYTDVSNMSRLARQRLLGRSWSVPVIRHLFAPLKEYFACVLE*

The amino acid sequence of dCas9-NEW XTEN-KRAB-NEW XTEN-(human)DNMT3L-DNMT3A (V31 scheme, SEQ ID NO: 82) is as follows:

MPKKKRKVMDKKYSIGLAIGTNSVGWAVITDEYKVPSKKFKVLGNTDRHSIKKNLIG
ALLFDSGETAEATRLKRTARRRYTRRKNRICYLQEIFSNEMAKVDDSFFHRLEESFLVEE
DKKHERHPIFGNIVDEVAYHEKYPTIYHLRKKLVDSTDKADLRLIYLALAHMIKFRGHFL
IEGDLNPDNSDVDKLFIQLVQTYNQLFEENPINASGVDAKAILSARLSKSRRLENLIAQLP
GEKKNGLFGNLIALSLGLTPNFKSNFDLAEDAKLQLSKDTYDDDLDNLLAQIGDQYADL
FLAAKNLSDAILLSDILRVNTEITKAPLSASMIKRYDEHHQDLTLLKALVRQQLPEKYKEI
FFDQSKNGYAGYIDGGASQEEFYKFIKPILEKMDGTEELLVKLNREDLLRKQRTFDNGSI
PHQIHLGELHAILRRQEDFYPFLKDNREKIEKILTFRIPYYVGPLARGNSRFAWMTRKSE
ETITPWNFEEVVDKGASAQSFIERMTNFDKNLPNEKVLPKHSLLYEYFTVYNELTKVKY
VTEGMRKPAFLSGEQKKAIVDLLFKTNRKVTVKQLKEDYFKKIECFDSVEISGVEDRFN
ASLGTYHDLLKIIKDKDFLDNEENEDILEDIVLTLTLFEDREMIEERLKTYAHLFDDKVM
KQLKRRRYTGWGRLSRKLINGIRDKQSGKTILDFLKSDGFANRNFMQLIHDDSLTFKEDI
QKAQVSGQGDSLHEHIANLAGSPAIKKGILQTVKVVDELVKVMGRHKPENIVIEMAREN
QTTQKGQKNSRERMKRIEEGIKELGSQILKEHPVENTQLQNEKLYLYYLQNGRDMYVDQ
ELDINRLSDYDVDAIVPQSFLKDDSIDNKVLTRSDKNRGKSDNVPSEEVVKKMKNYWRQ
LLNAKLITQRKFDNLTKAERGGLSELDKAGFIKRQLVETRQITKHVAQILDSRMNTKYDE
NDKLIREVKVITLKSKLVSDFRKDFQFYKVREINNYHHAHDAYLNAVVGTALIKKYPKLE
SEFVYGDYKVYDVRKMIAKSEQEIGKATAKYFFYSNIMNFFKTEITLANGEIRKRPLIETN
GETGEIVWDKGRDFATVRKVLSMPQVNIVKKTEVQTGGFSKESILPKRNSDKLIARKKD
WDPKKYGGFDSPTVAYSVLVVAKVEKGKSKKLKSVKELLGITIMERSSFEKNPIDFLEAK
GYKEVKKDLIIKLPKYSLFELENGRKRMLASAGELQKGNELALPSKYVNFLYLASHYEKL
KGSPEDNEQKQLFVEQHKHYLDEIIEQISEFSKRVILADANLDKVLSAYNKHRDKPIREQ
AENIIHLFTLTNLGAPAAFKYFDTTIDRKRYTSTKEVLDATLIHQSITGLYETRIDLSQLGG
DAYPYDVPDYASLGSGSPKKKRKVEDPKKKRKVDGGGPSSGAPPPSGGSPAGSPTSTEEG
TSESATPESGPGTSTEPSEGSAPGSPAGSPTSTEEGTSTEPSEGSAPGTSTEPSEGSGSETPGTSE

SATPESRTLVTFKDVFVDFTREEWKLLDTAQQIVYRNVMLENYKNLVSLGYQLTKPDVIL
RLEKGEEPSGSETPGTSESATPESGPGSPAGSPTSTEEGTSESATPESGPGSEPATSGSETPGTS
ESATPESGPGTSTEPSEGSAPGTSTEPSESATPESMFETVPVWRRQPVRVLSLFEDIKKEL
TSLGFLESGSDPGQLKHVVDVTDTVRKDVEEWGPFDLVYGATPPLGHTCDRPPSW
YLFQFHRLLQYARPKPGSPRPFFWMFVDNLVLNKEDLDVASRFLEMEPVTIPDVHG
GSLQNAVRVWSNIPAIRSSRHWALVSEEELSLLAQNKQSSKLAAKWPTKLVKNCFL
PLREYFKYFSTELTSSLSSGNSNANSRGPSFSSGLVPLSLRGSHMNHDQEFDPPKVYPPVP
AEKRKPIRVLSLFDGIATGLLVLKDLGIQVDRYIASEVCEDSITVGMVRHQGKIMYV
GDVRSVTQKHIQEWGPFDLVIGGSPCNDLSIVNPARKGLYEGTGRLFFEFYRLLHD
ARPKEGDDRPFFWLFENVVAMGVSDKRDISRFLESNPVMIDAKEVSAAHRARYFW
GNLPGMNRPLASTVNDKLELQECLEHGRIAKFSKVRTITTRSNSIKQGKDQHFPVF
MNEKEDILWCTEMERVFGFPVHYTDVSNMSRLARQRLLGRSWSVPVIRHLFAPLK
EYFACVLE*

The amino acid sequence of dCas9-NEW XTEN-(human)DNMT3L-DNMT3A-NEW XTEN-KRAB (V32 scheme, SEQ ID NO: 83) is as follows:

MVPAAKRVKLDGSDKKYSIGLAIGTNSVGWAVITDEYKVPSKKFKVLGNTDRHSIKK
NLIGALLFDSGETAEATRLKRTARRRYTRRKNRICYLQEIFSNEMAKVDDSFFHRLEESF
LVEEDKKHERHPIFGNIVDEVAYHEKYPTIYHLRKKLVDSTDKADLRLIYLALAHMIKFR
GHFLIEGDLNPDNSDVDKLFIQLVQTYNQLFEENPINASGVDAKAILSARLSKSRRLENLI
AQLPGEKKNGLFGNLIALSLGLTPNFKSNFDLAEDAKLQLSKDTYDDDLDNLLAQIGDQ
YADLFLAAKNLSDAILLSDILRVNTEITKAPLSASMIKRYDEHHQDLTLLKALVRQQLPEK
YKEIFFDQSKNGYAGYIDGGASQEEFYKFIKPILEKMDGTEELLVKLNREDLLRKQRTFD
NGSIPHQIHLGELHAILRRQEDFYPFLKDNREKIEKILTFRIPYYVGPLARGNSRFAWMT
RKSEETITPWNFEEVVDKGASAQSFIERMTNFDKNLPNEKVLPKHSLLYEYFTVYNELT
KVKYVTEGMRKPAFLSGEQKKAIVDLLFKTNRKVTVKQLKEDYFKKIECFDSVEISGVED
RFNASLGTYHDLLKIIKDKDFLDNEENEDILEDIVLTLTLFEDREMIEERLKTYAHLFDD
KVMKQLKRRRYTGWGRLSRKLINGIRDKQSGKTILDFLKSDGFANRNFMQLIHDDSLTF

KEDIQKAQVSGQGDSLHEHIANLAGSPAIKKGILQTVKVVDELVKVMGRHKPENIVIEM
ARENQTTQKGQKNSRERMKRIEEGIKELGSQILKEHPVENTQLQNEKLYLYYLQNGRDM
YVDQELDINRLSDYDVDAIVPQSFLKDDSIDNKVLTRSDKNRGKSDNVPSEEVVKKMKN
YWRQLLNAKLITQRKFDNLTKAERGGLSELDKAGFIKRQLVETRQITKHVAQILDSRMN
TKYDENDKLIREVKVITLKSKLVSDFRKDFQFYKVREINNYHHAHDAYLNAVVGTALIKK
YPKLESEFVYGDYKVYDVRKMIAKSEQEIGKATAKYFFYSNIMNFFKTEITLANGEIRKR
PLIETNGETGEIVWDKGRDFATVRKVLSMPQVNIVKKTEVQTGGFSKESILPKRNSDKLI
ARKKDWDPKKYGGFDSPTVAYSVLVVAKVEKGKSKKLKSVKELLGITIMERSSFEKNPID
FLEAKGYKEVKKDLIIKLPKYSLFELENGRKRMLASAGELQKGNELALPSKYVNFLYLAS
HYEKLKGSPEDNEQKQLFVEQHKHYLDEIIEQISEFSKRVILADANLDKVLSAYNKHRDK
PIREQAENIIHLFTLTNLGAPAAFKYFDTTIDRKRYTSTKEVLDATLIHQSITGLYETRIDL
SQLGGDAYPYDVPDYASLGSGSPKKKRKVEDPKKKRKVDGGGPSSGAPPPSGGSPAGSP
TSTEEGTSESATPESGPGTSTEPSEGSAPGSPAGSPTSTEEGTSTEPSEGSAPGTSTEPSEGSGSE
TPGTSESATPESMFETVPVWRRQPVRVLSLFEDIKKELTSLGFLESGSDPGQLKHVVD
VTDTVRKDVEEWGPFDLVYGATPPLGHTCDRPPSWYLFQFHRLLQYARPKPGSPR
PFFWMFVDNLVLNKEDLDVASRFLEMEPVTIPDVHGGSLQNAVRVWSNIPAIRSSR
HWALVSEEELSLLAQNKQSSKLAAKWPTKLVKNCFLPLREYFKYFSTELTSSLSSGN
SNANSRGPSFSSGLVPLSLRGSHMNHDQEFDPPKVYPPVPAEKRKPIRVLSLFDGIATGL
LVLKDLGIQVDRYIASEVCEDSITVGMVRHQGKIMYVGDVRSVTQKHIQEWGPFDL
VIGGSPCNDLSIVNPARKGLYEGTGRLFFEFYRLLHDARPKEGDDRPFFWLFENVV
AMGVSDKRDISRFLESNPVMIDAKEVSAAHRARYFWGNLPGMNRPLASTVNDKLE
LQECLEHGRIAKFSKVRTITTRSNSIKQGKDQHFPVFMNEKEDILWCTEMERVFGF
PVHYTDVSNMSRLARQRLLGRSWSVPVIRHLFAPLKEYFACVSGSETPGTSESATPES
GPGSPAGSPTSTEEGTSESATPESGPGSEPATSGSETPGTSESATPESGPGTSTEPSEGSAPGTS
TEPSESATPESRTLVTFKDVFVDFTREEWKLLDTAQQIVYRNVMLENYKNLVSLGYQLTK
PDVILRLEKGEEPLE*

The amino acid sequence of dCas9-XTEN80-ZIM3-XTEN80-DNMT3A-(human)DNMT3L (V33 scheme, SEQ ID NO: 84) is as follows:

MPKKKRKVMDKKYSIGLAIGTNSVGWAVITDEYKVPSKKFKVLGNTDRHSIKKNLIG
ALLFDSGETAEATRLKRTARRRYTRRKNRICYLQEIFSNEMAKVDDSFFHRLEESFLVEE
DKKHERHPIFGNIVDEVAYHEKYPTIYHLRKKLVDSTDKADLRLIYLALAHMIKFRGHFL
IEGDLNPDNSDVDKLFIQLVQTYNQLFEENPINASGVDAKAILSARLSKSRRLENLIAQLP
GEKKNGLFGNLIALSLGLTPNFKSNFDLAEDAKLQLSKDTYDDDLDNLLAQIGDQYADL
FLAAKNLSDAILLSDILRVNTEITKAPLSASMIKRYDEHHQDLTLLKALVRQQLPEKYKEI
FFDQSKNGYAGYIDGGASQEEFYKFIKPILEKMDGTEELLVKLNREDLLRKQRTFDNGSI
PHQIHLGELHAILRRQEDFYPFLKDNREKIEKILTFRIPYYVGPLARGNSRFAWMTRKSE
ETITPWNFEEVVDKGASAQSFIERMTNFDKNLPNEKVLPKHSLLYEYFTVYNELTKVKY
VTEGMRKPAFLSGEQKKAIVDLLFKTNRKVTVKQLKEDYFKKIECFDSVEISGVEDRFN
ASLGTYHDLLKIIKDKDFLDNEENEDILEDIVLTLTLFEDREMIEERLKTYAHLFDDKVM
KQLKRRRYTGWGRLSRKLINGIRDKQSGKTILDFLKSDGFANRNFMQLIHDDSLTFKEDI
QKAQVSGQGDSLHEHIANLAGSPAIKKGILQTVKVVDELVKVMGRHKPENIVIEMAREN
QTTQKGQKNSRERMKRIEEGIKELGSQILKEHPVENTQLQNEKLYLYYLQNGRDMYVDQ
ELDINRLSDYDVDAIVPQSFLKDDSIDNKVLTRSDKNRGKSDNVPSEEVVKKMKNYWRQ
LLNAKLITQRKFDNLTKAERGGLSELDKAGFIKRQLVETRQITKHVAQILDSRMNTKYDE
NDKLIREVKVITLKSKLVSDFRKDFQFYKVREINNYHHAHDAYLNAVVGTALIKKYPKLE
SEFVYGDYKVYDVRKMIAKSEQEIGKATAKYFFYSNIMNFFKTEITLANGEIRKRPLIETN
GETGEIVWDKGRDFATVRKVLSMPQVNIVKKTEVQTGGFSKESILPKRNSDKLIARKKD
WDPKKYGGFDSPTVAYSVLVVAKVEKGKSKKLKSVKELLGITIMERSSFEKNPIDFLEAK
GYKEVKKDLIIKLPKYSLFELENGRKRMLASAGELQKGNELALPSKYVNFLYLASHYEKL
KGSPEDNEQKQLFVEQHKHYLDEIIEQISEFSKRVILADANLDKVLSAYNKHRDKPIREQ
AENIIHLFTLTNLGAPAAFKYFDTTIDRKRYTSTKEVLDATLIHQSITGLYETRIDLSQLGG
DAYPYDVPDYASLGSGSPKKKRKVEDPKKKRKVDGGGPSSGAPPPSGGSPAGSPTSTEEG
TSESATPESGPGTSTEPSEGSAPGSPAGSPTSTEEGTSTEPSEGSAPGTSTEPSEMNNSQGRVT

FEDVTVNFTQGEWQRLNPEQRNLYRDVMLENYSNLVSVGQGETTKPDVILRLEQGKEP
WLEEEEVLGSGRAEKNGDIGGQIWKPKDVKESLSGSETPGTSEGGPSSGAPPPSGGSPAGS
PTSTEEGTSESATPESGPGTSTEPSEGSAPGSPAGSPTSTEEGTSTEPSEGSAPGTSTEPSESATP
ESGSNHDQEFDPPKVYPPVPAEKRKPIRVLSLFDGIATGLLVLKDLGIQVDRYIASEV
CEDSITVGMVRHQGKIMYVGDVRSVTQKHIQEWGPFDLVIGGSPCNDLSIVNPARK
GLYEGTGRLFFEFYRLLHDARPKEGDDRPFFWLFENVVAMGVSDKRDISRFLESNP
VMIDAKEVSAAHRARYFWGNLPGMNRPLASTVNDKLELQECLEHGRIAKFSKVRT
ITTRSNSIKQGKDQHFPVFMNEKEDILWCTEMERVFGFPVHYTDVSNMSRLARQRL
LGRSWSVPVIRHLFAPLKEYFACVSSGNSNANSRGPSFSSGLVPLSLRGSHMFETVPVW
RRQPVRVLSLFEDIKKELTSLGFLESGSDPGQLKHVVDVTDTVRKDVEEWGPFDLV
YGATPPLGHTCDRPPSWYLFQFHRLLQYARPKPGSPRPFFWMFVDNLVLNKEDLD
VASRFLEMEPVTIPDVHGGSLQNAVRVWSNIPAIRSSRHWALVSEEELSLLAQNKQS
SKLAAKWPTKLVKNCFLPLREYFKYFSMH*

The amino acid sequence of dCas9-BFP-ZIM3-XTEN80-DNMT3A-(human)DNMT3L (V34 scheme, SEQ ID NO: 85) is as follows (underlined dashed bold italic-BFP, SEQ ID NO: 55):

MPKKKRKVMDKKYSIGLAIGTNSVGWAVITDEYKVPSKKFKVLGNTDRHSIKKNLIG
ALLFDSGETAEATRLKRTARRRYTRRKNRICYLQEIFSNEMAKVDDSFFHRLEESFLVEE
DKKHERHPIFGNIVDEVAYHEKYPTIYHLRKKLVDSTDKADLRLIYLALAHMIKFRGHFL
IEGDLNPDNSDVDKLFIQLVQTYNQLFEENPINASGVDAKAILSARLSKSRRLENLIAQLP
GEKKNGLFGNLIALSLGLTPNFKSNFDLAEDAKLQLSKDTYDDDLDNLLAQIGDQYADL
FLAAKNLSDAILLSDILRVNTEITKAPLSASMIKRYDEHHQDLTLLKALVRQQLPEKYKEI
FFDQSKNGYAGYIDGGASQEEFYKFIKPILEKMDGTEELLVKLNREDLLRKQRTFDNGSI
PHQIHLGELHAILRRQEDFYPFLKDNREKIEKILTFRIPYYVGPLARGNSRFAWMTRKSE
ETITPWNFEEVVDKGASAQSFIERMTNFDKNLPNEKVLPKHSLLYEYFTVYNELTKVKY
VTEGMRKPAFLSGEQKKAIVDLLFKTNRKVTVKQLKEDYFKKIECFDSVEISGVEDRFN
ASLGTYHDLLKIIKDKDFLDNEENEDILEDIVLTLTLFEDREMIEERLKTYAHLFDDKVM
KQLKRRRYTGWGRLSRKLINGIRDKQSGKTILDFLKSDGFANRNFMQLIHDDSLTFKEDI

QKAQVSGQGDSLHEHIANLAGSPAIKKGILQTVKVVDELVKVMGRHKPENIVIEMAREN
QTTQKGQKNSRERMKRIEEGIKELGSQILKEHPVENTQLQNEKLYLYYLQNGRDMYVDQ
ELDINRLSDYDVDAIVPQSFLKDDSIDNKVLTRSDKNRGKSDNVPSEEVVKKMKNYWRQ
LLNAKLITQRKFDNLTKAERGGLSELDKAGFIKRQLVETRQITKHVAQILDSRMNTKYDE
NDKLIREVKVITLKSKLVSDFRKDFQFYKVREINNYHHAHDAYLNAVVGTALIKKYPKLE
SEFVYGDYKVYDVRKMIAKSEQEIGKATAKYFFYSNIMNFFKTEITLANGEIRKRPLIETN
GETGEIVWDKGRDFATVRKVLSMPQVNIVKKTEVQTGGFSKESILPKRNSDKLIARKKD
WDPKKYGGFDSPTVAYSVLVVAKVEKGKSKKLKSVKELLGITIMERSSFEKNPIDFLEAK
GYKEVKKDLIIKLPKYSLFELENGRKRMLASAGELQKGNELALPSKYVNFLYLASHYEKL
KGSPEDNEQKQLFVEQHKHYLDEIIEQISEFSKRVILADANLDKVLSAYNKHRDKPIREQ
AENIIHLFTLTNLGAPAAFKYFDTTIDRKRYTSTKEVLDATLIHQSITGLYETRIDLSQLGG
DAYPYDVPDYASLGSGSPKKKRKVEDPKKKRKVDGGGPSSGAPPPSGGSPAGSPTSTEEG
TSESATPESGPGTHVSELIKENMHMKLYMEGTVDNHHFKCTSEGEGKPYEGTQTMRIKV
VEGGPLPFAFDILATSFLYGSKTFINHTQGIPDFFKQSFPEGFTWERVTTYEDGGVLTAT
QDTSLQDGCLIYNVKIRGVNFTSNGPVMQKKTLGWEAFTETLYPADGGLEGRNDMALK
LVGGSHLIANIKTTYRSKKPAKNLKMPGVYYVDYRLERIKEANNETYVEQHEVAVARYC
DLPSKLGHKLNGSAPGTSTEPSEMNNSQGRVTFEDVTVNFTQGEWQRLNPEQRNLYRDV
MLENYSNLVSVGQGETTKPDVILRLEQGKEPWLEEEEVLGSGRAEKNGDIGGQIWKPK
DVKESLSGSETPGTSEGGPSSGAPPPSGGSPAGSPTSTEEGTSESATPESGPGTSTEPSEGSAP
GSPAGSPTSTEEGTSTEPSEGSAPGTSTEPSESATPESGSNHDQEFDPPKVYPPVPAEKRKP
IRVLSLFDGIATGLLVLKDLGIQVDRYIASEVCEDSITVGMVRHQGKIMYVGDVRSV
TQKHIQEWGPFDLVIGGSPCNDLSIVNPARKGLYEGTGRLFFEFYRLLHDARPKEG
DDRPFFWLFENVVAMGVSDKRDISRFLESNPVMIDAKEVSAAHRARYFWGNLPGM
NRPLASTVNDKLELQECLEHGRIAKFSKVRTITTRSNSIKQGKDQHFPVFMNEKEDI
LWCTEMERVFGFPVHYTDVSNMSRLARQRLLGRSWSVPVIRHLFAPLKEYFACVSS
GNSNANSRGPSFSSGLVPLSLRGSHMFETVPVWRRQPVRVLSLFEDIKKELTSLGFLES
GSDPGQLKHVVDVTDTVRKDVEEWGPFDLVYGATPPLGHTCDRPPSWYLFQFHRL

LQYARPKPGSPRPFFWMFVDNLVLNKEDLDVASRFLEMEPVTIPDVHGGSLQNAVR

VWSNIPAIRSSRHWALVSEEELSLLAQNKQSSKLAAKWPTKLVKNCFLPLREYFKY

FSMH*

The amino acid sequence of dCas9-BFP-ZIM3-XTEN80-(human)DNMT3L-DNMT3A (V35 scheme, SEQ ID NO: 86) is as follows (underlined dashed bold italic-BFP, SEQ ID NO: 55):

MPKKKRKVMDKKYSIGLAIGTNSVGWAVITDEYKVPSKKFKVLGNTDRHSIKKNLIG
ALLFDSGETAEATRLKRTARRRYTRRKNRICYLQEIFSNEMAKVDDSFFHRLEESFLVEE
DKKHERHPIFGNIVDEVAYHEKYPTIYHLRKKLVDSTDKADLRLIYLALAHMIKFRGHFL
IEGDLNPDNSDVDKLFIQLVQTYNQLFEENPINASGVDAKAILSARLSKSRRLENLIAQLP
GEKKNGLFGNLIALSLGLTPNFKSNFDLAEDAKLQLSKDTYDDDLDNLLAQIGDQYADL
FLAAKNLSDAILLSDILRVNTEITKAPLSASMIKRYDEHHQDLTLLKALVRQQLPEKYKEI
FFDQSKNGYAGYIDGGASQEEFYKFIKPILEKMDGTEELLVKLNREDLLRKQRTFDNGSI
PHQIHLGELHAILRRQEDFYPFLKDNREKIEKILTFRIPYYVGPLARGNSRFAWMTRKSE
ETITPWNFEEVVDKGASAQSFIERMTNFDKNLPNEKVLPKHSLLYEYFTVYNELTKVKY
VTEGMRKPAFLSGEQKKAIVDLLFKTNRKVTVKQLKEDYFKKIECFDSVEISGVEDRFN
ASLGTYHDLLKIIKDKDFLDNEENEDILEDIVLTLTLFEDREMIEERLKTYAHLFDDKVM
KQLKRRRYTGWGRLSRKLINGIRDKQSGKTILDFLKSDGFANRNFMQLIHDDSLTFKEDI
QKAQVSGQGDSLHEHIANLAGSPAIKKGILQTVKVVDELVKVMGRHKPENIVIEMAREN
QTTQKGQKNSRERMKRIEEGIKELGSQILKEHPVENTQLQNEKLYLYYLQNGRDMYVDQ
ELDINRLSDYDVDAIVPQSFLKDDSIDNKVLTRSDKNRGKSDNVPSEEVVKKMKNYWRQ
LLNAKLITQRKFDNLTKAERGGLSELDKAGFIKRQLVETRQITKHVAQILDSRMNTKYDE
NDKLIREVKVITLKSKLVSDFRKDFQFYKVREINNYHHAHDAYLNAVVGTALIKKYPKLE
SEFVYGDYKVYDVRKMIAKSEQEIGKATAKYFFYSNIMNFFKTEITLANGEIRKRPLIETN
GETGEIVWDKGRDFATVRKVLSMPQVNIVKKTEVQTGGFSKESILPKRNSDKLIARKKD
WDPKKYGGFDSPTVAYSVLVVAKVEKGKSKKLKSVKELLGITIMERSSFEKNPIDFLEAK
GYKEVKKDLIIKLPKYSLFELENGRKRMLASAGELQKGNELALPSKYVNFLYLASHYEKL
KGSPEDNEQKQLFVEQHKHYLDEIIEQISEFSKRVILADANLDKVLSAYNKHRDKPIREQ

AENIIHLFTLTNLGAPAAFKYFDTTIDRKRYTSTKEVLDATLIHQSITGLYETRIDLSQLGG
DAYPYDVPDYASLGSGSPKKKRKVEDPKKKRKVDGGGPSSGAPPPSGGSPAGSPTSTEEG
TSESATPESGPGTHVSELIKENMHMKLYMEGTVDNHHFKCTSEGEGKPYEGTQTMRIKV
VEGGPLPFAFDILATSFLYGSKTFINHTQGIPDFFKQSFPEGFTWERVTTYEDGGVLTAT
QDTSLQDGCLIYNVKIRGVNFTSNGPVMQKKTLGWEAFTETLYPADGGLEGRNDMALK
LVGGSHLIANIKTTYRSKKPAKNLKMPGVYYVDYRLERIKEANNETYVEQHEVAVARYC
DLPSKLGHKLNGSAPGTSTEPSEMNNSQGRVTFEDVTVNFTQGEWQRLNPEQRNLYRDV
MLENYSNLVSVGQGETTKPDVILRLEQGKEPWLEEEEVLGSGRAEKNGDIGGQIWKPK
DVKESLSGSETPGTSEGGPSSGAPPPSGGSPAGSPTSTEEGTSESATPESGPGTSTEPSEGSAP
GSPAGSPTSTEEGTSTEPSEGSAPGTSTEPSESATPESMFETVPVWRRQPVRVLSLFEDIKK
ELTSLGFLESGSDPGQLKHVVDVTDTVRKDVEEWGPFDLVYGATPPLGHTCDRPPS
WYLFQFHRLLQYARPKPGSPRPFFWMFVDNLVLNKEDLDVASRFLEMEPVTIPDV
HGGSLQNAVRVWSNIPAIRSSRHWALVSEEELSLLAQNKQSSKLAAKWPTKLVKN
CFLPLREYFKYFSTELTSSLSSGNSNANSRGPSFSSGLVPLSLRGSHMNHDQEFDPPKVYP
PVPAEKRKPIRVLSLFDGIATGLLVLKDLGIQVDRYIASEVCEDSITVGMVRHQGKI
MYVGDVRSVTQKHIQEWGPFDLVIGGSPCNDLSIVNPARKGLYEGTGRLFFEFYRL
LHDARPKEGDDRPFFWLFENVVAMGVSDKRDISRFLESNPVMIDAKEVSAAHRARY
FWGNLPGMNRPLASTVNDKLELQECLEHGRIAKFSKVRTITTRSNSIKQGKDQHFP
VFMNEKEDILWCTEMERVFGFPVHYTDVSNMSRLARQRLLGRSWSVPVIRHLFAP
LKEYFACVLE*

The amino acid sequence of dCas9-BFP-ZNF582-XTEN80-(human)DNMT3L-DNMT3A (V36 scheme, SEQ ID NO: 87) is as follows (underlined dashed bold italic-BFP, SEQ ID NO: 55):

MPKKKRKVMDKKYSIGLAIGTNSVGWAVITDEYKVPSKKFKVLGNTDRHSIKKNLIG
ALLFDSGETAEATRLKRTARRRYTRRKNRICYLQEIFSNEMAKVDDSFFHRLEESFLVEE
DKKHERHPIFGNIVDEVAYHEKYPTIYHLRKKLVDSTDKADLRLIYLALAHMIKFRGHFL
IEGDLNPDNSDVDKLFIQLVQTYNQLFEENPINASGVDAKAILSARLSKSRRLENLIAQLP

GEKKNGLFGNLIALSLGLTPNFKSNFDLAEDAKLQLSKDTYDDDLDNLLAQIGDQYADL
FLAAKNLSDAILLSDILRVNTEITKAPLSASMIKRYDEHHQDLTLLKALVRQQLPEKYKEI
FFDQSKNGYAGYIDGGASQEEFYKFIKPILEKMDGTEELLVKLNREDLLRKQRTFDNGSI
PHQIHLGELHAILRRQEDFYPFLKDNREKIEKILTFRIPYYVGPLARGNSRFAWMTRKSE
ETITPWNFEEVVDKGASAQSFIERMTNFDKNLPNEKVLPKHSLLYEYFTVYNELTKVKY
VTEGMRKPAFLSGEQKKAIVDLLFKTNRKVTVKQLKEDYFKKIECFDSVEISGVEDRFN
ASLGTYHDLLKIIKDKDFLDNEENEDILEDIVLTLTLFEDREMIEERLKTYAHLFDDKVM
KQLKRRRYTGWGRLSRKLINGIRDKQSGKTILDFLKSDGFANRNFMQLIHDDSLTFKEDI
QKAQVSGQGDSLHEHIANLAGSPAIKKGILQTVKVVDELVKVMGRHKPENIVIEMAREN
QTTQKGQKNSRERMKRIEEGIKELGSQILKEHPVENTQLQNEKLYLYYLQNGRDMYVDQ
ELDINRLSDYDVDAIVPQSFLKDDSIDNKVLTRSDKNRGKSDNVPSEEVVKKMKNYWRQ
LLNAKLITQRKFDNLTKAERGGLSELDKAGFIKRQLVETRQITKHVAQILDSRMNTKYDE
NDKLIREVKVITLKSKLVSDFRKDFQFYKVREINNYHHAHDAYLNAVVGTALIKKYPKLE
SEFVYGDYKVYDVRKMIAKSEQEIGKATAKYFFYSNIMNFFKTEITLANGEIRKRPLIETN
GETGEIVWDKGRDFATVRKVLSMPQVNIVKKTEVQTGGFSKESILPKRNSDKLIARKKD
WDPKKYGGFDSPTVAYSVLVVAKVEKGKSKKLKSVKELLGITIMERSSFEKNPIDFLEAK
GYKEVKKDLIIKLPKYSLFELENGRKRMLASAGELQKGNELALPSKYVNFLYLASHYEKL
KGSPEDNEQKQLFVEQHKHYLDEIIEQISEFSKRVILADANLDKVLSAYNKHRDKPIREQ
AENIIHLFTLTNLGAPAAFKYFDTTIDRKRYTSTKEVLDATLIHQSITGLYETRIDLSQLGG
DAYPYDVPDYASLGSGSPKKKRKVEDPKKKRKVDGGGPSSGAPPPSGGSPAGSPTSTEEG
TSESATPESGPGTHVSELIKENMHMKLYMEGTVDNHHFKCTSEGEGKPYEGTQTMRIKV
VEGGPLPFAFDILATSFLYGSKTFINHTQGIPDFFKQSFPEGFTWERVTTYEDGGVLTAT
QDTSLQDGCLIYNVKIRGVNFTSNGPVMQKKTLGWEAFTETLYPADGGLEGRNDMALK
LVGGSHLIANIKTTYRSKKPAKNLKMPGVYYVDYRLERIKEANNETYVEQHEVAVARYC
DLPSKLGHKLNGSAPGTSTEPSERTLELFRDVAIVFSQEEWQWLAPAQRDLYRDVMLET
YSNLVSLGLAVSKPDVISFLEQGKEPWMSGSETPGTSEGGPSSGAPPPSGGSPAGSPTSTEE
GTSESATPESGPGTSTEPSEGSAPGSPAGSPTSTEEGTSTEPSEGSAPGTSTEPSESATPESMFE

TVPVWRRQPVRVLSLFEDIKKELTSLGFLESGSDPGQLKHVVDVTDTVRKDVEEW
GPFDLVYGATPPLGHTCDRPPSWYLFQFHRLLQYARPKPGSPRPFFWMFVDNLVL
NKEDLDVASRFLEMEPVTIPDVHGGSLQNAVRVWSNIPAIRSSRHWALVSEEELSLL
AQNKQSSKLAAKWPTKLVKNCFLPLREYFKYFSTELTSSLSSGNSNANSRGPSFSSGLV
PLSLRGSHMNHDQEFDPPKVYPPVPAEKRKPIRVLSLFDGIATGLLVLKDLGIQVDRY
IASEVCEDSITVGMVRHQGKIMYVGDVRSVTQKHIQEWGPFDLVIGGSPCNDLSIVN
PARKGLYEGTGRLFFEFYRLLHDARPKEGDDRPFFWLFENVVAMGVSDKRDISRFL
ESNPVMIDAKEVSAAHRARYFWGNLPGMNRPLASTVNDKLELQECLEHGRIAKFS
KVRTITTRSNSIKQGKDQHFPVFMNEKEDILWCTEMERVFGFPVHYTDVSNMSRLA
RQRLLGRSWSVPVIRHLFAPLKEYFACVLE*

The amino acid sequence of dCas9-BFP-ZNF324-XTEN80-(human)DNMT3L-DNMT3A (V37 scheme, SEQ ID NO: 88) is as follows (underlined dashed bold italic-BFP, SEQ ID NO: 55):

MPKKKRKVMDKKYSIGLAIGTNSVGWAVITDEYKVPSKKFKVLGNTDRHSIKKNLIG
ALLFDSGETAEATRLKRTARRRYTRRKNRICYLQEIFSNEMAKVDDSFFHRLEESFLVEE
DKKHERHPIFGNIVDEVAYHEKYPTIYHLRKKLVDSTDKADLRLIYLALAHMIKFRGHFL
IEGDLNPDNSDVDKLFIQLVQTYNQLFEENPINASGVDAKAILSARLSKSRRLENLIAQLP
GEKKNGLFGNLIALSLGLTPNFKSNFDLAEDAKLQLSKDTYDDDLDNLLAQIGDQYADL
FLAAKNLSDAILLSDILRVNTEITKAPLSASMIKRYDEHHQDLTLLKALVRQQLPEKYKEI
FFDQSKNGYAGYIDGGASQEEFYKFIKPILEKMDGTEELLVKLNREDLLRKQRTFDNGSI
PHQIHLGELHAILRRQEDFYPFLKDNREKIEKILTFRIPYYVGPLARGNSRFAWMTRKSE
ETITPWNFEEVVDKGASAQSFIERMTNFDKNLPNEKVLPKHSLLYEYFTVYNELTKVKY
VTEGMRKPAFLSGEQKKAIVDLLFKTNRKVTVKQLKEDYFKKIECFDSVEISGVEDRFN
ASLGTYHDLLKIIKDKDFLDNEENEDILEDIVLTLTLFEDREMIEERLKTYAHLFDDKVM
KQLKRRRYTGWGRLSRKLINGIRDKQSGKTILDFLKSDGFANRNFMQLIHDDSLTFKEDI
QKAQVSGQGDSLHEHIANLAGSPAIKKGILQTVKVVDELVKVMGRHKPENIVIEMAREN
QTTQKGQKNSRERMKRIEEGIKELGSQILKEHPVENTQLQNEKLYLYYLQNGRDMYVDQ

ELDINRLSDYDVDAIVPQSFLKDDSIDNKVLTRSDKNRGKSDNVPSEEVVKKMKNYWRQ
LLNAKLITQRKFDNLTKAERGGLSELDKAGFIKRQLVETRQITKHVAQILDSRMNTKYDE
NDKLIREVKVITLKSKLVSDFRKDFQFYKVREINNYHHAHDAYLNAVVGTALIKKYPKLE
SEFVYGDYKVYDVRKMIAKSEQEIGKATAKYFFYSNIMNFFKTEITLANGEIRKRPLIETN
GETGEIVWDKGRDFATVRKVLSMPQVNIVKKTEVQTGGFSKESILPKRNSDKLIARKKD
WDPKKYGGFDSPTVAYSVLVVAKVEKGKSKKLKSVKELLGITIMERSSFEKNPIDFLEAK
GYKEVKKDLIIKLPKYSLFELENGRKRMLASAGELQKGNELALPSKYVNFLYLASHYEKL
KGSPEDNEQKQLFVEQHKHYLDEIIEQISEFSKRVILADANLDKVLSAYNKHRDKPIREQ
AENIIHLFTLTNLGAPAAFKYFDTTIDRKRYTSTKEVLDATLIHQSITGLYETRIDLSQLGG
DAYPYDVPDYASLGSGSPKKKRKVEDPKKKRKVDGGGPSSGAPPPSGGSPAGSPTSTEEG
TSESATPESGPGTHVSELIKENMHMKLYMEGTVDNHHFKCTSEGEGKPYEGTQTMRIKV
VEGGPLPFAFDILATSFLYGSKTFINHTQGIPDFFKQSFPEGFTWERVTTYEDGGVLTAT
QDTSLQDGCLIYNVKIRGVNFTSNGPVMQKKTLGWEAFTETLYPADGGLEGRNDMALK
LVGGSHLIANIKTTYRSKKPAKNLKMPGVYYVDYRLERIKEANNETYVEQHEVAVARYC
DLPSKLGHKLNGSAPGTSTEPSERTLMAFEDVAVYFSQEEWGLLDTAQRALYRRVMLDN
FALVASLGLSTSRPRVVIQLERGEEPWVSGSETPGTSEGGPSSGAPPPSGGSPAGSPTSTEE
GTSESATPESGPGTSTEPSEGSAPGSPAGSPTSTEEGTSTEPSEGSAPGTSTEPSESATPESMFE
TVPVWRRQPVRVLSLFEDIKKELTSLGFLESGSDPGQLKHVVDVTDTVRKDVEEW
GPFDLVYGATPPLGHTCDRPPSWYLFQFHRLLQYARPKPGSPRPFFWMFVDNLVL
NKEDLDVASRFLEMEPVTIPDVHGGSLQNAVRVWSNIPAIRSSRHWALVSEEELSLL
AQNKQSSKLAAKWPTKLVKNCFLPLREYFKYFSTELTSSLSSGNSNANSRGPSFSSGLV
PLSLRGSHMNHDQEFDPPKVYPPVPAEKRKPIRVLSLFDGIATGLLVLKDLGIQVDRY
IASEVCEDSITVGMVRHQGKIMYVGDVRSVTQKHIQEWGPFDLVIGGSPCNDLSIVN
PARKGLYEGTGRLFFEFYRLLHDARPKEGDDRPFFWLFENVVAMGVSDKRDISRFL
ESNPVMIDAKEVSAAHRARYFWGNLPGMNRPLASTVNDKLELQECLEHGRIAKFS
KVRTITTRSNSIKQGKDQHFPVFMNEKEDILWCTEMERVFGFPVHYTDVSNMSRLA
RQRLLGRSWSVPVIRHLFAPLKEYFACVLE*

The amino acid sequence of dCas9-XTEN80-DNMT3L-DNMT3A-XTEN16-KRAB-XTEN16-KRAB (V50 scheme, SEQ ID NO: 101) is as follows:

MVPAAKRVKLDGSDKKYSIGLAIGTNSVGWAVITDEYKVPSKKFKVLGNTDRHSIKK
NLIGALLFDSGETAEATRLKRTARRRYTRRKNRICYLQEIFSNEMAKVDDSFFHRLEESF
LVEEDKKHERHPIFGNIVDEVAYHEKYPTIYHLRKKLVDSTDKADLRLIYLALAHMIKFR
GHFLIEGDLNPDNSDVDKLFIQLVQTYNQLFEENPINASGVDAKAILSARLSKSRRLENLI
AQLPGEKKNGLFGNLIALSLGLTPNFKSNFDLAEDAKLQLSKDTYDDDLDNLLAQIGDQ
YADLFLAAKNLSDAILLSDILRVNTEITKAPLSASMIKRYDEHHQDLTLLKALVRQQLPEK
YKEIFFDQSKNGYAGYIDGGASQEEFYKFIKPILEKMDGTEELLVKLNREDLLRKQRTFD
NGSIPHQIHLGELHAILRRQEDFYPFLKDNREKIEKILTFRIPYYVGPLARGNSRFAWMT
RKSEETITPWNFEEVVDKGASAQSFIERMTNFDKNLPNEKVLPKHSLLYEYFTVYNELT
KVKYVTEGMRKPAFLSGEQKKAIVDLLFKTNRKVTVKQLKEDYFKKIECFDSVEISGVED
RFNASLGTYHDLLKIIKDKDFLDNEENEDILEDIVLTLTLFEDREMIEERLKTYAHLFDD
KVMKQLKRRRYTGWGRLSRKLINGIRDKQSGKTILDFLKSDGFANRNFMQLIHDDSLTF
KEDIQKAQVSGQGDSLHEHIANLAGSPAIKKGILQTVKVVDELVKVMGRHKPENIVIEM
ARENQTTQKGQKNSRERMKRIEEGIKELGSQILKEHPVENTQLQNEKLYLYYLQNGRDM
YVDQELDINRLSDYDVDAIVPQSFLKDDSIDNKVLTRSDKNRGKSDNVPSEEVVKKMKN
YWRQLLNAKLITQRKFDNLTKAERGGLSELDKAGFIKRQLVETRQITKHVAQILDSRMN
TKYDENDKLIREVKVITLKSKLVSDFRKDFQFYKVREINNYHHAHDAYLNAVVGTALIKK
YPKLESEFVYGDYKVYDVRKMIAKSEQEIGKATAKYFFYSNIMNFFKTEITLANGEIRKR
PLIETNGETGEIVWDKGRDFATVRKVLSMPQVNIVKKTEVQTGGFSKESILPKRNSDKLI
ARKKDWDPKKYGGFDSPTVAYSVLVVAKVEKGKSKKLKSVKELLGITIMERSSFEKNPID
FLEAKGYKEVKKDLIIKLPKYSLFELENGRKRMLASAGELQKGNELALPSKYVNFLYLAS
HYEKLKGSPEDNEQKQLFVEQHKHYLDEIIEQISEFSKRVILADANLDKVLSAYNKHRDK
PIREQAENIIHLFTLTNLGAPAAFKYFDTTIDRKRYTSTKEVLDATLIHQSITGLYETRIDL
SQLGGDAYPYDVPDYASLGSGSPKKKRKVEDPKKKRKVDGGGPSSGAPPPSGGSPAGSP
TSTEEGTSESATPESGPGTSTEPSEGSAPGSPAGSPTSTEEGTSTEPSEGSAPGTSTEPSEMGP

MEIYKTVSAWKRQPVRVLSLFRNIDKVLKSLGFLESGSGSGGGTLKYVEDVTNVVR
RDVEKWGPFDLVYGSTQPLGSSCDRCPGWYMFQFHRILQYALPRQESQRPFFWIF
MDNLLLTEDDQETTTRFLQTEAVTLQDVRGRDYQNAMRVWSNIPGLKSKHAPLTP
KEEEYLQAQVRSRSKLDAPKVDLLVKNCLLPLREYFKYFSQNSLPLSSGNSNANSRGP
SFSSGLVPLSLRGSHMNHDQEFDPPKVYPPVPAEKRKPIRVLSLFDGIATGLLVLKDLG
IQVDRYIASEVCEDSITVGMVRHQGKIMYVGDVRSVTQKHIQEWGPFDLVIGGSPC
NDLSIVNPARKGLYEGTGRLFFEFYRLLHDARPKEGDDRPFFWLFENVVAMGVSD
KRDISRFLESNPVMIDAKEVSAAHRARYFWGNLPGMNRPLASTVNDKLELQECLEH
GRIAKFSKVRTITTRSNSIKQGKDQHFPVFMNEKEDILWCTEMERVFGFPVHYTDV
SNMSRLARQRLLGRSWSVPVIRHLFAPLKEYFACVDGSGSETPGTSESATPESRTLVTF
KDVFVDFTREEWKLLDTAQQIVYRNVMLENYKNLVSLGYQLTKPDVILRLEKGEEPGSG
SETPGTSESATPESRTLVTFKDVFVDFTREEWKLLDTAQQIVYRNVMLENYKNLVSLGYQ
LTKPDVILRLEKGEEP*

The amino acid sequence of dCas9-XTEN80-DNMT3L-DNMT3A-XTEN16-KRAB-XTEN16-KRAB-XTEN16-KRAB (V51 scheme, SEQ ID NO: 102) is as follows:

MVPAAKRVKLDGSDKKYSIGLAIGTNSVGWAVITDEYKVPSKKFKVLGNTDRHSIKK
NLIGALLFDSGETAEATRLKRTARRRYTRRKNRICYLQEIFSNEMAKVDDSFFHRLEESF
LVEEDKKHERHPIFGNIVDEVAYHEKYPTIYHLRKKLVDSTDKADLRLIYLALAHMIKFR
GHFLIEGDLNPDNSDVDKLFIQLVQTYNQLFEENPINASGVDAKAILSARLSKSRRLENLI
AQLPGEKKNGLFGNLIALSLGLTPNFKSNFDLAEDAKLQLSKDTYDDDLDNLLAQIGDQ
YADLFLAAKNLSDAILLSDILRVNTEITKAPLSASMIKRYDEHHQDLTLLKALVRQQLPEK
YKEIFFDQSKNGYAGYIDGGASQEEFYKFIKPILEKMDGTEELLVKLNREDLLRKQRTFD
NGSIPHQIHLGELHAILRRQEDFYPFLKDNREKIEKILTFRIPYYVGPLARGNSRFAWMT
RKSEETITPWNFEEVVDKGASAQSFIERMTNFDKNLPNEKVLPKHSLLYEYFTVYNELT
KVKYVTEGMRKPAFLSGEQKKAIVDLLFKTNRKVTVKQLKEDYFKKIECFDSVEISGVED
RFNASLGTYHDLLKIIKDKDFLDNEENEDILEDIVLTLTLFEDREMIEERLKTYAHLFDD
KVMKQLKRRRYTGWGRLSRKLINGIRDKQSGKTILDFLKSDGFANRNFMQLIHDDSLTF

KEDIQKAQVSGQGDSLHEHIANLAGSPAIKKGILQTVKVVDELVKVMGRHKPENIVIEM
ARENQTTQKGQKNSRERMKRIEEGIKELGSQILKEHPVENTQLQNEKLYLYYLQNGRDM
YVDQELDINRLSDYDVDAIVPQSFLKDDSIDNKVLTRSDKNRGKSDNVPSEEVVKKMKN
YWRQLLNAKLITQRKFDNLTKAERGGLSELDKAGFIKRQLVETRQITKHVAQILDSRMN
TKYDENDKLIREVKVITLKSKLVSDFRKDFQFYKVREINNYHHAHDAYLNAVVGTALIKK
YPKLESEFVYGDYKVYDVRKMIAKSEQEIGKATAKYFFYSNIMNFFKTEITLANGEIRKR
PLIETNGETGEIVWDKGRDFATVRKVLSMPQVNIVKKTEVQTGGFSKESILPKRNSDKLI
ARKKDWDPKKYGGFDSPTVAYSVLVVAKVEKGKSKKLKSVKELLGITIMERSSFEKNPID
FLEAKGYKEVKKDLIIKLPKYSLFELENGRKRMLASAGELQKGNELALPSKYVNFLYLAS
HYEKLKGSPEDNEQKQLFVEQHKHYLDEIIEQISEFSKRVILADANLDKVLSAYNKHRDK
PIREQAENIIHLFTLTNLGAPAAFKYFDTTIDRKRYTSTKEVLDATLIHQSITGLYETRIDL
SQLGGDAYPYDVPDYASLGSGSPKKKRKVEDPKKKRKVDGGGPSSGAPPPSGGSPAGSP
TSTEEGTSESATPESGPGTSTEPSEGSAPGSPAGSPTSTEEGTSTEPSEGSAPGTSTEPSEMGP
MEIYKTVSAWKRQPVRVLSLFRNIDKVLKSLGFLESGSGSGGGTLKYVEDVTNVVR
RDVEKWGPFDLVYGSTQPLGSSCDRCPGWYMFQFHRILQYALPRQESQRPFFWIF
MDNLLLTEDDQETTTRFLQTEAVTLQDVRGRDYQNAMRVWSNIPGLKSKHAPLTP
KEEEYLQAQVRSRSKLDAPKVDLLVKNCLLPLREYFKYFSQNSLPLSSGNSNANSRGP
SFSSGLVPLSLRGSHMNHDQEFDPPKVYPPVPAEKRKPIRVLSLFDGIATGLLVLKDLG
IQVDRYIASEVCEDSITVGMVRHQGKIMYVGDVRSVTQKHIQEWGPFDLVIGGSPC
NDLSIVNPARKGLYEGTGRLFFEFYRLLHDARPKEGDDRPFFWLFENVVAMGVSD
KRDISRFLESNPVMIDAKEVSAAHRARYFWGNLPGMNRPLASTVNDKLELQECLEH
GRIAKFSKVRTITTRSNSIKQGKDQHFPVFMNEKEDILWCTEMERVFGFPVHYTDV
SNMSRLARQRLLGRSWSVPVIRHLFAPLKEYFACVDGSGSETPGTSESATPESRTLVTF
KDVFVDFTREEWKLLDTAQQIVYRNVMLENYKNLVSLGYQLTKPDVILRLEKGEEPGSG
SETPGTSESATPESRTLVTFKDVFVDFTREEWKLLDTAQQIVYRNVMLENYKNLVSLGYQ
LTKPDVILRLEKGEEPGSGSETPGTSESATPESRTLVTFKDVFVDFTREEWKLLDTAQQIV
YRNVMLENYKNLVSLGYQLTKPDVILRLEKGEEP*

The amino acid sequence of dCas9-XTEN80-KRAB-NEW XTEN-DNMT3L-DNMT3A-XTEN16-KRAB (V52 scheme, SEQ ID NO: 103) is as follows:

MVPAAKRVKLDGSDKKYSIGLAIGTNSVGWAVITDEYKVPSKKFKVLGNTDRHSIKK
NLIGALLFDSGETAEATRLKRTARRRYTRRKNRICYLQEIFSNEMAKVDDSFFHRLEESF
LVEEDKKHERHPIFGNIVDEVAYHEKYPTIYHLRKKLVDSTDKADLRLIYLALAHMIKFR
GHFLIEGDLNPDNSDVDKLFIQLVQTYNQLFEENPINASGVDAKAILSARLSKSRRLENLI
AQLPGEKKNGLFGNLIALSLGLTPNFKSNFDLAEDAKLQLSKDTYDDDLDNLLAQIGDQ
YADLFLAAKNLSDAILLSDILRVNTEITKAPLSASMIKRYDEHHQDLTLLKALVRQQLPEK
YKEIFFDQSKNGYAGYIDGGASQEEFYKFIKPILEKMDGTEELLVKLNREDLLRKQRTFD
NGSIPHQIHLGELHAILRRQEDFYPFLKDNREKIEKILTFRIPYYVGPLARGNSRFAWMT
RKSEETITPWNFEEVVDKGASAQSFIERMTNFDKNLPNEKVLPKHSLLYEYFTVYNELT
KVKYVTEGMRKPAFLSGEQKKAIVDLLFKTNRKVTVKQLKEDYFKKIECFDSVEISGVED
RFNASLGTYHDLLKIIKDKDFLDNEENEDILEDIVLTLTLFEDREMIEERLKTYAHLFDD
KVMKQLKRRRYTGWGRLSRKLINGIRDKQSGKTILDFLKSDGFANRNFMQLIHDDSLTF
KEDIQKAQVSGQGDSLHEHIANLAGSPAIKKGILQTVKVVDELVKVMGRHKPENIVIEM
ARENQTTQKGQKNSRERMKRIEEGIKELGSQILKEHPVENTQLQNEKLYLYYLQNGRDM
YVDQELDINRLSDYDVDAIVPQSFLKDDSIDNKVLTRSDKNRGKSDNVPSEEVVKKMKN
YWRQLLNAKLITQRKFDNLTKAERGGLSELDKAGFIKRQLVETRQITKHVAQILDSRMN
TKYDENDKLIREVKVITLKSKLVSDFRKDFQFYKVREINNYHHAHDAYLNAVVGTALIKK
YPKLESEFVYGDYKVYDVRKMIAKSEQEIGKATAKYFFYSNIMNFFKTEITLANGEIRKR
PLIETNGETGEIVWDKGRDFATVRKVLSMPQVNIVKKTEVQTGGFSKESILPKRNSDKLI
ARKKDWDPKKYGGFDSPTVAYSVLVVAKVEKGKSKKLKSVKELLGITIMERSSFEKNPID
FLEAKGYKEVKKDLIIKLPKYSLFELENGRKRMLASAGELQKGNELALPSKYVNFLYLAS
HYEKLKGSPEDNEQKQLFVEQHKHYLDEIIEQISEFSKRVILADANLDKVLSAYNKHRDK
PIREQAENIIHLFTLTNLGAPAAFKYFDTTIDRKRYTSTKEVLDATLIHQSITGLYETRIDL
SQLGGDAYPYDVPDYASLGSGSPKKKRKVEDPKKKRKVDGGGPSSGAPPPSGGSPAGSP
TSTEEGTSESATPESGPGTSTEPSEGSAPGSPAGSPTSTEEGTSTEPSEGSAPGTSTEPSERTLVT

FKDVFVDFTREEWKLLDTAQQIVYRNVMLENYKNLVSLGYQLTKPDVILRLEKGEEPSG
SETPGTSESATPESGPGSPAGSPTSTEEGTSESATPESGPGSEPATSGSETPGTSESATPESGPGT
STEPSEGSAPGTSTEPSESATPESMGPMEIYKTVSAWKRQPVRVLSLFRNIDKVLKSLGF
LESGSGSGGGTLKYVEDVTNVVRRDVEKWGPFDLVYGSTQPLGSSCDRCPGWYMF
QFHRILQYALPRQESQRPFFWIFMDNLLLTEDDQETTTRFLQTEAVTLQDVRGRDY
QNAMRVWSNIPGLKSKHAPLTPKEEEYLQAQVRSRSKLDAPKVDLLVKNCLLPLR
EYFKYFSQNSLPLSSGNSNANSRGPSFSSGLVPLSLRGSHMNHDQEFDPPKVYPPVPAEK
RKPIRVLSLFDGIATGLLVLKDLGIQVDRYIASEVCEDSITVGMVRHQGKIMYVGDV
RSVTQKHIQEWGPFDLVIGGSPCNDLSIVNPARKGLYEGTGRLFFEFYRLLHDARP
KEGDDRPFFWLFENVVAMGVSDKRDISRFLESNPVMIDAKEVSAAHRARYFWGNL
PGMNRPLASTVNDKLELQECLEHGRIAKFSKVRTITTRSNSIKQGKDQHFPVFMNE
KEDILWCTEMERVFGFPVHYTDVSNMSRLARQRLLGRSWSVPVIRHLFAPLKEYFA
CVDGSGSETPGTSESATPESRTLVTFKDVFVDFTREEWKLLDTAQQIVYRNVMLENYKNL
VSLGYQLTKPDVILRLEKGEEP*

The amino acid sequence of dCas9-XTEN80-ZIM3-NEW XTEN-DNMT3L-DNMT3A-XTEN16-KRAB (V53 scheme, SEQ ID NO: 104) is as follows:

MVPAAKRVKLDGSDKKYSIGLAIGTNSVGWAVITDEYKVPSKKFKVLGNTDRHSIKK
NLIGALLFDSGETAEATRLKRTARRRYTRRKNRICYLQEIFSNEMAKVDDSFFHRLEESF
LVEEDKKHERHPIFGNIVDEVAYHEKYPTIYHLRKKLVDSTDKADLRLIYLALAHMIKFR
GHFLIEGDLNPDNSDVDKLFIQLVQTYNQLFEENPINASGVDAKAILSARLSKSRRLENLI
AQLPGEKKNGLFGNLIALSLGLTPNFKSNFDLAEDAKLQLSKDTYDDDLDNLLAQIGDQ
YADLFLAAKNLSDAILLSDILRVNTEITKAPLSASMIKRYDEHHQDLTLLKALVRQQLPEK
YKEIFFDQSKNGYAGYIDGGASQEEFYKFIKPILEKMDGTEELLVKLNREDLLRKQRTFD
NGSIPHQIHLGELHAILRRQEDFYPFLKDNREKIEKILTFRIPYYVGPLARGNSRFAWMT
RKSEETITPWNFEEVVDKGASAQSFIERMTNFDKNLPNEKVLPKHSLLYEYFTVYNELT
KVKYVTEGMRKPAFLSGEQKKAIVDLLFKTNRKVTVKQLKEDYFKKIECFDSVEISGVED
RFNASLGTYHDLLKIIKDKDFLDNEENEDILEDIVLTLTLFEDREMIEERLKTYAHLFDD

KVMKQLKRRRYTGWGRLSRKLINGIRDKQSGKTILDFLKSDGFANRNFMQLIHDDSLTF
KEDIQKAQVSGQGDSLHEHIANLAGSPAIKKGILQTVKVVDELVKVMGRHKPENIVIEM
ARENQTTQKGQKNSRERMKRIEEGIKELGSQILKEHPVENTQLQNEKLYLYYLQNGRDM
YVDQELDINRLSDYDVDAIVPQSFLKDDSIDNKVLTRSDKNRGKSDNVPSEEVVKKMKN
YWRQLLNAKLITQRKFDNLTKAERGGLSELDKAGFIKRQLVETRQITKHVAQILDSRMN
TKYDENDKLIREVKVITLKSKLVSDFRKDFQFYKVREINNYHHAHDAYLNAVVGTALIKK
YPKLESEFVYGDYKVYDVRKMIAKSEQEIGKATAKYFFYSNIMNFFKTEITLANGEIRKR
PLIETNGETGEIVWDKGRDFATVRKVLSMPQVNIVKKTEVQTGGFSKESILPKRNSDKLI
ARKKDWDPKKYGGFDSPTVAYSVLVVAKVEKGKSKKLKSVKELLGITIMERSSFEKNPID
FLEAKGYKEVKKDLIIKLPKYSLFELENGRKRMLASAGELQKGNELALPSKYVNFLYLAS
HYEKLKGSPEDNEQKQLFVEQHKHYLDEIIEQISEFSKRVILADANLDKVLSAYNKHRDK
PIREQAENIIHLFTLTNLGAPAAFKYFDTTIDRKRYTSTKEVLDATLIHQSITGLYETRIDL
SQLGGDAYPYDVPDYASLGSGSPKKKRKVEDPKKKRKVDGGGPSSGAPPPSGGSPAGSP
TSTEEGTSESATPESGPGTSTEPSEGSAPGSPAGSPTSTEEGTSTEPSEGSAPGTSTEPSEMNNS
QGRVTFEDVTVNFTQGEWQRLNPEQRNLYRDVMLENYSNLVSVGQGETTKPDVILRLE
QGKEPWLEEEEVLGSGRAEKNGDIGGQIWKPKDVKESLSGSETPGTSESATPESGPGSPA
GSPTSTEEGTSESATPESGPGSEPATSGSETPGTSESATPESGPGTSTEPSEGSAPGTSTEPSESA
TPESMGPMEIYKTVSAWKRQPVRVLSLFRNIDKVLKSLGFLESGSGSGGGTLKYVE
DVTNVVRRDVEKWGPFDLVYGSTQPLGSSCDRCPGWYMFQFHRILQYALPRQESQ
RPFFWIFMDNLLLTEDDQETTTRFLQTEAVTLQDVRGRDYQNAMRVWSNIPGLKS
KHAPLTPKEEEYLQAQVRSRSKLDAPKVDLLVKNCLLPLREYFKYFSQNSLPLSSGN
SNANSRGPSFSSGLVPLSLRGSHMNHDQEFDPPKVYPPVPAEKRKPIRVLSLFDGIATGL
LVLKDLGIQVDRYIASEVCEDSITVGMVRHQGKIMYVGDVRSVTQKHIQEWGPFDL
VIGGSPCNDLSIVNPARKGLYEGTGRLFFEFYRLLHDARPKEGDDRPFFWLFENVV
AMGVSDKRDISRFLESNPVMIDAKEVSAAHRARYFWGNLPGMNRPLASTVNDKLE
LQECLEHGRIAKFSKVRTITTRSNSIKQGKDQHFPVFMNEKEDILWCTEMERVFGF
PVHYTDVSNMSRLARQRLLGRSWSVPVIRHLFAPLKEYFACVDGSGSETPGTSESATP

ESRTLVTFKDVFVDFTREEWKLLDTAQQIVYRNVMLENYKNLVSLGYQLTKPDVILRLE

KGEEP*

In other embodiments, the fusion described in the present application may comprise one or more effector domains, and the effector domains are N-terminal or C-terminal to the nucleic acid-binding domain.

For example, the effector domain that is C-terminal to the nucleic acid-binding domain may comprise at least one type of epigenetic modification domain. Specifically, the effector domain that is C-terminal to the nucleic acid-binding domain may comprise at least one epigenetic modification domain that provides histone modification.

For example, the effector domain that is N-terminal to the nucleic acid-binding domain may comprise one or more of the epigenetic modification domain and the transcriptional regulation domain. Specifically, the effector domain that is N-terminal to the nucleic acid-binding domain may comprise at least one type of epigenetic modification domain, at least one transcriptional regulation domain, or at least one type of epigenetic modification domain and at least one transcriptional regulation domain. More specifically, the effector domain that is N-terminal to the nucleic acid-binding domain may comprise one or more of an epigenetic modification domain that provides histone modification, an epigenetic modification domain that provides DNA modification, and a transcriptional repressor domain, such as at least one epigenetic modification domain that provides histone modification, at least one epigenetic modification domain that provides DNA modification, at least one transcriptional repressor domain, at least one epigenetic modification domain that provides histone modification and at least one epigenetic modification domain that provides DNA modification, at least one epigenetic modification domain that provides histone modification and at least one transcriptional regulation domain, and at least one epigenetic modification domain that provides DNA modification and at least one transcriptional regulation domain.

In some embodiments of the above cases, the effector domain that is N-terminal to the nucleic acid-binding domain comprises: (1) the epigenetic modification domain that provides histone modification; (2) the transcriptional repressor domain; (3) the epigenetic modification domain that provides DNA modification; or (4) the transcriptional repressor domain and the epigenetic modification domain that provides DNA modification. Specifically, in the embodiments of these cases, the effector domain that is N-terminal to the nucleic acid-binding domain comprises: (1) one or more epigenetic modification domains selected from EZH2, HDAC3, HDAC1, EHMT2, PRMT1, PRMT5, SETDB1, hSIRT1, HP1a, LSD1, and functionally active fragments thereof; (2) KRAB or a functionally active fragment thereof; (3) DNMT3A, DNMT3L, or a combination of DNMT3A and DNMT3L; or (4) a combination of KRAB and DNMT3A, a combination of KRAB and DNMT3L, or a combination of KRAB, DNMT3A, and DNMT3L.

The following are some exemplary fusions of the present application, and the components in the amino acid sequences of the fusion are as follows: bold-epigenetic modification domains that provide DNA modification (such as DNMT), underlined bold-epigenetic modification domains that provide histone modification (such as HDAC), italic-linker sequence, bold italic-transcriptional regulation domain, underlined bold italic-dCas9 or TALE, and * is any amino acid.

The amino acid sequence of DNMT3A-DNMT3L-XTEN80-dCas9-XTEN16-ZIM3 (V17 scheme, SEQ ID NO: 68) is as follows:

MPAAKRVKLDGSNHDQEFDPPKVYPPVPAEKRKPIRVLSLFDGIATGLLVLKDL
GIQVDRYIASEVCEDSITVGMVRHQGKIMYVGDVRSVTQKHIQEWGPFDLVIGGSP
CNDLSIVNPARKGLYEGTGRLFFEFYRLLHDARPKEGDDRPFFWLFENVVAMGVSD
KRDISRFLESNPVMIDAKEVSAAHRARYFWGNLPGMNRPLASTVNDKLELQECLEH
GRIAKFSKVRTITTRSNSIKQGKDQHFPVFMNEKEDILWCTEMERVFGFPVHYTDV
SNMSRLARQRLLGRSWSVPVIRHLFAPLKEYFACVSSGNSNANSRGPSFSSGLVPLSLRG
SHMGPMEIYKTVSAWKRQPVRVLSLFRNIDKVLKSLGFLESGSGSGGGTLKYVEDV
TNVVRRDVEKWGPFDLVYGSTQPLGSSCDRCPGWYMFQFHRILQYALPRQESQRP
FFWIFMDNLLLTEDDQETTTRFLQTEAVTLQDVRGRDYQNAMRVWSNIPGLKSKH
APLTPKEEEYLQAQVRSRSKLDAPKVDLLVKNCLLPLREYFKYFSQNSLPLGGPSSG
APPPSGGSPAGSPTSTEEGTSESATPESGPGTSTEPSEGSAPGSPAGSPTSTEEGTSTEPSEGSA

PGTSTEPSEPKKKRKVMDKKYSIGLAIGTNSVGWAVITDEYKVPSKKFKVLGNTDRHSIK
KNLIGALLFDSGETAEATRLKRTARRRYTRRKNRICYLQEIFSNEMAKVDDSFFHRLEES
FLVEEDKKHERHPIFGNIVDEVAYHEKYPTIYHLRKKLVDSTDKADLRLIYLALAHMIKF
RGHFLIEGDLNPDNSDVDKLFIQLVQTYNQLFEENPINASGVDAKAILSARLSKSRRLEN
LIAQLPGEKKNGLFGNLIALSLGLTPNFKSNFDLAEDAKLQLSKDTYDDDLDNLLAQIGD
QYADLFLAAKNLSDAILLSDILRVNTEITKAPLSASMIKRYDEHHQDLTLLKALVRQQLP
EKYKEIFFDQSKNGYAGYIDGGASQEEFYKFIKPILEKMDGTEELLVKLNREDLLRKQRT
FDNGSIPHQIHLGELHAILRRQEDFYPFLKDNREKIEKILTFRIPYYVGPLARGNSRFAW
MTRKSEETITPWNFEEVVDKGASAQSFIERMTNFDKNLPNEKVLPKHSLLYEYFTVYNE
LTKVKYVTEGMRKPAFLSGEQKKAIVDLLFKTNRKVTVKQLKEDYFKKIECFDSVEISGV
EDRFNASLGTYHDLLKIIKDKDFLDNEENEDILEDIVLTLTLFEDREMIEERLKTYAHLF
DDKVMKQLKRRRYTGWGRLSRKLINGIRDKQSGKTILDFLKSDGFANRNFMQLIHDDSL
TFKEDIQKAQVSGQGDSLHEHIANLAGSPAIKKGILQTVKVVDELVKVMGRHKPENIVIE
MARENQTTQKGQKNSRERMKRIEEGIKELGSQILKEHPVENTQLQNEKLYLYYLQNGRD
MYVDQELDINRLSDYDVDAIVPQSFLKDDSIDNKVLTRSDKNRGKSDNVPSEEVVKKMK
NYWRQLLNAKLITQRKFDNLTKAERGGLSELDKAGFIKRQLVETRQITKHVAQILDSRM
NTKYDENDKLIREVKVITLKSKLVSDFRKDFQFYKVREINNYHHAHDAYLNAVVGTALIK
KYPKLESEFVYGDYKVYDVRKMIAKSEQEIGKATAKYFFYSNIMNFFKTEITLANGEIRK
RPLIETNGETGEIVWDKGRDFATVRKVLSMPQVNIVKKTEVQTGGFSKESILPKRNSDKL
IARKKDWDPKKYGGFDSPTVAYSVLVVAKVEKGKSKKLKSVKELLGITIMERSSFEKNPI
DFLEAKGYKEVKKDLIIKLPKYSLFELENGRKRMLASAGELQKGNELALPSKYVNFLYLA
SHYEKLKGSPEDNEQKQLFVEQHKHYLDEIIEQISEFSKRVILADANLDKVLSAYNKHRD
KPIREQAENIIHLFTLTNLGAPAAFKYFDTTIDRKRYTSTKEVLDATLIHQSITGLYETRID
LSQLGGDAYPYDVPDYASLGSGSPKKKRKVEDPKKKRKVDGSGSETPGTSESATPESMN
NSQGRVTFEDVTVNFTQGEWQRLNPEQRNLYRDVMLENYSNLVSVGQGETTKPDVILR
LEQGKEPWLEEEEVLGSGRAEKNGDIGGQIWKPKDVKESLLE*

The amino acid sequence of DNMT3A-(human)DNMT3L-XTEN80-dCas9-XTEN16-KRAB (V18 scheme, SEQ ID NO: 69) is as follows:

MPAAKRVKLDGSNHDQEFDPPKVYPPVPAEKRKPIRVLSLFDGIATGLLVLKDL
GIQVDRYIASEVCEDSITVGMVRHQGKIMYVGDVRSVTQKHIQEWGPFDLVIGGSP
CNDLSIVNPARKGLYEGTGRLFFEFYRLLHDARPKEGDDRPFFWLFENVVAMGVSD
KRDISRFLESNPVMIDAKEVSAAHRARYFWGNLPGMNRPLASTVNDKLELQECLEH
GRIAKFSKVRTITTRSNSIKQGKDQHFPVFMNEKEDILWCTEMERVFGFPVHYTDV
SNMSRLARQRLLGRSWSVPVIRHLFAPLKEYFACVSSGNSNANSRGPSFSSGLVPLSLRG
SHMFETVPVWRRQPVRVLSLFEDIKKELTSLGFLESGSDPGQLKHVVDVTDTVRKD
VEEWGPFDLVYGATPPLGHTCDRPPSWYLFQFHRLLQYARPKPGSPRPFFWMFVD
NLVLNKEDLDVASRFLEMEPVTIPDVHGGSLQNAVRVWSNIPAIRSSRHWALVSEE
ELSLLAQNKQSSKLAAKWPTKLVKNCFLPLREYFKYFSGGPSSGAPPPSGGSPAGSPT
STEEGTSESATPESGPGTSTEPSEGSAPGSPAGSPTSTEEGTSTEPSEGSAPGTSTEPSEPKKKR
KVMDKKYSIGLAIGTNSVGWAVITDEYKVPSKKFKVLGNTDRHSIKKNLIGALLFDSGET
AEATRLKRTARRRYTRRKNRICYLQEIFSNEMAKVDDSFFHRLEESFLVEEDKKHERHP
IFGNIVDEVAYHEKYPTIYHLRKKLVDSTDKADLRLIYLALAHMIKFRGHFLIEGDLNPD
NSDVDKLFIQLVQTYNQLFEENPINASGVDAKAILSARLSKSRRLENLIAQLPGEKKNGL
FGNLIALSLGLTPNFKSNFDLAEDAKLQLSKDTYDDDLDNLLAQIGDQYADLFLAAKNL
SDAILLSDILRVNTEITKAPLSASMIKRYDEHHQDLTLLKALVRQQLPEKYKEIFFDQSKN
GYAGYIDGGASQEEFYKFIKPILEKMDGTEELLVKLNREDLLRKQRTFDNGSIPHQIHLG
ELHAILRRQEDFYPFLKDNREKIEKILTFRIPYYVGPLARGNSRFAWMTRKSEETITPWN
FEEVVDKGASAQSFIERMTNFDKNLPNEKVLPKHSLLYEYFTVYNELTKVKYVTEGMRK
PAFLSGEQKKAIVDLLFKTNRKVTVKQLKEDYFKKIECFDSVEISGVEDRFNASLGTYHD
LLKIIKDKDFLDNEENEDILEDIVLTLTLFEDREMIEERLKTYAHLFDDKVMKQLKRRRY
TGWGRLSRKLINGIRDKQSGKTILDFLKSDGFANRNFMQLIHDDSLTFKEDIQKAQVSG
QGDSLHEHIANLAGSPAIKKGILQTVKVVDELVKVMGRHKPENIVIEMARENQTTQKGQ
KNSRERMKRIEEGIKELGSQILKEHPVENTQLQNEKLYLYYLQNGRDMYVDQELDINRL

SDYDVDAIVPQSFLKDDSIDNKVLTRSDKNRGKSDNVPSEEVVKKMKNYWRQLLNAKLI
TQRKFDNLTKAERGGLSELDKAGFIKRQLVETRQITKHVAQILDSRMNTKYDENDKLIR
EVKVITLKSKLVSDFRKDFQFYKVREINNYHHAHDAYLNAVVGTALIKKYPKLESEFVYG
DYKVYDVRKMIAKSEQEIGKATAKYFFYSNIMNFFKTEITLANGEIRKRPLIETNGETGEI
VWDKGRDFATVRKVLSMPQVNIVKKTEVQTGGFSKESILPKRNSDKLIARKKDWDPKKY
GGFDSPTVAYSVLVVAKVEKGKSKKLKSVKELLGITIMERSSFEKNPIDFLEAKGYKEVK
KDLIIKLPKYSLFELENGRKRMLASAGELQKGNELALPSKYVNFLYLASHYEKLKGSPED
NEQKQLFVEQHKHYLDEIIEQISEFSKRVILADANLDKVLSAYNKHRDKPIREQAENIIHL
FTLTNLGAPAAFKYFDTTIDRKRYTSTKEVLDATLIHQSITGLYETRIDLSQLGGDAYPYD
VPDYASLGSGSPKKKRKVEDPKKKRKVDGSGSETPGTSESATPESRTLVTFKDVFVDFTR
EEWKLLDTAQQIVYRNVMLENYKNLVSLGYQLTKPDVILRLEKGEEP*

The amino acid sequence of HDAC3-dCas9-EZH2 (V57 scheme, SEQ ID NO: 108) is as follows:

MPAAKRVKLDGSAKTVAYFYDPDVGNFHYGAGHPMKPHRLALTHSLVLHYGL
YKKMIVFKPYQASQHDMCRFHSEDYIDFLQRVSPTNMQGFTKSLNAFNVGDDCPV
FPGLFEFCSRYTGASLQGATQLNNKICDIAINWAGGLHHAKKFEASGFCYVNDIVIG
ILELLKYHPRVLYIDIDIHHGDGVQEAFYLTDRVMTVSFHKYGNYFFPGTGDMYEV
GAESGRYYCLNVPLRDGIDDQSYKHLFQPVINQVVDFYQPTCIVLQCGADSLGCDR
LGCFNLSIRGHGECVEYVKSFNIPLLVLGGGGYTVRNVARCWTYETSLLVEEAISEE
LPYSEYFEYFAPDFTLHPDVSTRIENQNSRQYLDQIRQTIFENLGGPSSGAPPPSGGSPA
GSPTSTEEGTSESATPESGPGTSTEPSEGSAPGSPAGSPTSTEEGTSTEPSEGSAPGTSTEPSEPK
KKRKVMDKKYSIGLAIGTNSVGWAVITDEYKVPSKKFKVLGNTDRHSIKKNLIGALLFDS
GETAEATRLKRTARRRYTRRKNRICYLQEIFSNEMAKVDDSFFHRLEESFLVEEDKKHE
RHPIFGNIVDEVAYHEKYPTIYHLRKKLVDSTDKADLRLIYLALAHMIKFRGHFLIEGDL
NPDNSDVDKLFIQLVQTYNQLFEENPINASGVDAKAILSARLSKSRRLENLIAQLPGEKK
NGLFGNLIALSLGLTPNFKSNFDLAEDAKLQLSKDTYDDDLDNLLAQIGDQYADLFLAA
KNLSDAILLSDILRVNTEITKAPLSASMIKRYDEHHQDLTLLKALVRQQLPEKYKEIFFDQ

SKNGYAGYIDGGASQEEFYKFIKPILEKMDGTEELLVKLNREDLLRKQRTFDNGSIPHQI
HLGELHAILRRQEDFYPFLKDNREKIEKILTFRIPYYVGPLARGNSRFAWMTRKSEETIT
PWNFEEVVDKGASAQSFIERMTNFDKNLPNEKVLPKHSLLYEYFTVYNELTKVKYVTEG
MRKPAFLSGEQKKAIVDLLFKTNRKVTVKQLKEDYFKKIECFDSVEISGVEDRFNASLGT
YHDLLKIIKDKDFLDNEENEDILEDIVLTLTLFEDREMIEERLKTYAHLFDDKVMKQLK
RRRYTGWGRLSRKLINGIRDKQSGKTILDFLKSDGFANRNFMQLIHDDSLTFKEDIQKA
QVSGQGDSLHEHIANLAGSPAIKKGILQTVKVVDELVKVMGRHKPENIVIEMARENQTT
QKGQKNSRERMKRIEEGIKELGSQILKEHPVENTQLQNEKLYLYYLQNGRDMYVDQEL
DINRLSDYDVDAIVPQSFLKDDSIDNKVLTRSDKNRGKSDNVPSEEVVKKMKNYWRQLL
NAKLITQRKFDNLTKAERGGLSELDKAGFIKRQLVETRQITKHVAQILDSRMNTKYDEN
DKLIREVKVITLKSKLVSDFRKDFQFYKVREINNYHHAHDAYLNAVVGTALIKKYPKLES
EFVYGDYKVYDVRKMIAKSEQEIGKATAKYFFYSNIMNFFKTEITLANGEIRKRPLIETN
GETGEIVWDKGRDFATVRKVLSMPQVNIVKKTEVQTGGFSKESILPKRNSDKLIARKKD
WDPKKYGGFDSPTVAYSVLVVAKVEKGKSKKLKSVKELLGITIMERSSFEKNPIDFLEAK
GYKEVKKDLIIKLPKYSLFELENGRKRMLASAGELQKGNELALPSKYVNFLYLASHYEKL
KGSPEDNEQKQLFVEQHKHYLDEIIEQISEFSKRVILADANLDKVLSAYNKHRDKPIREQ
AENIIHLFTLTNLGAPAAFKYFDTTIDRKRYTSTKEVLDATLIHQSITGLYETRIDLSQLGG
DAYPYDVPDYASLGSGSPKKKRKVEDPKKKRKVDGSGSETPGTSESATPESRTLGQTGK
KSEKGPVCWRKRVKSEYMRLRQLKRFRRADEVKSMFSSNRQKILERTEILNQEWK
QRRIQPVHILTSVSSLRGTRECSVTSDLDFPTQVIPLKTLNAVASVPIMYSWSPLQQN
FMVEDETVLHNIPYMGDEVLDQDGTFIEELIKNYDGKVHGDRECGFINDEIFVELV
NALGQYNDDDDDDDGDDPEEREEKQKDLEDHRDDKESRPPRKFPSDKIFEAISSMF
PDKGTAEELKEKYKELTEQQLPGALPPECTPNIDGPNAKSVQREQSLHSFHTLFCR
RCFKYDCFLHPFHATPNTYKRKNTETALDNKPCGPQCYQHLEGAKEFAAALTAER
IKTPPKRPGGRRRGRLPNNSSRPSTPTINVLESKDTDSDREAGTETGGENNDKEEEE
KKDETSSSSEANSRCQTPIKMKPNIEPPENVEWSGAEASMFRVLIGTYYDNFCAIAR
LIGTKTCRQVYEFRVKESSIIAPAPAEDVDTPPRKKKRKHRLWAAHCRKIQLKKDG

SSNHVYNYQPCDHPRQPCDSSCPCVIAQNFCEKFCQCSSECQNRFPGCRCKAQCNT
KQCPCYLAVRECDPDLCLTCGAADHWDSKNVSCKNCSIQRGSKKHLLLAPSDVAG
WGIFIKDPVQKNEFISEYCGEIISQDEADRRGKVYDKYMCSFLFNLNNDFVVDATRK
GNKIRFANHSVNPNCYAKVMMVNGDHRIGIFAKRAIQTGEELFFDYRYSQADALKY
VGIEREMEIPEGSG*

The amino acid sequence of KRAB-dCas9-EZH2 (V58 scheme, SEQ ID NO: 109) is as follows:

MPAAKRVKLDGSRTLVTFKDVFVDFTREEWKLLDTAQQIVYRNVMLENYKNLVSLG
YQLTKPDVILRLEKGEEPGGPSSGAPPPSGGSPAGSPTSTEEGTSESATPESGPGTSTEPSEGS
APGSPAGSPTSTEEGTSTEPSEGSAPGTSTEPSEPKKKRKVMDKKYSIGLAIGTNSVGWAVIT
DEYKVPSKKFKVLGNTDRHSIKKNLIGALLFDSGETAEATRLKRTARRRYTRRKNRICYL
QEIFSNEMAKVDDSFFHRLEESFLVEEDKKHERHPIFGNIVDEVAYHEKYPTIYHLRKK
LVDSTDKADLRLIYLALAHMIKFRGHFLIEGDLNPDNSDVDKLFIQLVQTYNQLFEENPI
NASGVDAKAILSARLSKSRRLENLIAQLPGEKKNGLFGNLIALSLGLTPNFKSNFDLAED
AKLQLSKDTYDDDLDNLLAQIGDQYADLFLAAKNLSDAILLSDILRVNTEITKAPLSASMI
KRYDEHHQDLTLLKALVRQQLPEKYKEIFFDQSKNGYAGYIDGGASQEEFYKFIKPILEK
MDGTEELLVKLNREDLLRKQRTFDNGSIPHQIHLGELHAILRRQEDFYPFLKDNREKIE
KILTFRIPYYVGPLARGNSRFAWMTRKSEETITPWNFEEVVDKGASAQSFIERMTNFDK
NLPNEKVLPKHSLLYEYFTVYNELTKVKYVTEGMRKPAFLSGEQKKAIVDLLFKTNRKV
TVKQLKEDYFKKIECFDSVEISGVEDRFNASLGTYHDLLKIIKDKDFLDNEENEDILEDIV
LTLTLFEDREMIEERLKTYAHLFDDKVMKQLKRRRYTGWGRLSRKLINGIRDKQSGKTI
LDFLKSDGFANRNFMQLIHDDSLTFKEDIQKAQVSGQGDSLHEHIANLAGSPAIKKGIL
QTVKVVDELVKVMGRHKPENIVIEMARENQTTQKGQKNSRERMKRIEEGIKELGSQILK
EHPVENTQLQNEKLYLYYLQNGRDMYVDQELDINRLSDYDVDAIVPQSFLKDDSIDNKV
LTRSDKNRGKSDNVPSEEVVKKMKNYWRQLLNAKLITQRKFDNLTKAERGGLSELDKA
GFIKRQLVETRQITKHVAQILDSRMNTKYDENDKLIREVKVITLKSKLVSDFRKDFQFYK
VREINNYHHAHDAYLNAVVGTALIKKYPKLESEFVYGDYKVYDVRKMIAKSEQEIGKAT
AKYFFYSNIMNFFKTEITLANGEIRKRPLIETNGETGEIVWDKGRDFATVRKVLSMPQVN

IVKKTEVQTGGFSKESILPKRNSDKLIARKKDWDPKKYGGFDSPTVAYSVLVVAKVEKGK
SKKLKSVKELLGITIMERSSFEKNPIDFLEAKGYKEVKKDLIIKLPKYSLFELENGRKRML
ASAGELQKGNELALPSKYVNFLYLASHYEKLKGSPEDNEQKQLFVEQHKHYLDEIIEQIS
EFSKRVILADANLDKVLSAYNKHRDKPIREQAENIIHLFTLTNLGAPAAFKYFDTTIDRK
RYTSTKEVLDATLIHQSITGLYETRIDLSQLGGDAYPYDVPDYASLGSGSPKKKRKVEDP
KKKRKVDGSGSETPGTSESATPESRTLGQTGKKSEKGPVCWRKRVKSEYMRLRQLKR
FRRADEVKSMFSSNRQKILERTEILNQEWKQRRIQPVHILTSVSSLRGTRECSVTSD
LDFPTQVIPLKTLNAVASVPIMYSWSPLQQNFMVEDETVLHNIPYMGDEVLDQDGT
FIEELIKNYDGKVHGDRECGFINDEIFVELVNALGQYNDDDDDDDGDDPEEREEKQ
KDLEDHRDDKESRPPRKFPSDKIFEAISSMFPDKGTAEELKEKYKELTEQQLPGALP
PECTPNIDGPNAKSVQREQSLHSFHTLFCRRCFKYDCFLHPFHATPNTYKRKNTET
ALDNKPCGPQCYQHLEGAKEFAAALTAERIKTPPKRPGGRRRGRLPNNSSRPSTPTI
NVLESKDTDSDREAGTETGGENNDKEEEEKKDETSSSSEANSRCQTPIKMKPNIEPP
ENVEWSGAEASMFRVLIGTYYDNFCAIARLIGTKTCRQVYEFRVKESSIIAPAPAED
VDTPPRKKKRKHRLWAAHCRKIQLKKDGSSNHVYNYQPCDHPRQPCDSSCPCVIA
QNFCEKFCQCSSECQNRFPGCRCKAQCNTKQCPCYLAVRECDPDLCLTCGAADH
WDSKNVSCKNCSIQRGSKKHLLLAPSDVAGWGIFIKDPVQKNEFISEYCGEIISQDE
ADRRGKVYDKYMCSFLFNLNNDFVVDATRKGNKIRFANHSVNPNCYAKVMMVNG
DHRIGIFAKRAIQTGEELFFDYRYSQADALKYVGIEREMEIPEGSG*

The amino acid sequence of KRAB-DNMT3A-dCas9-EZH2 (V59 scheme, SEQ ID NO: 110) is as follows:

MPAAKRVKLDGSRTLVTFKDVFVDFTREEWKLLDTAQQIVYRNVMLENYKNLVSLG
YQLTKPDVILRLEKGEEPGGPSSGAPPPSGGSPAGSPTSTEEGTSESATPESGPGTSTEPSEGS
APGSPAGSPTSTEEGTSTEPSEGSAPGTSTEPSEMNHDQEFDPPKVYPPVPAEKRKPIRVL
SLFDGIATGLLVLKDLGIQVDRYIASEVCEDSITVGMVRHQGKIMYVGDVRSVTQK
HIQEWGPFDLVIGGSPCNDLSIVNPARKGLYEGTGRLFFEFYRLLHDARPKEGDDR
PFFWLFENVVAMGVSDKRDISRFLESNPVMIDAKEVSAAHRARYFWGNLPGMNRP

LASTVNDKLELQECLEHGRIAKFSKVRTITTRSNSIKQGKDQHFPVFMNEKEDILW
CTEMERVFGFPVHYTDVSNMSRLARQRLLGRSWSVPVIRHLFAPLKEYFACVGGPS
SGAPPPSGGSPAGSPTSTEEGTSESATPESGPGTSTEPSEGSAPGSPAGSPTSTEEGTSTEPSEG
SAPGTSTEPSEPKKKRKVMDKKYSIGLAIGTNSVGWAVITDEYKVPSKKFKVLGNTDRHSI
KKNLIGALLFDSGETAEATRLKRTARRRYTRRKNRICYLQEIFSNEMAKVDDSFFHRLEE
SFLVEEDKKHERHPIFGNIVDEVAYHEKYPTIYHLRKKLVDSTDKADLRLIYLALAHMIK
FRGHFLIEGDLNPDNSDVDKLFIQLVQTYNQLFEENPINASGVDAKAILSARLSKSRRLE
NLIAQLPGEKKNGLFGNLIALSLGLTPNFKSNFDLAEDAKLQLSKDTYDDDLDNLLAQIG
DQYADLFLAAKNLSDAILLSDILRVNTEITKAPLSASMIKRYDEHHQDLTLLKALVRQQL
PEKYKEIFFDQSKNGYAGYIDGGASQEEFYKFIKPILEKMDGTEELLVKLNREDLLRKQR
TFDNGSIPHQIHLGELHAILRRQEDFYPFLKDNREKIEKILTFRIPYYVGPLARGNSRFA
WMTRKSEETITPWNFEEVVDKGASAQSFIERMTNFDKNLPNEKVLPKHSLLYEYFTVYN
ELTKVKYVTEGMRKPAFLSGEQKKAIVDLLFKTNRKVTVKQLKEDYFKKIECFDSVEISG
VEDRFNASLGTYHDLLKIIKDKDFLDNEENEDILEDIVLTLTLFEDREMIEERLKTYAHL
FDDKVMKQLKRRRYTGWGRLSRKLINGIRDKQSGKTILDFLKSDGFANRNFMQLIHDDS
LTFKEDIQKAQVSGQGDSLHEHIANLAGSPAIKKGILQTVKVVDELVKVMGRHKPENIVI
EMARENQTTQKGQKNSRERMKRIEEGIKELGSQILKEHPVENTQLQNEKLYLYYLQNGR
DMYVDQELDINRLSDYDVDAIVPQSFLKDDSIDNKVLTRSDKNRGKSDNVPSEEVVKKM
KNYWRQLLNAKLITQRKFDNLTKAERGGLSELDKAGFIKRQLVETRQITKHVAQILDSR
MNTKYDENDKLIREVKVITLKSKLVSDFRKDFQFYKVREINNYHHAHDAYLNAVVGTAL
IKKYPKLESEFVYGDYKVYDVRKMIAKSEQEIGKATAKYFFYSNIMNFFKTEITLANGEIR
KRPLIETNGETGEIVWDKGRDFATVRKVLSMPQVNIVKKTEVQTGGFSKESILPKRNSDK
LIARKKDWDPKKYGGFDSPTVAYSVLVVAKVEKGKSKKLKSVKELLGITIMERSSFEKNP
IDFLEAKGYKEVKKDLIIKLPKYSLFELENGRKRMLASAGELQKGNELALPSKYVNFLYL
ASHYEKLKGSPEDNEQKQLFVEQHKHYLDEIIEQISEFSKRVILADANLDKVLSAYNKHR
DKPIREQAENIIHLFTLTNLGAPAAFKYFDTTIDRKRYTSTKEVLDATLIHQSITGLYETRI
DLSQLGGDAYPYDVPDYASLGSGSPKKKRKVEDPKKKRKVDGSGSETPGTSESATPESRT

LGQTGKKSEKGPVCWRKRVKSEYMRLRQLKRFRRADEVKSMFSSNRQKILERTEI
LNQEWKQRRIQPVHILTSVSSLRGTRECSVTSDLDFPTQVIPLKTLNAVASVPIMYS
WSPLQQNFMVEDETVLHNIPYMGDEVLDQDGTFIEELIKNYDGKVHGDRECGFIN
DEIFVELVNALGQYNDDDDDDDGDDPEEREEKQKDLEDHRDDKESRPPRKFPSDKI
FEAISSMFPDKGTAEELKEKYKELTEQQLPGALPPECTPNIDGPNAKSVQREQSLHS
FHTLFCRRCFKYDCFLHPFHATPNTYKRKNTETALDNKPCGPQCYQHLEGAKEFA
AALTAERIKTPPKRPGGRRRGRLPNNSSRPSTPTINVLESKDTDSDREAGTETGGEN
NDKEEEEKKDETSSSSEANSRCQTPIKMKPNIEPPENVEWSGAEASMFRVLIGTYYD
NFCAIARLIGTKTCRQVYEFRVKESSIIAPAPAEDVDTPPRKKKRKHRLWAAHCRKI
QLKKDGSSNHVYNYQPCDHPRQPCDSSCPCVIAQNFCEKFCQCSSECQNRFPGCRC
KAQCNTKQCPCYLAVRECDPDLCLTCGAADHWDSKNVSCKNCSIQRGSKKHLLLA
PSDVAGWGIFIKDPVQKNEFISEYCGEIISQDEADRRGKVYDKYMCSFLFNLNNDFV
VDATRKGNKIRFANHSVNPNCYAKVMMVNGDHRIGIFAKRAIQTGEELFFDYRYSQ
ADALKYVGIEREMEIPEGSG*

The amino acid sequence of KRAB-DNMT3A-dCas9-HDAC3 (V60 scheme, SEQ ID NO: 111) is as follows:

MPAAKRVKLDGSRTLVTFKDVFVDFTREEWKLLDTAQQIVYRNVMLENYKNLVSLG
YQLTKPDVILRLEKGEEPGGPSSGAPPPSGGSPAGSPTSTEEGTSESATPESGPGTSTEPSEGS
APGSPAGSPTSTEEGTSTEPSEGSAPGTSTEPSEMNHDQEFDPPKVYPPVPAEKRKPIRVL
SLFDGIATGLLVLKDLGIQVDRYIASEVCEDSITVGMVRHQGKIMYVGDVRSVTQK
HIQEWGPFDLVIGGSPCNDLSIVNPARKGLYEGTGRLFFEFYRLLHDARPKEGDDR
PFFWLFENVVAMGVSDKRDISRFLESNPVMIDAKEVSAAHRARYFWGNLPGMNRP
LASTVNDKLELQECLEHGRIAKFSKVRTITTRSNSIKQGKDQHFPVFMNEKEDILW
CTEMERVFGFPVHYTDVSNMSRLARQRLLGRSWSVPVIRHLFAPLKEYFACVGGPS
SGAPPPSGGSPAGSPTSTEEGTSESATPESGPGTSTEPSEGSAPGSPAGSPTSTEEGTSTEPSEG
SAPGTSTEPSEPKKKRKVMDKKYSIGLAIGTNSVGWAVITDEYKVPSKKFKVLGNTDRHSI
KKNLIGALLFDSGETAEATRLKRTARRRYTRRKNRICYLQEIFSNEMAKVDDSFFHRLEE

SFLVEEDKKHERHPIFGNIVDEVAYHEKYPTIYHLRKKLVDSTDKADLRLIYLALAHMIK
FRGHFLIEGDLNPDNSDVDKLFIQLVQTYNQLFEENPINASGVDAKAILSARLSKSRRLE
NLIAQLPGEKKNGLFGNLIALSLGLTPNFKSNFDLAEDAKLQLSKDTYDDDLDNLLAQIG
DQYADLFLAAKNLSDAILLSDILRVNTEITKAPLSASMIKRYDEHHQDLTLLKALVRQQL
PEKYKEIFFDQSKNGYAGYIDGGASQEEFYKFIKPILEKMDGTEELLVKLNREDLLRKQR
TFDNGSIPHQIHLGELHAILRRQEDFYPFLKDNREKIEKILTFRIPYYVGPLARGNSRFA
WMTRKSEETITPWNFEEVVDKGASAQSFIERMTNFDKNLPNEKVLPKHSLLYEYFTVYN
ELTKVKYVTEGMRKPAFLSGEQKKAIVDLLFKTNRKVTVKQLKEDYFKKIECFDSVEISG
VEDRFNASLGTYHDLLKIIKDKDFLDNEENEDILEDIVLTLTLFEDREMIEERLKTYAHL
FDDKVMKQLKRRRYTGWGRLSRKLINGIRDKQSGKTILDFLKSDGFANRNFMQLIHDDS
LTFKEDIQKAQVSGQGDSLHEHIANLAGSPAIKKGILQTVKVVDELVKVMGRHKPENIVI
EMARENQTTQKGQKNSRERMKRIEEGIKELGSQILKEHPVENTQLQNEKLYLYYLQNGR
DMYVDQELDINRLSDYDVDAIVPQSFLKDDSIDNKVLTRSDKNRGKSDNVPSEEVVKKM
KNYWRQLLNAKLITQRKFDNLTKAERGGLSELDKAGFIKRQLVETRQITKHVAQILDSR
MNTKYDENDKLIREVKVITLKSKLVSDFRKDFQFYKVREINNYHHAHDAYLNAVVGTAL
IKKYPKLESEFVYGDYKVYDVRKMIAKSEQEIGKATAKYFFYSNIMNFFKTEITLANGEIR
KRPLIETNGETGEIVWDKGRDFATVRKVLSMPQVNIVKKTEVQTGGFSKESILPKRNSDK
LIARKKDWDPKKYGGFDSPTVAYSVLVVAKVEKGKSKKLKSVKELLGITIMERSSFEKNP
IDFLEAKGYKEVKKDLIIKLPKYSLFELENGRKRMLASAGELQKGNELALPSKYVNFLYL
ASHYEKLKGSPEDNEQKQLFVEQHKHYLDEIIEQISEFSKRVILADANLDKVLSAYNKHR
DKPIREQAENIIHLFTLTNLGAPAAFKYFDTTIDRKRYTSTKEVLDATLIHQSITGLYETRI
DLSQLGGDAYPYDVPDYASLGSGSPKKKRKVEDPKKKRKVDGSGSETPGTSESATPESRT
LAKTVAYFYDPDVGNFHYGAGHPMKPHRLALTHSLVLHYGLYKKMIVFKPYQASQ
HDMCRFHSEDYIDFLQRVSPTNMQGFTKSLNAFNVGDDCPVFPGLFEFCSRYTGAS
LQGATQLNNKICDIAINWAGGLHHAKKFEASGFCYVNDIVIGILELLKYHPRVLYID
IDIHHGDGVQEAFYLTDRVMTVSFHKYGNYFFPGTGDMYEVGAESGRYYCLNVPL
RDGIDDQSYKHLFQPVINQVVDFYQPTCIVLQCGADSLGCDRLGCFNLSIRGHGEC

VEYVKSFNIPLLVLGGGGYTVRNVARCWTYETSLLVEEAISEELPYSEYFEYFAPDF
TLHPDVSTRIENQNSRQYLDQIRQTIFENLKMLNHAPSVQIHDVPADLLTYDRTDEA
DAEERGPEENYSRPEAPNEFYDGDHDNDKESDVEIEGSG*

The amino acid sequence of DNMT3A-DNMT3L-XTEN80-dCas9-XTEN16-EZH2x11 (V61 scheme, SEQ ID NO: 112) is as follows:

MPAAKRVKLDGSNHDQEFDPPKVYPPVPAEKRKPIRVLSLFDGIATGLLVLKDL
GIQVDRYIASEVCEDSITVGMVRHQGKIMYVGDVRSVTQKHIQEWGPFDLVIGGSP
CNDLSIVNPARKGLYEGTGRLFFEFYRLLHDARPKEGDDRPFFWLFENVVAMGVSD
KRDISRFLESNPVMIDAKEVSAAHRARYFWGNLPGMNRPLASTVNDKLELQECLEH
GRIAKFSKVRTITTRSNSIKQGKDQHFPVFMNEKEDILWCTEMERVFGFPVHYTDV
SNMSRLARQRLLGRSWSVPVIRHLFAPLKEYFACVSSGNSNANSRGPSFSSGLVPLSLRG
SHMGPMEIYKTVSAWKRQPVRVLSLFRNIDKVLKSLGFLESGSGSGGGTLKYVEDV
TNVVRRDVEKWGPFDLVYGSTQPLGSSCDRCPGWYMFQFHRILQYALPRQESQRP
FFWIFMDNLLLTEDDQETTTRFLQTEAVTLQDVRGRDYQNAMRVWSNIPGLKSKH
APLTPKEEEYLQAQVRSRSKLDAPKVDLLVKNCLLPLREYFKYFSQNSLPLGGPSSG
APPPSGGSPAGSPTSTEEGTSESATPESGPGTSTEPSEGSAPGSPAGSPTSTEEGTSTEPSEGSA
PGTSTEPSEPKKKRKVMDKKYSIGLAIGTNSVGWAVITDEYKVPSKKFKVLGNTDRHSIK
KNLIGALLFDSGETAEATRLKRTARRRYTRRKNRICYLQEIFSNEMAKVDDSFFHRLEES
FLVEEDKKHERHPIFGNIVDEVAYHEKYPTIYHLRKKLVDSTDKADLRLIYLALAHMIKF
RGHFLIEGDLNPDNSDVDKLFIQLVQTYNQLFEENPINASGVDAKAILSARLSKSRRLEN
LIAQLPGEKKNGLFGNLIALSLGLTPNFKSNFDLAEDAKLQLSKDTYDDDLDNLLAQIGD
QYADLFLAAKNLSDAILLSDILRVNTEITKAPLSASMIKRYDEHHQDLTLLKALVRQQLP
EKYKEIFFDQSKNGYAGYIDGGASQEEFYKFIKPILEKMDGTEELLVKLNREDLLRKQRT
FDNGSIPHQIHLGELHAILRRQEDFYPFLKDNREKIEKILTFRIPYYVGPLARGNSRFAW
MTRKSEETITPWNFEEVVDKGASAQSFIERMTNFDKNLPNEKVLPKHSLLYEYFTVYNE
LTKVKYVTEGMRKPAFLSGEQKKAIVDLLFKTNRKVTVKQLKEDYFKKIECFDSVEISGV
EDRFNASLGTYHDLLKIIKDKDFLDNEENEDILEDIVLTLTLFEDREMIEERLKTYAHLF

DDKVMKQLKRRRYTGWGRLSRKLINGIRDKQSGKTILDFLKSDGFANRNFMQLIHDDSL
TFKEDIQKAQVSGQGDSLHEHIANLAGSPAIKKGILQTVKVVDELVKVMGRHKPENIVIE
MARENQTTQKGQKNSRERMKRIEEGIKELGSQILKEHPVENTQLQNEKLYLYYLQNGRD
MYVDQELDINRLSDYDVDAIVPQSFLKDDSIDNKVLTRSDKNRGKSDNVPSEEVVKKMK
NYWRQLLNAKLITQRKFDNLTKAERGGLSELDKAGFIKRQLVETRQITKHVAQILDSRM
NTKYDENDKLIREVKVITLKSKLVSDFRKDFQFYKVREINNYHHAHDAYLNAVVGTALIK
KYPKLESEFVYGDYKVYDVRKMIAKSEQEIGKATAKYFFYSNIMNFFKTEITLANGEIRK
RPLIETNGETGEIVWDKGRDFATVRKVLSMPQVNIVKKTEVQTGGFSKESILPKRNSDKL
IARKKDWDPKKYGGFDSPTVAYSVLVVAKVEKGKSKKLKSVKELLGITIMERSSFEKNPI
DFLEAKGYKEVKKDLIIKLPKYSLFELENGRKRMLASAGELQKGNELALPSKYVNFLYLA
SHYEKLKGSPEDNEQKQLFVEQHKHYLDEIIEQISEFSKRVILADANLDKVLSAYNKHRD
KPIREQAENIIHLFTLTNLGAPAAFKYFDTTIDRKRYTSTKEVLDATLIHQSITGLYETRID
LSQLGGDAYPYDVPDYASLGSGSPKKKRKVEDPKKKRKVDGSGSETPGTSESATPESMD
QMPNLFRGSKACIHFIRSSVDDVLSMTASYIVSAVIPSMQHPTHIRGRTQKQLWTTS
LVDHSVTSICHSYLGTKWVYIACWILDRRFNEWHLQKEGAKEFAAALTAERIKTPP
KRPGGRRRGRLPNNSSRPSTPTISVLESKDTDSDREAGTETGGENNDKEEEEKKDET
SSSSEANSRCQTPIKMKPNIEPPENVEWSGAEASMFRVLIGTYYDNFCAIARLIGTKT
CRQVYEFRVKESSIIAPVPTEDVDTPPRKKKRKHRLWAAHCRKIQLKKDGSSNHVY
NYQPCDHPRQPCDSSCPCVIAQNFCEKFCQCSSECQNRFPGCRCKAQCNTKQCPCY
LAVRECDPDLCLTCGAADHWDSKNVSCKNCSIQRGSKKHLLLAPSDVAGWGIFIKD
PVQKNEFISEYCGEIISQDEADRRGKVYDKYMCSFLFNLNNDFVVDATRKGNKIRFA
NHSVNPNCYAKVMMVNGDHRIGIFAKRAIQTGEELFFDYRYSQADALKYVGIERE
MEIP*

The amino acid sequence of DNMT3A-DNMT3L-XTEN80-dCas9-XTEN16-EZH2 (V62 scheme, SEQ ID NO: 113) is as follows:

MPAAKRVKLDGSNHDQEFDPPKVYPPVPAEKRKPIRVLSLFDGIATGLLVLKDL
GIQVDRYIASEVCEDSITVGMVRHQGKIMYVGDVRSVTQKHIQEWGPFDLVIGGSP

CNDLSIVNPARKGLYEGTGRLFFEFYRLLHDARPKEGDDRPFFWLFENVVAMGVSD
KRDISRFLESNPVMIDAKEVSAAHRARYFWGNLPGMNRPLASTVNDKLELQECLEH
GRIAKFSKVRTITTRSNSIKQGKDQHFPVFMNEKEDILWCTEMERVFGFPVHYTDV
SNMSRLARQRLLGRSWSVPVIRHLFAPLKEYFACVSSGNSNANSRGPSFSSGLVPLSLRG
SHMGPMEIYKTVSAWKRQPVRVLSLFRNIDKVLKSLGFLESGSGSGGGTLKYVEDV
TNVVRRDVEKWGPFDLVYGSTQPLGSSCDRCPGWYMFQFHRILQYALPRQESQRP
FFWIFMDNLLLTEDDQETTTRFLQTEAVTLQDVRGRDYQNAMRVWSNIPGLKSKH
APLTPKEEEYLQAQVRSRSKLDAPKVDLLVKNCLLPLREYFKYFSQNSLPLGGPSSG
APPPSGGSPAGSPTSTEEGTSESATPESGPGTSTEPSEGSAPGSPAGSPTSTEEGTSTEPSEGSA
PGTSTEPSEPKKKRKVMDKKYSIGLAIGTNSVGWAVITDEYKVPSKKFKVLGNTDRHSIK
KNLIGALLFDSGETAEATRLKRTARRRYTRRKNRICYLQEIFSNEMAKVDDSFFHRLEES
FLVEEDKKHERHPIFGNIVDEVAYHEKYPTIYHLRKKLVDSTDKADLRLIYLALAHMIKF
RGHFLIEGDLNPDNSDVDKLFIQLVQTYNQLFEENPINASGVDAKAILSARLSKSRRLEN
LIAQLPGEKKNGLFGNLIALSLGLTPNFKSNFDLAEDAKLQLSKDTYDDDLDNLLAQIGD
QYADLFLAAKNLSDAILLSDILRVNTEITKAPLSASMIKRYDEHHQDLTLLKALVRQQLP
EKYKEIFFDQSKNGYAGYIDGGASQEEFYKFIKPILEKMDGTEELLVKLNREDLLRKQRT
FDNGSIPHQIHLGELHAILRRQEDFYPFLKDNREKIEKILTFRIPYYVGPLARGNSRFAW
MTRKSEETITPWNFEEVVDKGASAQSFIERMTNFDKNLPNEKVLPKHSLLYEYFTVYNE
LTKVKYVTEGMRKPAFLSGEQKKAIVDLLFKTNRKVTVKQLKEDYFKKIECFDSVEISGV
EDRFNASLGTYHDLLKIIKDKDFLDNEENEDILEDIVLTLTLFEDREMIEERLKTYAHLF
DDKVMKQLKRRRYTGWGRLSRKLINGIRDKQSGKTILDFLKSDGFANRNFMQLIHDDSL
TFKEDIQKAQVSGQGDSLHEHIANLAGSPAIKKGILQTVKVVDELVKVMGRHKPENIVIE
MARENQTTQKGQKNSRERMKRIEEGIKELGSQILKEHPVENTQLQNEKLYLYYLQNGRD
MYVDQELDINRLSDYDVDAIVPQSFLKDDSIDNKVLTRSDKNRGKSDNVPSEEVVKKMK
NYWRQLLNAKLITQRKFDNLTKAERGGLSELDKAGFIKRQLVETRQITKHVAQILDSRM
NTKYDENDKLIREVKVITLKSKLVSDFRKDFQFYKVREINNYHHAHDAYLNAVVGTALIK
KYPKLESEFVYGDYKVYDVRKMIAKSEQEIGKATAKYFFYSNIMNFFKTEITLANGEIRK

RPLIETNGETGEIVWDKGRDFATVRKVLSMPQVNIVKKTEVQTGGFSKESILPKRNSDKL
IARKKDWDPKKYGGFDSPTVAYSVLVVAKVEKGKSKKLKSVKELLGITIMERSSFEKNPI
DFLEAKGYKEVKKDLIIKLPKYSLFELENGRKRMLASAGELQKGNELALPSKYVNFLYLA
SHYEKLKGSPEDNEQKQLFVEQHKHYLDEIIEQISEFSKRVILADANLDKVLSAYNKHRD
KPIREQAENIIHLFTLTNLGAPAAFKYFDTTIDRKRYTSTKEVLDATLIHQSITGLYETRID
LSQLGGDAYPYDVPDYASLGSGSPKKKRKVEDPKKKRKVDGSGSETPGTSESATPESRTL
GQTGKKSEKGPVCWRKRVKSEYMRLRQLKRFRRADEVKSMFSSNRQKILERTEIL
NQEWKQRRIQPVHILTSVSSLRGTRECSVTSDLDFPTQVIPLKTLNAVASVPIMYSW
SPLQQNFMVEDETVLHNIPYMGDEVLDQDGTFIEELIKNYDGKVHGDRECGFINDE
IFVELVNALGQYNDDDDDDDGDDPEEREEKQKDLEDHRDDKESRPPRKFPSDKIFE
AISSMFPDKGTAEELKEKYKELTEQQLPGALPPECTPNIDGPNAKSVQREQSLHSFH
TLFCRRCFKYDCFLHPFHATPNTYKRKNTETALDNKPCGPQCYQHLEGAKEFAAA
LTAERIKTPPKRPGGRRRGRLPNNSSRPSTPTINVLESKDTDSDREAGTETGGENND
KEEEEKKDETSSSSEANSRCQTPIKMKPNIEPPENVEWSGAEASMFRVLIGTYYDNF
CAIARLIGTKTCRQVYEFRVKESSIIAPAPAEDVDTPPRKKKRKHRLWAAHCRKIQL
KKDGSSNHVYNYQPCDHPRQPCDSSCPCVIAQNFCEKFCQCSSECQNRFPGCRCKA
QCNTKQCPCYLAVRECDPDLCLTCGAADHWDSKNVSCKNCSIQRGSKKHLLLAPS
DVAGWGIFIKDPVQKNEFISEYCGEIISQDEADRRGKVYDKYMCSFLFNLNNDFVVD
ATRKGNKIRFANHSVNPNCYAKVMMVNGDHRIGIFAKRAIQTGEELFFDYRYSQAD
ALKYVGIEREMEIPEGSG*

The amino acid sequence of DNMT3A-DNMT3L-XTEN80-dCas9-XTEN16-EZH2 (core) (V63 scheme, SEQ ID NO: 114) is as follows:

MPAAKRVKLDGSNHDQEFDPPKVYPPVPAEKRKPIRVLSLFDGIATGLLVLKDL
GIQVDRYIASEVCEDSITVGMVRHQGKIMYVGDVRSVTQKHIQEWGPFDLVIGGSP
CNDLSIVNPARKGLYEGTGRLFFEFYRLLHDARPKEGDDRPFFWLFENVVAMGVSD
KRDISRFLESNPVMIDAKEVSAAHRARYFWGNLPGMNRPLASTVNDKLELQECLEH
GRIAKFSKVRTITTRSNSIKQGKDQHFPVFMNEKEDILWCTEMERVFGFPVHYTDV

SNMSRLARQRLLGRSWSVPVIRHLFAPLKEYFACVSSGNSNANSRGPSFSSGLVPLSLRG
SHMGPMEIYKTVSAWKRQPVRVLSLFRNIDKVLKSLGFLESGSGSGGGTLKYVEDV
TNVVRRDVEKWGPFDLVYGSTQPLGSSCDRCPGWYMFQFHRILQYALPRQESQRP
FFWIFMDNLLLTEDDQETTTRFLQTEAVTLQDVRGRDYQNAMRVWSNIPGLKSKH
APLTPKEEEYLQAQVRSRSKLDAPKVDLLVKNCLLPLREYFKYFSQNSLPLGGPSSG
APPPSGGSPAGSPTSTEEGTSESATPESGPGTSTEPSEGSAPGSPAGSPTSTEEGTSTEPSEGSA
PGTSTEPSEPKKKRKVMDKKYSIGLAIGTNSVGWAVITDEYKVPSKKFKVLGNTDRHSIK
KNLIGALLFDSGETAEATRLKRTARRRYTRRKNRICYLQEIFSNEMAKVDDSFFHRLEES
FLVEEDKKHERHPIFGNIVDEVAYHEKYPTIYHLRKKLVDSTDKADLRLIYLALAHMIKF
RGHFLIEGDLNPDNSDVDKLFIQLVQTYNQLFEENPINASGVDAKAILSARLSKSRRLEN
LIAQLPGEKKNGLFGNLIALSLGLTPNFKSNFDLAEDAKLQLSKDTYDDDLDNLLAQIGD
QYADLFLAAKNLSDAILLSDILRVNTEITKAPLSASMIKRYDEHHQDLTLLKALVRQQLP
EKYKEIFFDQSKNGYAGYIDGGASQEEFYKFIKPILEKMDGTEELLVKLNREDLLRKQRT
FDNGSIPHQIHLGELHAILRRQEDFYPFLKDNREKIEKILTFRIPYYVGPLARGNSRFAW
MTRKSEETITPWNFEEVVDKGASAQSFIERMTNFDKNLPNEKVLPKHSLLYEYFTVYNE
LTKVKYVTEGMRKPAFLSGEQKKAIVDLLFKTNRKVTVKQLKEDYFKKIECFDSVEISGV
EDRFNASLGTYHDLLKIIKDKDFLDNEENEDILEDIVLTLTLFEDREMIEERLKTYAHLF
DDKVMKQLKRRRYTGWGRLSRKLINGIRDKQSGKTILDFLKSDGFANRNFMQLIHDDSL
TFKEDIQKAQVSGQGDSLHEHIANLAGSPAIKKGILQTVKVVDELVKVMGRHKPENIVIE
MARENQTTQKGQKNSRERMKRIEEGIKELGSQILKEHPVENTQLQNEKLYLYYLQNGRD
MYVDQELDINRLSDYDVDAIVPQSFLKDDSIDNKVLTRSDKNRGKSDNVPSEEVVKKMK
NYWRQLLNAKLITQRKFDNLTKAERGGLSELDKAGFIKRQLVETRQITKHVAQILDSRM
NTKYDENDKLIREVKVITLKSKLVSDFRKDFQFYKVREINNYHHAHDAYLNAVVGTALIK
KYPKLESEFVYGDYKVYDVRKMIAKSEQEIGKATAKYFFYSNIMNFFKTEITLANGEIRK
RPLIETNGETGEIVWDKGRDFATVRKVLSMPQVNIVKKTEVQTGGFSKESILPKRNSDKL
IARKKDWDPKKYGGFDSPTVAYSVLVVAKVEKGKSKKLKSVKELLGITIMERSSFEKNPI
DFLEAKGYKEVKKDLIIKLPKYSLFELENGRKRMLASAGELQKGNELALPSKYVNFLYLA

SHYEKLKGSPEDNEQKQLFVEQHKHYLDEIIEQISEFSKRVILADANLDKVLSAYNKHRD
KPIREQAENIIHLFTLTNLGAPAAFKYFDTTIDRKRYTSTKEVLDATLIHQSITGLYETRID
LSQLGGDAYPYDVPDYASLGSGSPKKKRKVEDPKKKRKVDGSGSETPGTSESATPESRTL
EDVDTPPRKKKRKHRLWAAHCRKIQLKKDGSSNHVYNYQPCDHPRQPCDSSCPCV
IAQNFCEKFCQCSSECQNRFPGCRCKAQCNTKQCPCYLAVRECDPDLCLTCGAAD
HWDSKNVSCKNCSIQRGSKKHLLLAPSDVAGWGIFIKDPVQKNEFISEYCGEIISQD
EADRRGKVYDKYMCSFLFNLNNDFVVDATRKGNKIRFANHSVNPNCYAKVMMVN
GDHRIGIFAKRAIQTGEELFFDYRYSQADALKYVGIEREMEIPEGSG*

The amino acid sequence of DNMT3A-DNMT3L-XTEN80-dCas9-XTEN16-HDAC3 (V64 scheme, SEQ ID NO: 115) is as follows:

MPAAKRVKLDGSNHDQEFDPPKVYPPVPAEKRKPIRVLSLFDGIATGLLVLKDL
GIQVDRYIASEVCEDSITVGMVRHQGKIMYVGDVRSVTQKHIQEWGPFDLVIGGSP
CNDLSIVNPARKGLYEGTGRLFFEFYRLLHDARPKEGDDRPFFWLFENVVAMGVSD
KRDISRFLESNPVMIDAKEVSAAHRARYFWGNLPGMNRPLASTVNDKLELQECLEH
GRIAKFSKVRTITTRSNSIKQGKDQHFPVFMNEKEDILWCTEMERVFGFPVHYTDV
SNMSRLARQRLLGRSWSVPVIRHLFAPLKEYFACVSSGNSNANSRGPSFSSGLVPLSLRG
SHMGPMEIYKTVSAWKRQPVRVLSLFRNIDKVLKSLGFLESGSGSGGGTLKYVEDV
TNVVRRDVEKWGPFDLVYGSTQPLGSSCDRCPGWYMFQFHRILQYALPRQESQRP
FFWIFMDNLLLTEDDQETTTRFLQTEAVTLQDVRGRDYQNAMRVWSNIPGLKSKH
APLTPKEEEYLQAQVRSRSKLDAPKVDLLVKNCLLPLREYFKYFSQNSLPLGGPSSG
APPPSGGSPAGSPTSTEEGTSESATPESGPGTSTEPSEGSAPGSPAGSPTSTEEGTSTEPSEGSA
PGTSTEPSEPKKKRKVMDKKYSIGLAIGTNSVGWAVITDEYKVPSKKFKVLGNTDRHSIK
KNLIGALLFDSGETAEATRLKRTARRRYTRRKNRICYLQEIFSNEMAKVDDSFFHRLEES
FLVEEDKKHERHPIFGNIVDEVAYHEKYPTIYHLRKKLVDSTDKADLRLIYLALAHMIKF
RGHFLIEGDLNPDNSDVDKLFIQLVQTYNQLFEENPINASGVDAKAILSARLSKSRRLEN
LIAQLPGEKKNGLFGNLIALSLGLTPNFKSNFDLAEDAKLQLSKDTYDDDLDNLLAQIGD
QYADLFLAAKNLSDAILLSDILRVNTEITKAPLSASMIKRYDEHHQDLTLLKALVRQQLP

EKYKEIFFDQSKNGYAGYIDGGASQEEFYKFIKPILEKMDGTEELLVKLNREDLLRKQRT
FDNGSIPHQIHLGELHAILRRQEDFYPFLKDNREKIEKILTFRIPYYVGPLARGNSRFAW
MTRKSEETITPWNFEEVVDKGASAQSFIERMTNFDKNLPNEKVLPKHSLLYEYFTVYNE
LTKVKYVTEGMRKPAFLSGEQKKAIVDLLFKTNRKVTVKQLKEDYFKKIECFDSVEISGV
EDRFNASLGTYHDLLKIIKDKDFLDNEENEDILEDIVLTLTLFEDREMIEERLKTYAHLF
DDKVMKQLKRRRYTGWGRLSRKLINGIRDKQSGKTILDFLKSDGFANRNFMQLIHDDSL
TFKEDIQKAQVSGQGDSLHEHIANLAGSPAIKKGILQTVKVVDELVKVMGRHKPENIVIE
MARENQTTQKGQKNSRERMKRIEEGIKELGSQILKEHPVENTQLQNEKLYLYYLQNGRD
MYVDQELDINRLSDYDVDAIVPQSFLKDDSIDNKVLTRSDKNRGKSDNVPSEEVVKKMK
NYWRQLLNAKLITQRKFDNLTKAERGGLSELDKAGFIKRQLVETRQITKHVAQILDSRM
NTKYDENDKLIREVKVITLKSKLVSDFRKDFQFYKVREINNYHHAHDAYLNAVVGTALIK
KYPKLESEFVYGDYKVYDVRKMIAKSEQEIGKATAKYFFYSNIMNFFKTEITLANGEIRK
RPLIETNGETGEIVWDKGRDFATVRKVLSMPQVNIVKKTEVQTGGFSKESILPKRNSDKL
IARKKDWDPKKYGGFDSPTVAYSVLVVAKVEKGKSKKLKSVKELLGITIMERSSFEKNPI
DFLEAKGYKEVKKDLIIKLPKYSLFELENGRKRMLASAGELQKGNELALPSKYVNFLYLA
SHYEKLKGSPEDNEQKQLFVEQHKHYLDEIIEQISEFSKRVILADANLDKVLSAYNKHRD
KPIREQAENIIHLFTLTNLGAPAAFKYFDTTIDRKRYTSTKEVLDATLIHQSITGLYETRID
LSQLGGDAYPYDVPDYASLGSGSPKKKRKVEDPKKKRKVDGSGSETPGTSESATPESRTL
AKTVAYFYDPDVGNFHYGAGHPMKPHRLALTHSLVLHYGLYKKMIVFKPYQASQ
HDMCRFHSEDYIDFLQRVSPTNMQGFTKSLNAFNVGDDCPVFPGLFEFCSRYTGAS
LQGATQLNNKICDIAINWAGGLHHAKKFEASGFCYVNDIVIGILELLKYHPRVLYID
IDIHHGDGVQEAFYLTDRVMTVSFHKYGNYFFPGTGDMYEVGAESGRYYCLNVPL
RDGIDDQSYKHLFQPVINQVVDFYQPTCIVLQCGADSLGCDRLGCFNLSIRGHGEC
VEYVKSFNIPLLVLGGGGYTVRNVARCWTYETSLLVEEAISEELPYSEYFEYFAPDF
TLHPDVSTRIENQNSRQYLDQIRQTIFENLKMLNHAPSVQIHDVPADLLTYDRTDEA
DAEERGPEENYSRPEAPNEFYDGDHDNDKESDVEIEGSG*

The amino acid sequence of DNMT3A-DNMT3L-XTEN80-dCas9-XTEN16-HDAC3(core 1) (V65 scheme, SEQ ID NO: 116) is as follows:

MPAAKRVKLDGSNHDQEFDPPKVYPPVPAEKRKPIRVLSLFDGIATGLLVLKDL
GIQVDRYIASEVCEDSITVGMVRHQGKIMYVGDVRSVTQKHIQEWGPFDLVIGGSP
CNDLSIVNPARKGLYEGTGRLFFEFYRLLHDARPKEGDDRPFFWLFENVVAMGVSD
KRDISRFLESNPVMIDAKEVSAAHRARYFWGNLPGMNRPLASTVNDKLELQECLEH
GRIAKFSKVRTITTRSNSIKQGKDQHFPVFMNEKEDILWCTEMERVFGFPVHYTDV
SNMSRLARQRLLGRSWSVPVIRHLFAPLKEYFACVSSGNSNANSRGPSFSSGLVPLSLRG
SHMGPMEIYKTVSAWKRQPVRVLSLFRNIDKVLKSLGFLESGSGSGGGTLKYVEDV
TNVVRRDVEKWGPFDLVYGSTQPLGSSCDRCPGWYMFQFHRILQYALPRQESQRP
FFWIFMDNLLLTEDDQETTTRFLQTEAVTLQDVRGRDYQNAMRVWSNIPGLKSKH
APLTPKEEEYLQAQVRSRSKLDAPKVDLLVKNCLLPLREYFKYFSQNSLPLGGPSSG
APPPSGGSPAGSPTSTEEGTSESATPESGPGTSTEPSEGSAPGSPAGSPTSTEEGTSTEPSEGSA
PGTSTEPSEPKKKRKVMDKKYSIGLAIGTNSVGWAVITDEYKVPSKKFKVLGNTDRHSIK
KNLIGALLFDSGETAEATRLKRTARRRYTRRKNRICYLQEIFSNEMAKVDDSFFHRLEES
FLVEEDKKHERHPIFGNIVDEVAYHEKYPTIYHLRKKLVDSTDKADLRLIYLALAHMIKF
RGHFLIEGDLNPDNSDVDKLFIQLVQTYNQLFEENPINASGVDAKAILSARLSKSRRLEN
LIAQLPGEKKNGLFGNLIALSLGLTPNFKSNFDLAEDAKLQLSKDTYDDDLDNLLAQIGD
QYADLFLAAKNLSDAILLSDILRVNTEITKAPLSASMIKRYDEHHQDLTLLKALVRQQLP
EKYKEIFFDQSKNGYAGYIDGGASQEEFYKFIKPILEKMDGTEELLVKLNREDLLRKQRT
FDNGSIPHQIHLGELHAILRRQEDFYPFLKDNREKIEKILTFRIPYYVGPLARGNSRFAW
MTRKSEETITPWNFEEVVDKGASAQSFIERMTNFDKNLPNEKVLPKHSLLYEYFTVYNE
LTKVKYVTEGMRKPAFLSGEQKKAIVDLLFKTNRKVTVKQLKEDYFKKIECFDSVEISGV
EDRFNASLGTYHDLLKIIKDKDFLDNEENEDILEDIVLTLTLFEDREMIEERLKTYAHLF
DDKVMKQLKRRRYTGWGRLSRKLINGIRDKQSGKTILDFLKSDGFANRNFMQLIHDDSL
TFKEDIQKAQVSGQGDSLHEHIANLAGSPAIKKGILQTVKVVDELVKVMGRHKPENIVIE
MARENQTTQKGQKNSRERMKRIEEGIKELGSQILKEHPVENTQLQNEKLYLYYLQNGRD

MYVDQELDINRLSDYDVDAIVPQSFLKDDSIDNKVLTRSDKNRGKSDNVPSEEVVKKMK
NYWRQLLNAKLITQRKFDNLTKAERGGLSELDKAGFIKRQLVETRQITKHVAQILDSRM
NTKYDENDKLIREVKVITLKSKLVSDFRKDFQFYKVREINNYHHAHDAYLNAVVGTALIK
KYPKLESEFVYGDYKVYDVRKMIAKSEQEIGKATAKYFFYSNIMNFFKTEITLANGEIRK
RPLIETNGETGEIVWDKGRDFATVRKVLSMPQVNIVKKTEVQTGGFSKESILPKRNSDKL
IARKKDWDPKKYGGFDSPTVAYSVLVVAKVEKGKSKKLKSVKELLGITIMERSSFEKNPI
DFLEAKGYKEVKKDLIIKLPKYSLFELENGRKRMLASAGELQKGNELALPSKYVNFLYLA
SHYEKLKGSPEDNEQKQLFVEQHKHYLDEIIEQISEFSKRVILADANLDKVLSAYNKHRD
KPIREQAENIIHLFTLTNLGAPAAFKYFDTTIDRKRYTSTKEVLDATLIHQSITGLYETRID
LSQLGGDAYPYDVPDYASLGSGSPKKKRKVEDPKKKRKVDGSGSETPGTSESATPESRTL
AKTVAYFYDPDVGNFHYGAGHPMKPHRLALTHSLVLHYGLYKKMIVFKPYQASQ
HDMCRFHSEDYIDFLQRVSPTNMQGFTKSLNAFNVGDDCPVFPGLFEFCSRYTGAS
LQGATQLNNKICDIAINWAGGLHHAKKFEASGFCYVNDIVIGILELLKYHPRVLYID
IDIHHGDGVQEAFYLTDRVMTVSFHKYGNYFFPGTGDMYEVGAESGRYYCLNVPL
RDGIDDQSYKHLFQPVINQVVDFYQPTCIVLQCGADSLGCDRLGCFNLSIRGHGEC
VEYVKSFNIPLLVLGGGGYTVRNVARCWTYETSLLVEEGSG*

The amino acid sequence of DNMT3A-DNMT3L-XTEN80-dCas9-XTEN16-HDAC3(core2) (V66 scheme, SEQ ID NO: 117) is as follows:

MPAAKRVKLDGSNHDQEFDPPKVYPPVPAEKRKPIRVLSLFDGIATGLLVLKDL
GIQVDRYIASEVCEDSITVGMVRHQGKIMYVGDVRSVTQKHIQEWGPFDLVIGGSP
CNDLSIVNPARKGLYEGTGRLFFEFYRLLHDARPKEGDDRPFFWLFENVVAMGVSD
KRDISRFLESNPVMIDAKEVSAAHRARYFWGNLPGMNRPLASTVNDKLELQECLEH
GRIAKFSKVRTITTRSNSIKQGKDQHFPVFMNEKEDILWCTEMERVFGFPVHYTDV
SNMSRLARQRLLGRSWSVPVIRHLFAPLKEYFACVSSGNSNANSRGPSFSSGLVPLSLRG
SHMGPMEIYKTVSAWKRQPVRVLSLFRNIDKVLKSLGFLESGSGSGGGTLKYVEDV
TNVVRRDVEKWGPFDLVYGSTQPLGSSCDRCPGWYMFQFHRILQYALPRQESQRP
FFWIFMDNLLLTEDDQETTTRFLQTEAVTLQDVRGRDYQNAMRVWSNIPGLKSKH

APLTPKEEEYLQAQVRSRSKLDAPKVDLLVKNCLLPLREYFKYFSQNSLPLGGPSSG
APPPSGGSPAGSPTSTEEGTSESATPESGPGTSTEPSEGSAPGSPAGSPTSTEEGTSTEPSEGSA
PGTSTEPSEPKKKRKVMDKKYSIGLAIGTNSVGWAVITDEYKVPSKKFKVLGNTDRHSIK
KNLIGALLFDSGETAEATRLKRTARRRYTRRKNRICYLQEIFSNEMAKVDDSFFHRLEES
FLVEEDKKHERHPIFGNIVDEVAYHEKYPTIYHLRKKLVDSTDKADLRLIYLALAHMIKF
RGHFLIEGDLNPDNSDVDKLFIQLVQTYNQLFEENPINASGVDAKAILSARLSKSRRLEN
LIAQLPGEKKNGLFGNLIALSLGLTPNFKSNFDLAEDAKLQLSKDTYDDDLDNLLAQIGD
QYADLFLAAKNLSDAILLSDILRVNTEITKAPLSASMIKRYDEHHQDLTLLKALVRQQLP
EKYKEIFFDQSKNGYAGYIDGGASQEEFYKFIKPILEKMDGTEELLVKLNREDLLRKQRT
FDNGSIPHQIHLGELHAILRRQEDFYPFLKDNREKIEKILTFRIPYYVGPLARGNSRFAW
MTRKSEETITPWNFEEVVDKGASAQSFIERMTNFDKNLPNEKVLPKHSLLYEYFTVYNE
LTKVKYVTEGMRKPAFLSGEQKKAIVDLLFKTNRKVTVKQLKEDYFKKIECFDSVEISGV
EDRFNASLGTYHDLLKIIKDKDFLDNEENEDILEDIVLTLTLFEDREMIEERLKTYAHLF
DDKVMKQLKRRRYTGWGRLSRKLINGIRDKQSGKTILDFLKSDGFANRNFMQLIHDDSL
TFKEDIQKAQVSGQGDSLHEHIANLAGSPAIKKGILQTVKVVDELVKVMGRHKPENIVIE
MARENQTTQKGQKNSRERMKRIEEGIKELGSQILKEHPVENTQLQNEKLYLYYLQNGRD
MYVDQELDINRLSDYDVDAIVPQSFLKDDSIDNKVLTRSDKNRGKSDNVPSEEVVKKMK
NYWRQLLNAKLITQRKFDNLTKAERGGLSELDKAGFIKRQLVETRQITKHVAQILDSRM
NTKYDENDKLIREVKVITLKSKLVSDFRKDFQFYKVREINNYHHAHDAYLNAVVGTALIK
KYPKLESEFVYGDYKVYDVRKMIAKSEQEIGKATAKYFFYSNIMNFFKTEITLANGEIRK
RPLIETNGETGEIVWDKGRDFATVRKVLSMPQVNIVKKTEVQTGGFSKESILPKRNSDKL
IARKKDWDPKKYGGFDSPTVAYSVLVVAKVEKGKSKKLKSVKELLGITIMERSSFEKNPI
DFLEAKGYKEVKKDLIIKLPKYSLFELENGRKRMLASAGELQKGNELALPSKYVNFLYLA
SHYEKLKGSPEDNEQKQLFVEQHKHYLDEIIEQISEFSKRVILADANLDKVLSAYNKHRD
KPIREQAENIIHLFTLTNLGAPAAFKYFDTTIDRKRYTSTKEVLDATLIHQSITGLYETRID
LSQLGGDAYPYDVPDYASLGSGSPKKKRKVEDPKKKRKVDGSGSETPGTSESATPESRTL
AKTVAYFYDPDVGNFHYGAGHPMKPHRLALTHSLVLHYGLYKKMIVFKPYQASQ

HDMCRFHSEDYIDFLQRVSPTNMQGFTKSLNAFNVGDDCPVFPGLFEFCSRYTGAS

LQGATQLNNKICDIAINWAGGLHHAKKFEASGFCYVNDIVIGILELLKYHPRVLYID

IDIHHGDGVQEAFYLTDRVMTVSFHKYGNYFFPGTGDMYEVGAESGRYYCLNVPL

RDGIDDQSYKHLFQPVINQVVDFYQPTCIVLQCGADSLGCDRLGCFNLSIRGHGEC

VEYVKSFNIPLLVLGGGGYTVRNVARCWTYETSLLVEEAISEELPYSEYFEYFAPDF

TLHPDVSTRIENQNSRQYLDQIRQTIFENLEGSG*

The amino acid sequence of DNMT3A-DNMT3L-XTEN80-dCas9-XTEN16-EHMT2 (V67 scheme, SEQ ID NO: 118) is as follows:

MPAAKRVKLDGSNHDQEFDPPKVYPPVPAEKRKPIRVLSLFDGIATGLLVLKDL
GIQVDRYIASEVCEDSITVGMVRHQGKIMYVGDVRSVTQKHIQEWGPFDLVIGGSP
CNDLSIVNPARKGLYEGTGRLFFEFYRLLHDARPKEGDDRPFFWLFENVVAMGVSD
KRDISRFLESNPVMIDAKEVSAAHRARYFWGNLPGMNRPLASTVNDKLELQECLEH
GRIAKFSKVRTITTRSNSIKQGKDQHFPVFMNEKEDILWCTEMERVFGFPVHYTDV
SNMSRLARQRLLGRSWSVPVIRHLFAPLKEYFACVSSGNSNANSRGPSFSSGLVPLSLRG
SHMGPMEIYKTVSAWKRQPVRVLSLFRNIDKVLKSLGFLESGSGSGGGTLKYVEDV
TNVVRRDVEKWGPFDLVYGSTQPLGSSCDRCPGWYMFQFHRILQYALPRQESQRP
FFWIFMDNLLLTEDDQETTTRFLQTEAVTLQDVRGRDYQNAMRVWSNIPGLKSKH
APLTPKEEEYLQAQVRSRSKLDAPKVDLLVKNCLLPLREYFKYFSQNSLPLGGPSSG
APPPSGGSPAGSPTSTEEGTSESATPESGPGTSTEPSEGSAPGSPAGSPTSTEEGTSTEPSEGSA
PGTSTEPSEPKKKRKVMDKKYSIGLAIGTNSVGWAVITDEYKVPSKKFKVLGNTDRHSIK
KNLIGALLFDSGETAEATRLKRTARRRYTRRKNRICYLQEIFSNEMAKVDDSFFHRLEES
FLVEEDKKHERHPIFGNIVDEVAYHEKYPTIYHLRKKLVDSTDKADLRLIYLALAHMIKF
RGHFLIEGDLNPDNSDVDKLFIQLVQTYNQLFEENPINASGVDAKAILSARLSKSRRLEN
LIAQLPGEKKNGLFGNLIALSLGLTPNFKSNFDLAEDAKLQLSKDTYDDDLDNLLAQIGD
QYADLFLAAKNLSDAILLSDILRVNTEITKAPLSASMIKRYDEHHQDLTLLKALVRQQLP
EKYKEIFFDQSKNGYAGYIDGGASQEEFYKFIKPILEKMDGTEELLVKLNREDLLRKQRT
FDNGSIPHQIHLGELHAILRRQEDFYPFLKDNREKIEKILTFRIPYYVGPLARGNSRFAW

MTRKSEETITPWNFEEVVDKGASAQSFIERMTNFDKNLPNEKVLPKHSLLYEYFTVYNE
LTKVKYVTEGMRKPAFLSGEQKKAIVDLLFKTNRKVTVKQLKEDYFKKIECFDSVEISGV
EDRFNASLGTYHDLLKIIKDKDFLDNEENEDILEDIVLTLTLFEDREMIEERLKTYAHLF
DDKVMKQLKRRRYTGWGRLSRKLINGIRDKQSGKTILDFLKSDGFANRNFMQLIHDDSL
TFKEDIQKAQVSGQGDSLHEHIANLAGSPAIKKGILQTVKVVDELVKVMGRHKPENIVIE
MARENQTTQKGQKNSRERMKRIEEGIKELGSQILKEHPVENTQLQNEKLYLYYLQNGRD
MYVDQELDINRLSDYDVDAIVPQSFLKDDSIDNKVLTRSDKNRGKSDNVPSEEVVKKMK
NYWRQLLNAKLITQRKFDNLTKAERGGLSELDKAGFIKRQLVETRQITKHVAQILDSRM
NTKYDENDKLIREVKVITLKSKLVSDFRKDFQFYKVREINNYHHAHDAYLNAVVGTALIK
KYPKLESEFVYGDYKVYDVRKMIAKSEQEIGKATAKYFFYSNIMNFFKTEITLANGEIRK
RPLIETNGETGEIVWDKGRDFATVRKVLSMPQVNIVKKTEVQTGGFSKESILPKRNSDKL
IARKKDWDPKKYGGFDSPTVAYSVLVVAKVEKGKSKKLKSVKELLGITIMERSSFEKNPI
DFLEAKGYKEVKKDLIIKLPKYSLFELENGRKRMLASAGELQKGNELALPSKYVNFLYLA
SHYEKLKGSPEDNEQKQLFVEQHKHYLDEIIEQISEFSKRVILADANLDKVLSAYNKHRD
KPIREQAENIIHLFTLTNLGAPAAFKYFDTTIDRKRYTSTKEVLDATLIHQSITGLYETRID
LSQLGGDAYPYDVPDYASLGSGSPKKKRKVEDPKKKRKVDGSGSETPGTSESATPESRTN
RAIRTEKIICRDVARGYENVPIPCVNGVDGEPCPEDYKYISENCETSTMNIDRNITHL
QHCTCVDDCSSSNCLCGQLSIRCWYDKDGRLLQEFNKIEPPLIFECNQACSCWRNC
KNRVVQSGIKVRLQLYRTAKMGWGVRALQTIPQGTFICEYVGELISDAEADVRED
DSYLFDLDNKDGEVYCIDARYYGNISRFINHLCDPNIIPVRVFMLHQDLRFPRIAFFS
SRDIRTGEELGFDYGDRFWDIKSKYFTCQCGSEKCKHSAEAIALEQSRLARLDPHPE
LLPELGSLPPVNTEGSG*

The amino acid sequence of DNMT3A-DNMT3L-XTEN80-dCas9-XTEN16-HDAC1 (V68 scheme, SEQ ID NO: 119) is as follows:

MPAAKRVKLDGSTGPATMNHDQEFDPPKVYPPVPAEKRKPIRVLSLFDGIATGLL
VLKDLGIQVDRYIASEVCEDSITVGMVRHQGKIMYVGDVRSVTQKHIQEWGPFDL
VIGGSPCNDLSIVNPARKGLYEGTGRLFFEFYRLLHDARPKEGDDRPFFWLFENVV

AMGVSDKRDISRFLESNPVMIDAKEVSAAHRARYFWGNLPGMNRPLASTVNDKLE
LQECLEHGRIAKFSKVRTITTRSNSIKQGKDQHFPVFMNEKEDILWCTEMERVFGF
PVHYTDVSNMSRLARQRLLGRSWSVPVIRHLFAPLKEYFACVSSGNSNANSRGPSFSS
GLVPLSLRGSHMGPMEIYKTVSAWKRQPVRVLSLFRNIDKVLKSLGFLESGSGSGGG
TLKYVEDVTNVVRRDVEKWGPFDLVYGSTQPLGSSCDRCPGWYMFQFHRILQYAL
PRQESQRPFFWIFMDNLLLTEDDQETTTRFLQTEAVTLQDVRGRDYQNAMRVWSN
IPGLKSKHAPLTPKEEEYLQAQVRSRSKLDAPKVDLLVKNCLLPLREYFKYFSQNSL
PLGGPSSGAPPPSGGSPAGSPTSTEEGTSESATPESGPGTSTEPSEGSAPGSPAGSPTSTEEGTS
TEPSEGSAPGTSTEPSEPKKKRKVMDKKYSIGLAIGTNSVGWAVITDEYKVPSKKFKVLGN
TDRHSIKKNLIGALLFDSGETAEATRLKRTARRRYTRRKNRICYLQEIFSNEMAKVDDSF
FHRLEESFLVEEDKKHERHPIFGNIVDEVAYHEKYPTIYHLRKKLVDSTDKADLRLIYLA
LAHMIKFRGHFLIEGDLNPDNSDVDKLFIQLVQTYNQLFEENPINASGVDAKAILSARLS
KSRRLENLIAQLPGEKKNGLFGNLIALSLGLTPNFKSNFDLAEDAKLQLSKDTYDDDLD
NLLAQIGDQYADLFLAAKNLSDAILLSDILRVNTEITKAPLSASMIKRYDEHHQDLTLLKA
LVRQQLPEKYKEIFFDQSKNGYAGYIDGGASQEEFYKFIKPILEKMDGTEELLVKLNRED
LLRKQRTFDNGSIPHQIHLGELHAILRRQEDFYPFLKDNREKIEKILTFRIPYYVGPLARG
NSRFAWMTRKSEETITPWNFEEVVDKGASAQSFIERMTNFDKNLPNEKVLPKHSLLYEY
FTVYNELTKVKYVTEGMRKPAFLSGEQKKAIVDLLFKTNRKVTVKQLKEDYFKKIECFD
SVEISGVEDRFNASLGTYHDLLKIIKDKDFLDNEENEDILEDIVLTLTLFEDREMIEERLK
TYAHLFDDKVMKQLKRRRYTGWGRLSRKLINGIRDKQSGKTILDFLKSDGFANRNFMQ
LIHDDSLTFKEDIQKAQVSGQGDSLHEHIANLAGSPAIKKGILQTVKVVDELVKVMGRH
KPENIVIEMARENQTTQKGQKNSRERMKRIEEGIKELGSQILKEHPVENTQLQNEKLYLY
YLQNGRDMYVDQELDINRLSDYDVDAIVPQSFLKDDSIDNKVLTRSDKNRGKSDNVPSE
EVVKKMKNYWRQLLNAKLITQRKFDNLTKAERGGLSELDKAGFIKRQLVETRQITKHVA
QILDSRMNTKYDENDKLIREVKVITLKSKLVSDFRKDFQFYKVREINNYHHAHDAYLNA
VVGTALIKKYPKLESEFVYGDYKVYDVRKMIAKSEQEIGKATAKYFFYSNIMNFFKTEITL
ANGEIRKRPLIETNGETGEIVWDKGRDFATVRKVLSMPQVNIVKKTEVQTGGFSKESILP

KRNSDKLIARKKDWDPKKYGGFDSPTVAYSVLVVAKVEKGKSKKLKSVKELLGITIMERS
SFEKNPIDFLEAKGYKEVKKDLIIKLPKYSLFELENGRKRMLASAGELQKGNELALPSKY
VNFLYLASHYEKLKGSPEDNEQKQLFVEQHKHYLDEIIEQISEFSKRVILADANLDKVLS
AYNKHRDKPIREQAENIIHLFTLTNLGAPAAFKYFDTTIDRKRYTSTKEVLDATLIHQSIT
GLYETRIDLSQLGGDAYPYDVPDYASLGSGSPKKKRKVEDPKKKRKVDGSGSETPGTSE
SATPESRTLEMAQTQGTRRKVCYYYDGDVGNYYYGQGHPMKPHRIRMTHNLLLNY
GLYRKMEIYRPHKANAEEMTKYHSDDYIKFLRSIRPDNMSEYSKQMQRFNVGEDC
PVFDGLFEFCQLSTGGSVASAVKLNKQQTDIAVNWAGGLHHAKKSEASGFCYVNDI
VLAILELLKYHQRVLYIDIDIHHGDGVEEAFYTTDRVMTVSFHKYGEYFPGTGDLR
DIGAGKGKYYAVNYPLRDGIDDESYEAIFKPVMSKVMEMFQPSAVVLQCGSDSLSG
DRLGCFNLTIKGHAKCVEFVKSFNLPMLMLGGGGYTIRNVARCWTYETAVALDTE
IPNELPYNDYFEYFGPDFKLHISPSNMTNQNTNEYLEKIKQRLFENLRMLPHAPGVQ
MQAIPEDAIPEESGDEDEDDPDKRISICSSDKRIACEEEFSDSEEEGEGGRKNSSNFK
KAKRVKTEDEKEKDPEEKKEVTEEEKTKEEKPEAKGVKEEVKLA*

The amino acid sequence of DNMT3A-DNMT3L-XTEN80-dCas9-XTEN16-PRMT1 (V69 scheme, SEQ ID NO: 120) is as follows:

MPAAKRVKLDGSTGPATMNHDQEFDPPKVYPPVPAEKRKPIRVLSLFDGIATGLL
VLKDLGIQVDRYIASEVCEDSITVGMVRHQGKIMYVGDVRSVTQKHIQEWGPFDL
VIGGSPCNDLSIVNPARKGLYEGTGRLFFEFYRLLHDARPKEGDDRPFFWLFENVV
AMGVSDKRDISRFLESNPVMIDAKEVSAAHRARYFWGNLPGMNRPLASTVNDKLE
LQECLEHGRIAKFSKVRTITTRSNSIKQGKDQHFPVFMNEKEDILWCTEMERVFGF
PVHYTDVSNMSRLARQRLLGRSWSVPVIRHLFAPLKEYFACVSSGNSNANSRGPSFSS
GLVPLSLRGSHMGPMEIYKTVSAWKRQPVRVLSLFRNIDKVLKSLGFLESGSGSGGG
TLKYVEDVTNVVRRDVEKWGPFDLVYGSTQPLGSSCDRCPGWYMFQFHRILQYAL
PRQESQRPFFWIFMDNLLLTEDDQETTTRFLQTEAVTLQDVRGRDYQNAMRVWSN
IPGLKSKHAPLTPKEEEYLQAQVRSRSKLDAPKVDLLVKNCLLPLREYFKYFSQNSL
PLGGPSSGAPPPSGGSPAGSPTSTEEGTSESATPESGPGTSTEPSEGSAPGSPAGSPTSTEEGTS

TEPSEGSAPGTSTEPSEPKKKRKVMDKKYSIGLAIGTNSVGWAVITDEYKVPSKKFKVLGN
TDRHSIKKNLIGALLFDSGETAEATRLKRTARRRYTRRKNRICYLQEIFSNEMAKVDDSF
FHRLEESFLVEEDKKHERHPIFGNIVDEVAYHEKYPTIYHLRKKLVDSTDKADLRLIYLA
LAHMIKFRGHFLIEGDLNPDNSDVDKLFIQLVQTYNQLFEENPINASGVDAKAILSARLS
KSRRLENLIAQLPGEKKNGLFGNLIALSLGLTPNFKSNFDLAEDAKLQLSKDTYDDDLD
NLLAQIGDQYADLFLAAKNLSDAILLSDILRVNTEITKAPLSASMIKRYDEHHQDLTLLKA
LVRQQLPEKYKEIFFDQSKNGYAGYIDGGASQEEFYKFIKPILEKMDGTEELLVKLNRED
LLRKQRTFDNGSIPHQIHLGELHAILRRQEDFYPFLKDNREKIEKILTFRIPYYVGPLARG
NSRFAWMTRKSEETITPWNFEEVVDKGASAQSFIERMTNFDKNLPNEKVLPKHSLLYEY
FTVYNELTKVKYVTEGMRKPAFLSGEQKKAIVDLLFKTNRKVTVKQLKEDYFKKIECFD
SVEISGVEDRFNASLGTYHDLLKIIKDKDFLDNEENEDILEDIVLTLTLFEDREMIEERLK
TYAHLFDDKVMKQLKRRRYTGWGRLSRKLINGIRDKQSGKTILDFLKSDGFANRNFMQ
LIHDDSLTFKEDIQKAQVSGQGDSLHEHIANLAGSPAIKKGILQTVKVVDELVKVMGRH
KPENIVIEMARENQTTQKGQKNSRERMKRIEEGIKELGSQILKEHPVENTQLQNEKLYLY
YLQNGRDMYVDQELDINRLSDYDVDAIVPQSFLKDDSIDNKVLTRSDKNRGKSDNVPSE
EVVKKMKNYWRQLLNAKLITQRKFDNLTKAERGGLSELDKAGFIKRQLVETRQITKHVA
QILDSRMNTKYDENDKLIREVKVITLKSKLVSDFRKDFQFYKVREINNYHHAHDAYLNA
VVGTALIKKYPKLESEFVYGDYKVYDVRKMIAKSEQEIGKATAKYFFYSNIMNFFKTEITL
ANGEIRKRPLIETNGETGEIVWDKGRDFATVRKVLSMPQVNIVKKTEVQTGGFSKESILP
KRNSDKLIARKKDWDPKKYGGFDSPTVAYSVLVVAKVEKGKSKKLKSVKELLGITIMERS
SFEKNPIDFLEAKGYKEVKKDLIIKLPKYSLFELENGRKRMLASAGELQKGNELALPSKY
VNFLYLASHYEKLKGSPEDNEQKQLFVEQHKHYLDEIIEQISEFSKRVILADANLDKVLS
AYNKHRDKPIREQAENIIHLFTLTNLGAPAAFKYFDTTIDRKRYTSTKEVLDATLIHQSIT
GLYETRIDLSQLGGDAYPYDVPDYASLGSGSPKKKRKVEDPKKKRKVDGSGSETPGTSE
SATPESRTLEMAAAEAANCIMENFVATLANGMSLQPPLEEVSCGQAESSEKPNAEDM
TSKDYYFDSYAHFGIHEEMLKDEVRTLTYRNSMFHNRHLFKDKVVLDVGSGTGIL
CMFAAKAGARKVIGIECSSISDYAVKIVKANKLDHVVTIIKGKVEEVELPVEKVDIII

SEWMGYCLFYESMLNTVLYARDKWLAPDGLIFPDRATLYVTAIEDRQYKDYKIHW

WENVYGFDMSCIKDVAIKEPLVDVVDPKQLVTNACLIKEVDIYTVKVEDLTFTSPFC

LQVKRNDYVHALVAYFNIEFTRCHKRTGFSTSPESPYTHWKQTVFYMEDYLTVKT

GEEIFGTIGMRPNAKNNRDLDFTIDLDFKGQLCELSCSTDYRMR*

The amino acid sequence of DNMT3A-DNMT3L-XTEN80-dCas9-XTEN16-SETDB1 (V70 scheme, SEQ ID NO: 121) is as follows:

MPAAKRVKLDGSTGPATMNHDQEFDPPKVYPPVPAEKRKPIRVLSLFDGIATGLL
VLKDLGIQVDRYIASEVCEDSITVGMVRHQGKIMYVGDVRSVTQKHIQEWGPFDL
VIGGSPCNDLSIVNPARKGLYEGTGRLFFEFYRLLHDARPKEGDDRPFFWLFENVV
AMGVSDKRDISRFLESNPVMIDAKEVSAAHRARYFWGNLPGMNRPLASTVNDKLE
LQECLEHGRIAKFSKVRTITTRSNSIKQGKDQHFPVFMNEKEDILWCTEMERVFGF
PVHYTDVSNMSRLARQRLLGRSWSVPVIRHLFAPLKEYFACVSSGNSNANSRGPSFSS
GLVPLSLRGSHMGPMEIYKTVSAWKRQPVRVLSLFRNIDKVLKSLGFLESGSGSGGG
TLKYVEDVTNVVRRDVEKWGPFDLVYGSTQPLGSSCDRCPGWYMFQFHRILQYAL
PRQESQRPFFWIFMDNLLLTEDDQETTTRFLQTEAVTLQDVRGRDYQNAMRVWSN
IPGLKSKHAPLTPKEEEYLQAQVRSRSKLDAPKVDLLVKNCLLPLREYFKYFSQNSL
PLGGPSSGAPPPSGGSPAGSPTSTEEGTSESATPESGPGTSTEPSEGSAPGSPAGSPTSTEEGTS
TEPSEGSAPGTSTEPSEPKKKRKVMDKKYSIGLAIGTNSVGWAVITDEYKVPSKKFKVLGN
TDRHSIKKNLIGALLFDSGETAEATRLKRTARRRYTRRKNRICYLQEIFSNEMAKVDDSF
FHRLEESFLVEEDKKHERHPIFGNIVDEVAYHEKYPTIYHLRKKLVDSTDKADLRLIYLA
LAHMIKFRGHFLIEGDLNPDNSDVDKLFIQLVQTYNQLFEENPINASGVDAKAILSARLS
KSRRLENLIAQLPGEKKNGLFGNLIALSLGLTPNFKSNFDLAEDAKLQLSKDTYDDDLD
NLLAQIGDQYADLFLAAKNLSDAILLSDILRVNTEITKAPLSASMIKRYDEHHQDLTLLKA
LVRQQLPEKYKEIFFDQSKNGYAGYIDGGASQEEFYKFIKPILEKMDGTEELLVKLNRED
LLRKQRTFDNGSIPHQIHLGELHAILRRQEDFYPFLKDNREKIEKILTFRIPYYVGPLARG
NSRFAWMTRKSEETITPWNFEEVVDKGASAQSFIERMTNFDKNLPNEKVLPKHSLLYEY
FTVYNELTKVKYVTEGMRKPAFLSGEQKKAIVDLLFKTNRKVTVKQLKEDYFKKIECFD

SVEISGVEDRFNASLGTYHDLLKIIKDKDFLDNEENEDILEDIVLTLTLFEDREMIEERLK
TYAHLFDDKVMKQLKRRRYTGWGRLSRKLINGIRDKQSGKTILDFLKSDGFANRNFMQ
LIHDDSLTFKEDIQKAQVSGQGDSLHEHIANLAGSPAIKKGILQTVKVVDELVKVMGRH
KPENIVIEMARENQTTQKGQKNSRERMKRIEEGIKELGSQILKEHPVENTQLQNEKLYLY
YLQNGRDMYVDQELDINRLSDYDVDAIVPQSFLKDDSIDNKVLTRSDKNRGKSDNVPSE
EVVKKMKNYWRQLLNAKLITQRKFDNLTKAERGGLSELDKAGFIKRQLVETRQITKHVA
QILDSRMNTKYDENDKLIREVKVITLKSKLVSDFRKDFQFYKVREINNYHHAHDAYLNA
VVGTALIKKYPKLESEFVYGDYKVYDVRKMIAKSEQEIGKATAKYFFYSNIMNFFKTEITL
ANGEIRKRPLIETNGETGEIVWDKGRDFATVRKVLSMPQVNIVKKTEVQTGGFSKESILP
KRNSDKLIARKKDWDPKKYGGFDSPTVAYSVLVVAKVEKGKSKKLKSVKELLGITIMERS
SFEKNPIDFLEAKGYKEVKKDLIIKLPKYSLFELENGRKRMLASAGELQKGNELALPSKY
VNFLYLASHYEKLKGSPEDNEQKQLFVEQHKHYLDEIIEQISEFSKRVILADANLDKVLS
AYNKHRDKPIREQAENIIHLFTLTNLGAPAAFKYFDTTIDRKRYTSTKEVLDATLIHQSIT
GLYETRIDLSQLGGDAYPYDVPDYASLGSGSPKKKRKVEDPKKKRKVDGSGSETPGTSE
SATPESRTLETPPPQVAYSKERIPGKGVFINTGPEFLVGCDCKDGCRDKSKCACHQL
TIQATACTPGGQINPNSGYQYKRLEECLPTGVYECNKRCKCDPNMCTNRLVQHGL
QVRLQLFKTQNKGWGIRCLDDIAKGSFVCIYAGKILTDDFADKEGLEMGDEYFAN
LDHIESVENFKEGYESDAPCSSDSSGVDLKDQEDGNSGTEDPEESNDDSSDDNFCKD
EDFSTSSVWRSYATRRQTRGQKENGLSETTSKDSHPPDLGPPHIPVPPSIPVGGCNPP
SSEETPKNKVASWLSCNSVSEGGFADSDSHSSFKTNEGGEGRAGGSRMEAEKASTS
GLGIKDEGDIKQAKKEDTDDRNKMSVVTESSRNYGYNPSPVKPEGLRRPPSKTSMH
QSRRLMASAQSNPDDVLTLSSSTESEGESGTSRKPTAGQTSATAVDSDDIQTISSGSE
GDDFEDKKNMTGPMKRQVAVKSTRGFALKSTHGIAIKSTNMASVDKGESAPVRKN
TRQFYDGEESCYIIDAKLEGNLGRYLNHSCSPNLFVQNVFVDTHDLRFPWVAFFAS
KRIRAGTELTWDYNYEVGSVEGKELLCCCGAIECRGRLL*

The amino acid sequence of DNMT3A-DNMT3L-XTEN80-dCas9-XTEN16-hSIRT1 (V71 scheme, SEQ ID NO: 122) is as follows:

MPAAKRVKLDGSTGPATMNHDQEFDPPKVYPPVPAEKRKPIRVLSLFDGIATGLL
VLKDLGIQVDRYIASEVCEDSITVGMVRHQGKIMYVGDVRSVTQKHIQEWGPFDL
VIGGSPCNDLSIVNPARKGLYEGTGRLFFEFYRLLHDARPKEGDDRPFFWLFENVV
AMGVSDKRDISRFLESNPVMIDAKEVSAAHRARYFWGNLPGMNRPLASTVNDKLE
LQECLEHGRIAKFSKVRTITTRSNSIKQGKDQHFPVFMNEKEDILWCTEMERVFGF
PVHYTDVSNMSRLARQRLLGRSWSVPVIRHLFAPLKEYFACVSSGNSNANSRGPSFSS
GLVPLSLRGSHMGPMEIYKTVSAWKRQPVRVLSLFRNIDKVLKSLGFLESGSGSGGG
TLKYVEDVTNVVRRDVEKWGPFDLVYGSTQPLGSSCDRCPGWYMFQFHRILQYAL
PRQESQRPFFWIFMDNLLLTEDDQETTTRFLQTEAVTLQDVRGRDYQNAMRVWSN
IPGLKSKHAPLTPKEEEYLQAQVRSRSKLDAPKVDLLVKNCLLPLREYFKYFSQNSL
PLGGPSSGAPPPSGGSPAGSPTSTEEGTSESATPESGPGTSTEPSEGSAPGSPAGSPTSTEEGTS
TEPSEGSAPGTSTEPSEPKKKRKVMDKKYSIGLAIGTNSVGWAVITDEYKVPSKKFKVLGN
TDRHSIKKNLIGALLFDSGETAEATRLKRTARRRYTRRKNRICYLQEIFSNEMAKVDDSF
FHRLEESFLVEEDKKHERHPIFGNIVDEVAYHEKYPTIYHLRKKLVDSTDKADLRLIYLA
LAHMIKFRGHFLIEGDLNPDNSDVDKLFIQLVQTYNQLFEENPINASGVDAKAILSARLS
KSRRLENLIAQLPGEKKNGLFGNLIALSLGLTPNFKSNFDLAEDAKLQLSKDTYDDDLD
NLLAQIGDQYADLFLAAKNLSDAILLSDILRVNTEITKAPLSASMIKRYDEHHQDLTLLKA
LVRQQLPEKYKEIFFDQSKNGYAGYIDGGASQEEFYKFIKPILEKMDGTEELLVKLNRED
LLRKQRTFDNGSIPHQIHLGELHAILRRQEDFYPFLKDNREKIEKILTFRIPYYVGPLARG
NSRFAWMTRKSEETITPWNFEEVVDKGASAQSFIERMTNFDKNLPNEKVLPKHSLLYEY
FTVYNELTKVKYVTEGMRKPAFLSGEQKKAIVDLLFKTNRKVTVKQLKEDYFKKIECFD
SVEISGVEDRFNASLGTYHDLLKIIKDKDFLDNEENEDILEDIVLTLTLFEDREMIEERLK
TYAHLFDDKVMKQLKRRRYTGWGRLSRKLINGIRDKQSGKTILDFLKSDGFANRNFMQ
LIHDDSLTFKEDIQKAQVSGQGDSLHEHIANLAGSPAIKKGILQTVKVVDELVKVMGRH
KPENIVIEMARENQTTQKGQKNSRERMKRIEEGIKELGSQILKEHPVENTQLQNEKLYLY
YLQNGRDMYVDQELDINRLSDYDVDAIVPQSFLKDDSIDNKVLTRSDKNRGKSDNVPSE
EVVKKMKNYWRQLLNAKLITQRKFDNLTKAERGGLSELDKAGFIKRQLVETRQITKHVA

QILDSRMNTKYDENDKLIREVKVITLKSKLVSDFRKDFQFYKVREINNYHHAHDAYLNA
VVGTALIKKYPKLESEFVYGDYKVYDVRKMIAKSEQEIGKATAKYFFYSNIMNFFKTEITL
ANGEIRKRPLIETNGETGEIVWDKGRDFATVRKVLSMPQVNIVKKTEVQTGGFSKESILP
KRNSDKLIARKKDWDPKKYGGFDSPTVAYSVLVVAKVEKGKSKKLKSVKELLGITIMERS
SFEKNPIDFLEAKGYKEVKKDLIIKLPKYSLFELENGRKRMLASAGELQKGNELALPSKY
VNFLYLASHYEKLKGSPEDNEQKQLFVEQHKHYLDEIIEQISEFSKRVILADANLDKVLS
AYNKHRDKPIREQAENIIHLFTLTNLGAPAAFKYFDTTIDRKRYTSTKEVLDATLIHQSIT
GLYETRIDLSQLGGDAYPYDVPDYASLGSGSPKKKRKVEDPKKKRKVDGSGSETPGTSE
SATPESRTLETILKDLLPETIPPPELDDMTLWQIVINILSEPPKRKKRKDINTIEDAVKL
LQECKKIIVLTGAGVSVSCGIPDFRSRDGIYARLAVDFPDLPDPQAMFDIEYFRKDPR
PFFKFAKEIYPGQFQPSLCHKFIALSDKEGKLLRNYTQNIDTLEQVAGIQRIIQCHGS
FATASCLICKYKVDCEAVRGDIFNQVVPRCPRCPADEPLAIMKPEIVFFGENLPEQF
HRAMKYDKDEVDLLIVIGSSLKVRPVALIPSSIPHEVPQILINREPLPHLHFDVELLG
DCDVIINELCHRLGGEYAKL*

The amino acid sequence of DNMT3A-DNMT3L-XTEN80-dCas9-XTEN16-PRMT5 (V72 scheme, SEQ ID NO: 123) is as follows:

MPAAKRVKLDGSTGPATMNHDQEFDPPKVYPPVPAEKRKPIRVLSLFDGIATGLL
VLKDLGIQVDRYIASEVCEDSITVGMVRHQGKIMYVGDVRSVTQKHIQEWGPFDL
VIGGSPCNDLSIVNPARKGLYEGTGRLFFEFYRLLHDARPKEGDDRPFFWLFENVV
AMGVSDKRDISRFLESNPVMIDAKEVSAAHRARYFWGNLPGMNRPLASTVNDKLE
LQECLEHGRIAKFSKVRTITTRSNSIKQGKDQHFPVFMNEKEDILWCTEMERVFGF
PVHYTDVSNMSRLARQRLLGRSWSVPVIRHLFAPLKEYFACVSSGNSNANSRGPSFSS
GLVPLSLRGSHMGPMEIYKTVSAWKRQPVRVLSLFRNIDKVLKSLGFLESGSGSGGG
TLKYVEDVTNVVRRDVEKWGPFDLVYGSTQPLGSSCDRCPGWYMFQFHRILQYAL
PRQESQRPFFWIFMDNLLLTEDDQETTTRFLQTEAVTLQDVRGRDYQNAMRVWSN
IPGLKSKHAPLTPKEEEYLQAQVRSRSKLDAPKVDLLVKNCLLPLREYFKYFSQNSL
PLGGPSSGAPPPSGGSPAGSPTSTEEGTSESATPESGPGTSTEPSEGSAPGSPAGSPTSTEEGTS

TEPSEGSAPGTSTEPSEPKKKRKVMDKKYSIGLAIGTNSVGWAVITDEYKVPSKKFKVLGN
TDRHSIKKNLIGALLFDSGETAEATRLKRTARRRYTRRKNRICYLQEIFSNEMAKVDDSF
FHRLEESFLVEEDKKHERHPIFGNIVDEVAYHEKYPTIYHLRKKLVDSTDKADLRLIYLA
LAHMIKFRGHFLIEGDLNPDNSDVDKLFIQLVQTYNQLFEENPINASGVDAKAILSARLS
KSRRLENLIAQLPGEKKNGLFGNLIALSLGLTPNFKSNFDLAEDAKLQLSKDTYDDDLD
NLLAQIGDQYADLFLAAKNLSDAILLSDILRVNTEITKAPLSASMIKRYDEHHQDLTLLKA
LVRQQLPEKYKEIFFDQSKNGYAGYIDGGASQEEFYKFIKPILEKMDGTEELLVKLNRED
LLRKQRTFDNGSIPHQIHLGELHAILRRQEDFYPFLKDNREKIEKILTFRIPYYVGPLARG
NSRFAWMTRKSEETITPWNFEEVVDKGASAQSFIERMTNFDKNLPNEKVLPKHSLLYEY
FTVYNELTKVKYVTEGMRKPAFLSGEQKKAIVDLLFKTNRKVTVKQLKEDYFKKIECFD
SVEISGVEDRFNASLGTYHDLLKIIKDKDFLDNEENEDILEDIVLTLTLFEDREMIEERLK
TYAHLFDDKVMKQLKRRRYTGWGRLSRKLINGIRDKQSGKTILDFLKSDGFANRNFMQ
LIHDDSLTFKEDIQKAQVSGQGDSLHEHIANLAGSPAIKKGILQTVKVVDELVKVMGRH
KPENIVIEMARENQTTQKGQKNSRERMKRIEEGIKELGSQILKEHPVENTQLQNEKLYLY
YLQNGRDMYVDQELDINRLSDYDVDAIVPQSFLKDDSIDNKVLTRSDKNRGKSDNVPSE
EVVKKMKNYWRQLLNAKLITQRKFDNLTKAERGGLSELDKAGFIKRQLVETRQITKHVA
QILDSRMNTKYDENDKLIREVKVITLKSKLVSDFRKDFQFYKVREINNYHHAHDAYLNA
VVGTALIKKYPKLESEFVYGDYKVYDVRKMIAKSEQEIGKATAKYFFYSNIMNFFKTEITL
ANGEIRKRPLIETNGETGEIVWDKGRDFATVRKVLSMPQVNIVKKTEVQTGGFSKESILP
KRNSDKLIARKKDWDPKKYGGFDSPTVAYSVLVVAKVEKGKSKKLKSVKELLGITIMERS
SFEKNPIDFLEAKGYKEVKKDLIIKLPKYSLFELENGRKRMLASAGELQKGNELALPSKY
VNFLYLASHYEKLKGSPEDNEQKQLFVEQHKHYLDEIIEQISEFSKRVILADANLDKVLS
AYNKHRDKPIREQAENIIHLFTLTNLGAPAAFKYFDTTIDRKRYTSTKEVLDATLIHQSIT
GLYETRIDLSQLGGDAYPYDVPDYASLGSGSPKKKRKVEDPKKKRKVDGSGSETPGTSE
SATPESRTLEMAAMAVGGAGGSRVSSGRDLNCVPEIADTLGAVAKQGFDFLCMPVF
HPRFKREFIQEPAKNRPGPQTRSDLLLSGRDWNTLIVGKLSPWIRPDSKVEKIRRNS
EAAMLQELNFGAYLGLPAFLLPLNQEDNTNLARVLTNHIHTGHHSSMFWMRVPLV

APEDLRDDIIENAPTTHTEEYSGEEKTWMWWHNFRTLCDYSKRIAVALEIGADLPS
NHVIDRWLGEPIKAAILPTSIFLTNKKGFPVLSKMHQRLIFRLLKLEVQFIITGTNHH
SEKEFCSYLQYLEYLSQNRPPPNAYELFAKGYEDYLQSPLQPLMDNLESQTYEVFE
KDPIKYSQYQQAIYKCLLDRVPEEEKDTNVQVLMVLGAGRGPLVNASLRAAKQAD
RRIKLYAVEKNPNAVVTLENWQFEEWGSQVTVVSSDMREWVAPEKADIIVSELLGS
FADNELSPECLDGAQHFLKDDGVSIPGEYTSFLAPISSSKLYNEVRACREKDRDPEA
QFEMPYVVRLHNFHQLSAPQPCFTFSHPNRDPMIDNNRYCTLEFPVEVNTVLHGFA
GYFETVLYQDITLSIRPETHSPGMFSWFPILFPIKQPITVREGQTICVRFWRCSNSKK
VWYEWAVTAPVCSAIHNPTGRSYTIGL*

The amino acid sequence of DNMT3A-DNMT3L-XTEN80-dCas9-XTEN16-HP1a (V73 scheme, SEQ ID NO: 124) is as follows:

MPAAKRVKLDGSTGPATMNHDQEFDPPKVYPPVPAEKRKPIRVLSLFDGIATGLL
VLKDLGIQVDRYIASEVCEDSITVGMVRHQGKIMYVGDVRSVTQKHIQEWGPFDL
VIGGSPCNDLSIVNPARKGLYEGTGRLFFEFYRLLHDARPKEGDDRPFFWLFENVV
AMGVSDKRDISRFLESNPVMIDAKEVSAAHRARYFWGNLPGMNRPLASTVNDKLE
LQECLEHGRIAKFSKVRTITTRSNSIKQGKDQHFPVFMNEKEDILWCTEMERVFGF
PVHYTDVSNMSRLARQRLLGRSWSVPVIRHLFAPLKEYFACVSSGNSNANSRGPSFSS
GLVPLSLRGSHMGPMEIYKTVSAWKRQPVRVLSLFRNIDKVLKSLGFLESGSGSGGG
TLKYVEDVTNVVRRDVEKWGPFDLVYGSTQPLGSSCDRCPGWYMFQFHRILQYAL
PRQESQRPFFWIFMDNLLLTEDDQETTTRFLQTEAVTLQDVRGRDYQNAMRVWSN
IPGLKSKHAPLTPKEEEYLQAQVRSRSKLDAPKVDLLVKNCLLPLREYFKYFSQNSL
PLGGPSSGAPPPSGGSPAGSPTSTEEGTSESATPESGPGTSTEPSEGSAPGSPAGSPTSTEEGTS
TEPSEGSAPGTSTEPSEPKKKRKVMDKKYSIGLAIGTNSVGWAVITDEYKVPSKKFKVLGN
TDRHSIKKNLIGALLFDSGETAEATRLKRTARRRYTRRKNRICYLQEIFSNEMAKVDDSF
FHRLEESFLVEEDKKHERHPIFGNIVDEVAYHEKYPTIYHLRKKLVDSTDKADLRLIYLA
LAHMIKFRGHFLIEGDLNPDNSDVDKLFIQLVQTYNQLFEENPINASGVDAKAILSARLS
KSRRLENLIAQLPGEKKNGLFGNLIALSLGLTPNFKSNFDLAEDAKLQLSKDTYDDDLD

NLLAQIGDQYADLFLAAKNLSDAILLSDILRVNTEITKAPLSASMIKRYDEHHQDLTLLKA
LVRQQLPEKYKEIFFDQSKNGYAGYIDGGASQEEFYKFIKPILEKMDGTEELLVKLNRED
LLRKQRTFDNGSIPHQIHLGELHAILRRQEDFYPFLKDNREKIEKILTFRIPYYVGPLARG
NSRFAWMTRKSEETITPWNFEEVVDKGASAQSFIERMTNFDKNLPNEKVLPKHSLLYEY
FTVYNELTKVKYVTEGMRKPAFLSGEQKKAIVDLLFKTNRKVTVKQLKEDYFKKIECFD
SVEISGVEDRFNASLGTYHDLLKIIKDKDFLDNEENEDILEDIVLTLTLFEDREMIEERLK
TYAHLFDDKVMKQLKRRRYTGWGRLSRKLINGIRDKQSGKTILDFLKSDGFANRNFMQ
LIHDDSLTFKEDIQKAQVSGQGDSLHEHIANLAGSPAIKKGILQTVKVVDELVKVMGRH
KPENIVIEMARENQTTQKGQKNSRERMKRIEEGIKELGSQILKEHPVENTQLQNEKLYLY
YLQNGRDMYVDQELDINRLSDYDVDAIVPQSFLKDDSIDNKVLTRSDKNRGKSDNVPSE
EVVKKMKNYWRQLLNAKLITQRKFDNLTKAERGGLSELDKAGFIKRQLVETRQITKHVA
QILDSRMNTKYDENDKLIREVKVITLKSKLVSDFRKDFQFYKVREINNYHHAHDAYLNA
VVGTALIKKYPKLESEFVYGDYKVYDVRKMIAKSEQEIGKATAKYFFYSNIMNFFKTEITL
ANGEIRKRPLIETNGETGEIVWDKGRDFATVRKVLSMPQVNIVKKTEVQTGGFSKESILP
KRNSDKLIARKKDWDPKKYGGFDSPTVAYSVLVVAKVEKGKSKKLKSVKELLGITIMERS
SFEKNPIDFLEAKGYKEVKKDLIIKLPKYSLFELENGRKRMLASAGELQKGNELALPSKY
VNFLYLASHYEKLKGSPEDNEQKQLFVEQHKHYLDEIIEQISEFSKRVILADANLDKVLS
AYNKHRDKPIREQAENIIHLFTLTNLGAPAAFKYFDTTIDRKRYTSTKEVLDATLIHQSIT
GLYETRIDLSQLGGDAYPYDVPDYASLGSGSPKKKRKVEDPKKKRKVDGSGSETPGTSE
SATPESRTLGKKTKRTADSSSSEDEEEYVVEKVLDRRVVKGQVEYLLKWKGFSEEH
NTWEPEKNLDCPELISEFMKKYKKMKEGENNKPREKSESNKRKSNFSNSADDIKSK
KKREQSNDIARGFERGLEPEKIIGATDSCGDLMFLMKWKDTDEADLVLAKEANVK
CPQIVIAFYEERLTWHAYPEDAENKEKETAKS*

The amino acid sequence of DNMT3A-DNMT3L-XTEN80-dCas9-XTEN16-PRMT5(short) (V74 scheme, SEQ ID NO: 125) is as follows:

MPAAKRVKLDGSTGPATMNHDQEFDPPKVYPPVPAEKRKPIRVLSLFDGIATGLL

VLKDLGIQVDRYIASEVCEDSITVGMVRHQGKIMYVGDVRSVTQKHIQEWGPFDL

VIGGSPCNDLSIVNPARKGLYEGTGRLFFEFYRLLHDARPKEGDDRPFFWLFENVV
AMGVSDKRDISRFLESNPVMIDAKEVSAAHRARYFWGNLPGMNRPLASTVNDKLE
LQECLEHGRIAKFSKVRTITTRSNSIKQGKDQHFPVFMNEKEDILWCTEMERVFGF
PVHYTDVSNMSRLARQRLLGRSWSVPVIRHLFAPLKEYFACVSSGNSNANSRGPSFSS
GLVPLSLRGSHMGPMEIYKTVSAWKRQPVRVLSLFRNIDKVLKSLGFLESGSGSGGG
TLKYVEDVTNVVRRDVEKWGPFDLVYGSTQPLGSSCDRCPGWYMFQFHRILQYAL
PRQESQRPFFWIFMDNLLLTEDDQETTTRFLQTEAVTLQDVRGRDYQNAMRVWSN
IPGLKSKHAPLTPKEEEYLQAQVRSRSKLDAPKVDLLVKNCLLPLREYFKYFSQNSL
PLGGPSSGAPPPSGGSPAGSPTSTEEGTSESATPESGPGTSTEPSEGSAPGSPAGSPTSTEEGTS
TEPSEGSAPGTSTEPSEPKKKRKVMDKKYSIGLAIGTNSVGWAVITDEYKVPSKKFKVLGN
TDRHSIKKNLIGALLFDSGETAEATRLKRTARRRYTRRKNRICYLQEIFSNEMAKVDDSF
FHRLEESFLVEEDKKHERHPIFGNIVDEVAYHEKYPTIYHLRKKLVDSTDKADLRLIYLA
LAHMIKFRGHFLIEGDLNPDNSDVDKLFIQLVQTYNQLFEENPINASGVDAKAILSARLS
KSRRLENLIAQLPGEKKNGLFGNLIALSLGLTPNFKSNFDLAEDAKLQLSKDTYDDDLD
NLLAQIGDQYADLFLAAKNLSDAILLSDILRVNTEITKAPLSASMIKRYDEHHQDLTLLKA
LVRQQLPEKYKEIFFDQSKNGYAGYIDGGASQEEFYKFIKPILEKMDGTEELLVKLNRED
LLRKQRTFDNGSIPHQIHLGELHAILRRQEDFYPFLKDNREKIEKILTFRIPYYVGPLARG
NSRFAWMTRKSEETITPWNFEEVVDKGASAQSFIERMTNFDKNLPNEKVLPKHSLLYEY
FTVYNELTKVKYVTEGMRKPAFLSGEQKKAIVDLLFKTNRKVTVKQLKEDYFKKIECFD
SVEISGVEDRFNASLGTYHDLLKIIKDKDFLDNEENEDILEDIVLTLTLFEDREMIEERLK
TYAHLFDDKVMKQLKRRRYTGWGRLSRKLINGIRDKQSGKTILDFLKSDGFANRNFMQ
LIHDDSLTFKEDIQKAQVSGQGDSLHEHIANLAGSPAIKKGILQTVKVVDELVKVMGRH
KPENIVIEMARENQTTQKGQKNSRERMKRIEEGIKELGSQILKEHPVENTQLQNEKLYLY
YLQNGRDMYVDQELDINRLSDYDVDAIVPQSFLKDDSIDNKVLTRSDKNRGKSDNVPSE
EVVKKMKNYWRQLLNAKLITQRKFDNLTKAERGGLSELDKAGFIKRQLVETRQITKHVA
QILDSRMNTKYDENDKLIREVKVITLKSKLVSDFRKDFQFYKVREINNYHHAHDAYLNA
VVGTALIKKYPKLESEFVYGDYKVYDVRKMIAKSEQEIGKATAKYFFYSNIMNFFKTEITL

ANGEIRKRPLIETNGETGEIVWDKGRDFATVRKVLSMPQVNIVKKTEVQTGGFSKESILP
KRNSDKLIARKKDWDPKKYGGFDSPTVAYSVLVVAKVEKGKSKKLKSVKELLGITIMERS
SFEKNPIDFLEAKGYKEVKKDLIIKLPKYSLFELENGRKRMLASAGELQKGNELALPSKY
VNFLYLASHYEKLKGSPEDNEQKQLFVEQHKHYLDEIIEQISEFSKRVILADANLDKVLS
AYNKHRDKPIREQAENIIHLFTLTNLGAPAAFKYFDTTIDRKRYTSTKEVLDATLIHQSIT
GLYETRIDLSQLGGDAYPYDVPDYASLGSGSPKKKRKVEDPKKKRKVDGSGSETPGTSE
SATPESRTLEPPPNAYELFAKGYEDYLQSPLQPLMDNLESQTYEVFEKDPIKYSQYQQ
AIYKCLLDRVPEEEKDTNVQVLMVLGAGRGPLVNASLRAAKQADRRIKLYAVEKN
PNAVVTLENWQFEEWGSQVTVVSSDMREWVAPEKADIIVSELLGSFADNELSPECL
DGAQHFLKDDGVSIPGEYTSFLAPISSSKLYNEVRACREKDRDPEAQFEMPYVVRL
HNFHQLSAPQPCFTFSHPNRDPMIDNNRYCTLEFPVEVNTVLHGFAGYFETVLYQDI
TLSIRPETHSPGMFSWFPILFPIKQPITVREGQTICVRFWRCSNSKKVWYEWAVTAP
VCSAIHNPTGRSYTIGL*

The amino acid sequence of DNMT3A-DNMT3L-XTEN80-dCas9-XTEN16-LSD1 (V75 scheme, SEQ ID NO: 126) is as follows:

MNHDQEFDPPKVYPPVPAEKRKPIRVLSLFDGIATGLLVLKDLGIQVDRYIASEV
CEDSITVGMVRHQGKIMYVGDVRSVTQKHIQEWGPFDLVIGGSPCNDLSIVNPARK
GLYEGTGRLFFEFYRLLHDARPKEGDDRPFFWLFENVVAMGVSDKRDISRFLESNP
VMIDAKEVSAAHRARYFWGNLPGMNRPLASTVNDKLELQECLEHGRIAKFSKVRT
ITTRSNSIKQGKDQHFPVFMNEKEDILWCTEMERVFGFPVHYTDVSNMSRLARQRL
LGRSWSVPVIRHLFAPLKEYFACVSSGNSNANSRGPSFSSGLVPLSLRGSHMGPMEIYKT
VSAWKRQPVRVLSLFRNIDKVLKSLGFLESGSGSGGGTLKYVEDVTNVVRRDVEK
WGPFDLVYGSTQPLGSSCDRCPGWYMFQFHRILQYALPRQESQRPFFWIFMDNLL
LTEDDQETTTRFLQTEAVTLQDVRGRDYQNAMRVWSNIPGLKSKHAPLTPKEEEY
LQAQVRSRSKLDAPKVDLLVKNCLLPLREYFKYFSQNSLPLGGPSSGAPPPSGGSPAG
SPTSTEEGTSESATPESGPGTSTEPSEGSAPGSPAGSPTSTEEGTSTEPSEGSAPGTSTEPSEPKK
KRKVMDKKYSIGLAIGTNSVGWAVITDEYKVPSKKFKVLGNTDRHSIKKNLIGALLFDSG

ETAEATRLKRTARRRYTRRKNRICYLQEIFSNEMAKVDDSFFHRLEESFLVEEDKKHER
HPIFGNIVDEVAYHEKYPTIYHLRKKLVDSTDKADLRLIYLALAHMIKFRGHFLIEGDLN
PDNSDVDKLFIQLVQTYNQLFEENPINASGVDAKAILSARLSKSRRLENLIAQLPGEKKN
GLFGNLIALSLGLTPNFKSNFDLAEDAKLQLSKDTYDDDLDNLLAQIGDQYADLFLAAK
NLSDAILLSDILRVNTEITKAPLSASMIKRYDEHHQDLTLLKALVRQQLPEKYKEIFFDQS
KNGYAGYIDGGASQEEFYKFIKPILEKMDGTEELLVKLNREDLLRKQRTFDNGSIPHQIH
LGELHAILRRQEDFYPFLKDNREKIEKILTFRIPYYVGPLARGNSRFAWMTRKSEETITP
WNFEEVVDKGASAQSFIERMTNFDKNLPNEKVLPKHSLLYEYFTVYNELTKVKYVTEG
MRKPAFLSGEQKKAIVDLLFKTNRKVTVKQLKEDYFKKIECFDSVEISGVEDRFNASLGT
YHDLLKIIKDKDFLDNEENEDILEDIVLTLTLFEDREMIEERLKTYAHLFDDKVMKQLK
RRRYTGWGRLSRKLINGIRDKQSGKTILDFLKSDGFANRNFMQLIHDDSLTFKEDIQKA
QVSGQGDSLHEHIANLAGSPAIKKGILQTVKVVDELVKVMGRHKPENIVIEMARENQTT
QKGQKNSRERMKRIEEGIKELGSQILKEHPVENTQLQNEKLYLYYLQNGRDMYVDQEL
DINRLSDYDVDAIVPQSFLKDDSIDNKVLTRSDKNRGKSDNVPSEEVVKKMKNYWRQLL
NAKLITQRKFDNLTKAERGGLSELDKAGFIKRQLVETRQITKHVAQILDSRMNTKYDEN
DKLIREVKVITLKSKLVSDFRKDFQFYKVREINNYHHAHDAYLNAVVGTALIKKYPKLES
EFVYGDYKVYDVRKMIAKSEQEIGKATAKYFFYSNIMNFFKTEITLANGEIRKRPLIETN
GETGEIVWDKGRDFATVRKVLSMPQVNIVKKTEVQTGGFSKESILPKRNSDKLIARKKD
WDPKKYGGFDSPTVAYSVLVVAKVEKGKSKKLKSVKELLGITIMERSSFEKNPIDFLEAK
GYKEVKKDLIIKLPKYSLFELENGRKRMLASAGELQKGNELALPSKYVNFLYLASHYEKL
KGSPEDNEQKQLFVEQHKHYLDEIIEQISEFSKRVILADANLDKVLSAYNKHRDKPIREQ
AENIIHLFTLTNLGAPAAFKYFDTTIDRKRYTSTKEVLDATLIHQSITGLYETRIDLSQLGG
DAYPYDVPDYASLGSGSEDPKKKRKVDGSGSETPGTSESATPESRTLLSGKKAAAAAAA
AAAAATGTEAGPGTAGGSENGSEVAAQPAGLSGPAEVGPGAVGERTPRKKEPPRA
SPPGGLAEPPGSAGPQAGPTVVPGSATPMETGIAETPEGRRTSRRKRAKVEYREMD
ESLANLSEDEYYSEEERNAKAEKEKKLPPPPPQAPPEEENESEPEEPSGQAGGLQDD
SSGGYGDGQASGVEGAAFQSRLPHDRMTSQEAACFPDIISGPQQTQKVFLFIRNRTL

QLWLDNPKIQLTFEATLQQLEAPYNSDTVLVHRVHSYLERHGLINFGIYKRIKPLPT
KKTGKVIIIGSGVSGLAAARQLQSFGMDVTLLEARDRVGGRVATFRKGNYVADLG
AMVVTGLGGNPMAVVSKQVNMELAKIKQKCPLYEANGQADTVKVPKEKDEMVE
QEFNRLLEATSYLSHQLDFNVLNNKPVSLGQALEVVIQLQEKHVKDEQIEHWKKIV
KTQEELKELLNKMVNLKEKIKELHQQYKEASEVKPPRDITAEFLVKSKHRDLTAL
CKEYDELAETQGKLEEKLQELEANPPSDVYLSSRDRQILDWHFANLEFANATPLST
LSLKHWDQDDDFEFTGSHLTVRNGYSCVPVALAEGLDIKLNTAVRQVRYTASGCE
VIAVNTRSTSQTFIYKCDAVLCTLPLGVLKQQPPAVQFVPPLPEWKTSAVQRMGFG
NLNKVVLCFDRVFWDPSVNLFGHVGSTTASRGELFLFWNLYKAPILLALVAGEAA
GIMENISDDVIVGRCLAILKGIFGSSAVPQPKETVVSRWRADPWARGSYSYVAAGSS
GNDYDLMAQPITPGPSIPGAPQPIPRLFFAGEHTIRNYPATVHGALLSGLREAGRIAD
QFLGAMYTLPRQATPGVPAQQSPSMEGSG*

The amino acid sequence of DNMT3A-DNMT3L-XTEN80-TALE8-XTEN16-KRAB (V76 scheme, SEQ ID NO: 355) is as follows:

MKRTADGSEFESPKKKRKVGSNHDQEFDPPKVYPPVPAEKRKPIRVLSLFDGIAT
GLLVLKDLGIQVDRYIASEVCEDSITVGMVRHQGKIMYVGDVRSVTQKHIQEWGP
FDLVIGGSPCNDLSIVNPARKGLYEGTGRLFFEFYRLLHDARPKEGDDRPFFWLFEN
VVAMGVSDKRDISRFLESNPVMIDAKEVSAAHRARYFWGNLPGMNRPLASTVNDK
LELQECLEHGRIAKFSKVRTITTRSNSIKQGKDQHFPVFMNEKEDILWCTEMERVF
GFPVHYTDVSNMSRLARQRLLGRSWSVPVIRHLFAPLKEYFACVSSGNSNANSRGPSF
SSGLVPLSLRGSHMGPMEIYKTVSAWKRQPVRVLSLFRNIDKVLKSLGFLESGSGSGG
GTLKYVEDVTNVVRRDVEKWGPFDLVYGSTQPLGSSCDRCPGWYMFQFHRILQY
ALPRQESQRPFFWIFMDNLLLTEDDQETTTRFLQTEAVTLQDVRGRDYQNAMRVW
SNIPGLKSKHAPLTPKEEEYLQAQVRSRSKLDAPKVDLLVKNCLLPLREYFKYFSQ
NSLPLGGPSSGAPPPSGGSPAGSPTSTEEGTSESATPESGPGTSTEPSEGSAPGSPAGSPTSTEE
GTSTEPSEGSAPGTSTEPSEVDLRTLGYSQQQQEKIKPKVRSTVAQHHEALVGHGFTHAHI
VALSQHPAALGTVAVKYQDMIAALPEATHEAIVGVGKQWSGARALEALLTVAGELRGPP

LQLDTGQLLKIAKRGGVTAVEAVHAWRNALTGAPLNLTPDQVVAIASNGGGKQALETV
QRLLPVLCQDHGLTPDQVVAIASHDGGKQALETVQRLLPVLCQDHGLTPDQVVAIASNI
GGKQALETVQRLLPVLCQDHGLTPDQVVAIASNNGGKQALETVQRLLPVLCQDHGLTP
DQVVAIASNGGGKQALETVQRLLPVLCQDHGLTPDQVVAIASNGGGKQALETVQRLLPV
LCQDHGLTPDQVVAIASHDGGKQALETVQRLLPVLCQDHGLTPDQVVAIASNIGGKQAL
ETVQRLLPVLCQDHGLTPDQVVAIASNNGGKQALETVQRLLPVLCQDHGLTPDQVVAIA
SNNGGKQALETVQRLLPVLCQDHGLTPDQVVAIASNNGGKQALETVQRLLPVLCQDHG
LTPDQVVAIASNGGGKQALETVQRLLPVLCQDHGLTPDQVVAIASHDGGKQALETVQRL
LPVLCQDHGLTPDQVVAIASNGGGKQALETVQRLLPVLCQDHGLTPDQVVAIASNNGGK
QALETVQRLLPVLCQDHGLTPDQVVAIASNIGGKQALETVQRLLPVLCQDHGLTPDQVV
AIASNNGGKQALETVQRLLPVLCQDHGLTPDQVVAIASHDGGKQALESIVAQLSRPDPA
LAALTNDHLVALACLGGRPAMDAVKKGLPHAPELIRRVNRRIGERTSHRVAYPYDVPD
YASLGSGSPKKKRKVEDPKKKRKVDGSGSETPGTSESATPESRTLVTFKDVFVDFTREE
WKLLDTAQQIVYRNVMLENYKNLVSLGYQLTKPDVILRLEKGEEPLEDGD*

The amino acid sequence of DNMT3A-DNMT3L-XTEN80-TALE6-XTEN16-KRAB (V77 scheme, SEQ ID NO: 356) is as follows:

MKRTADGSEFESPKKKRKVGSNHDQEFDPPKVYPPVPAEKRKPIRVLSLFDGIAT
GLLVLKDLGIQVDRYIASEVCEDSITVGMVRHQGKIMYVGDVRSVTQKHIQEWGP
FDLVIGGSPCNDLSIVNPARKGLYEGTGRLFFEFYRLLHDARPKEGDDRPFFWLFEN
VVAMGVSDKRDISRFLESNPVMIDAKEVSAAHRARYFWGNLPGMNRPLASTVNDK
LELQECLEHGRIAKFSKVRTITTRSNSIKQGKDQHFPVFMNEKEDILWCTEMERVF
GFPVHYTDVSNMSRLARQRLLGRSWSVPVIRHLFAPLKEYFACVSSGNSNANSRGPSF
SSGLVPLSLRGSHMGPMEIYKTVSAWKRQPVRVLSLFRNIDKVLKSLGFLESGSGSGG
GTLKYVEDVTNVVRRDVEKWGPFDLVYGSTQPLGSSCDRCPGWYMFQFHRILQY
ALPRQESQRPFFWIFMDNLLLTEDDQETTTRFLQTEAVTLQDVRGRDYQNAMRVW
SNIPGLKSKHAPLTPKEEEYLQAQVRSRSKLDAPKVDLLVKNCLLPLREYFKYFSQ
NSLPLGGPSSGAPPPSGGSPAGSPTSTEEGTSESATPESGPGTSTEPSEGSAPGSPAGSPTSTEE

GTSTEPSEGSAPGTSTEPSEVDLRTLGYSQQQQEKIKPKVRSTVAQHHEALVGHGFTHAHI
VALSQHPAALGTVAVKYQDMIAALPEATHEAIVGVGKQWSGARALEALLTVAGELRGPP
LQLDTGQLLKIAKRGGVTAVEAVHAWRNALTGAPLNLTPDQVVAIASNGGGKQALETV
QRLLPVLCQDHGLTPDQVVAIASNNGGKQALETVQRLLPVLCQDHGLTPDQVVAIASNN
GGKQALETVQRLLPVLCQDHGLTPDQVVAIASNIGGKQALETVQRLLPVLCQDHGLTPD
QVVAIASNNGGKQALETVQRLLPVLCQDHGLTPDQVVAIASNGGGKQALETVQRLLPVL
CQDHGLTPDQVVAIASHDGGKQALETVQRLLPVLCQDHGLTPDQVVAIASNGGGKQAL
ETVQRLLPVLCQDHGLTPDQVVAIASNNGGKQALETVQRLLPVLCQDHGLTPDQVVAIA
SNNGGKQALETVQRLLPVLCQDHGLTPDQVVAIASHDGGKQALETVQRLLPVLCQDHG
LTPDQVVAIASNIGGKQALETVQRLLPVLCQDHGLTPDQVVAIASNGGGKQALETVQRL
LPVLCQDHGLTPDQVVAIASHDGGKQALETVQRLLPVLCQDHGLTPDQVVAIASHDGG
KQALETVQRLLPVLCQDHGLTPDQVVAIASHDGGKQALETVQRLLPVLCQDHGLTPDQ
VVAIASNIGGKQALETVQRLLPVLCQDHGLTPDQVVAIASHDGGKQALESIVAQLSRPDP
ALAALTNDHLVALACLGGRPAMDAVKKGLPHAPELIRRVNRRIGERTSHRVAYPYDVP
DYASLGSGSPKKKRKVEDPKKKRKVDGSGSETPGTSESATPESRTLVTFKDVFVDFTRE
EWKLLDTAQQIVYRNVMLENYKNLVSLGYQLTKPDVILRLEKGEEPLEDGD*

Without intending to be bound by any theory, the following examples are only intended to illustrate the fusion protein, preparation method and use of the present application, etc., and are not intended to limit the scope of the invention of the present application.

### EXAMPLES

### Example 1

### Design and construction of plasmids containing the fusions of the present application

The amino acid sequence of the epigenetic modification editor (including Dnmt3a CD, Dnmt3l CD, dSpCas9 or TALE, KRAB) with HA epitope, P2A and BFP was optimized and synthesized by Genscript into a nucleic acid sequence suitable for mammalian expression, and then the nucleic acid sequence was cloned into the pLV-CAG vector with CAG promoter and WPRE, and the complete epigenetic modification editor and self-cleaved BFP were expressed by the CAG promoter.

When optimizing the order of different elements, different fragments were amplified using PCR primers with homology arms, and then the different fragments were sequentially recombined into a specific order using NEBuilder reagent.

When optimizing different functional elements, the different functional elements were optimized and synthesized into nucleic acid sequences suitable for mammalian expression by Genscript. First, the vector other than the element to be replaced was amplified by PCR, and then the element to be replaced was amplified from the sequence synthesized by the company, and the homologous arm sequence was introduced at the same time. Finally, the different elements were recombined into the vector through the NEBuilder reagent to construct the final expression plasmid.

### Example 2

### The fusions of the present application were capable of inhibiting expression of the VEGFA gene

Two groups of experiments were designed to evaluate the effect of the fusion provided in the present application (comprising the amino acid sequences as set forth in SEQ ID NOs: 337-342) for inhibiting the expression of the target gene. In each group, 7 editor plasmids and 1 control were constructed according to the method of example 1. The two groups used two different gRNAs (comprising the nucleotide sequences as set forth in SEQ ID NOs: 332 and 333) targeting the mouse VEGFA gene, and 7 different editor plasmids were co-transfected into the HEK293T cell line using PEI (tools: 350 ng of editor, 150 ng of gRNA, 48-well plate). The reporter gene of mouse VEGFA was inserted into the 293T cell line, and GFP expression was regulated by the regulatory region of VEGFA.

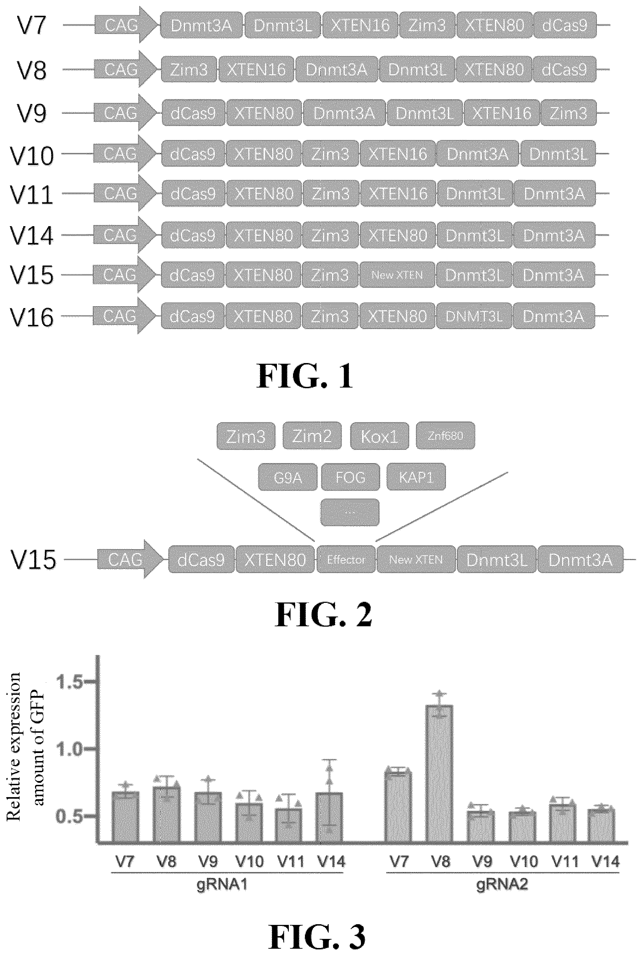

The average green fluorescence intensity of different samples was analyzed 11 days after transfection, and divided by the fluorescence intensity of the control group (transfected with control NT gRNA, whose sequence was as set forth in SEQ ID NO: 364) to obtain the relative inhibition efficiency (FIG. 3). The results showed that the fusions of the present application had achieved higher or comparable inhibition efficiency on GFP expression compared with the control group.

### Example 3

### The fusions of the present application were capable of inhibiting expression of the CTLA-1 gene

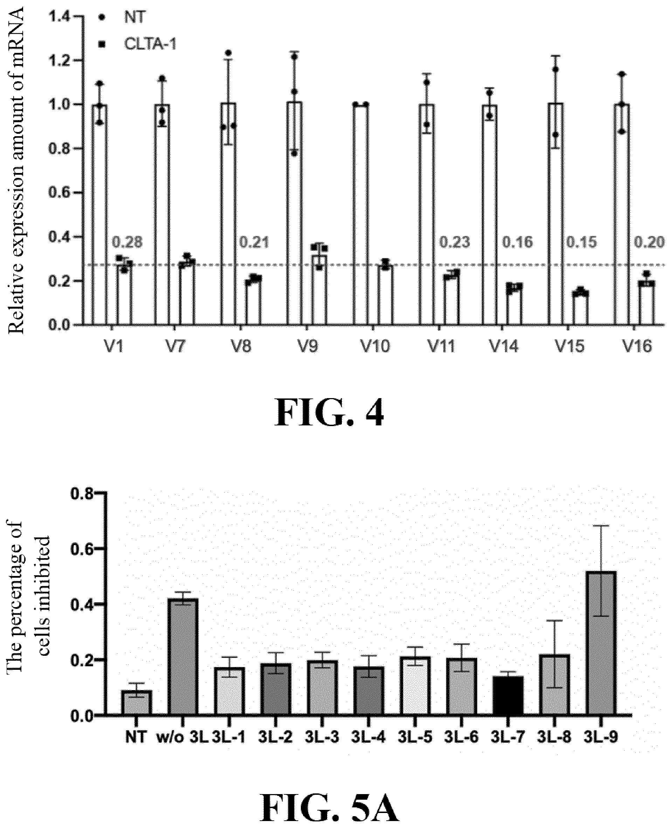

Different editing tools (comprising the amino acid sequences as set forth in SEQ ID NOs: 337-344) were constructed according to the method of example 1, and a control group V1 (the N-terminus and C-terminus of the nucleic acid-binding domain were fused with a methylation factor and a transcriptional repressor, respectively, comprising the amino acid sequence as set forth in SEQ ID NO: 328) was set up. These editing tools and a gRNA targeting the human CLTA gene (comprising the nucleotide sequence as set forth in SEQ ID NO: 334) or a control gRNA (NT gRNA, whose sequence was as set forth in SEQ ID NO: 364) were co-transfected into the HEK293T cell line (tools: 700 ng of editor, 300 ng of gRNA, 24-well plate), and the transfection-positive cells were sorted 72 hours after transfection, and the total RNA was extracted with Trizol. The relative expression amount of CLTA was quantified by qPCR, and the relative inhibition efficiency of different tools was calculated (FIG. 4). The results showed that the fusions of the present application achieved a comparable inhibitory efficiency on the mRNA transcribed from the CLTA gene compared with the V1 group.

### Example 4

### The fusions of the present application were capable of inhibiting expression of the CD151 gene

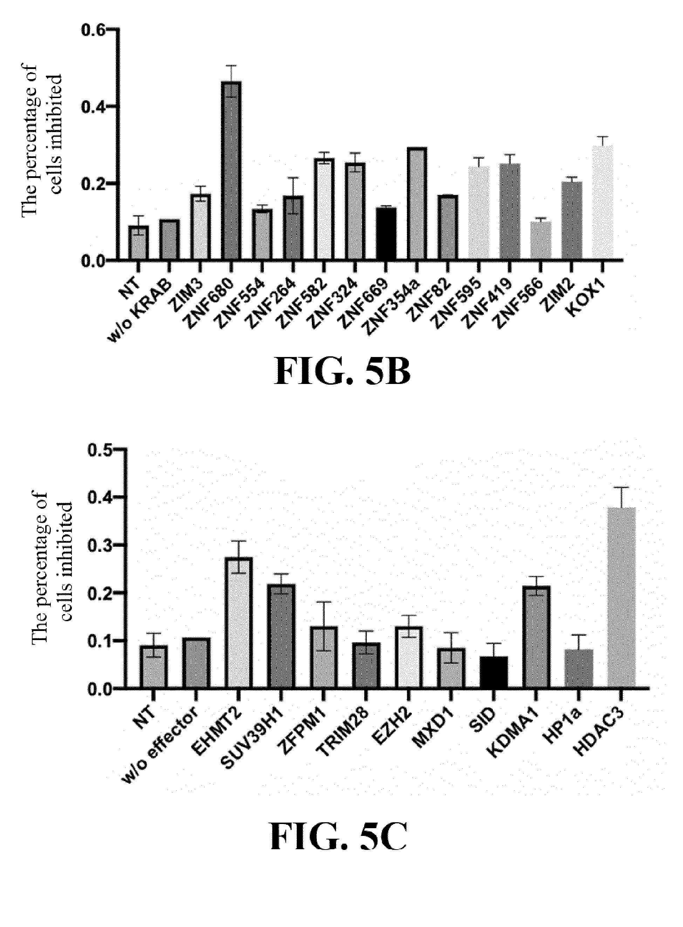

Different versions of epigenetic editing tools of the V15' scheme (the original amino acid sequence was as set forth in SEQ ID NO: 343) were constructed according to the method of example 1, wherein (1) the effector domain contained DNMT3L factors from different sources (such as the amino acid sequences as set forth in SEQ ID NOs: 13-21), such as versions 3L-1 to 3L-9 as shown in FIG. 5A; (2) the effector domain contained different zinc finger protein-based transcriptional repressors (such as the amino acid sequences as set forth in SEQ ID NOs: 22-35), such as versions for example ZIM3 and ZNF680 as shown in FIG. 5B; and (3) the effector domain contained different types of transcriptional repressors (such as the amino acid sequences as set forth in SEQ ID NOs: 36-46), such as versions for example EHMT2 and SUV39H1 as shown in FIG. 5C. The above-mentioned different editing tools and gRNA plasmids targeting CD151 or control gRNA (NT gRNA, whose sequence was as set forth in SEQ ID NO: 364) were transfected into HEK293T cells (tools: 700 ng, 300 ng of gRNA, 24-well plates). After 48 hours of transfection, transfection-positive cells were sorted using a flow sorter for continued culture. 9 days after transfection, the cells were harvested and the expression amount of CD151 was measured by flow cytometer. When the expression amount of CD151 measured was lower than that of untransfected cells, these cells were defined as suppressed cells. The effects of different versions of epigenetic editing tools were compared based on the proportion of suppressed cells to all cells (FIGs. 5A-5C). The results showed that the fusions of the present application could achieve higher or comparable inhibition effects when containing different methylation factors or containing different transcriptional repressors compared with the control group (NT gRNA and the group without fusion with DNMT3L factors or transcriptional repressors).

### Example 5

### The fusions of the present application were capable of inhibiting expression of the PCSK9 gene

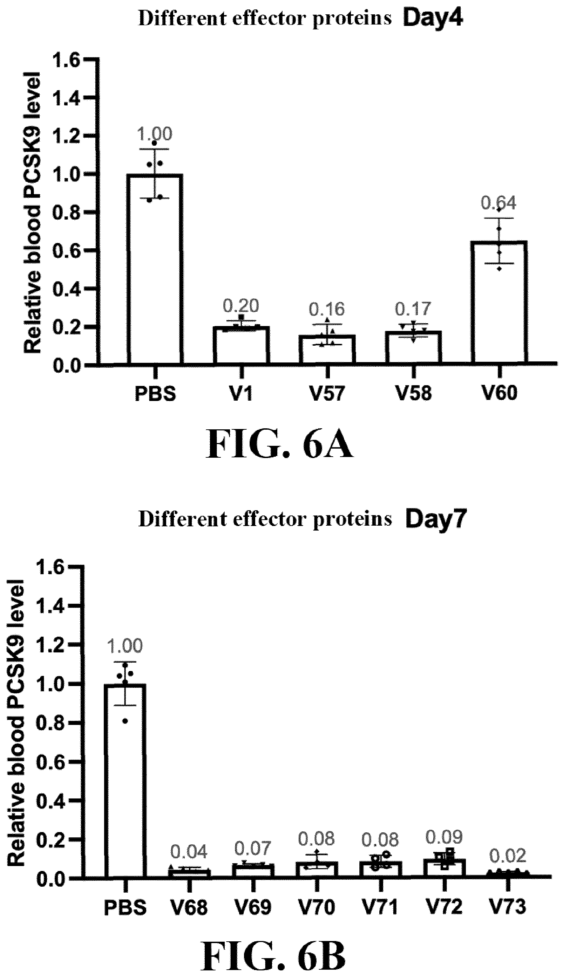

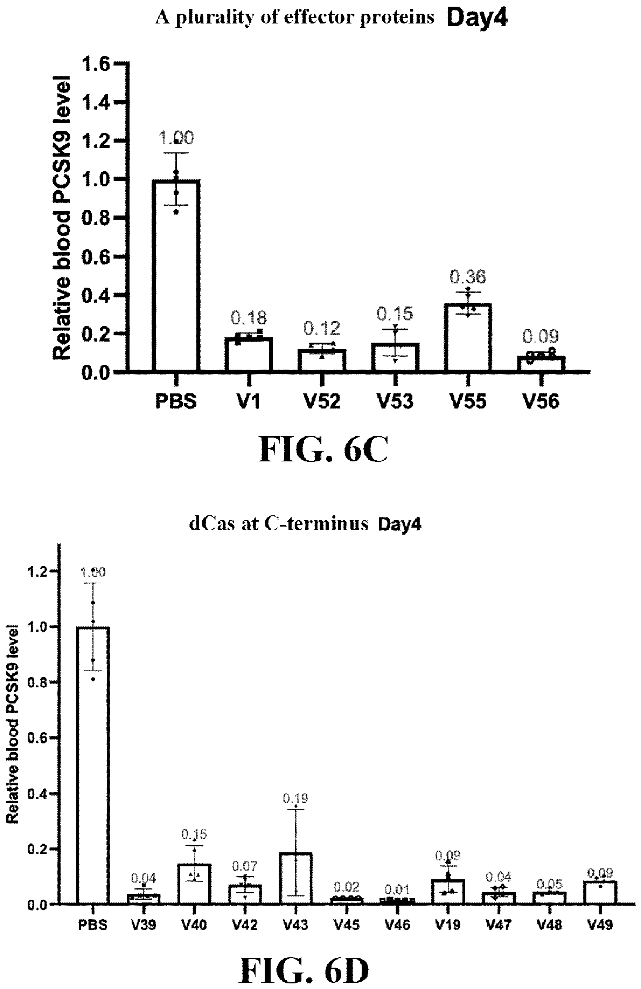

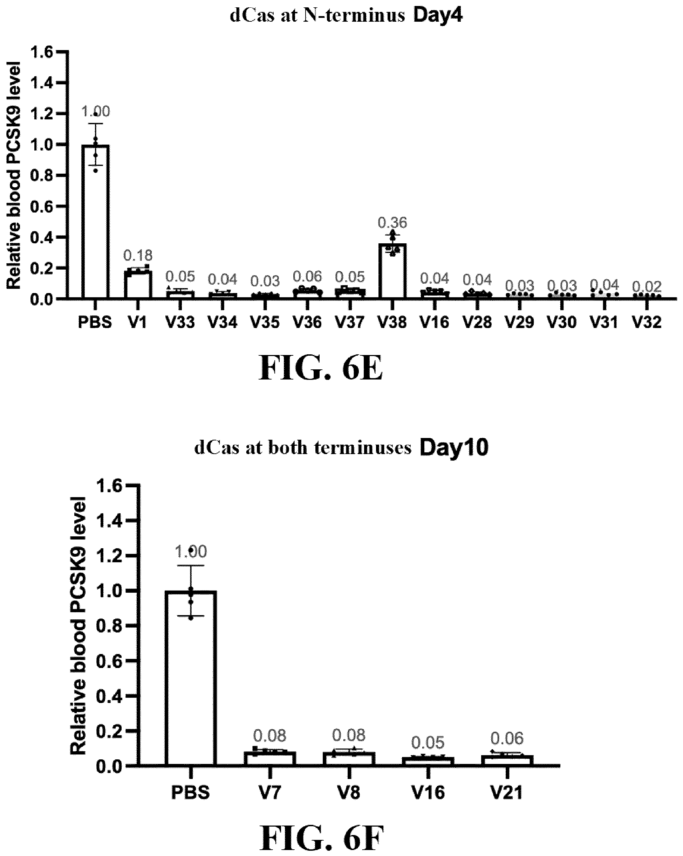

Different versions of the tool were transcribed into mRNAs in vitro (the sequences of the mRNAs were as set forth in SEQ ID NOs: 261-327), and then the obtained mRNAs were mixed with chemically synthesized sgRNAs at a 1:1 mass ratio (the sequences of the sgRNAs were SEQ ID NOs: 335 and 336) to prepare LNPs (References for LNPs: Musunuru, K., Chadwick, A.C., Mizoguchi, T.et al. In vivo CRISPR base editing of PCSK9 durably lowers cholesterol in primates. Nature 593, 429-434 (2021).). The prepared LNPs were injected into mice through the tail vein at a dose of 4.5 mg per kg body weight. Blood was collected from the cheeks of mice 4-10 days after the injection, and the content of PCSK9 protein in the blood was measured by Elisa. The PBS group was a control group injected with an equal volume of PBS. The results were shown in FIGs. 6A-6F. The fusions provided by the present application had a significant inhibition effect on the expression of the PCSK9 gene.

### Example 6

### The fusions of the present application were capable of inhibiting expression of the PCSK9 gene

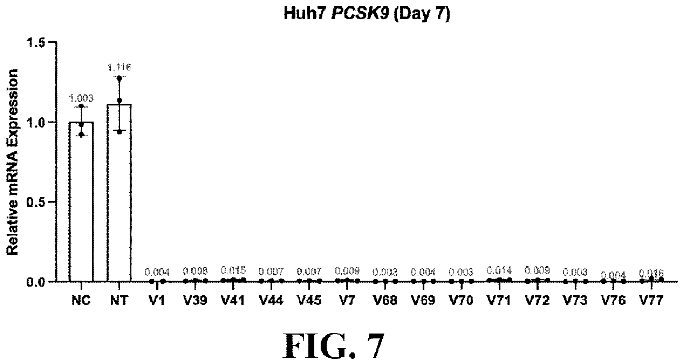

According to the methods for transcribing mRNAs (see SEQ ID NOs: 261-327 for the sequences of the mRNAs) in vitro, chemically synthesizing a mixture of sgRNA/mRNA at a mass ratio of 1:1 (the sequence of the sgRNA was as set forth in SEQ ID NO: 363), and preparing LNPs in example 5, the prepared LNPs was added to Huh7 cells (1.25 ug/mL dose). 7 days after the addition of LNPs, all cells were harvested, total RNA was extracted with Trizol, the relative expression amount of PCSK9 was quantified by qPCR, and the relative inhibition efficiency of different tools was calculated. The results were shown in FIG. 7, where the NC group was a control without LNPs, and the NT group was a control with NT gRNA (whose sequence was as set forth in SEQ ID NO: 364). It could be seen that the fusions provided by the present application had a significant inhibition effect on the expression of the PCSK9 gene.

## Claims

1. A fusion comprising a nucleic acid-binding domain and one or more effector domains, wherein the effector domain comprises one or more of epigenetic modification domains and/or transcriptional regulation domains and the fusion comprises at least two types of epigenetic modification domains and/or transcriptional regulation domains.

2. The fusion according to claim 1, wherein the nucleic acid-binding domain is a DNA-binding domain.

3. The fusion according to claim 2, wherein the DNA-binding domain is selected from a TALE domain, a zinc finger domain, a tetR domain, a large-range nuclease, a Cas protein, an Argonaute (Ago) protein, and a homolog or modified form thereof.

4. The fusion according to any one of claims 2-3, wherein the DNA-binding domain is capable of binding to a target sequence of a target locus.

5. The fusion according to claim 4, wherein the DNA-binding domain is capable of binding to a guide RNA.

6. The fusion according to claim 5, wherein the guide RNA is capable of specifically recognizing and hybridizing to the target sequence of the target locus.

7. The fusion according to any one of claims 2-6, wherein the DNA-binding domain is a Cas protein, and the Cas protein is a class II Cas nuclease.

8. The fusion according to claim 7, wherein the Cas protein is selected from a class II type II Cas nuclease and a class II type V Cas nuclease.

9. The fusion according to any one of claims 2-8, wherein the DNA-binding domain is a Cas9 protein.

10. The fusion according to claim 9, wherein the Cas9 protein is a dead Cas9 protein (dCas9).

11. The fusion according to any one of claims 1-6, wherein the nucleic acid-binding domain comprises an amino acid sequence as set forth in any one of SEQ ID NOs: 1-7, 353 and 354.

12. The fusion according to any one of claims 1-11, wherein the epigenetic modification domain is selected from DNA deamination activity, DNA methyltransferase activity, DNA demethylase activity, DNA amination activity, DNA oxidation activity, DNA helicase activity, histone acetyltransferase activity, histone deacetylase activity, histone methyltransferase activity, histone demethylase activity, histone kinase activity, histone phosphatase activity, histone ubiquitin ligase activity, and histone deubiquitination activity.

13. The fusion according to any one of claims 1-12, wherein the epigenetic modification domain comprises a DNA methyltransferase and/or a functionally active fragment thereof.

14. The fusion according to claim 13, wherein the DNA methyltransferase is selected from DNMT3A, DNMT3B, Dnmt3c, DNMT1, DNMT2 and DNMT3L.

15. The fusion according to any one of claims 1-14, wherein the epigenetic modification domain comprises a plurality of DNA methyltransferases and/or functionally active fragments thereof, and the plurality of DNA methyltransferases and/or functionally active fragments thereof are linked via a linker sequence.

16. The fusion according to any one of claims 1-15, wherein the epigenetic modification domain comprises at least one DNMT3A and at least one DNMT3L.

17. The fusion according to any one of claims 13-15, wherein the DNA methyltransferase comprises an amino acid sequence as set forth in any one of SEQ ID NOs: 8-21.

18. The fusion according to any one of claims 1-17, wherein the transcriptional regulation domain is a transcriptional activation domain or a transcriptional repressor domain.

19. The fusion according to claim 18, wherein the transcriptional repressor domain is selected from KRAB, ZIM3, ZNF680, ZNF554, ZNF264, ZNF582, ZNF324, ZNF669, ZNF354A, ZNF82, ZNF595, ZNF419, ZNF566, ZIM2, EHMT2, SUV39H1, ZFPM1, TRIM28, EZH2, MXD1, SID, LSD1, HP1a, HDAC3, ZNF436, ZNF257, ZNF675, ZNF490, ZNF320, ZNF331, ZNF816, ZNF41, ZNF189, ZNF528, ZNF543, ZNF140, ZNF610, ZNF350, ZNF8, ZNF30, ZNF98, ZNF677, ZNF596, ZNF214, ZNF37A, ZNF34, ZNF250, ZNF547, ZNF273, ZFP82, ZNF224, ZNF33A, ZNF45, ZNF175, ZNF184, ZFP28-1, ZFP28-2, ZNF18, ZNF213, ZNF394, ZFP1, ZFP14, ZNF416, ZNF557, ZNF729, ZNF254, ZNF764, ZNF785, ZNF10, CBX5, RYBP, YAF2, MGA, CBX1, SCMH1, MPP8, SUMO3, HERC2, BIN1, PCGF2, TOX, FOXA1, FOXA2, IRF2BP1, IRF2BP2, IRF2BPL IRF-2BP1_2 N-terminal domain, HOXA13, HOXB13, HOXC13, HOXA11, HOXC11, HOXC10, HOXA10, HOXB9, HOXA9, ZFP28, ZN334, ZN568, ZN37A, ZN181, ZN510, ZN862, ZN140, ZN208, ZN248, ZN571, ZN699, ZN726, ZIK1, ZNF2, Z705F, ZNF14, ZN471, ZN624, ZNF84, ZNF7, ZN891, ZN337, Z705G, ZN529, ZN729, ZN419, Z705A, ZN302, ZN486, ZN621, ZN688, ZN33A, ZN554, ZN878, ZN772, ZN224, ZN184, ZN544, ZNF57, ZN283, ZN549, ZN211, ZN615, ZN253, ZN226, ZN730, Z585A, ZN732, ZN681, ZN667, ZN649, ZN470, ZN484, ZN431, ZN382, ZN254, ZN124, ZN607, ZN317, ZN620, ZN141, ZN584, ZN540, ZN75D, ZN555, ZN658, ZN684, RBAK, ZN829, ZN582, ZN112, ZN716, HKR1, ZN350, ZN480, ZN416, ZNF92, ZN100, ZN736, ZNF74, ZN443, ZN195, ZN530, ZN782, ZN791, ZN331, Z354C, ZN157, ZN727, ZN550, ZN793, ZN235, ZN724, ZN573, ZN577, ZN789, ZN718, ZN300, ZN383, ZN429, ZN677, ZN850, ZN454, ZN257, ZN264, ZN485, ZN737, ZNF44, ZN596, ZN565, ZN543, ZFP69, SUMO1, ZNF12, ZN169, ZN433, ZN175, ZN347, ZNF25, ZN519, Z585B, ZN517, ZN846, ZN230, ZNF66, ZN713, ZN816, ZN426, ZN674, ZN627, ZNF20, Z587B, ZN316, ZN233, ZN611, ZN556, ZN234, ZN560, ZNF77, ZN682, ZN614, ZN785, ZN445, ZFP30, ZN225, ZN551, ZN610, ZN528, ZN284, ZN418, ZN490, ZN805, Z780B, ZN763, ZN285, ZNF85, ZN223, ZNF90, ZN557, ZN425, ZN229, ZN606, ZN155, ZN222, ZN442, ZNF91, ZN135, ZN778, ZN534, ZN586, ZN567, ZN440, ZN583, ZN441, ZNF43, ZN589, ZN563, ZN561, ZN136, ZN630, ZN527, ZN333, Z324B, ZN786, ZN709, ZN792, ZN599, ZN613, ZF69B, ZN799, ZN569, ZN564, ZN546, ZFP92, ZN723, ZN439, ZFP57, ZNF19, ZN404, ZN274, CBX3, ZN250, ZN570, ZN675, ZN695, ZN548, ZN132, ZN738, ZN420, ZN626, ZN559, ZN460, ZN268, ZN304, ZN605, ZN844, SUMO5, ZN101, ZN783, ZN417, ZN182, ZN823, ZN177, ZN197, ZN717, ZN669, ZN256, ZN251, CBX4, CDY2, CDYL2, ZN562, ZN461, Z324A, ZN766, ID2, ZN214, CBX7, ID1, CREM, SCX, ASCL1, ZN764, SCML2, TWST1, CREB1, TERF1, ID3, CBX8, GSX1, NKX22, ATF1, TWST2, ZNF17, TOX3, TOX4, ZMYM3, I2BP1, RHXF1, SSX2, I2BPL, ZN680, TRI68, HXA13, PHC3, TCF24, HXB13, HEY1, PHC2, ZNF81, FIGLA, SAM11, KMT2B, HEY2, JDP2, HXC13, ASCL4, HHEX, GSX2, ETV7, ASCL3, PHC1, OTP, I2BP2, VGLL2, HXA11, PDLI4, ASCL2, CDX4, ZN860, LMBL4, PDIP3, NKX25, CEBPB, ISL1, CDX2, PROP1, SIN3B, SMBT1, HXC11, HXC10, PRS6A, VSX1, NKX23, MTG16, HMX3, HMX1, KIF22, CSTF2, CEBPE, DLX2, PPARG, PRIC1, UNC4, BARX2, ALX3, TCF15, TERA, VSX2, HXD12, CDX1, TCF23, ALX1, HXA10, RX, CXXC5, SCML1, NFIL3, DLX6, MTG8, CEBPD, SEC13, FIP1, ALX4, LHX3, PRIC2, MAGI3, NELL1, PRRX1, MTG8R, RAX2, DLX3, DLX1, NKX26, NAB1, SAMD7, PITX3, WDR5, MEOX2, NAB2, DHX8, CBX6, EMX2, CPSF6, HXC12, KDM4B, LMBL3, PHX2A, EMX1, NC2B, DLX4, SRY, ZN777, ZN398, GATA3, BSH, SF3B4, TEAD1, TEAD3, RGAP1, PHF1, GATA2, FOXO3, ZN212, IRX4, ZBED6, LHX4, SIN3A, RBBP7, NKX61, R51A1, MB3L1, DLX5, NOTC1, TERF2, ZN282, RGS12, ZN840, SPI2B, PAX7, NKX62, ASXL2, FOXO1, GATA1, ZMYM5, LRP1, MIXL1, SGT1, LMCD1, CEBPA, SOX14, WTIP, PRP19, NKX11, RBBP4, DMRT2, SMCA2, and a functionally active fragment thereof.

20. The fusion according to claim 18 or 19, wherein the transcriptional repressor domain comprises an amino acid sequence as set forth in any one of SEQ ID NOs: 22-46.

21. The fusion according to any one of claims 1-20, wherein one or more of the nucleic acid-binding domain, the epigenetic modification domain and the transcriptional regulation domain are linked via a linker sequence.

22. The fusion according to claim 21, wherein the linker sequence comprises at least 16 amino acids.

23. The fusion according to claim 21 or 22, wherein the linker sequence comprises an XTEN linker sequence.

24. The fusion according to any one of claims 21-23, wherein the linker sequence comprises one or more fragment sequences truncated from an amino acid sequence as set forth in SEQ ID NO: 59, and the one or more fragment sequences comprise no less than 16 consecutive amino acids.

25. The fusion according to any one of claims 21-24, wherein the linker sequence comprises a GS linking peptide, and the GS linking peptide comprises the sequence (GS)ₐ(GGS)_{b}(GGGS)_{c}(GGGGS)_{d}, in which G represents a glycine residue (Gly), S represents a serine residue (Ser), and a, b, c and d represent integers greater than or equal to 0.

26. The fusion according to any one of claims 21-25, wherein the linker sequence comprises an amino acid sequence selected from one or more of SEQ ID NOs: 47-58.

27. The fusion according to any one of claims 1-26, wherein the one or more effector domains are N-terminal or C-terminal to the nucleic acid-binding domain.

28. The fusion according to claim 27, wherein the effector domain comprises at least one type of the epigenetic modification domain and at least one type of the transcriptional regulation domain.

29. The fusion according to claim 27 or 28, wherein the epigenetic modification domain comprises DNMT3A and DNMT3L, and the C-terminus of the DNMT3A is linked to the N-terminus of the DNMT3L.

30. The fusion according to claim 27 or 28, wherein the epigenetic modification domain comprises DNMT3A and DNMT3L, and the C-terminus of the DNMT3L is linked to the N-terminus of the DNMT3A.

31. The fusion according to any one of claims 27-30, wherein the transcriptional regulation domain comprises a transcriptional repressor domain.

32. The fusion according to any one of claims 27-31, wherein the transcriptional regulation domain comprises a zinc finger protein-based transcription factor or a functionally active fragment thereof.

33. The fusion according to claim 32, wherein the zinc finger protein-based transcription factor comprises KRAB.

34. The fusion according to claim 32 or 33, wherein the zinc finger protein-based transcription factor is selected from ZIM3 KRAB and KOX1 KRAB.

35. The fusion according to any one of claims 27-34, wherein the transcriptional regulation domain comprises two or more of the zinc finger protein-based transcription factors or functionally active fragments thereof described above.

36. The fusion according to any one of claims 27-35, wherein the transcriptional regulation domain comprises two or more of the KRAB domains described above, and the two or more of the KRAB domains described above are of the same type or of different types.

37. The fusion according to claim 36, wherein the two or more of the KRAB domains described above are linked via an XTEN linker sequence.

38. The fusion according to any one of claims 27-37, wherein the one or more effector domains are N-terminal to the nucleic acid-binding domain, and the epigenetic editing domain in the effector domain is N-terminal or C-terminal to the transcriptional regulation domain.

39. The fusion according to any one of claims 27-38, wherein the fusion comprises the following substances linked in sequence from the N-terminus to the C-terminus:
(1) at least one type of the epigenetic editing domain, at least one type of the transcriptional regulation domain and the nucleic acid-binding domain;
(2) at least one type of the transcriptional regulation domain, at least one type of the epigenetic editing domain and the nucleic acid-binding domain; or
(3) at least one type of the transcriptional regulation domain, at least one type of the epigenetic editing domain, at least one type of the transcriptional regulation domain and the nucleic acid-binding domain.

40. The fusion according to any one of claims 27-39, wherein the fusion comprises the following substances linked in sequence from the N-terminus to the C-terminus:
(1) at least one DNA methyltransferase domain, at least one transcriptional repressor domain and the nucleic acid-binding domain;
(2) at least one transcriptional repressor domain, at least one DNA methyltransferase domain and the nucleic acid-binding domain; or
(3) at least one transcriptional repressor domain, at least one DNA methyltransferase domain, at least one transcriptional repressor domain and the nucleic acid-binding domain.

41. The fusion according to any one of claims 27-40, wherein the fusion comprises the following substances linked in sequence from the N-terminus to the C-terminus:
(1) one or a combination of DNMT3A and DNMT3L, one or more of the zinc finger protein-based transcription factors described above, and dCas9;
(2) one or more of the zinc finger protein-based transcription factors described above, one or a combination of DNMT3A and DNMT3L, and dCas9; or
(3) one or more of the zinc finger protein-based transcription factors described above, one or a combination of DNMT3A and DNMT3L, one or more of the zinc finger protein-based transcription factors described above, and dCas9.

42. The fusion according to any one of claims 27-41, wherein the fusion comprises the following domains: DNMT3A-DNMT3L-KRAB-dCas9, KRAB-DNMT3A-DNMT3L-dCas9, DNMT3A-DNMT3L-ZNF582-dCas9, DNMT3A-DNMT3L-ZNF324-dCas9, DNMT3A-DNMT3L-ZNF680-dCas9, DNMT3A-DNMT3L-ZNF354a-dCas9, DNMT3A-DNMT3L-ZNF419-dCas9, KRAB-DNMT3A-DNMT3L-KRAB-dCas9, KRAB-KRAB-DNMT3A-DNMT3L-dCas9, or DNMT3A-DNMT3L-KRAB-KRAB-dCas9, wherein - indicates that the domains of the fusion are each directly and/or indirectly linked, and the domains are arranged in the order from N-terminus to C-terminus.

43. The fusion according to any one of claims 27-37, wherein the one or more effector domains are C-terminal to the nucleic acid-binding domain, and the epigenetic editing domain in the effector domain is N-terminal or C-terminal to the transcriptional regulation domain.

44. The fusion according to any one of claims 27-37 and 43, wherein the fusion comprises the following substances linked in sequence from the N-terminus to the C-terminus:
(1) the nucleic acid-binding domain, at least one type of the epigenetic editing domain and at least one type of the transcriptional regulation domain;
(2) the nucleic acid-binding domain, at least one type of the transcriptional regulation domain and at least one type of the epigenetic editing domain; or
(3) the nucleic acid-binding domain, at least one type of the transcriptional regulation domain, at least one type of the epigenetic editing domain and at least one type of the transcriptional regulation domain.

45. The fusion according to any one of claims 27-37 and 43-44, wherein the fusion comprises the following substances linked in sequence from the N-terminus to the C-terminus:
(1) the nucleic acid-binding domain, at least one DNA methyltransferase domain and at least one transcriptional repressor domain;
(2) the nucleic acid-binding domain, at least one transcriptional repressor domain and at least one DNA methyltransferase domain; or
(3) the nucleic acid-binding domain, at least one transcriptional repressor domain, at least one DNA methyltransferase domain and at least one transcriptional repressor domain.

46. The fusion according to any one of claims 27-37 and 43-45, wherein the fusion comprises the following substances linked in sequence from the N-terminus to the C-terminus:
(1) dCas9, one or a combination of DNMT3A and DNMT3L, and one or more of the zinc finger protein-based transcription factors described above;
(2) dCas9, one or more of the zinc finger protein-based transcription factors described above, and one or a combination of DNMT3A and DNMT3L; or
(3) dCas9, one or more of the zinc finger protein-based transcription factors described above, one or a combination of DNMT3A and DNMT3L, and one or more of the zinc finger protein-based transcription factors described above.

47. The fusion according to any one of claims 27-37 and 43-46, wherein the fusion comprises the following domains: dCas9-DNMT3A-DNMT3L-KRAB, dCas9-DNMT3L-DNMT3A-KRAB, dCas9-KRAB-DNMT3A-DNMT3L, dCas9-KRAB-DNMT3L-DNMT3A, dCas9-ZNF582-DNMT3L-DNMT3A, dCas9-ZNF324-DNMT3L-DNMT3A, dCas9-DNMT3L-DNMT3A-KRAB-KRAB, dCas9-DNMT3L-DNMT3A-KRAB-KRAB-KRAB, or dCas9-KRAB-DNMT3L-DNMT3A-KRAB, wherein - indicates that the domains of the fusion are each directly and/or indirectly linked, and the domains are arranged in the order from N-terminus to C-terminus.

48. The fusion according to any one of claims 27-47, wherein the fusion comprises an amino acid sequence as set forth in any one of SEQ ID NOs: 60-67, 70-107 and 337-344.

49. The fusion according to any one of claims 1-26, wherein the fusion comprises one or more effector domains, and the effector domains are N-terminal and C-terminal to the nucleic acid-binding domain.

50. The fusion according to claim 49, wherein the one or more effector domains comprise at least one epigenetic editing domain.

51. The fusion according to claim 50, wherein the one or more effector domains comprise at least one epigenetic editing domain that provides histone modification.

52. The fusion according to any one of claims 49-51, wherein the effector domain that is C-terminal to the nucleic acid-binding domain comprises at least one type of epigenetic modification domain.

53. The fusion according to any one of claims 49-52, wherein the effector domain that is C-terminal to the nucleic acid-binding domain comprises at least one epigenetic modification domain that provides histone modification.

54. The fusion according to any one of claims 49-53, wherein the effector domain that is N-terminal to the nucleic acid-binding domain comprises one or more of the epigenetic modification domain and the transcriptional regulation domain.

55. The fusion according to any one of claims 49-54, wherein the effector domain that is N-terminal to the nucleic acid-binding domain comprises one or more of the epigenetic modification domain that provides histone modification, the epigenetic modification domain that provides DNA modification, and the transcriptional repressor domain.

56. The fusion according to any one of claims 49-55, wherein the effector domain that is N-terminal to the nucleic acid-binding domain comprises:
(1) the epigenetic modification domain that provides histone modification;
(2) the transcriptional repressor domain;
(3) the epigenetic modification domain that provides DNA modification; or
(4) the transcriptional repressor domain and the epigenetic modification domain that provides DNA modification.

57. The fusion according to any one of claims 49-56, wherein the effector domain that is N-terminal to the nucleic acid-binding domain comprises:
(1) one or more epigenetic modification domains selected from EZH2, HDAC3, HDAC1, EHMT2, PRMT1, PRMT5, SETDB1, hSIRT1, HP1a, LSD1, and functionally active fragments thereof;
(2) KRAB or a functionally active fragment thereof;
(3) DNMT3A, DNMT3L, or a combination of DNMT3A and DNMT3L; or
(4) a combination of KRAB and DNMT3A, a combination of KRAB and DNMT3L, or a combination of KRAB, DNMT3A, and DNMT3L.

58. The fusion according to any one of claims 49-57, wherein the fusion comprises the following substances linked in sequence from the N-terminus to the C-terminus:
(1) the epigenetic modification domain that provides histone modification, the nucleic acid-binding domain and the epigenetic modification domain that provides histone modification;
(2) the transcriptional repressor domain, the nucleic acid-binding domain and the epigenetic modification domain that provides histone modification;
(3) the epigenetic modification domain that provides DNA modification, the nucleic acid-binding domain and the epigenetic modification domain that provides histone modification;
(4) the transcriptional repressor domain, the epigenetic modification domain that provides DNA modification, the nucleic acid-binding domain, and the epigenetic modification domain that provides histone modification; or
(5) the epigenetic modification domain that provides DNA modification, the transcriptional repressor domain, the nucleic acid-binding domain, and the epigenetic modification domain that provides histone modification.

59. The fusion according to claim 58, wherein the epigenetic modification domain that provides histone modification is selected from EZH2, HDAC3, HDAC1, EHMT2, PRMT1, PRMT5, SETDB1, hSIRT1, HP1a, LSD1, and a functionally active fragment thereof.

60. The fusion according to claim 58, wherein the epigenetic modification domain that provides DNA modification is selected from DNMT3A, DNMT3L, a combination of DNMT3A and DNMT3L, or a functionally active fragment thereof.

61. The fusion according to claim 58, wherein the transcriptional repressor domain is KRAB and/or a functionally active fragment thereof.

62. The fusion according to any one of claims 49-61, wherein the fusion comprises the following domains: DNMT3A-DNMT3L-dCas9-KRAB, HDAC3-dCas9-EZH2, KRAB-dCas9-EZH2, KRAB-DNMT3A-dCas9-EZH2, KRAB-DNMT3A-dCas9-HDAC3, DNMT3A-DNMT3L-dCas9-(EZH2)ₙ₌₁₋₁₁, DNMT3A-DNMT3L-dCas9-HDAC3, DNMT3A-DNMT3L-dCas9-EHMT2, DNMT3A-DNMT3L-dCas9-HDAC1, DNMT3A-DNMT3L-dCas9-PRMT1, DNMT3A-DNMT3L-dCas9-SETDB1, DNMT3A-DNMT3L-dCas9-hSIRT1, DNMT3A-DNMT3L-dCas9-PRMT5, DNMT3A-DNMT3L-dCas9-HP1a, DNMT3A-DNMT3L-dCas9-LSD1, or DNMT3A-DNMT3L-TALE-KRAB, wherein - indicates that the domains of the fusion are each directly and/or indirectly linked, and the domains are arranged in the order from N-terminus to C-terminus.

63. The fusion according to any one of claims 49-62, wherein the fusion comprises an amino acid sequence as set forth in any one of SEQ ID NOs: 68, 69, 108-126, 355 and 356.

64. The fusion according to any one of claims 49-63, wherein the fusion further comprises a nuclear localization signal and/or a tag domain.

65. A nucleic acid encoding the fusion according to any one of claims 1-64.

66. A recombinant vector comprising the nucleic acid according to claim 65.

67. A delivery vector comprising the fusion according to any one of claims 1-64, the nucleic acid according to claim 65, and/or the recombinant vector according to claim 66, and optionally comprising a liposome and/or a lipid nanoparticle.

68. A composition comprising the fusion according to any one of claims 1-64, the nucleic acid according to claim 65, the recombinant vector according to claim 66, and/or the delivery vector according to claim 67.

69. A cell comprising the fusion according to any one of claims 1-64, the nucleic acid according to claim 65, the recombinant vector according to claim 66, the delivery vector according to claim 67, and/or the composition according to claim 68.

70. A kit comprising the fusion according to any one of claims 1-64, the nucleic acid according to claim 65, the recombinant vector according to claim 66, the delivery vector according to claim 67, the composition according to claim 68, and/or the cell according to claim 69.

71. A method for regulating the expression of a target gene, wherein the method comprises administering the fusion according to any one of claims 1-64, the nucleic acid according to claim 65, the recombinant vector according to claim 66, the delivery vector according to claim 67, the composition according to claim 68, the cell according to claim 69, and/or the kit according to claim 70.

72. The method according to claim 71, wherein the method comprises introducing the fusion, the nucleic acid, the recombinant vector, the delivery vector, the composition, the cell, and/or the kit into a cell containing the target gene.

73. The method according to claim 71, wherein the method comprises contacting the fusion, the nucleic acid, the recombinant vector, the delivery vector, and/or the composition with a regulatory element near and/or of the target gene.

74. The method according to claim 73, wherein the regulatory element comprises a core promoter, a proximal promoter, a distal enhancer, a silencer, an insulator element, a boundary element, and/or a locus control region.

75. A method for treating or alleviating a disease or condition associated with abnormal expression and/or abnormal activity of a target gene, wherein the method comprises administering to a subject in need thereof an effective amount of the fusion according to any one of claims 1-64, the nucleic acid according to claim 65, the recombinant vector according to claim 66, the delivery vector according to claim 67, the composition according to claim 68, the cell according to claim 69, and/or the kit according to claim 70.
